# EUROPEAN PATENT APPLICATION

(11) **EP 3 854 789 A1**
(43) Date of publication of application: **28.07.2021**
(21) Application number: 20153134.0
(22) Date of filing: 22.01.2020
(51) Int. Cl.: C07D 273/00, C07D 255/02, A61K 31/395, A61P 11/06, A61P 29/00

(54) **MACROCYCLIC COMPOUNDS USEFUL AS CHITINASE INHIBITORS**

(71) Applicant: CYGNET BIOSCIENCES B.V., Willemstad (CW)
(72) Inventor: The designation of the inventor has not yet been filed
(74) Representative: Capasso, Olga

(57) **Abstract**

The present invention relates to macrocyclic compounds of formula (I) and their use as chitinase inhibitors as well as to pharmaceutical compositions and methods of preparation thereof. The compounds can in particular be used in the treatment, prevention and/or amelioration of asthma.

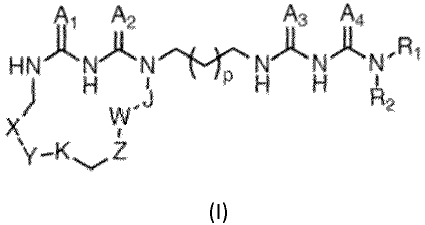

## Description

### TECHNICAL FIELD OF THE INVENTION

The present invention refers to macrocyclic compounds useful as chitinase inhibitors. Said compounds can thus be used in the treatment, prevention and/or amelioration of any condition that would be ameliorated by chitinase inhibition, such as IL-13 and/or Th-2-mediated diseases. The present invention also concerns pharmaceutical compositions comprising said compounds and methods of preparing them.

### BACKGROUND OF THE INVENTION

### Asthma Disease

Asthma is a chronic lung disease that obstructs the airways reducing the flow of air into and out of the lungs.

According to World Health Organization (WHO), 235 million people suffer from asthma globally.¹ Asthma is characterized by shortness of breath, wheezing and chest tightness, often associated with coughing; physiologically, by reversible narrowing of the bronchial airways and an increase in bronchial responsiveness to inhaled stimuli; pathologically, by lymphocytic and eosinophilic inflammation and remodelling of the bronchial mucosa with thickening of the lamina reticularis beneath the airway epithelium and hyperplasia of the cells of all structural elements of the airway wall-vessels, smooth muscle, secretory glands and goblet cells.

Asthma affects people of all ages, but it most often starts during childhood, when it can usually be controlled with inhaled medications, but it is often associated with other allergic disorders. Patients with persistent disease into adulthood, or those who have onset in adulthood, are more likely to have more frequent and severe episodes that require intensification of therapy.²

The main causes of asthma are not completely clear, but the strongest risk factor for its development are inhaled asthma triggers, such as indoor allergens, for example house dust mites, carpets, pollution and pet dander, outdoor allergens such as pollens and moulds, but also tobacco smoke and chemical irritants in the workplace.

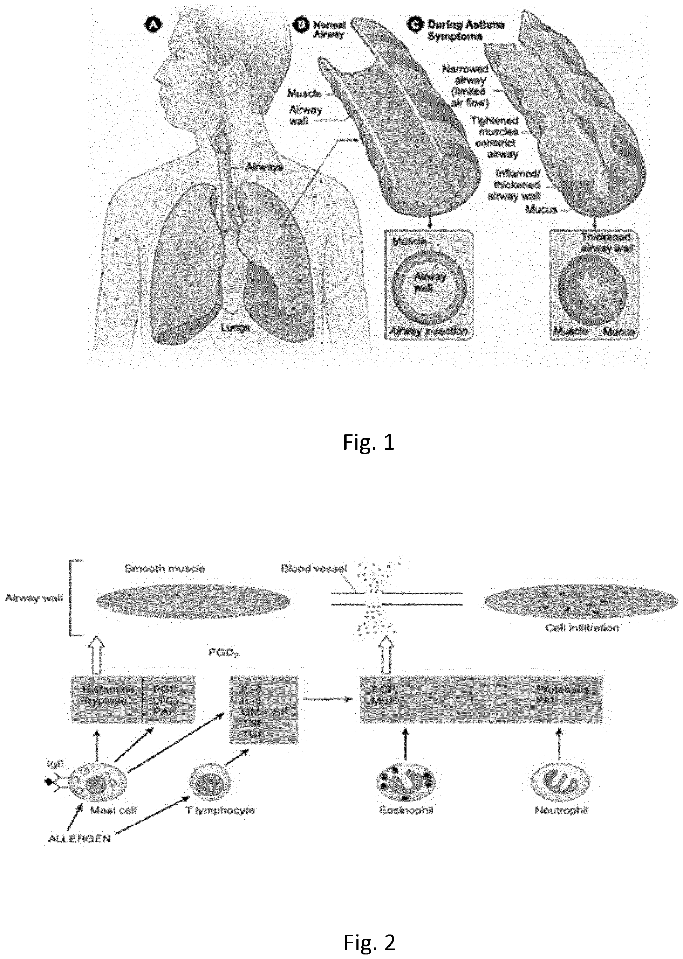

In mild asthma, symptoms may occur only occasionally, as on exposure to allergens or certain pollutants, on exercise, or after viral upper infection. More severe forms of asthma are associated with frequent attacks of wheezing dyspnea, especially at night, or with chronic airway narrowing, causing chronic respiratory impairment. People who have asthma have inflamed airways, that are swollen and very sensitive, and when some substances are inhaled the airways react strongly. The smooth muscle contracts, this narrows the airways causing less air to flow into the lungs. Epithelial cells produce more mucus than usual forming a plug in the airway lumen. Mucus is a sticky and thick liquid that can further narrow the airways **(****Figure 1****).**³

Asthma is mediated by reaginic immunoglobulin (IgE). The most common allergens, that cause IgE production, are proteins from house dust mite, cockroach, animal dander, moulds and pollens. Once produced, IgE antibodies bind to mast cells in the airway mucosa. On re-exposure to a specific allergen, the antibody exposed on the surface of the mast cells binds the antigen and consequently triggers the synthesis and release of mediators stored in the cells' granules, such as histamine, tryptase, leukotrienes C₄ and D₄ and prostaglandin D₂. These mediators diffuse through the airway mucosa, causing the muscle contraction and vascular leakage responsible for the acute bronchoconstriction of the "early asthmatic response". This response is often followed in 3-6 hours by a second, the "late asthmatic response", associated with an influx of inflammatory cells into the bronchial mucosa and with an increased bronchial reactivity. The mediators responsible for this late response are cytokines produced by Th2 lymphocytes, especially interleukins 5, 9 and 13. These cytokines attract and activate eosinophils, stimulate IgE production by B lymphocytes and stimulate mucus production by bronchial epithelial cells **(****Figure 2****).** Most asthma attacks are not caused by inhalation of allergens, rather by viral respiratory infection; moreover, bronchospasm can be induced by non-allergenic stimuli such as distilled water, exercise, cold air and rapid respiratory manoeuvres.

The exaggerated reactivity of the airways appears to be fundamental to asthma's pathogenesis, because it is ubiquitous in patients with asthma and its degree roughly correlates with the clinical severity of the disease.

The mechanisms underlying bronchial hyper-reactivity are somehow related to inflammation of the airway mucosa. The agents that increase bronchial reactivity also cause airway inflammation. The increase in reactivity due to allergen inhalation is associated with an increase in both eosinophils and polymorphonuclear leukocytes in bronchial lavage fluid.

Whatever the mechanism responsible for bronchial hyper-reactivity, bronchoconstriction itself seems to result also from the activation of neural and humoral pathways from released mediators. This hypothesis suggests that asthma may be treated by drugs with different modes of action.⁴

### Chitinases

A common characteristic of bacteria, fungi, arthropods, plants and humans is the production of chitinases, enzymes that are involved in the degradation and turnover of chitin. Chitinases are chitinolytic enzymes belonging to the glycosyl hydrolases family (GH) characterized for the selective hydrolysis of 6-1,4 linkage of N-acetyl glucosamine (GluNAc) present in the chitin chains.

Chitin is the second most abundant carbohydrate polymer in nature after cellulose and is the major component of fungal cell walls, invertebrate exoskeletons and crustacean shells. It is a polymer of N-acetylglucosamine, linked with 6-1,4-glycosidic bonds.

The chitin deposition is regulated by biosynthesis and degradation. Chitinolytic enzymes catalyse the hydrolysis reaction of glycosidic bonds of chitin bringing it down to its oligomers and monomers. They are present in a wide range of organisms, including organisms that do not contain chitin, such as bacteria, viruses, plants and animals, including mammals, playing an important physiological and ecological role. In particular, despite the fact that mammals do not synthesize chitin, the genome of mammals encodes different chitinases belonging to Glycosyl Hydrolases (GH) 18 family. The human chitinases presenting catalytic activity (the so-called "true chitinases") are two: chitotriosidase (CHIT1 or CHIT-1), the first identified, and acidic mammalian chitinase (AMCase), named after its acidic pH optimum. In addition to "true chitinases", human genome encodes the homologous enzymatically inactive proteins, named chitinase-like lectins (chi-lectins), such as human chitinase-3-like protein-1 (CHI3L1/HC-gp39/YKL-40), human chitinase-3-like protein-2 (CHI3L2/YKL-39) and oviductins. They are secreted locally and into circulation and seem to be involved in inflammatory conditions.

### Acidic Mammalian Chitinase

Acidic Mammalian Chitinase, AMCase, is the second true chitinase protein discovered in humans. The gene that expresses AMCase derived from a duplication of the ancestor gene of the Chitinase proteins. This hypothesis is confirmed by the location of the AMCase gene on human chromosome 1 (1q13.1e21.3) and the sequence homology and conservation of intron exon boundaries with CHIT-1. AMCase contains a 30-kDa N-terminal catalytic domain that is able to cleave chitin, and it is mainly expressed in the gastrointestinal tract and lung of both mouse and human.⁵

Likewise to CHIT1, in addition to the N-terminal catalytic domain, AMCase contains a C-terminal chitinase binding domain (CBM). The enzyme shows an optimum of pH around 2.0, and is adapted to function in the extreme acidic environment of the stomach where it is able to play a role in defence and/or digestion of chitin-containing organisms. The abundancy of AMCase in the gastrointestinal tract and lungs suggests a possible role as a food processor in stomach and its involvement in lung inflammation.⁶ Airway epithelial cells express the highest percentage of AMCase. Beyond expression in the stomach, the presence of AMCase in the lungs suggests that this enzyme may have a dual function in digestion of chitinous substrates and host defence.

In 2004, Prof. Elias from Yale university demonstrated that AMCase is specifically upregulated in response to Th2 inflammation in the lung.⁵ Poorly controlled T helper (Th)-2 responses following exposure to allergens evolve to acute allergic sensitization and atopic asthma in individuals. Th2 cells secrete several cytokines, including IL-4, IL-5, IL-9 and IL-13, as well as chemokines such as thymus and activation regulated chemokine and macrophage-derived chemokine, leading to further Th2 cell recruitment and activation of B cells. The early up regulation of AMCase expression in undifferentiated monocytes treated with IL-4 suggested that an inhibition of AMCase may prevent Th2 immune response.⁵

More in detail, Kawada et al.⁷ described the involvement of the AMCase in the development of the Th2 related inflammatory pathologies explaining that originally, the inflammation related to Th2 was developed to combat parasitic infections, whereas allergy reactions, including atopic asthma, arise as a consequence of poorly controlled Th2-type immune responses elicited independently of parasitic infection.⁸ AMCase production is mediated by Interleukin (IL)-13 in both airway epithelial cells and macrophages. This event may trigger the development of airway hyper-responsiveness and inflammatory cell infiltration after exposure to an allergen in asthma patients and murine models of asthma. Expression of AMCase in the lung epithelial cells induces the production of monocyte chemoattractant protein (MCP-1) and eotaxin-1, and afterward it may cause the pathogenesis of asthma. Allosamidin significantly ameliorates Th2 inflammation and airway hypersensitivity, in part by inhibiting IL-13 pathway activation and chemokine production. Therefore, chitinases have been proposed as a potential therapeutic target in Th2-mediated inflammation.⁵

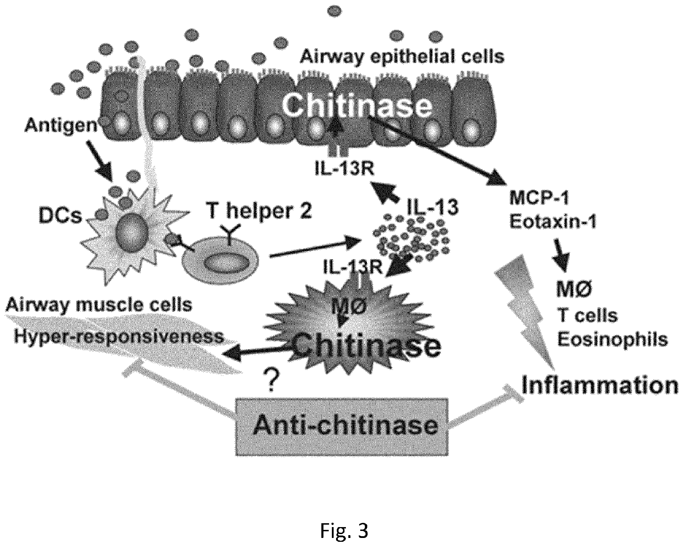

In particular, the antigen presenting cells such as dendritic cells (DC) actively uptake antigens, presumably containing chitin, and present these antigens to Th2 type of CD4+ T cells. These CD4+ T cells produce cytokines such as IL-4, IL-5 and IL-13. The last one plays a major role to induce the production of AMCase by both airway epithelial cells and macrophages, which express IL-13 receptor (IL-13R) on their surface. AMCase induces the production of monocyte chemoattractant protein-1 (MCP-1) and eotaxin-1, that improve the recruitment of immune system cells in the lung and further aggravates the inflammation and airway hyper-responsiveness. Antibodies Anti-AMCase and chitinase inhibitor, such as Allosamidin, reduce significantly both airway hyper-responsiveness and inflammation processes (**Figure 3**).⁷

In general, chitinases are mainly expressed and secreted by phagocytes (mainly neutrophils and macrophages) and are induced at sites of inflammation, infection, and tissue remodeling, suggesting that these proteins play active roles in anti-infective defense and repair responses.

Since its expression in epithelial cells of conjunctiva, AMCase may be associated to allergic ocular pathologies. In patients with vernal keratoconjunctivitis (VKC) and with seasonal allergic conjunctivitis (SAC) the level of AMCase activity in the tears was found significantly elevated. In dry eye syndrome, another non-allergic ocular pathology, an increased AMCase activity was documented and the specific mRNA expressed by epithelial conjunctival cells (Musumeci et al., 2009, Cornea, 28, 667-672). Then, AMCase may also be considered an important mediator in the pathogenesis of Th2 inflammation eye's diseases, suggesting its potential diagnostic and therapeutic utility.

AMCase is overexpressed in Chronic Rhinosinusitis which is a common condition that has been associated with hypertrophied adenoids in children and may be a target for new drug treatments under this condition (Kyung Wook Heo, Otolaryngology -- Head and Neck Surgery 2011 145: 660).

AMCase displays markedly increased chitinolytic activity in all nasal polyps (NPs) and may be important in NP pathogenesis, suggesting that inhibition of chitinolytic activity may be a novel therapeutic strategy for the treatment of NPs (Park SK AM J Rhinol Allergy 2011).

It has been proved in animal model, that Allosamidin, a pharmacologic inhibitor of AMCase, inhibits AMCase activity in the esophagus and reduces esophageal levels of eosinophils, and features of esophageal remodeling suggesting that allosamidin may be a novel therapy to investigate in Eosinophilic Esophagitis (Jae Youn Cho, Int Ummunopharmacol 2014, 18, 35-42).

WO 2014/202697 describes macrocyclic amidinourea derivatives to be used as chitinase inhibitors, specifically as *T. viride* chitinase inhibitors, having the following general formula:

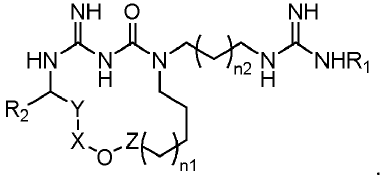

There is still the need to develop chitinase inhibitors which can, in particular, be used in the treatment, prevention and/or amelioration of conditions such as asthma.

### SUMMARY OF THE INVENTION

In the present invention, it was surprisingly found that compounds of formula (I) as defined below act as chitinase inhibitors, in particular of the human Acidic Mammalian Chitinase (AMCase). Advantageously, compounds of the invention can exhibit a good inhibition constant (Kᵢ) in the micromolar range against human AMCase and/or a percentage of inhibition of AMCase of at least 70% at a fixed concentration. Structurally, compounds of formula (I) include a 13-16 membered macrocycle, a linker moiety labelled as M-Q with variable chain length and a terminal moiety.

Therefore, it is an object of the present invention a compound of formula (I): 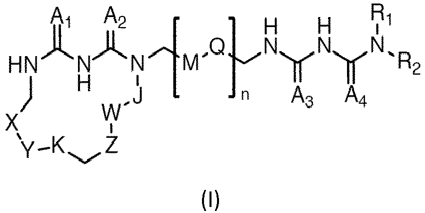 or a salt, a solvate, a tautomer or a stereoisomer thereof,
wherein:
n is a number from 0 to 3;
J is a linear saturated or unsaturated C₁-C₄ alkyl chain;
K is -CH₂-, O or S;
A₁, A₂, A₃, A₄ are the same or different and are each independently selected from the group consisting of: NH, O and S;
R₁ and R₂ are the same or different and are each independently selected from the group consisting of: H, substituted or unsubstituted C₁-C₆ alkyl, substituted or unsubstituted C₂-C₆ alkenyl, substituted or unsubstituted C₂-C₆ alkynyl, substituted or unsubstituted aryl and substituted or unsubstituted heteroaryl, wherein the substituted C₁-C₆ alkyl, the substituted C₂-C₆ alkenyl, the substituted C₂-C₆ alkynyl, the substituted aryl and the substituted heteroaryl are each independently substituted by one or more substituents each independently selected from the group consisting of: halogen, NO₂, substituted or unsubstituted phenyl, OR_{A}, C(O)OR_{B}, OC(O)R_{B}, SR_{A}, SO₃H, SOR_{A}, SO₂R_{A}, NR_{A}R_{B}, OP(O)(OR_{A}), OP(O)(OR_{A})(OR_{B}), OC(O)NR_{A}R_{B}, NR_{A}C(O)OR_{B}, C(O)NR_{A}R_{B}, NR_{A}C(O)R_{B}, N(COR_{A})(COR_{B}), C(O)NR_{A}C(O)R_{B}, C(O)ONR_{A}R_{B}, C(NOR_{A})R_{B}, C(O)R_{A}, CN, haloalkyl, CF₃, OCF₃, substituted or unsubstituted cycloalkyl, substituted or unsubstituted C₁-C₆ alkyl, substituted or unsubstituted C₂-C₆ alkenyl and substituted or unsubstituted C₂-C₆ alkynyl;
or R₁ and R₂ together with the nitrogen to which they are attached form a 4-7 membered saturated, partially unsaturated or aromatic ring optionally containing one or more additional heteroatoms independently selected from N, S and O, the ring being optionally substituted by one, two or more groups independently selected from: halogen, NO₂, substituted or unsubstituted phenyl, OR_{A}, C(O)OR_{B}, OC(O)R_{B}, SR_{A}, SO₃H, SOR_{A}, SO₂R_{A}, NR_{A}R_{B}, OP(O)(OR_{A}), OP(O)(OR_{A})(OR_{B}), OC(O)NR_{A}R_{B}, NR_{A}C(O)OR_{B}, C(O)NR_{A}R_{B}, NR_{A}C(O)R_{B}, N(COR_{A})(COR_{B}), C(O)NR_{A}C(O)R_{B}, C(O)ONR_{A}R_{B}, C(NOR_{A})R_{B}, C(O)R_{A}, CN, haloalkyl, CF₃, OCF₃, substituted or unsubstituted cycloalkyl, substituted or unsubstituted C₁-C₆ alkyl, substituted or unsubstituted C₂-C₆ alkenyl and substituted or unsubstituted C₂-C₆ alkynyl;
R_{A} and R_{B} are the same or different and are each independently selected from: H, unsubstituted or substituted C₁-C₆ alkyl, unsubstituted or substituted aralkyl, unsubstituted or substituted aryl, haloalkyl, or R_{A} and R_{B} together with the nitrogen, sulfur, or oxygen to which they are attached, form a 4-7 membered saturated, partially unsaturated or aromatic ring optionally containing one or more additional heteroatoms independently selected from N, S and O the ring being optionally substituted by one, two or more groups independently selected from halogen, substituted or unsubstituted C₁-C₆ alkyl, haloalkyl, OH and alkoxy;
X-Y, Z-W and M-Q are the same or different and are each independently selected from the group consisting of: -CH₂CH₂-, substituted or unsubstituted aryl and substituted or unsubstituted heteroaryl, wherein the substituted aryl and the substituted heteroaryl are each independently substituted by one or more substituents each independently selected from the group consisting of: halogen, NO₂, substituted or unsubstituted phenyl, OR_{A}, C(O)OR_{B}, OC(O)R_{B}, SR_{A}, SO₃H, SOR_{A}, SO₂R_{A}, NR_{A}R_{B}, OP(O)(OR_{A}), OP(O)(OR_{A})(OR_{B}), OC(O)NR_{A}R_{B}, NR_{A}C(O)OR_{B}, C(O)NR_{A}R_{B}, NR_{A}C(O)R_{B}, N(COR_{A})(COR_{B}), C(O)NR_{A}C(O)R_{B}, C(O)ONR_{A}R_{B}, C(NOR_{A})R_{B}, C(O)R_{A}, CN, haloalkyl, CF₃, OCF₃, substituted or unsubstituted cycloalkyl, substituted or unsubstituted C₁-C₆ alkyl, substituted or unsubstituted C₂-C₆ alkenyl and substituted or unsubstituted C₂-C₆ alkynyl. In a preferred embodiment, compound 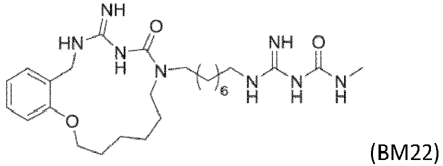 and the trifluoroacetate salt thereof are excluded from formula (I). In another preferred embodiment, said compound, salt, solvate, tautomer or stereoisomer is an Acidic Mammalian Chitinase (AMCase) inhibitor and/or a chitotriosidase (CHIT-1) inhibitor.

Preferably, X-Y and Z-W are the same or different and are each independently selected from the group consisting of: substituted or unsubstituted aryl and substituted or unsubstituted heteroaryl. It is a further object of the present invention a compound of formula (I): 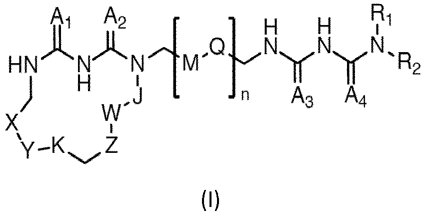 or a salt, a solvate, a tautomer or a stereoisomer thereof,
wherein:
n is a number from 0 to 3;
J is a linear saturated or unsaturated C₁-C₄ alkyl chain;
K is -CH₂-, O or S;
A₁, A₂, A₃, A₄ are the same or different and are each independently selected from the group consisting of: NH, O and S;
R₁ and R₂ are the same or different and are each independently selected from the group consisting of: H, substituted or unsubstituted C₁-C₆ alkyl, substituted or unsubstituted C₂-C₆ alkenyl, substituted or unsubstituted C₂-C₆ alkynyl, substituted or unsubstituted aryl and substituted or unsubstituted heteroaryl, wherein the substituted C₁-C₆ alkyl, the substituted C₂-C₆ alkenyl, the substituted C₂-C₆ alkynyl, the substituted aryl and the substituted heteroaryl are each independently substituted by one or more substituents each independently selected from the group consisting of: halogen, NO₂, substituted or unsubstituted phenyl, OR_{A}, C(O)OR_{B}, OC(O)R_{B}, SR_{A}, SO₃H, SOR_{A}, SO₂R_{A}, NR_{A}R_{B}, OP(O)(OR_{A}), OP(O)(OR_{A})(OR_{B}), OC(O)NR_{A}R_{B}, NR_{A}C(O)OR_{B}, C(O)NR_{A}R_{B}, NR_{A}C(O)R_{B}, N(COR_{A})(COR_{B}), C(O)NR_{A}C(O)R_{B}, C(O)ONR_{A}R_{B}, C(NOR_{A})R_{B}, C(O)R_{A}, CN, haloalkyl, CF₃, OCF₃, substituted or unsubstituted cycloalkyl, substituted or unsubstituted C₁-C₆ alkyl, substituted or unsubstituted C₂-C₆ alkenyl and substituted or unsubstituted C₂-C₆ alkynyl;
or R₁ and R₂ together with the nitrogen to which they are attached form a 4-7 membered saturated, partially unsaturated or aromatic ring optionally containing one or more additional heteroatoms independently selected from N, S and O, the ring being optionally substituted by one, two or more groups independently selected from: halogen, NO₂, substituted or unsubstituted phenyl, OR_{A}, C(O)OR_{B}, OC(O)R_{B}, SR_{A}, SO₃H, SOR_{A}, SO₂R_{A}, NR_{A}R_{B}, OP(O)(OR_{A}), OP(O)(OR_{A})(OR_{B}), OC(O)NR_{A}R_{B}, NR_{A}C(O)OR_{B}, C(O)NR_{A}R_{B}, NR_{A}C(O)R_{B}, N(COR_{A})(COR_{B}), C(O)NR_{A}C(O)R_{B}, C(O)ONR_{A}R_{B}, C(NOR_{A})R_{B}, C(O)R_{A}, CN, haloalkyl, CF₃, OCF₃, substituted or unsubstituted cycloalkyl, substituted or unsubstituted C₁-C₆ alkyl, substituted or unsubstituted C₂-C₆ alkenyl and substituted or unsubstituted C₂-C₆ alkynyl;
R_{A} and R_{B} are the same or different and are each independently selected from: H, unsubstituted or substituted C₁-C₆ alkyl, unsubstituted or substituted aralkyl, unsubstituted or substituted aryl, haloalkyl, or R_{A} and R_{B} together with the nitrogen, sulfur, or oxygen to which they are attached, form a 4-7 membered saturated, partially unsaturated or aromatic ring optionally containing one or more additional heteroatoms independently selected from N, S and O the ring being optionally substituted by one, two or more groups independently selected from halogen, substituted or unsubstituted C₁-C₆ alkyl, haloalkyl, OH and alkoxy;
M-Q is selected from the group consisting of: -CH₂CH₂-, substituted or unsubstituted aryl and substituted or unsubstituted heteroaryl, wherein the substituted aryl and the substituted heteroaryl are each independently substituted by one or more substituents each independently selected from the group consisting of: halogen, NO₂, substituted or unsubstituted phenyl, OR_{A}, C(O)OR_{B}, OC(O)R_{B}, SR_{A}, SO₃H, SOR_{A}, SO₂R_{A}, NR_{A}R_{B}, OP(O)(OR_{A}), OP(O)(OR_{A})(OR_{B}), OC(O)NR_{A}R_{B}, NR_{A}C(O)OR_{B}, C(O)NR_{A}R_{B}, NR_{A}C(O)R_{B}, N(COR_{A})(COR_{B}), C(O)NR_{A}C(O)R_{B}, C(O)ONR_{A}R_{B}, C(NOR_{A})R_{B}, C(O)R_{A}, CN, haloalkyl, CF₃, OCF₃, substituted or unsubstituted cycloalkyl, substituted or unsubstituted C₁-C₆ alkyl, substituted or unsubstituted C₂-C₆ alkenyl and substituted or unsubstituted C₂-C₆ alkynyl;
X-Y and Z-W are the same or different and are each independently selected from the group consisting of: substituted or unsubstituted aryl and substituted or unsubstituted heteroaryl, wherein the substituted aryl and the substituted heteroaryl are each independently substituted by one or more substituents each independently selected from the group consisting of: halogen, NO₂, substituted or unsubstituted phenyl, OR_{A}, C(O)OR_{B}, OC(O)R_{B}, SR_{A}, SO₃H, SOR_{A}, SO₂R_{A}, NR_{A}R_{B}, OP(O)(OR_{A}), OP(O)(OR_{A})(OR_{B}), OC(O)NR_{A}R_{B}, NR_{A}C(O)OR_{B}, C(O)NR_{A}R_{B}, NR_{A}C(O)R_{B}, N(COR_{A})(COR_{B}), C(O)NR_{A}C(O)R_{B}, C(O)ONR_{A}R_{B}, C(NOR_{A})R_{B}, C(O)R_{A}, CN, haloalkyl, CF₃, OCF₃, substituted or unsubstituted cycloalkyl, substituted or unsubstituted C₁-C₆ alkyl, substituted or unsubstituted C₂-C₆ alkenyl and substituted or unsubstituted C₂-C₆ alkynyl. Preferably, said compound, salt, solvate, tautomer or stereoisomer is a chitinase inhibitor. More preferably, said compound, salt, solvate, tautomer or stereoisomer is an Acidic Mammalian Chitinase (AMCase) inhibitor and/or a chitotriosidase (CHIT-1) inhibitor.

As used herein, any reference to "the compound(s) of the invention", "the compound(s) of formula (I)" or "the compound(s) as defined above" includes also any pharmaceutically acceptable salt, tautomer, solvate, or stereoisomer thereof.

The following preferred embodiments refer to both above-identified objects of the present invention. All the following preferred embodiments may be combined amongst themselves in any possible way that would give rise to a chemically stable compound.

Preferably, M-Q is -CH₂CH₂- such that in a preferred embodiment, the present invention provides a compound having general formula (II): 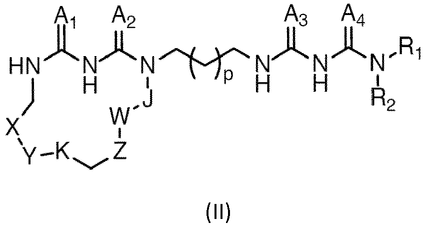 wherein p is a number from 0 to 6. Preferably, p is 2, 4 or 6. More preferably, p is 6. It is to be understood that the value of p corresponds to the value of n times two (p = n x 2). Then, all the following preferred embodiments also refer to the compound of formula (II), wherein p = n x 2.

Preferably, X-Y and Z-W are the same or different and are each substituted aryl or substituted heteroaryl. Still preferably, X-Y and/or Z-W is

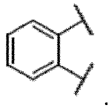

Preferably, X-Y and/or Z-W are each independently substituted by one or more substituents independently selected from the group consisting of: halogen (more preferably fluorine and chlorine), OR_{A} (more preferably OC₁-C₆haloalkyl such as OCF₃ or OC₁-C₆alkyl such as OCH₃) and C₁-C₆ alkyl (preferably CH₃).

Preferably, the compound as defined above is characterized by at least one of the following:
- A₁ is NH,
- A₂ is O,
- A₃ is NH,
- A₄ is O or S.

More preferably, A₁ is NH, A₂ is O, A₃ is NH and A₄ is O.

In a preferred embodiment, A₃ is NH, A₄ is O, R₁ is H and R₂ is methyl.

Preferably, the compound as defined above is characterized by at least one of the following:
- R₁ and R₂ are independently H, methyl or CH₂CH₂OH,
- n is 1, 2 or 3,
- J is a C₁ alkyl chain, a C₂ alkyl chain or a C₃ alkyl chain,
- K is -CH₂- or O.

More preferably, the compound as defined above is characterized by at least one of the following:
- R₁ is H and R₂ is methyl or vice versa,
- n is 3,
- J is a C₃ alkyl chain,
- K is O.

Preferably, R₁ and R₂ are independently H or methyl, n is 1, 2 or 3, J is a C₁ alkyl chain, a C₂ alkyl chain or a C₃ alkyl chain and K is -CH₂- or O. More preferably, R₁ is H and R₂ is methyl or vice versa, n is 3, J is a C₃ alkyl chain and K is O.

Preferably, R₁ is methyl and X-Y and Z-W are the same or different and are each substituted aryl or substituted heteroaryl.

It is a further object of the present invention provides a compound of general formula: 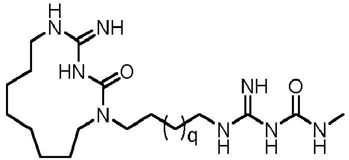 wherein q is a number from 0 to 5; or 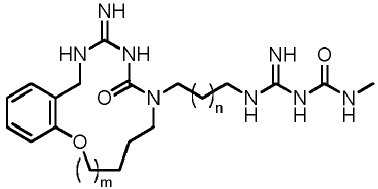 wherein n is a number from 0 to 6 and m is a number from 1 to 3; or 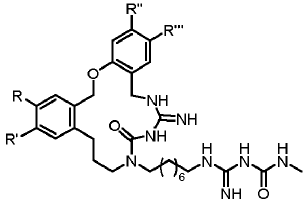 wherein R, R', R" and R''' are present or absent and, when present, are the same or different and are each independently selected from the group consisting of: halogen, NO₂, substituted or unsubstituted phenyl, OR_{A}, C(O)OR_{B}, OC(O)R_{B}, SR_{A}, SO₃H, SOR_{A}, SO₂R_{A}, NR_{A}R_{B}, OP(O)(OR_{A}), OP(O)(OR_{A})(OR_{B}), OC(O)NR_{A}R_{B}, NR_{A}C(O)OR_{B}, C(O)NR_{A}R_{B}, NR_{A}C(O)R_{B}, N(COR_{A})(COR_{B}), C(O)NR_{A}C(O)R_{B}, C(O)ONR_{A}R_{B}, C(NOR_{A})R_{B}, C(O)R_{A}, CN, haloalkyl, CF₃, OCF₃, substituted or unsubstituted cycloalkyl, substituted or unsubstituted C₁-C₆ alkyl, substituted or unsubstituted C₂-C₆ alkenyl and substituted or unsubstituted C₂-C₆ alkynyl. Preferably, R, R', R" and R''' are each independently selected from the group consisting of: hydrogen, halogen (more preferably fluorine and chlorine), OR_{A} (more preferably OC₁-C₆haloalkyl such as CF₃ or OC₁-C₆alkyl such as CH₃) and C₁-C₆ alkyl (preferably CH₃).

Preferably, the compound as defined above is selected from:

| | | |
|---|---|---|
| **BM41** | 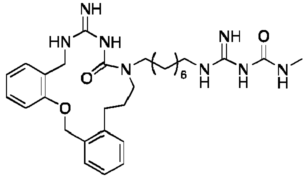 | 1-[N-(8-{16-imino-14-oxo-2-oxa-13,15,17-triazatricyclo[17.4.0.0^{4,9}]tricosa-1(19),4(9),5,7,20,22-hexaen-13-yl}octyl)carbamimidoyl]-3-methylurea |
| **BM46** | 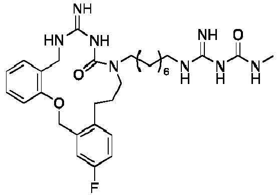 | 1-[N-(8-{6-fluoro-16-imino-14-oxo-2-oxa-13,15,17-triazatricyclo[17.4.0.0^{4,9}]tricosa-1(19),4(9),5,7,20,22-hexaen-13-yl}octyl)carbamimidoyl]-3-methylurea |
| **BM43** | 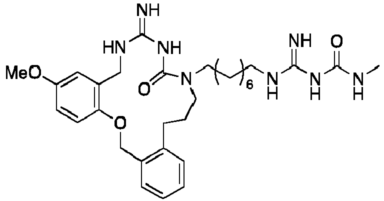 | 1-[N-(8-{16-imino-21-methoxy-14-oxo-2-oxa-13,15, 17-triazatricyclo[17.4.0.0^{4,9}]tricosa-1(19),4(9),5,7,20,22-hexaen-13-yl}octyl)carbamimidoyl]-3-methylurea |
| **BM55** | 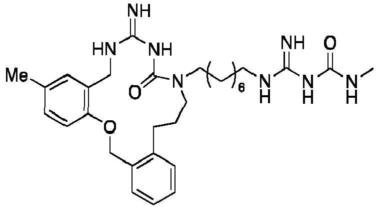 | 1-[N-(8-{16-imino-21-methyl-14-oxo-2-oxa-13,15,17-triazatricyclo[17.4.0.0^{4,9}]tricosa-1(19),4(9),5,7,20,22-hexaen-13-yl}octyl)carbamimidoyl]-3-methylurea |
| **BM54** | 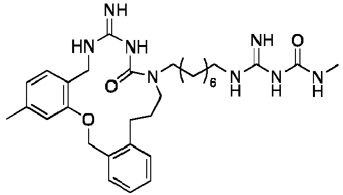 | 1-[N-(8-{16-imino-22-methyl-14-oxo-2-oxa-13,15,17-triazatricyclo[17.4.0.0^{4,9}]tricosa-1(19),4(9),5,7,20,22-hexaen-13-yl}octyl)carbamimidoyl]-3-methylurea |
| **BM38** | 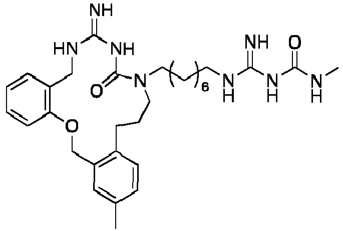 | 1-[N-(8-{16-imino-6-methyl-14-oxo-2-oxa-13,15,17-triazatricyclo[17.4.0.0^{4,9}]tricosa-1(19),4(9),5,7,20,22-hexaen-13-yl}octyl)carbamimidoyl]-3-methylurea |

| | | |
|---|---|---|
| **BM47** | 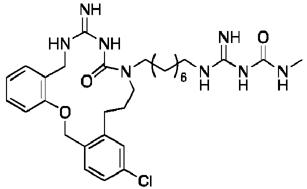 | 1-[N-(8-{7-chloro-16-imino-14-oxo-2-oxa-13,15,17-triazatricyclo[17.4.0.0^{4,9}]tricosa-1(19),4(9),5,7,20,22-hexaen-13-yl}octyl)carbamimidoyl]-3-methylurea |
| **BM32** | 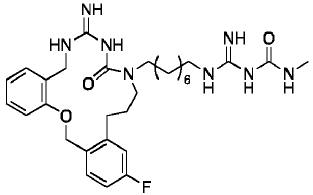 | 1-[N-(8-{7-fluoro-16-imino-14-oxo-2-oxa-13,15,17-triazatricyclo[17.4.0.0^{4,9}]tricosa-1(19),4(9),5,7,20,22-hexaen-13-yl}octyl)carbamimidoyl]-3-methylurea |
| **BM40** | 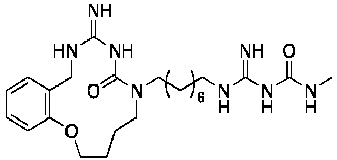 | 1-{N-[8-(9-imino-7-oxo-2,3,4,5,6,7,8,9,10,11-decahydro-1,6,8,10-benzoxatriazacyclotridecin-6-yl)octyl]carbamimidoyl}-3-methylurea |
| **BM39** | 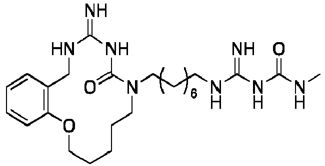 | 1-{N-[8-(10-imino-8-oxo-3,4,5,6,7,8,9,10,11,12-decahydro-2H-1,7,9,11-benzoxatriazacyclotetradecin-7-yl)octyl]carbamimidoyl}-3-methylurea |
| **BM52** | 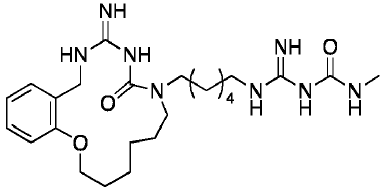 | 1-{N-[6-(11-imino-9-oxo-2,3,4,5,6,7,8,9,10,11,12,13-dodecahydro-1,8,10,12-benzoxatriazacyclopentadecin-8-yl)hexyl]carbamimidoyl}-3-methylurea |
| **BM50** | 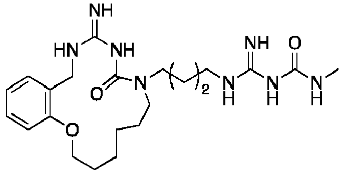 | 1-{N-[4-(11-imino-9-oxo-2,3,4,5,6,7,8,9,10,11,12,13-dodecahydro-1,8,10,12-benzoxatriazacyclopentadecin-8-yl)butyl]carbamimidoyl}-3-methylurea |
| **BM22** | 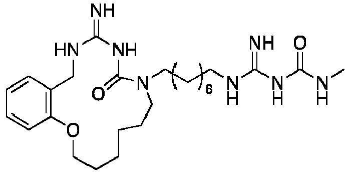 | 1-{N-[8-(11-imino-9-oxo-2,3,4,5,6,7,8,9,10,11,12,13-dodecahydro-1,8,10,12-benzoxatriazacyclopentadecin-8-yl)octyl]carbamimidoyl}-3-methylurea |
| **BM34** | 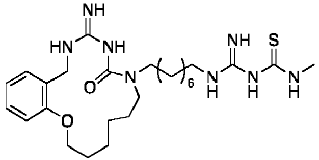 | 1-{N-[8-(11-imino-9-oxo-2,3,4,5,6,7,8,9,10,11,12,13-dodecahydro-1,8,10,12-benzoxatriazacyclopentadecin-8-yl)octyl]carbamimidoyl}-3-methylthiourea |

| | | |
|---|---|---|
| **BM35** | 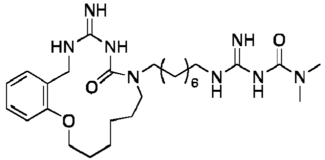 | 1-{N-[8-(11-imino-9-oxo-2,3,4,5,6,7,8,9,10,11,12,13-dodecahydro-1,8,10,12-benzoxatriazacyclopentadecin-8-yl)octyl]carbamimidoyl}-3,3-dimethylurea |
| **BM36** | 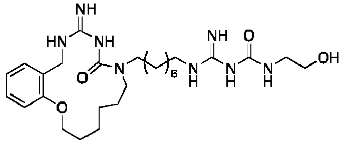 | 3-(2-hydroxyethyl)-1-{N-[8-(11-imino-9-oxo-2,3,4,5,6,7,8,9,10,11,12,13-dodecahydro-1,8,10,12-benzoxatriazacyclopentadecin-8-yl)octyl]carbamimidoyl}urea |
| **BM53** | 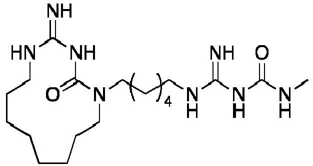 | 1-{N-[6-(4-imino-2-oxo-1,3,5-triazacyclotridecan-1-yl)hexyl]carbamimidoyl}-3-methylurea |
| **BM49** | 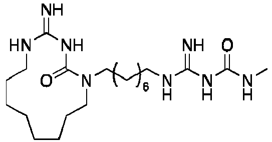 | 1-{N-[8-(4-imino-2-oxo-1,3,5-triazacyclotridecan-1-yl)octyl]carbamimidoyl}-3-methylurea |
| **BM45** | 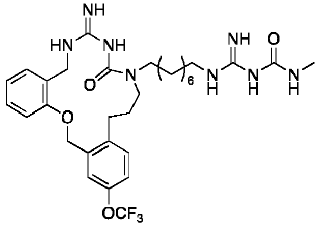 | 1-(N-{8-[16-imino-14-oxo-6-(trifluoromethoxy)-2-oxa-13,15,17-triazatricyclo[17.4.0.0^{4,9}]tricosa-1(19),4(9),5,7,20,22-hexaen-13-yl]octyl}carbamimidoyl)-3-methylurea |
| **BM56** | 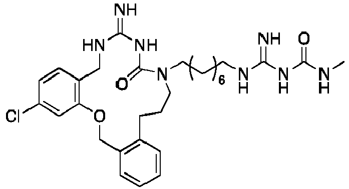 | 1-[N-(8-{22-chloro-16-imino-14-oxo-2-oxa-13,15,17-triazatricyclo[17.4.0.0^{4,9}]tricosa-1(19),4(9),5,7,20,22-hexaen-13-yl}octyl)carbamimidoyl]-3-methylurea |
| **BM57** | 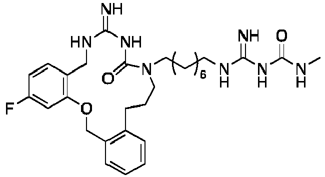 | 1-[N-(8-{22-fluoro-16-imino-14-oxo-2-oxa-13,15,17-triazatricyclo[17.4.0.0^{4,9}]tricosa-1(19),4(9),5,7,20,22-hexaen-13-yl}octyl)carbamimidoyl]-3-methylurea |

| | | |
|---|---|---|
| **BM58** | 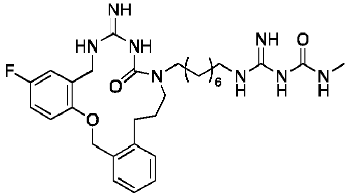 | 1-[N-(8-{21-fluoro-16-imino-14-oxo-2-oxa-13,15,17-triazatricyclo[17.4.0.0^{4,9}]tricosa-1(19),4(9),5,7,20,22-hexaen-13-yl}octyl)carbamimidoyl]-3-methylurea |
| **BM42** | 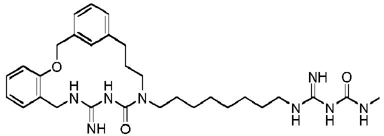 | 1-[N-(8-{12-imino-14-oxo-3-oxa-11,13,15-triazatricyclo[17.3.1.0^{4,9}]tricosa-1(23),4(9),5,7,19,21-hexaen-15-yl}octyl)carbamimidoyl]-3-methylurea |
| **BM48** | 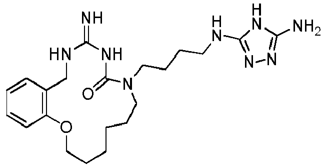 | 8-(4-((5-amino-4H-1,2,4-triazol-3-yl)amino)butyl)-11-imino-3,4,5,6,7,8,10,11,12,13-decahydrobenzo[*b*][1]oxa[5,7,9]triazacyclopentadecin-9(2*H*)-one |

Preferably, the compounds of the invention are chitinase inhibitors. Still preferably, the compounds of the invention are inhibitors of Acidic Mammalian Chitinase (AMCase) and/or chitotriosidase (CHIT-1), more preferably they are dual inhibitors of AMCase and CHIT-1. More in detail, the compounds of the present invention may act as inhibitors of the human chitinase known as human acidic mammalian chitinase, or human AMCase or human CHIA, which can be identified by those skilled in the art through the UniProtKB code Q9BZP6. More in detail, the compounds of the present invention may act as inhibitors of the human chitinase known as human chitotriosidase, or human CHIT1 or human CHIT-1, which can be identified by those skilled in the art through the UniProtKB code Q13231.

It is to be understood that, in the present invention, the terms "chitinase", "AMCase", "CHIT-1" and similar include the corresponding gene, mRNA, cDNA or the protein codified by them, including fragments, derivatives, variants, isoforms, etc. thereof.

As used herein, "chitinase" refers to any enzyme that binds and/or degrades chitin or, more in general, to any enzyme that is in any way involved in the process of chitin degradation and turnover. Preferred chitinases are enzymes that belong to the glycosyl hydrolases family (GH) and which are characterized for selectively hydrolysing glycosidic bonds of chitin breaking it down into its oligomers and monomers, in particular they selectively hydrolyse β-1,4 linkage of N-acetyl glucosamine (GluNAc). Other preferred chitinases are proteins that belong to the glycosyl hydrolases family (GH) and which are able to bind the chitin polymers or oligomers without hydrolysing the β-1,4 linkage of N-acetyl glucosamine (GluNAc). Examples of the latter type of preferred chitinases include but are not limited to chi-lectins. Preferably, "chitinase" as used herein does not include *T. viride* chitinase. Also preferably, "chitinase" as used herein does not include any fungal chitinase. More preferably, "chitinase" as used herein refers to a mammalian chitinase and/or to a chitinase belonging to the Glycosyl Hydrolases (GH) 18 family. Human chitinases are particularly preferred. Examples of preferred chitinases include: Acidic Mammalian Chitinase (AMCase), chitotriosidase (CHIT-1) and chitinase-like lectins (chi-lectins), such as human chitinase-3-like protein-1 (CHI3L1/HC-gp39/YKL-40), human chitinase-3-like protein-2 (CHI3L2/YKL-39), and oviductins. Still preferably, "chitinase" as used herein refers to Acidic Mammalian Chitinase (AMCase) and/or chitotriosidase (CHIT-1), in particular to human Acidic Mammalian Chitinase (AMCase) and/or human chitotriosidase (CHIT-1).

Chitinase inhibition may be measured according to a variety of methods known to the skilled person. In particular, chitinase inhibition can be measured by the inhibition constant (Ki) or by the percentage of inhibition, as described in the assays hereinbelow. Preferably, compounds of the invention exhibit a Ki for a chitinase lower than or equal to 50 µM and/or a percentage of inhibition of a chitinase equal to or greater than 70% at a fixed concentration such as 50 µM or 100 µM. In a preferred embodiment, the compound or a pharmaceutically acceptable salt, tautomer, solvate, or stereoisomer thereof as defined above is for use in inhibiting the activity of a chitinase. Inhibition of chitinase activity may be measured with respect to a proper control, such as a subject affected by a disease or disorder mediated by the activity of a chitinase or a subject throughout the course of a therapy for a disease or disorder mediated by the activity of a chitinase. Preferably, compounds of the invention inhibit chitinase activity by at least 10%, 20%, 30%, 40%, 50%, 60%, 70%, 80%, 90% in respect to a proper control. More preferably, compounds of the invention inhibit chitinase activity by approximately 10%, 20%, 30%, 40%, 50%, 60%, 70%, 80%, 90% in respect to a proper control.

Further, the activity of the chitinase enzyme can be determined *in vitro,* both in presence and in absence of a compound of the present invention, by those skilled in the art through a fluorescence assay which exploits a reagent that can be hydrolysed by the chitinase enzyme. Such reagent, upon hydrolysis, is able to release a compound emitting fluorescence. Such fluorescence can be detected by a fluorometer, for example a Perkin Elmer Envision Multilabel Reader 2014. More in detail, the reagent can be 4-methylumbelliferyl β-D-N,N',N"-triacetylchitotrioside, which upon hydrolysis releases the fluorescent compound 4-methylumbelliferone. Inhibition can then be evaluated as fluorescence with respect to a proper control, such as untreated chitinase enzyme or chitinase enzyme treated with a known chitinase inhibitor.

The activity of the chitinase enzyme can be also determined *in vivo,* both in presence and in absence of a compound of the present invention, by those skilled in the art through the evaluation of the expression of AMCase during pulmonary Th2-mediated inflammation in a subject, e.g. a mouse. Such evaluation can be performed through the measurement of quantity of AMCase protein and AMCase mRNA in the subject's pulmonary tissue, and through the measurement of the chitinase activity exerted by the bronchoalveolar lavage of such subject. The presence or absence of AMCase protein in both epithelial cells and macrophages can be evaluated through immunohistochemistry of the subject's lungs. Inhibition can then be evaluated by comparison to a proper control, such as untreated subject or subject treated with a known chitinase inhibitor.

The expression "wherein said compounds is a chitinase/AMCase/CHIT-1 inhibitor" indicates that the compound may be used as an inhibitor of such proteins. Then, the present invention also includes: the compound as defined above for use as a chitinase inhibitor as well as a method of inhibiting at least one chitinase comprising the step of contacting said chitinase with a compound as described herein. Preferably, the compound as defined above is for use in the treatment, prevention and/or amelioration of any disease, disorder or condition susceptible of being ameliorated by the inhibition of a chitinase, such as a disease or disorder mediated by the activity of a chitinase; a disease, disorder or condition associated with aberrant expression or activity of a chitinase; a disease, disorder or condition characterised by upregulation and/or overexpression of a chitinase. Another object of the present invention relates to an *in vitro* method of inhibiting a chitinase with the compound of the present invention. This may be useful, for instance, to evaluate whether any given compound is an inhibitor/activator of such a chitinase.

Therefore, the compounds of the invention can be used for the treatment of diseases and for carrying out biological assays, cellular assays, biochemical assays or the like.

In a preferred aspect, the compound as defined above is for use as a medicament. Preferably, said medicament is an inhibitor of a chitinase, wherein said chitinase is preferably Acidic Mammalian Chitinase (AMCase) and/or chitotriosidase (CHIT-1).

Preferably, the compound as defined above is for use in a method of treatment, prevention and/or amelioration of a Th-2-mediated disease, an IL-13-mediated disease, an IL-4-mediated disease, an IL-5-mediated disease or an IL-9-mediated disease. More preferably, the compound as defined above is for use as an inhibitor of a chitinase in a method of treatment, prevention and/or amelioration of a Th-2-mediated disease, an IL-13-mediated disease, an IL-4-mediated disease, an IL-5-mediated disease or an IL-9-mediated disease.

As used herein, the expression "Th-2-mediated disease" refers to any disease in whose pathogenesis Type 2 helper T cells are involved.

As used herein, the expressions "IL-4-mediated disease", "IL-5-mediated disease", "IL-9-mediated disease", "IL-13-mediated disease" refer to diseases whose pathogenesis involves and/or is mediated by cytokines and, more in detail, interleukins named, respectively, interleukin-4, interleukin-5, interleukin-9, interleukin-13. Examples of such diseases include but are not limited to: allergic diseases, tumors and central nervous system tumors, HIV infections for IL-4; allergic rhinitis for IL-5; large cell anaplastic lymphoma, Hodgkin's disease for IL-9; parasitosis, asthma, allergic lung disease for IL-13.

Preferably, the compound as defined above is for use in the treatment, prevention and/or amelioration of any one or more of: an airway disease, a lung disease, a chronic lung disease, an inflammatory disease, an infection, an immune response, tissue remodelling, an ocular pathology (allergic or non-allergic), an allergic disease or reaction, a disorder of the gastrointestinal tract, a dental disease, a neurologic disease, a metabolic disease, a liver disease, a kidney disease, a skin disease, a fibrotic disorder and/or cancer. More preferably, the compound as defined above is for use in the treatment, prevention and/or amelioration of any one or more of: asthma, bronchoconstriction, cough, shortness of breath, wheezing, chest tightness, wheezing dyspnea, airway narrowing, chronic airway narrowing, respiratory impairment, chronic respiratory impairment, bronchospasm, lung inflammation, airway inflammation, chronic inflammatory disease, idiopathic pulmonary fibrosis, interstitial lung disease, chronic obstructive pulmonary disease (COPD), airway hyper-responsiveness, bronchial hyper-reactivity, Th2 immune response, conjunctivitis, vernal keratoconjunctivitis, seasonal allergic conjunctivitis, dry eye syndrome, Th2 inflammation eye's disease, allergic rhinitis, seasonal allergic rhinitis, chronic rhinosinusitis, allergic lung disease, chronic lung disease, adenoid hypertrophy, a nasal polyp, eosinophilic esophagitis, a fungal infection, a parasitic infection, parasitosis, celiac disease, a food allergy, irritable bowel syndrome, irritable bowel disease, microscopic colitis, atopic eczema, atopic dermatitis, allergic contact dermatitis, eosinophilic otitis media, eosinophilic pneumonia, IgG4 mediated disease, cystic fibrosis, Hermansky-Pudlak syndrome, Alzheimer's disease, inflammatory bowel disease, ulcerative colitis, Crohn's disease, rheumatoid arthritis, osteoarthritis, psoriasis, scleroderma, multiple sclerosis, Sjogren's syndrome, atherosclerosis, sarcoidosis, periodontitis, insulin-dependent diabetes mellitus and non- insulin-dependent diabetes mellitus, non-alcoholic fatty liver disease, non-alcoholic steatohepatitis, hepatitis-C virus-induced fibrosis and cirrhosis, alcoholic fibrosis, cancer of the nervous system, glioblastoma, breast cancer, colon cancer, primary and metastatic lung cancer, mesothelioma, osteosarcoma, malignant melanoma, ovarian cancer, cervical cancer, prostate cancer, liver cancer, gastric cancer, metastatic renal cancer, leukemia, lymphoma, large cell anaplastic lymphoma, Hodgkin's disease, nephropathy (e.g. diabetic nephropathy), focal segmental glomerulosclerosis, tubulointerstitial fibrosis, postransplant fibrosis, and retroperitoneal fibrosis (e.g. Ormond's disease), fibrotic disorder, Gaucher disease, Fabry disease, lysosomal storage disorders, Niemann-Pick disease, nephropatic cysteinosis, X-linked globotiaosylceramidosis, polycystic ovary syndrome, endometriosis and HIV.

Still preferably, the compound as defined above is for use in a method of treatment, prevention and/or amelioration of any one or more of: asthma, bronchoconstriction, cough, shortness of breath, wheezing, chest tightness, wheezing dyspnea, airway narrowing, chronic airway narrowing, respiratory impairment, chronic respiratory impairment, bronchospasm, lung inflammation, idiopathic pulmonary fibrosis, allergic rhinitis, allergic lung disease, interstitial lung disease, chronic obstructive pulmonary disease (COPD), airway hyper-responsiveness, bronchial hyper-reactivity, Th2 immune response, conjunctivitis, sarcoidosis, vernal keratoconjunctivitis, seasonal allergic conjunctivitis, dry eye syndrome, chronic rhinosinusitis, chronic lung disease, adenoid hypertrophy, a nasal polyp, eosinophilic esophagitis, cancer, parasitosis.

More preferably, the compound as defined above is for use in a method of treatment, prevention and/or amelioration of asthma. "Asthma" as used in the present invention refers to a lung disease that obstructs at least one airway and/or reduces the flow of air into and out of the lungs and includes all types of asthma, including but not limited to: atopic asthma, allergic asthma, non-allergic asthma, adult-onset asthma, asthma-COPD overlap, exercise-induced bronchoconstriction, occupational asthma, etc. In particular, the treatment, prevention and/or amelioration of asthma refers to the treatment, prevention and/or amelioration of at least one asthma symptom, such as: bronchoconstriction, cough, shortness of breath, wheezing, chest tightness, wheezing dyspnea, chronic airway narrowing, chronic respiratory impairment, bronchospasm, lymphocytic and eosinophilic inflammation, remodelling of the bronchial mucosa, thickening of the lamina reticularis beneath the airway epithelium, hyperplasia of the cells of all structural elements of the airway wall-vessels, smooth muscle, secretory glands and goblet cells, etc. Still preferably, any of said uses is in combination with a further therapeutic agent.

In another preferred aspect, the present invention provides the combination of the compound as defined above with a further therapeutic agent.

In a further preferred aspect, the present invention provides a pharmaceutical composition comprising:
- the compound as defined above,
- a vehicle, and
- optionally a further therapeutic agent.

Preferably, said further therapeutic agent is another known therapy that is useful for the prevention/amelioration/treatment of any of the above-mentioned diseases, disorders or conditions. More preferably, said further therapeutic agent selected from the group consisting of: a chitinase inhibitor, an anti-chitinase antibody, a steroid, a membrane stabilizer, a 5LOX inhibitor, a leukotriene synthesis and receptor inhibitor, an inhibitor of IgE isotype switching or IgE synthesis, an IgG isotype switching or IgG synthesis, a b-agonist, an anti-Th2 cytokine, a tryptase inhibitor, aspirin, a COX inhibitor, methotrexate, an anti-TNF drug, retuxin, a PD4 inhibitor, a p38 inhibitor, a PDE4 inhibitor, a xanthine and an antihistamine.

In particular, the further therapeutic agent may be a dual AMCase-CHIT-1 inhibitor. Additionally or alternatively, the further therapeutic agent being a chitinase inhibitor may be a bisdionin such as Bisdionin C and Bisdionin F, allosamidin or a compound related thereto, cyclo(L-Arg-D-Pro), argifin or argadin. More preferably, said further therapeutic agent being a chitinase inhibitor is allosamidin or argifin.

Bisdionins such as Bisdionin C and Bisdionin F may be as described in: Sutherland, T. E. et al.; Analyzing Airway Inflammation with Chemical Biology: Dissection of Acidic Mammalian Chitinase Function with a Selective Drug-Like Inhibitor. Chemistry & Biology 2011, 18 (5), 569-579, herein incorporated by reference.

Allosamidin and related compounds may be as described in any one or more of: Sakuda, S. et al.; A. Search for Microbial Insect Growth Regulators. II. Allosamidin, a Novel Insect Chitinase Inhibitor. J. Antibiot. 1987, 40 (3), 296-300; Nishimoto, Y.; Isolation and Characterization of New Allosamidins. J. Antibiot. 1991, 44 (7), 716-722; Griffith, D. A.; Danishefsky, S. J. The Total Synthesis of Allosamidin. Expansions of the Methodology of Azaglycosylation Pursuant to the Total Synthesis of Allosamidin. a Surprising Enantiotopic Sense for a Lipase-Induced Deacetylation. J. Am. Chem. Soc. 1996, 118 (40), 9526-9538, all of which are herein incorporated by reference.

Cyclo(L-Arg-D-Pro) may be as described in any one or more of: Izumida, H. et al.; A Novel Chitinase Inhibitor From a Marine Bacterium, Pseudomonas Sp. J. Antibiot. 1996, 49 (1), 76-80; Izumida, H. et al.; The Effect of Chitinase Inhibitors, Cyclo(Arg-Pro) Against Cell Separation of Saccharomyces Cerevisiae and the Morphological Change of Candida Albicans. J. Antibiot. 1996, 49 (8), 829-831; Houston, D. R. et al.; The Cyclic Dipeptide CI-4 [Cyclo-(L-Arg-D-Pro)] Inhibits Family 18 Chitinases by Structural Mimicry of a Reaction Intermediate. Biochem. J. 2002, 368 (Pt 1), 23-27, all of which are herein incorporated by reference.

Argifin and Argadin may be as described in any one or more of: Andersen, O. A. et al.; Structure-Based Dissection of the Natural Product Cyclopentapeptide Chitinase Inhibitor Argifin. Chemistry & Biology 2008, 15 (3), 295-301; Rao, F. V. et al.; Specificity and Affinity of Natural Product Cyclopentapeptide Inhibitors Against a. Fumigatus, Human, and Bacterial Chitinases. Chemistry & Biology 2005, 12 (1), 65-76, all of which are herein incorporated by reference.

Additional chitinase inhibitors suitable for use according to the present invention are those developed by Oncoarendi Therapeutics and described in: Cole, D. C. et al.; Identification and Characterization of Acidic Mammalian Chitinase Inhibitors. J. Med. Chem. 2010, 53 (16), 6122-6128; Mazur, M. et al.; Targeting Acidic Mammalian Chitinase Is Effective in Animal Model of Asthma. J. Med. Chem. 2017, acs.jmedchem.7b01051-acs.jmedchem.7b01051.; WO2016/099331 and WO 2017/037670, all of which are herein incorporated by reference.

Preferably, said anti-chitinase antibody is produced as described in Goto, M. et al., Immunohistochemical demonstration of acidic mammalian chitinase in the mouse salivary gland and gastric mucosa. Archives of Oral Biology 2003, 10.1016/S0003-9969(03)00150-X or produced as anti-AMCase antiserum as described in Zhu, Z. et al., Acidic Mammalian Chitinase in AsthmaticTh2 Inflammation and IL-13 Pathway Activation. Science 2004, 10.1126/science.1095336.

As used herein "steroid" includes the agents described in: NK Ostrom, Allergy Asthma Proc. 2014 May-Jun;35 Suppl 1:S3-10, doi: 10.2500/aap.2014.35.3758; LM Montaño et al., Steroids. 2019 Oct 3;153:108509, doi: 10.1016/j.steroids.2019.108509; and PJ Barnes, J Biol Chem. 2011 Sep 23;286(38):32899-905, doi: 10.1074/jbc.R110.206466. Preferred examples of steroids are glucocorticosteroids and androgens.

As used herein "membrane stabilizer" includes the agents described in: P Peachell, Curr Opin Pharmacol. 2005 Jun;5(3):251-6, doi: 10.1016/j.coph.2005.03.001. Preferred examples of membrane stabilizers are ketotifen and cromones such as cromoglycate and nedocromil.

As used herein "a 5LOX inhibitor, a leukotriene synthesis and receptor inhibitor" includes the agents described in: PJ Barnes J Biol Chem. 2011 Sep 23;286(38):32899-905, doi: 10.1074/jbc.R110.206466. Preferred examples of 5LOX inhibitor, a leukotriene synthesis and receptor inhibitor are Leukotriene Modifiers.

As used herein "an inhibitor of IgE isotype switching or IgE synthesis" includes the agents described in: MY Ozkars et al., Allergol Immunopathol (Madr). 2018 May - Jun;46(3):226-234. doi: 10.1016/j.aller.2017.07.003.

As used herein "an IgG isotype switching or IgG synthesis" includes the agents described in: EM Egeskjold et al., Allergy. 1981 Nov;36(8):573-81, doi: 10.1111/j.1398-9995.1981.tb01875.x.

As used herein "b-agonists" includes the agents described in: PJ Barnes J Biol Chem. 2011 Sep 23;286(38):32899-905, doi: 10.1074/jbc.R110.206466. Preferred examples of b-agonists include β₂-adrenergic agonists.

As used herein "anti-Th2 cytokine" includes the agents described in: PM Hansbro et al., Expert Opin Investig Drugs. 2013 Jan;22(1):49-69. doi: 10.1517/13543784.2013.732997.

As used herein "tryptase inhibitor" includes the agents described in: KD Rice et al., Current Pharmaceutical Design. 2010;4(5):381-96 doi: 10.1002/chin.199901273 and in: RP Numerof et al., Expert Opin Investig Drugs. 1997 Jul;6(7):811-7.

As used herein "COX inhibitor" includes the agents described in: S Bacchi et al., Antiinflamm Antiallergy Agents Med Chem. 2012;11(1):52-64, doi: 10.2174/187152312803476255. Preferred examples of b-agonists include NSAIDs, non-steroidal anti-inflammatory drugs.

As used herein "anti-TNF drug" includes the agents described in: M Berry et al, Curr Opin Pharmacol. 2007 Jun;7(3):279-82, doi: 10.1016/j.coph.2007.03.001 and in: MY Ozkars et al., Allergol Immunopathol (Madr). 2018 May - Jun;46(3):226-234. doi: 10.1016/j.aller.2017.07.003.

As used herein "p38 inhibitor" includes the agents described in: P. Norman, Expert Opin Investig Drugs. 2015 Mar;24(3):383-92, doi: 10.1517/13543784.2015.1006358.

As used herein "PDE4 inhibitor" and "xanthine" include the agents described in: PJ Barnes J Biol Chem. 2011 Sep 23;286(38):32899-905, doi: 10.1074/jbc.R110.20646 and in: WM Brown. Treating COPD with PDE 4 inhibitors. Int J Chron Obstruct Pulmon Dis. 2007;2(4):517-33, PMID: 18268925; PMCID: PMC2699952.

As used herein "antihistamine" includes the agents described in: AW Jones, Clin Pharmacol Drug Dev. 2016 Jan;5(1):5-12. doi: 10.1002/cpdd.236 and NK Ostrom, Allergy Asthma Proc. 2014 May-Jun;35 Suppl 1:S3-10. doi: 10.2500/aap.2014.35.3758.

In a preferred aspect, the pharmaceutical composition as defined above is for use as an inhibitor of a chitinase, wherein said chitinase is preferably Acidic Mammalian Chitinase (AMCase) and/or chitotriosidase (CHIT-1). Preferably, the pharmaceutical composition is for use in a method of treatment, prevention and/or amelioration of any one or more of the diseases, disorders or conditions defined above, for instance said Th-2-mediated diseases, IL-13-mediated diseases, IL-4-mediated diseases, IL-5-mediated diseases or IL-9-mediated diseases, in particular asthma.

Compounds of the invention may be prepared in a variety of ways. These processes form further aspects of the invention. In particular, it is a further object of the invention a process for the preparation of the compound as defined above according to one of the following synthetic schemes:
- Scheme A, wherein X-Y, Z-W and M-Q are -CH₂CH₂-, K is -CH₂- and PG is a protecting group, comprising the following steps: 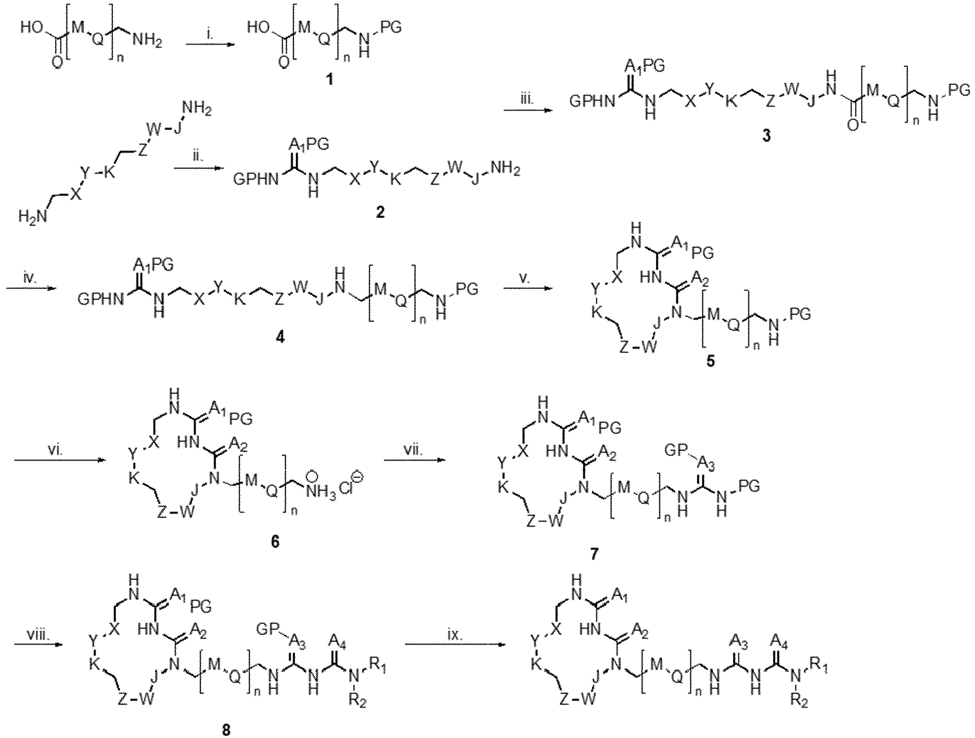
   (i) Protection of the amino acid of formula H₂NCH₂[M-Q]ₙCOOH to obtain a protected compound of formula 1;
   (ii) Addition-elimination reaction of the diamine of formula H₂NCH₂XYKCH₂ZWJNH₂ with a compound of formula 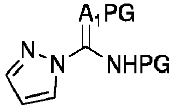 to obtain a compound of formula 2;
   (iii) Coupling of the protected compound of formula 1 with the compound of formula 2 to obtain an amide compound formula 3;
   (iv) Reduction of the amide compound of formula 3 to an amine compound of formula 4;
   (v) Intramolecular reaction of the compound of formula 4 to obtain a macrocyclic compound of formula 5;
   (vi) Deprotection of the macrocyclic compound of formula 5 to obtain a deprotected macrocyclic compound of formula 6;
   (vii) Addition-elimination reaction of the deprotected macrocyclic compound of formula 6 with a compound of formula 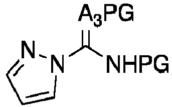 to obtain a compound of formula 7;
   (viii) Nucleophilic substitution of the compound of formula 7 with a compound of formula NR₁R₂ to obtain a compound of formula 8;
   (ix) Deprotection of the compound of formula 8 to obtain a compound as defined above;
or Scheme B, wherein X-Y is a substituted or unsubstituted aryl or a substituted or unsubstituted heteroaryl, M-Q is -CH₂CH₂-, K is O or S and PG is a protecting group, comprising the following steps: 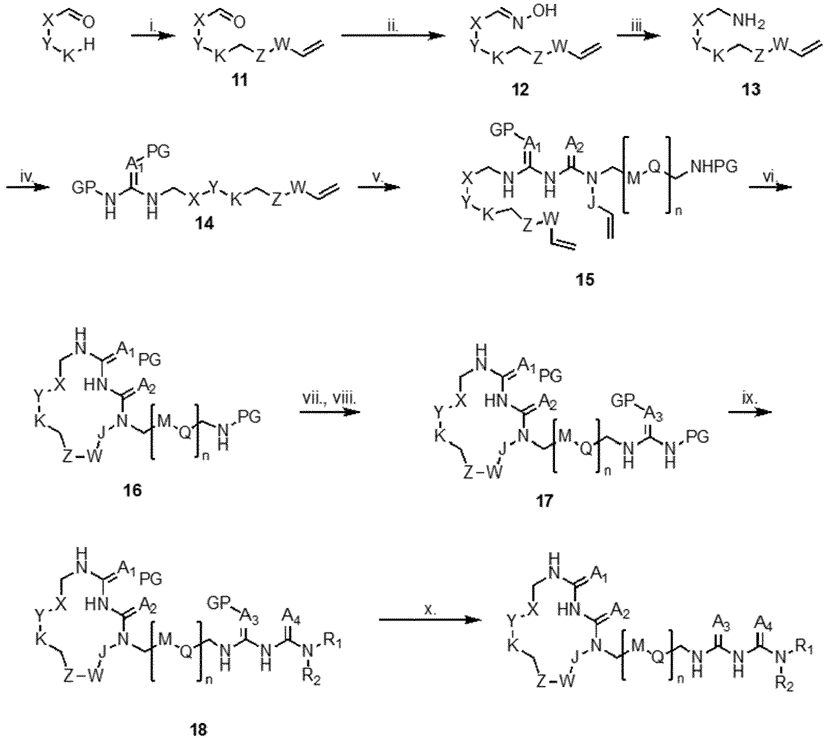
   (i) Reaction of the aldehyde of formula KH-Y-X-CHO with a bromoalkene of formula H₂CCHWZCH₂Br to obtain a compound of formula 11;
   (ii) Reaction of a compound of formula 11 with hydroxylamine or a salt thereof to obtain an oxime compound of formula 12;
   (iii) Reduction of the oxime compound of formula 12 to obtain an amine compound of formula 13;
   (iv) Addition-elimination reaction of the amine compound of formula 13 with a compound of formula 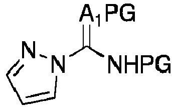 to obtain a compound of formula 14;
   (v) Reaction of a compound of formula 14 with a linker compound of formula H₂CCHJNHCH₂[M-Q]ₙCH₂NHPG to obtain a compound of formula 15;
   (vi) Intramolecular reaction of the compound of formula 15 to obtain a macrocyclic compound of formula 16;
   (vii)-(viii) Reduction of the macrocyclic compound of formula 16 and subsequent addition-elimination reaction with a compound of formula 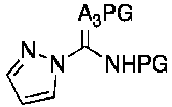 to obtain a macrocyclic compound of formula 17;
   (ix) Nucleophilic substitution of the compound of formula 17 with a compound of formula NR₁R₂ to obtain a compound of formula 18;
   (xi) Deprotection of the compound of formula 18 to obtain a compound as defined above;
or Scheme C, wherein X-Y and W-Z are a substituted or unsubstituted aryl or a substituted or unsubstituted heteroaryl, M-Q is -CH₂CH₂- and K is O or S, comprising the following steps: 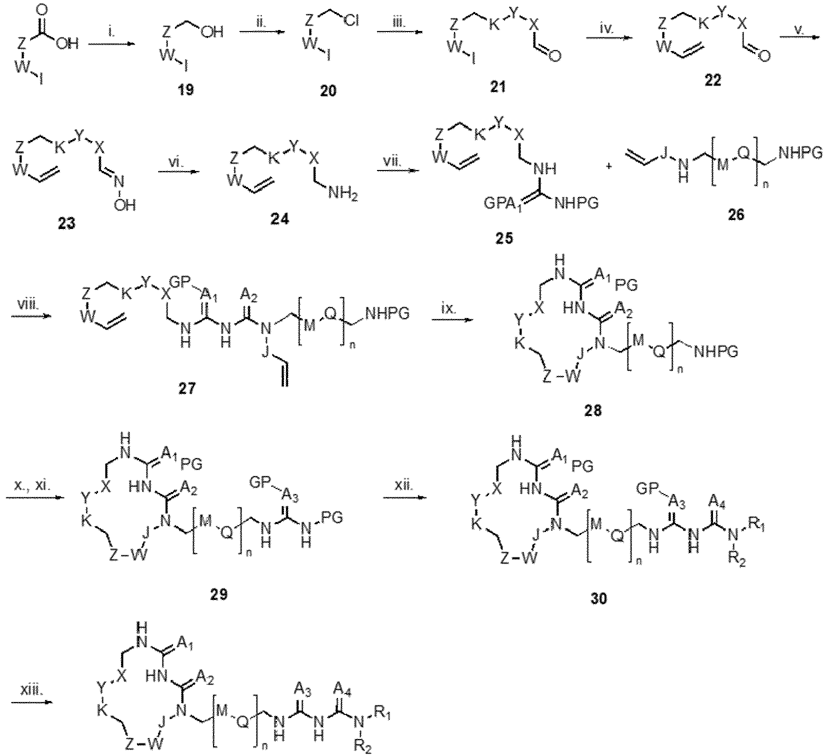
   (i) Reduction of the carboxylic acid of formula I-W-Z-COOH to obtain a primary alcohol of formula 19;
   (ii) Conversion of the primary alcohol of formula 19 to a chloride of formula 20;
   (iii) Nucleophilic substitution of the chloride of formula 20 with a compound of formula KH-Y-X-CHO to obtain a compound of formula 21;
   (iv) Conversion of the iodide of formula 21 to a vinyl compound of formula 22;
   (v) Reaction of a compound of formula 22 with hydroxylamine or a salt thereof to obtain an oxime compound of formula 23;
   (vi) Reduction of the oxime compound of formula 23 to obtain an amine compound of formula 24;
   (vii) Addition-elimination reaction of the amine compound of formula 24 with a compound of formula 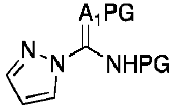 to obtain a compound of formula 25;
   (viii) Reaction of the compound of formula 25 with a linker compound of formula 26 to obtain a compound of formula 27;
   (ix) Intramolecular reaction of the compound of formula 27 to obtain a macrocyclic compound of formula 28;
   (x) and (xi) Reduction of the macrocyclic compound of formula 28 and subsequent addition-elimination reaction with a compound of formula 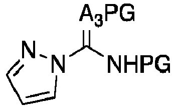 to obtain a macrocyclic compound of formula 29;
   (xii) Nucleophilic substitution of the compound of formula 29 with a compound of formula NR₁R₂ to obtain a compound of formula 30;
   (xiii) Deprotection of the compound of formula 30 to obtain a compound as defined above.

The present invention also provides intermediates of the compounds as defined above. In particular, it is an object of the present invention an intermediate of a compound as defined above having one of the following formulas: 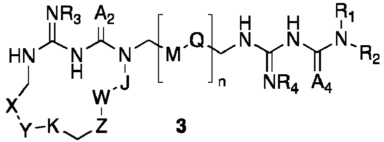 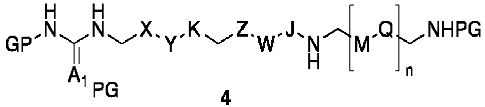 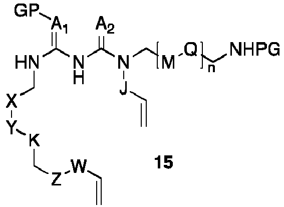 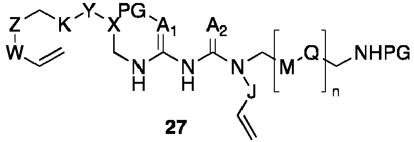 wherein n, J, K, A₁, A₂, A₃, A₄, R₁, R₂, X-Y, Z-W are as defined above, R₃ is H or a protecting group, PG and R₄ are a protecting group, preferably said protecting group is Boc or Cbz. It is to be understood that where a compound contains more than one protecting group, such protecting groups may be the same or different. Compounds of formula (III), 4, 15 and 27 are preferably useful as intermediates in the synthesis of compounds of formula (I) as defined above. In a preferred aspect, the compounds of formula (III), 4, 15 and 27 are for use as inhibitors of a chitinase, wherein said chitinase is preferably Acidic Mammalian Chitinase (AMCase) and/or chitotriosidase (CHIT-1). Preferably, the compounds of formula (III), 4, 15 and 27 are for use in a method of treatment, prevention and/or amelioration of any one or more of the diseases, disorders or conditions defined above, for instance said Th-2-mediated diseases, IL-13-mediated diseases, IL-4-mediated diseases, IL-5-mediated diseases or IL-9-mediated diseases, in particular asthma.

It is understood that substituents and substitution patterns on the compounds of the instant invention can be selected by one of ordinary skill in the art to provide compounds that are chemically stable and that can be readily synthesized by techniques known in the art, as well as by the methods set forth below, from readily available starting materials. If a substituent is itself substituted with more than one group, it is understood that these multiple groups may be on the same atom or on different atoms, so long as a stable structure results.

The expression "one or more substituents" refers in particular to 1, 2, 3, 4 or more substituents, in particular to 1, 2, 3 or 4 substituents, more in particular to 1, 2 or 3 substituents.

As used herein, the term "alkyl" refers to both branched and linear saturated aliphatic hydrocarbon chains containing the specified number of carbon atoms. For example, "C₁-C₆ alkyl" includes groups containing 1, 2, 3, 4, 5 or 6 carbon atoms in a linear or branched arrangement. "C₁-C₄ alkyl" includes groups containing 1, 2, 3 or 4 carbon atoms in a linear or branched arrangement. Preferred alkyl groups include: methyl, ethyl, n-propyl, i-propyl, n-butyl, t-butyl, i-butyl, pentyl, hexyl, and so on.

As used herein, the term "alkenyl" refers to both branched and linear aliphatic unsaturated hydrocarbon chains containing the specified number of carbon atoms and at least one carbon-carbon double bond. For example, "C₂-C₆ alkenyl" includes groups containing 2, 3, 4, 5 or 6 carbon atoms in a linear or branched arrangement and at least one carbon-carbon double bond. Preferred alkenyl groups include: ethenyl, propenyl, allyl, isobuthenyl, pentenyl, prenyl, esenyl, and so on.

As used herein, the term "alkynyl" refers to both branched and linear aliphatic unsaturated hydrocarbon chains containing the specified number of carbon atoms and at least one carbon-carbon triple bond. For example, "C₂-C₆ alkynyl" includes groups containing 2, 3, 4, 5 or 6 carbon atoms in a linear or branched arrangement and at least one carbon-carbon triple bond. Preferred alkynyl groups include: acetylenyl, ethynyl, propynyl, and so on.

As used herein, the term "halogen" includes fluorine, chlorine, bromine and iodine. Preferably, halogen refers to fluorine or chlorine.

As used herein, the term "aryl" or "aromatic ring" refers to monocyclic, bicyclic or polycyclic aromatic hydrocarbon groups, for example, benzene, naphthalene, anthracene, 1,2,3,4-tetrahydronaphthalene, indene, 2,3-dihydroindene, and pyrene. Polycyclic systems may have two or more cyclic rings in which two or more carbons are common to two adjoining rings (fused rings) wherein at least one of the rings is an aromatic hydrocarbon. The aromatic ring may be substituted at one or more ring positions with one or more substituents, such as halogen, azide, alkyl, aralkyl, alkenyl, alkynyl, cycloalkyl, cycloalkylalkyl, (cycloalkyl)alkoxyl, hydroxyl, alkoxyl, amino, nitro, sulfhydryl, imino, amido, phosphonate, phosphinate, carbonyl, carboxyl, silyl, ether, alkylthio, sulfonyl, aminosulfonyl, sulfonamido, ketone, aldehyde, ester, heterocyclyl, heterocyclylalkyl, aromatic or heteroaromatic moieties, aminoalkyl, haloalkyl, fluoroalkyl (such as trifluoromethyl), haloalkoxyl, cyano, or the like.

As used herein, the term "heteroaryl" refers to a monocyclic, bicyclic, and polycyclic aromatic group having 3 to 14 total atoms including one or more heteroatoms such as nitrogen, oxygen, or sulfur in the ring structure. More preferred heteroaryl groups have 5-10 ring members where from 1-4 of the ring members are hetero atoms selected from the group consisting of O, N, and S. Exemplary heteroaryl groups include but are not limited to, for example, azaindolyl, benzimidazolyl, benzofuranyl, benzoxazolyl, benzothiazolyl, benzothiadiazolyl, benzotriazolyl, benzoxadiazolyl, furanyl, imidazolyl, imidazopyridinyl, indolyl, indolinyl, indazolyl, isoindolinyl, isoxazolyl, isothiazolyl, isoquinolinyl, oxadiazolyl, oxazolyl, purinyl, pyranyl, pyrazinyl, pyrazolyl, pyridinyl, pyrimidinyl, pyrrolyl, pyrrolo[2,3-d]pyrimidinyl, pyrazolo[3,4-d]pyrimidinyl, quinolinyl, quinazolinyl, triazolyl, thiazolyl, thiophenyl, tetrahydroindolyl, tetrazolyl, thiadiazolyl, thienyl, thiomorpholinyl or tropanyl, and the like. The "heteroaryl" may be substituted at one or more ring positions with one or more substituents such as halogen, azide, alkyl, aralkyl, alkenyl, alkynyl, cycloalkyl, hydroxyl, alkoxyl, amino, nitro, sulfhydryl, imino, amido, phosphonate, phosphinate, carbonyl, carboxyl, silyl, ether, alkylthio, sulfonyl, sulfonamido, ketone, aldehyde, ester, heterocyclyl, aromatic or heteroaromatic moieties, fluoroalkyl (such as trifluromethyl), cyano, or the like. The term "heteroaryl" also includes polycyclic ring systems having two or more cyclic rings in which two or more carbons are common to two adjoining rings (fused rings) wherein at least one of the rings is an aromatic group having one or more heteroatoms in the ring structure. Representative examples of bicyclic heteroaryl include, but are not limited to, benzimidazolyl, benzofuranyl, benzothienyl, benzoxadiazolyl, benzoxathiadiazolyl, benzothiazolyl, cinnolinyl, 5,6-dihydroquinolin-2-yl, 5,6- dihydroisoquinolin-I-yl, furopyridinyl, indazolyl, indolyl, isoquinolinyl, naphthyridinyl, quinolinyl, purinyl, 5,6,7, 8-tetrahydroquinolin-2-yl, 5,6,7,8-tetrahydroquinolin-3-yl, 5,6,7,8-tetrahydroquinolin-4-yl, 5,6,7,8-tetrahydroisoquinolin-I-yl, thienopyridinyl, 4,5,6,7-tetrahydrobenzo c][I,2,5]oxadiazolyl, and 6,7-dihydrobenzo[c][I,2,5]oxadiazol-4(5H)-onyl.

The term "haloalkyl" as used herein refers to an alkyl group, as defined herein, wherein some or all of the hydrogens are replaced with halogen atoms. The term "haloalkoxyl" refers to an alkoxy group, as defined herein, wherein some or all of the hydrogens are replaced with halogen atoms. An exemplary haloalkyl group is trifluoromethyl.

As used herein, the term "alkoxy" refers to an alkyl group, as defined herein, appended to the parent molecular moiety through an oxygen atom. Representative examples of alkoxy include, but are not limited to, methoxy, ethoxy, propoxy, 2-propoxy, butoxy, tert-butoxy, pentyloxy, and hexyloxy.

As used herein, the term "cycloalkyl" refers to mono- or bicyclic or bridged saturated or partially (un)saturated carbocyclic rings, each having from 3 to 12 carbon atoms. Examples of monocyclic cycloalkyls include cyclopropyl, cyclobutyl, cyclopentyl, cyclopentenyl, cyclohexyl, cyclohexenyl, cycloheptyl, and cyclooctyl. Bicyclic cycloalkyl ring systems include bridged monocyclic rings and fused bicyclic rings. Bridged monocyclic rings contain a monocyclic cycloalkyl ring where two non-adjacent carbon atoms of the monocyclic ring are linked by an alkylene bridge of between one and three additional carbon atoms (i.e., a bridging group of the form -(CH2)ₙ-, where n is 1, 2, or 3). Representative examples of bicyclic ring systems include, but are not limited to, bicyclo[3.1.1]heptane, bicyclo[2.2.1]heptane, bicyclo[2.2.2]octane, bicyclo[3.2.2]nonane, bicyclo[3.3.1]nonane, and bicyclo[4.2.1]nonane. Fused bicyclic cycloalkyl ring systems contain a monocyclic cycloalkyl ring fused to either a phenyl, a monocyclic cycloalkyl, a monocyclic cycloalkenyl, a monocyclic heterocyclyl, or a monocyclic heteroaryl. The bridged or fused bicyclic cycloalkyl is attached to the parent molecular moiety through any carbon atom contained within the monocyclic cycloalkyl ring. Cycloalkyl groups are optionally substituted.

As used herein, the terms "aralkyl" or "arylalkyl" herein refer to an alkyl group, for example a C1-C6 alkyl group, substituted with an aryl group, wherein the moiety is appended to the parent molecule through the alkyl group. The aromatic ring and the alkyl group may be substituted at one or more positions with one or more substituents. An exemplary aralkyl group is benzyl.

When X-Y, Z-W and/or M-Q are substituted or unsubstituted aryl or substituted or unsubstituted heteroaryl, a variety of different connectivity patterns could occur, all of which are enclosed within the scope of the present invention. As a non-limiting example, preferred connectivity patterns for when X-Y, Z-W and/or M-Q are substituted or unsubstituted aryl or substituted or unsubstituted heteroaryl are shown below for 5 and 6-membered rings. It is to be understood that analogous examples where the ring is different from the depicted one are also within the scope of the present invention. It is also to be understood that any atom of the ring skeleton may be substituted or unsubstituted as long as a valid chemical structure is obtained.

Preferably, when X-Y, Z-W and/or M-Q are substituted or unsubstituted aryl or substituted or unsubstituted heteroaryl, such aryl or heteroaryl may be connected to the rest of the macrocycle as follows: 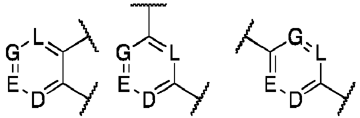 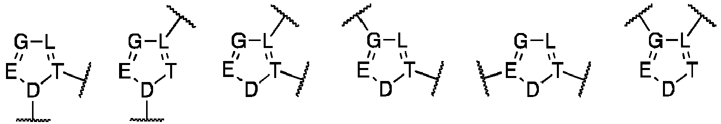 wherein D, E, G, L and T can be the same or different and are each independently N, O, S or C.

Also preferably, when X-Y, Z-W and/or M-Q are substituted or unsubstituted aryl or substituted or unsubstituted heteroaryl, such aryl or heteroaryl may be connected to the rest of the macrocycle as follows: 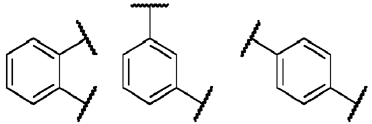 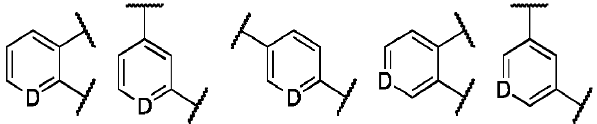 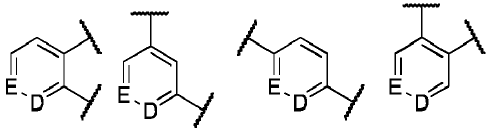 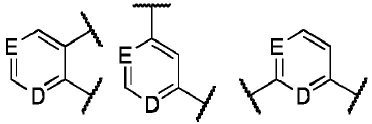 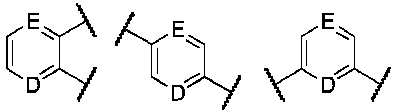 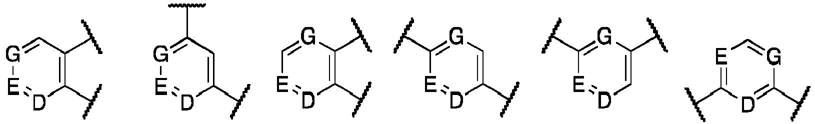 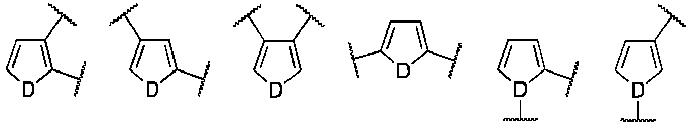 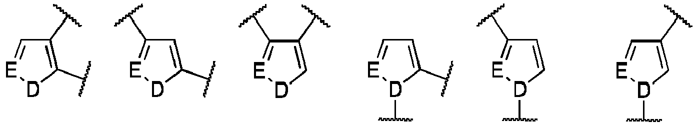 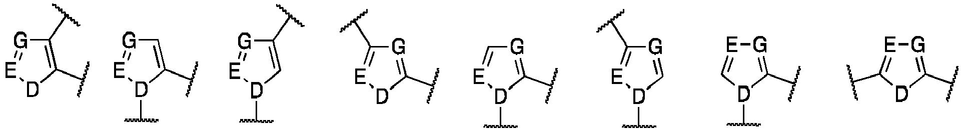 wherein D, E and G can be the same or different and are each independently N, O or S.

In a particularly preferred embodiment, X-Y, Z-W, and/or M-Q are as shown in any one of the above-depicted connectivity patterns. Still preferably, X-Y or Z-W are as shown in any one of the above-depicted connectivity patterns. More preferably, X-Y and Z-W are as shown in any one of the above-depicted connectivity patterns. When X-Y, Z-W and/or M-Q are aryl or heteroaryl, they may be connected to the rest of the macrocycle in the same way or in different ways.

Salts of the compounds of the invention are also encompassed within the scope of the invention. Because of their potential use in medicine, the salts of the compounds of the invention are preferably pharmaceutically acceptable. Suitable pharmaceutically acceptable salts comprise conventional nontoxic salts obtained by salification of a compound of the invention with inorganic acids (e.g. hydrochloric, hydrobromic, sulphuric, or phosphoric acids), or with organic acids (e.g. acetic, propionic, succinic, benzoic, sulfanilic, 2-acetoxy-benzoic, cinnamic, mandelic, salicylic, glycolic, lactic, oxalic, malic, maleic, malonic, fumaric, tartaric, citric, p-toluenesulfonic, methanesulfonic, ethanesulfonic, or naphthalensulfonic acids). For reviews on suitable pharmaceutical salts see Berge S. M. et al., J. Pharm. Sci. 1977, 66, 1-19; Gould P. L. Int. J. Pharm 1986, 33, 201-217; Bighley et al. Encyclopedia of Pharmaceutical Technology, Marcel Dekker Inc, New York 1996, Volume 13, page 453-497; and Remington "The Science and Practice of Pharmacy", Lippincott Williams & Wilkins, 2000.

In addition, base addition salts can be formed with a suitable inorganic or organic base such as triethylamine, ethanolamine, triethanolamine, dicyclohexylamine, ammonium hydroxide, pyridine. The term "inorganic base," as used herein, has its ordinary meaning as understood by one of ordinary skill in the art and broadly refers to an inorganic compound that can act as a proton acceptor. The term "organic base," as used herein, also has its ordinary meaning as understood by one of ordinary skill in the art and broadly refers to an organic compound that can act as a proton acceptor.

Other suitable pharmaceutically acceptable salts include pharmaceutically acceptable alkali-metal or alkaline-earth-metal salts such as sodium, potassium, calcium or magnesium salts; in particular pharmaceutically acceptable salts of one or more carboxylic acid moieties that may be present in the compound of the invention.

Other salts, which are not pharmaceutically acceptable, for example the trifluoroacetate salt, may be useful in the preparation of compounds of this invention and these form a further aspect of the invention. The invention includes within its scope all possible stoichiometric and nonstoichiometric forms of the salts of the compounds of the invention.

In addition, the compounds of the invention may exist in unsolvated as well as in solvated forms with pharmaceutically acceptable solvents such as water, EtOH and the like. Both unsolvated and solvated forms (i.e. solvates) are within the scope of the present invention.

Certain compounds of the invention may exist in stereoisomeric forms (e.g. they may contain one or more asymmetric carbon atoms). The individual stereoisomers (enantiomers and diastereomers) and mixtures of these are included within the scope of the present invention. The present invention also covers the individual isomers of the compounds defined above as well as mixtures with isomers thereof in which one or more chiral centers are inverted. Racemic mixtures may be separated to give their individual enantiomer using preparative HPLC using a column with chiral stationary phase or resolved to yield individual enantiomers utilizing methods known to those skilled in the art. In addition, chiral intermediate compounds may be resolved and used to prepare individual enantiomers.

The compounds of the invention or solvates/hydrates of the compounds of the invention or salts, may exist in one or more polymorphic forms. The invention extends to all such forms whether in a pure polymorphic form or when admixed with any other material, such as another polymorphic form.

The compounds of the invention may exist in a zwitterionic form, all of which are included within the scope of the present invention.

Likewise, it is understood that compounds of the invention may exist in tautomeric forms other than that shown in the formula and these are also included within the scope of the present invention.

It will be appreciated by those skilled in the art that certain protected derivatives of the compounds of the invention, which may be made prior to a final deprotection stage, may not possess pharmacological activity as such, but may, in certain instances, be administered orally or parenterally and thereafter metabolized in the body to form compounds defined in the first aspect which are pharmacologically active. Such derivatives may therefore be described as "prodrugs". All protected derivatives and prodrugs of compounds defined above are included within the scope of the invention. Examples of suitable pro-drugs for the compounds of the present invention are described in Drug of Today, Volume 19, Number 9, 1983, pp 499-538 and in Topics in Chemistry, Chapter 31, pp 306-316 and in "Design of Prodrugs" by H. Bundgaard, Elsevier, 1985, Chapter 1 (the disclosure in which document is incorporated herein by reference). It will be further appreciated by those skilled in the art that certain moieties, known to those skilled in the art as "pro-moieties", for described by H. Bundgaard, in "Design of Prodrugs" (the disclosure in which document is incorporated herein by reference) may be placed on appropriate functionalities when such functionalities are present within the compound defined in the first aspect. The invention also includes all suitable isotopic variations of a compound of the invention. An "isotopic variation" of a compound of the invention is defined as one in which at least one atom is replaced by an atom having the same atomic number but an atomic mass different from the atomic mass usually found in nature. Examples of isotopes that can be incorporated into compounds of the invention include isotopes such as ²H, ³H, ¹³C, ¹⁴C, ¹⁵N, ¹⁷O, ¹⁸O, ³¹P, ³²P, ³⁵S, ¹⁸F and ³⁶Cl, respectively. Certain isotopic variations of the invention, for example, those in which a radioactive isotope such as ³H or ¹⁴C is incorporated, are useful in drug and/or substrate tissue distribution studies. Further, substitution with isotopes such as deuterium ²H, may afford certain therapeutic advantages resulting from greater metabolic stability. Isotopic variations of the compounds of the invention can generally be prepared by conventional procedures such as by the illustrative methods or by the preparations described in the examples hereafter using appropriate isotopic variations of suitable reagents.

It is also an object of the present invention a kit comprising the compound or the pharmaceutical composition as defined above. Preferably, said kit is a device for inhalation.

The pharmaceutical compositions can be chosen based on the treatment requirements. Such compositions are prepared by blending and are suitably adapted for oral or parenteral administration, and as such can be administered in the form of preparation for inhalation, tablets, capsules, oral preparations, powders, granules, pills, injectable or infusible liquid solutions, suspensions, suppositories. Preferably, the pharmaceutical composition of the present invention is suitable for administration by inhalation. For instance, the pharmaceutical composition is in the form of a powder and/or can be delivered from any inhalation device known to one of ordinary skill in the art, such an inhaler, an insufflator, a nebulizer pressurized pack, an aerosol.

The compounds for the present invention can be administered in intranasal form via topical use of suitable intranasal vehicles and delivery devices, or via transdermal routes, using those forms of transdermal skin patches well known to those of ordinary skill in the art.

The oral solid compositions can be prepared by conventional methods of blending, filling or tableting. The blending operation can be repeated to distribute the active principle throughout compositions containing large quantities of fillers. Such operations are conventional. Tablets and capsules for oral administration are normally presented in unit dose form and contain conventional excipients such as binders, fillers (including cellulose, mannitol, lactose), diluents, tableting agents, lubricants (including magnesium stearate), detergents, disintegrants (e.g. polyvinylpyrrolidone and starch derivatives such as sodium glycolate starch), coloring agents, flavoring agents, and wetting agents (for example sodium lauryl sulfate).

Oral liquid preparations can be in the form of, for example, aqueous or oily suspensions, solutions, emulsions, syrups or elixirs, or can be presented as a dry product for reconstitution with water or with a suitable vehicle before use. Such liquid preparations can contain conventional additives such as suspending agents, for example sorbitol, syrup, methyl cellulose, gelatine, hydroxyethyl cellulose, carboxymethyl cellulose, aluminium stearate gel, or hydrogenated edible fats; emulsifying agents, such as lecithin, sorbitan monooleate, or acacia; non-aqueous vehicles (which can include edible oils), such as almond oil, fractionated coconut oil, oily esters such as esters of glycerine, propylene glycol, or ethyl alcohol; preservatives, such as methyl or propyl p-hydroxybenzoate or sorbic acid, and if desired, conventional flavouring or colouring agents. Oral formulations also include conventional slow-release formulations such as enterically coated tablets or granules.

For parenteral administration fluid unit dosages can be prepared, containing the compound and a sterile vehicle. The compound can be either suspended or dissolved, depending on the vehicle and concentration. The parenteral solutions are normally prepared by dissolving the compound in a vehicle, sterilising by filtration, filling suitable vials and sealing. Advantageously, adjuvants such as local anaesthetics, preservatives and buffering agents can also be dissolved in the vehicle. To increase the stability, the composition can be frozen after having filled the vials and removed the water under vacuum. Parenteral suspensions are prepared in substantially the same manner, except that the compound can be suspended in the vehicle instead of being dissolved and sterilized by exposure to ethylene oxide before suspending in the sterile vehicle. Advantageously, a surfactant or wetting agent can be included in the composition to facilitate uniform distribution of the compound of the invention. In order to increase bioavailability, the compounds can be pharmaceutically formulated in liposomes or in nanoparticles. Acceptable liposomes can be neutral, negatively, or positively charged, the charge being a function of the charge of the liposome components and pH of the liposome solution. Liposomes can be normally prepared using a mixture of Phospholipids and cholesterol. Suitable phospholipids include phosphatidylcholine, phosphatidylethanolamine, phosphatidic acid, phosphotidylglycerol, phosphatidylinositol. Polyethylene glycol can be added to improve the blood circulation time of liposomes. Acceptable nanoparticles include albumin nanoparticles and gold nanoparticles.

For buccal or sublingual administration, the compositions may be tablets, lozenges, pastilles, or gel. The compounds can be pharmaceutically formulated as suppositories or retention enemas, e.g. containing conventional suppositories bases such as cocoa butter, polyethylene glycol, or other glycerides, for a rectal administration.

Another means of administering the compounds of the invention regards topical treatment. Topical formulations can contain for example ointments, creams, lotions, gels, solutions, pastes and/or can contain liposomes, micelles and/or microspheres. Examples of ointments include oleaginous ointments such as vegetable oils, animal fats, semisolid hydrocarbons, emulsifiable ointments such as hydroxystearin sulfate, anhydrous lanolin, hydrophilic petrolatum, cetyl alcohol, glycerol monostearate, stearic acid, water soluble ointments containing polyethylene glycols of various molecular weights. Creams, as known to formulation experts, are viscous liquids or semisolid emulsions, and contain an oil phase, an emulsifier and an aqueous phase. The oil phase generally contains petrolatum and an alcohol such as cetyl or stearic alcohol. Formulations suitable for topical administration to the eye also include eye drops, wherein the active ingredient is dissolved or suspended in a suitable carrier, especially an aqueous solvent for the active ingredient.

A further method of administering the compounds of the invention regards transdermal delivery. Topical transdermal formulations comprise conventional aqueous and non-aqueous vectors, such as creams, oils, lotions or pastes or can be in the form of membranes or medicated patches.

Formulations are known in the art (Remington "The Science and Practice of Pharmacy", Lippincott Williams & Wilkins, 2000).

The compounds of the present invention may be employed for use in the treatment and/or prevention of the above-mentioned conditions alone as a sole therapy or in combination with other therapeutic agents either by separate administrations, or by including the two or more active principles in the same pharmaceutical formulation. The compounds may be administered simultaneously or sequentially. The combination can be administered as separate compositions (simultaneous, sequential) of the individual components of the treatment or as a single dosage form containing all agents. When the compounds of this invention are in combination with other active ingredients, the active ingredients may be separately formulated into single-ingredient preparations of one of the above-described forms and then provided as combined preparations, which are given at the same time or different times or may be formulated together into a two- or more ingredients preparation.

Compounds of the invention may be administered to a patient in a total daily dose of, for example, from 0.001 to 1000 mg/kg body weight daily. Dosage unit compositions may contain such amounts of submultiples thereof to make up the daily dose. The compound may also be administered weekly or any other day. The determination of optimum dosages for a particular patient is well known to one skilled in the art. As is common practice, the compositions are normally accompanied by written or printed instructions for use in the treatment in question.

The present invention will now be described with reference to the following illustrative, non-limiting figures and examples.
**Figure 1****: A:** Location of the lungs and the airways in the body. **B:** Cross-section of a normal airway. **C:** Cross-section of an airway during asthma symptoms.
**Figure 2****:** Immunopathogenesis of asthma.
**Figure 3****:** Schematic representation of the mechanism of chitinases mediated airway hyper-responsiveness and airway inflammation⁷.

### DETAILED DESCRIPTION OF THE INVENTION

### CHEMISTRY

### General

According to a further aspect of the invention there is provided a process for the preparation of compounds of the invention or salts thereof. The following schemes are examples of synthetic schemes that may be used to synthesise the compounds of the invention. In the following schemes reactive groups can be protected with protecting groups and deprotected according to well established techniques. In the following schemes unless otherwise indicated all substituents are as defined herein above in formula (I), (II) or (III). In the general descriptions of the schemes, reference is sometimes made to a compound labelled with a number which is not itself depicted in the schemes. Such reference refers to the compound(s) labelled with the same number and a letter in the schemes, e.g. a scheme refers to compounds **4a** and **4b** and its general description uses the number **4** to identify both compounds.

It will be understood by those skilled in the art that certain compounds of the invention can be converted into other compounds of the invention according to standard chemical methods.

For the synthesis of aliphatic compounds, the appropriate amino acid was protected with CbzCl and used in the coupling with the primary amine **2,** obtained previously by guanylation of 1,8-diaminooctane, with 1,3-Di-Boc-2-(trifluoromethylsulfonyl)guanidine, both commercially available, to afford the corresponding amide **3** which was then reduced to amine **4** with DIBAL-H. The presence of an unsubstituted di-Boc guanidine and a free amine group led to the formation of an intramolecular amidinourea through a very peculiar reaction.^{9,10} The proposed reaction mechanism involves the formation of an isocyanate intermediate from the Boc protecting group via a E1cB reaction. The lost *tert-*butoxide contributes to the deprotonation of the amine, which is free to do a nucleophilic attack on the isocyanate getting the macrocycle **5.** The next removal of the CbzCl with hydrogen atmosphere occurs using an H₂ atmosphere on Pd/C 10% with the addition of catalytic amount of hydrogen chloride 37% to ease the cleavage of the carbamate group and it was followed with a second guanylation in abundant DIPEA to de-salify the amine **6.** The guanylated compound **7** obtained undergoes the nucleophilic attack from methylamine with the same reaction mechanism for closing the cycle to furnish compound **8**. The desired compounds **BM49** and **BM53** were obtained after a final deprotection of **8** with trifluoroacetic acid at 10% in dry dichloromethane to remove the two Boc protecting group.

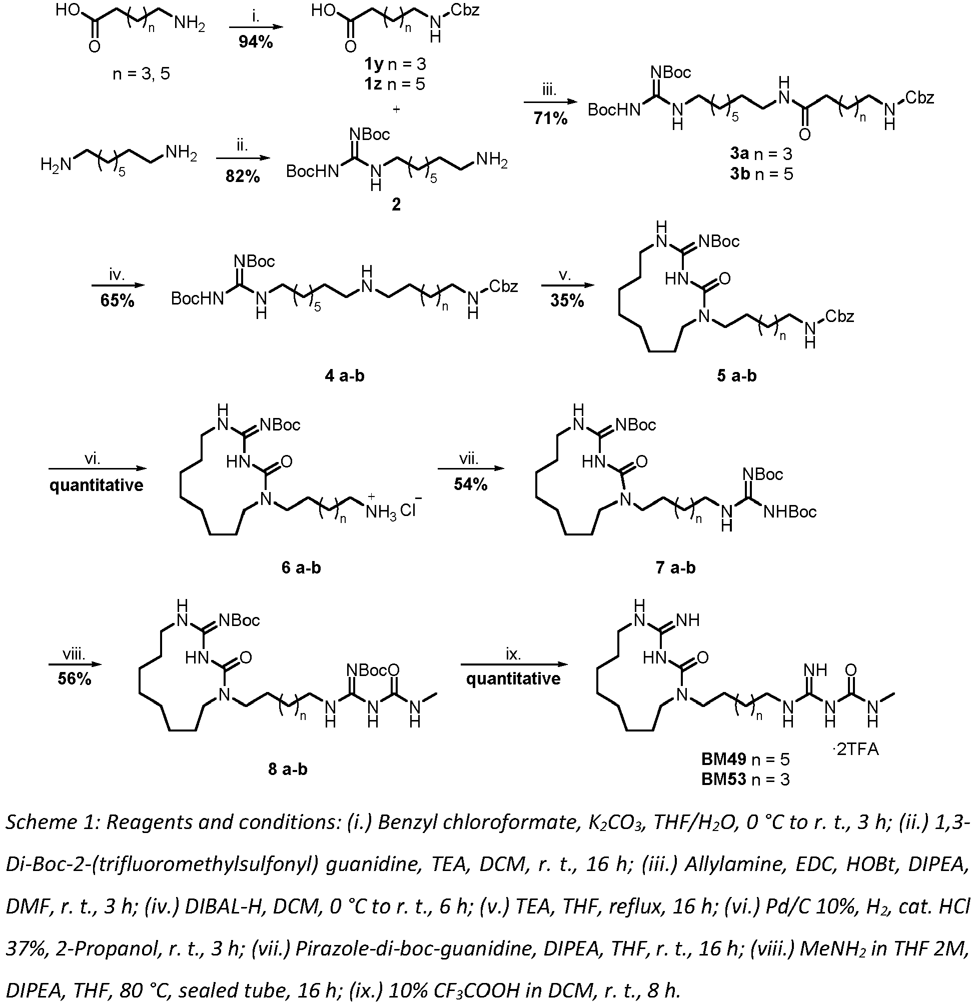

The linker moiety **10** (Scheme 2) is the common part of the other compounds reported in Scheme 3 and Scheme 5. It was prepared starting from an appropriate commercial amino acid, whose amino group is firstly protected with benzyl chloroformate. The free carboxylic group of **1** is then coupled with allylamine using EDC and HOBt in dry DMF, to obtain the corresponding amide **9.** Both passages show good yields. The amide **9,** lastly, is reduced to the secondary amine **10** with DIBAL-H.

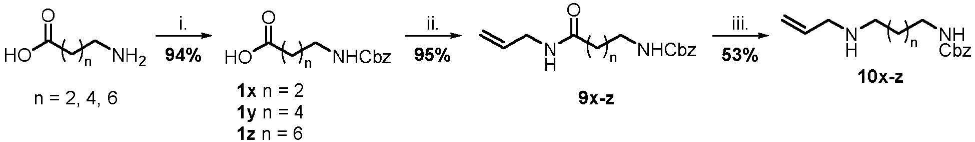

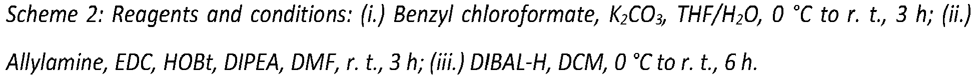

The synthesis of the monophenyl compounds starts from salicylaldehyde and bromo-alkene derivatives, both commercially available. The first step is the O-alkylation of the phenolic hydroxyl group, with potassium carbonate in presence of catalytic amount of sodium iodide in acetonitrile at reflux. The aldehyde group of **11** is then firstly converted in the oxime **12** with the addition-elimination of hydroxylamine hydrochloride in ethanol at reflux, in presence of pyridine, and subsequently reduced to primary amine **13** thanks to the Clemmensen reduction of the oxime, using metallic zinc and aqueous hydrogen chloride as source of hydrogen atoms, in THF at reflux. The amine is finally guanylated thanks to di-boc-pyrazole-carboxamidine in THF in presence of DIPEA to furnish intermediate **14.** Intermediate **14** and the linker moiety **10** are then joined through the amidinourea-forming reaction by simply putting them in THF at reflux in presence of a base with a mechanism similar to what described previously for closing the cycle. **15** undergoes the ring-closing metathesis,^{11,12} which is one of the most delicate synthetic passage of the route. The reaction is very diluted, in order to avoid the possibility of intermolecular reactions (2 mM of starting material) and it is accomplished with 2^{nd} generation Grubb's catalyst. This is a ruthenium-based catalyst which is more stable against air and moisture in respect of 1^{st} generation one, but it benefits from anyway a freshly degassed solvent to work properly: this can be appreciated both visually (the reaction mixture tends to turn dark brown from light pink) and from the lower yields (from a maximum of ^{∼}45% to 25% or less) if not correctly degassed. From the metathesis both the Z and the E isomer could be obtained, but the former is much more favored over the latter, to the point that in quite flexible systems like **16** it can be spotted both in TLC and in NMR, but in more rigid system such as the biphenyl derivatives it is almost absent. In any case, both isomers give the same product after the hydrogenation, so they are collected together. During this step, the Cbz protecting group is removed as well and the desired intermediate was guanylated to furnish compound 17 which underwent the steps already described to obtain **BM22, BM35, BM36, BM39, BM40, BM50** and **BM52.** The intermediate **17** can be Boc-deprotected with trifluoroacetic acid and subsequently reacted with methyl isothiocyanate. The final formation of the trifluoroacetate salt leads to the obtainment of **BM34.**

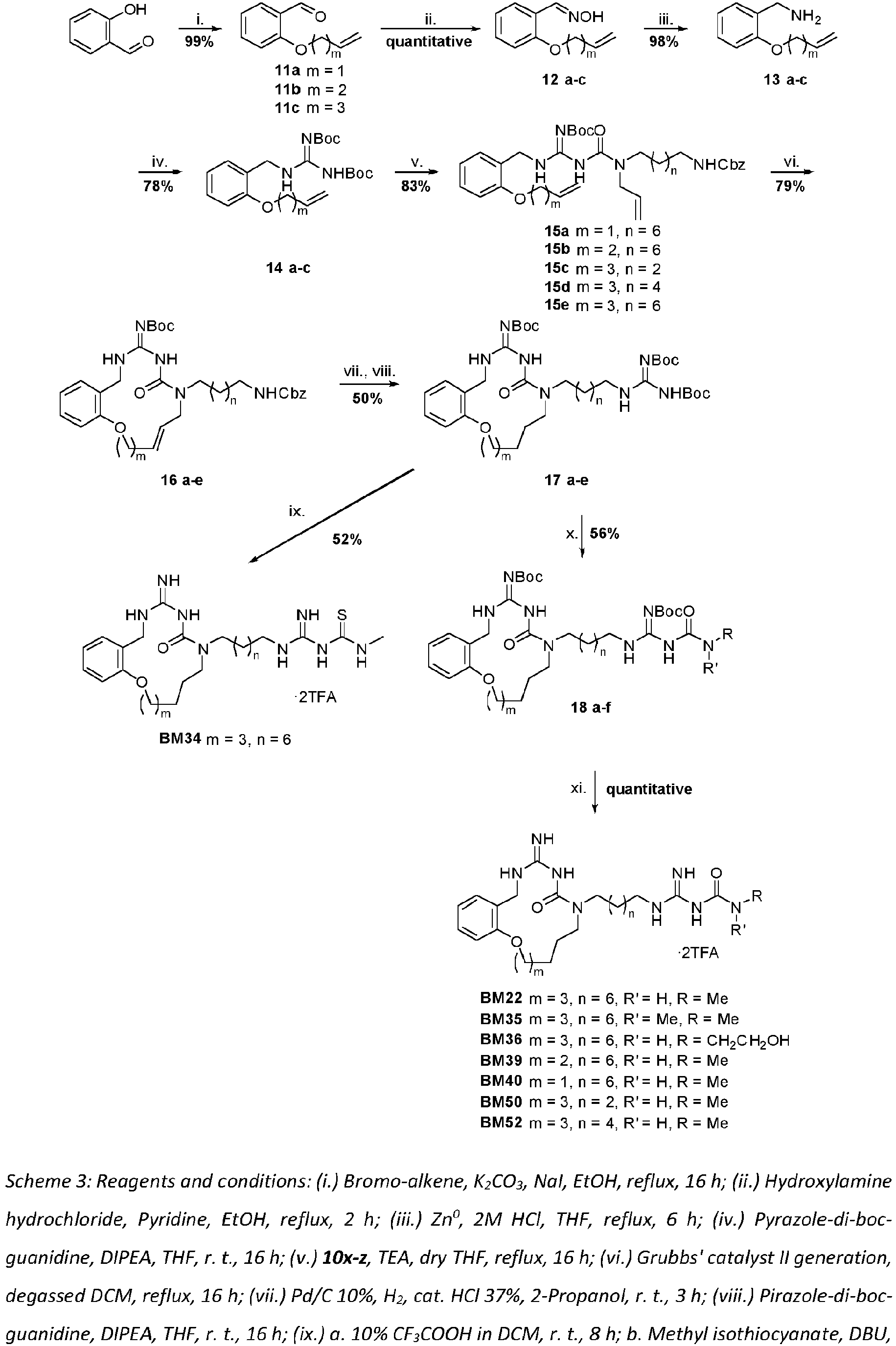

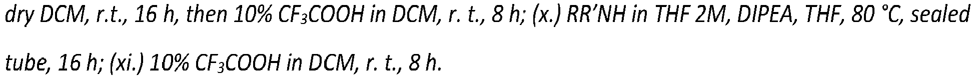

The synthesis of biphenyl compounds begins with the reduction of the carboxylic group to primary alcohol **19** with the borane-dimethylsulfide complex, in THF, in quantitative yield. Since the iodine atom is still present on the phenyl ring of **19,** a reaction in very mild conditions¹² was used to subsequently convert the alcohol in a good leaving group with Tosyl Chloride with dimethylaminopyridine and trimethylamine in DCM, which affords the corresponding chloride **20** instead of the expected tosyl group. This is due to the relatively high reactiveness of the benzylic position, which is affected by the presence of an electron-withdrawing group in ortho position on the phenyl ring, namely the iodine atom: the tosyl is readily substituted by the chloride ion released by Tosyl Chloride itself. **20** is then used for the O-alkylation of the appropriate 2-hydroxy-benzaldehyde, and the resulting intermediate **21** undergoes the Stille reaction with palladium acetate and triphenylphosphine for the introduction of the vinyl group. This last step furnishes the desired intermediate **22** with a much higher efficiency (Scheme 4).

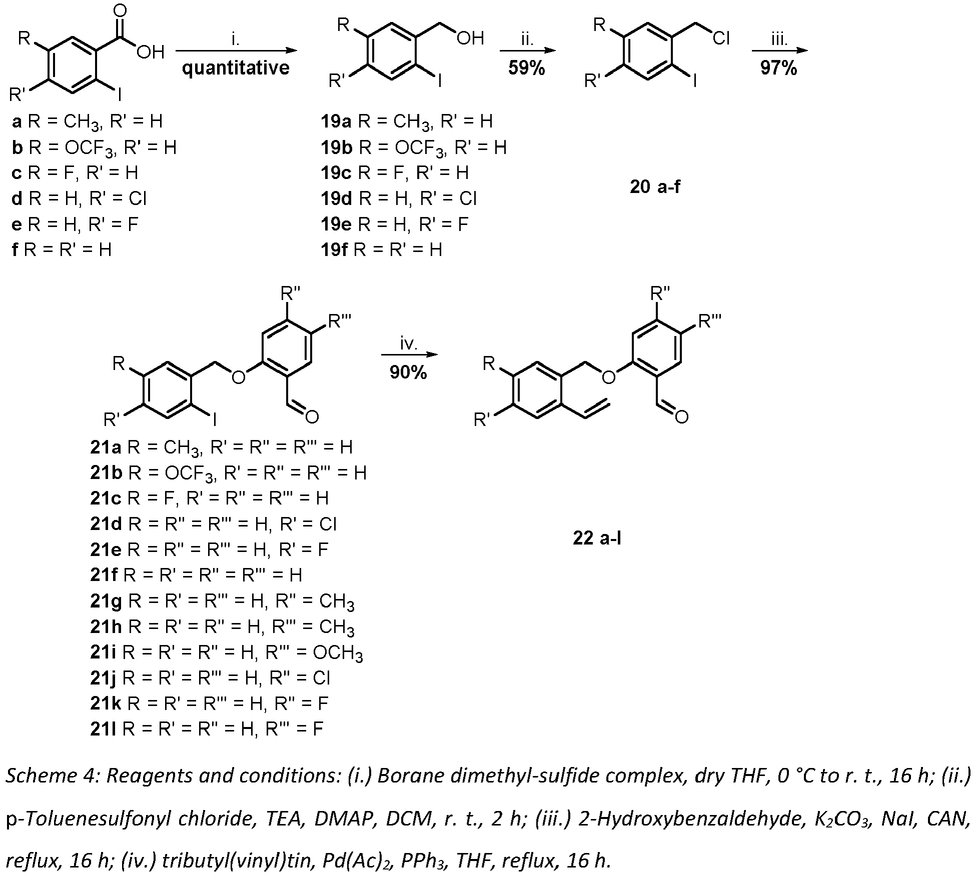

The desired biphenyl derivatives were obtained through the steps shown in Scheme 5 as already described above for Scheme 3.

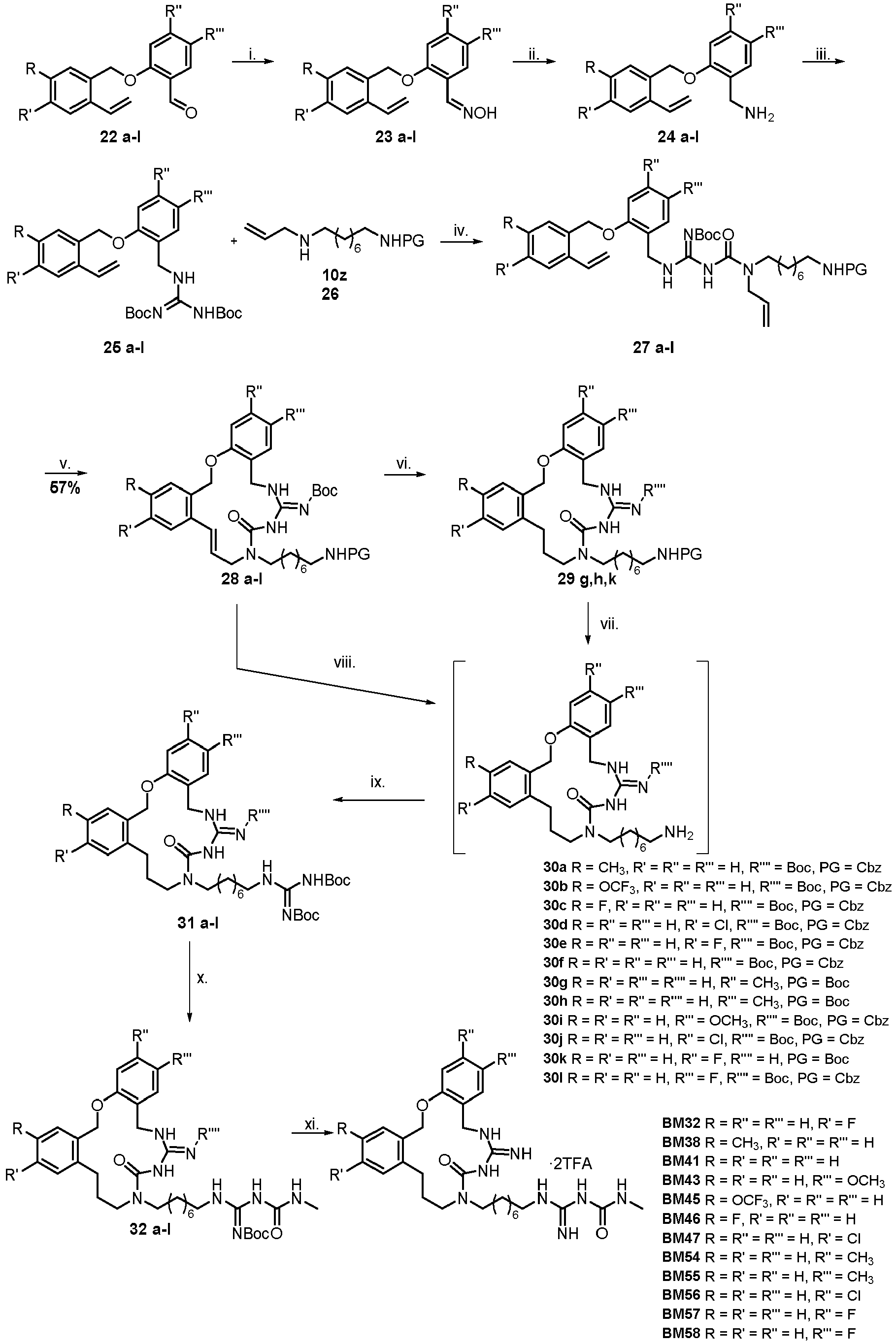

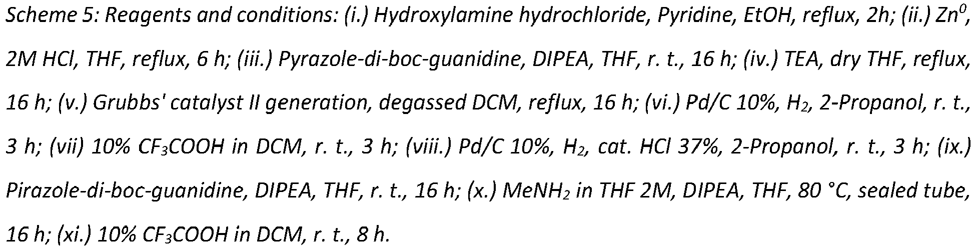

In the synthetic route of these di-phenyl compounds, two main challenges were observed: the first one was the ring-closing metathesis reaction that, as said before, gives low yields likely due to presence of a more rigid system, compared to that of the mono-phenyl derivatives that, indeed, have a more flexible sp³ carbon atom chain. The second one was the hydrogenation step with a catalytic amount of HCI 37% for Cbz cleavage, in which it was observed the formation of the product of ring cleavage at the benzyl position next to oxygen, sensitive to this reaction condition.

In order to solve the latter, the protecting group inserted in the first step of the linker moiety synthesis was changed with the Boc group (Scheme 6). In this way the hydrogenation could be carried out without the use of the acid, reducing only the double bond to obtain compound **29.** Then the protecting group was removed with TFA 10% in CH₂Cl₂ to obtain compound **30.**

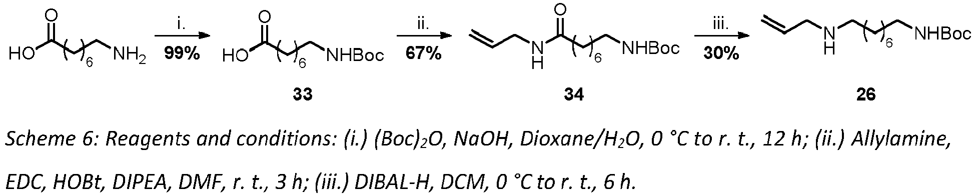

### Synthetic procedures

The following procedures illustrate the preparation of certain compounds of the invention or salts thereof.

All reagents were used as purchased from commercial suppliers without further purification. CH₂Cl₂ was dried over calcium hydride. THF were dried over Na/benzophenone prior to use. Anhydrous reactions were run under a positive pressure of dry N₂ or Ar.

### General procedure for the protection of amino acid.

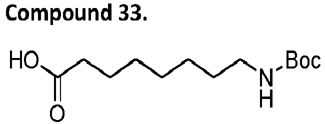

In a flask with magnetic stirrer, 8-aminooctanoic acid (Sigma-Aldrich S.r.l., 855294) (1000 mg, 6.29 mmol) was dissolved in dioxane/H₂O 2:1 (24 mL) and NaOH (252 mg, 6.29 mmol) was added. Then di-*tert-*butyldicarbonate (Sigma-Aldrich S.r.l., 361941) (1510 mg, 6.92 mmol) was added to the mixture on ice/NaCl bath. The mixture was stirred for 16 h at room temperature. The reaction was concentrated *in vacuo.* The residue was acidified with HCI IN and extracted with AcOEt. Organic layers were dried over anhydrous Na₂SO₄, filtered and evaporated *in vacuo.* The residue was purified by flash chromatography (Hex/AcOEt 5:5). **Yield:** 92%. **¹H-NMR** (400 MHz, CDCl₃): δ 4.57(bs, 1H), 3.10-2.98 (m, 2H), 2.29 (t, *J* = 7.2 Hz, 2H), 1.62-1.55 (m, 4H), 1.40 (s, 9H), 1.45-1.32 (m, 6H) ppm. **¹³C-NMR** (100 MHz, CDCl₃): δ 179.0, 156.6, 79.0, 40.5, 33.9, 29.8, 28.8, 28.3, 27.73, 26.4, 24.5 ppm.

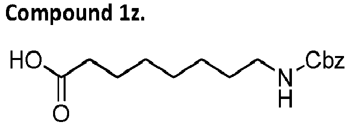

In a flask equipped with a magnetic stirrer, 8-aminooctanoic acid (Sigma-Aldrich S.r.l., 855294) (2000 mg, 14.58 mmol) was dissolved in THF/H₂O 1:1 (56 mL) and NaHCO₃ (1162 mg, 13.83 mmol) was added. Then benzyl-chloroformate (2.7 mL, 18.87 mmol) was slowly added to the mixture on ice/NaCl bath. The mixture was stirred for 3 h at r.t. and then quenched with AcOEt and H₂O. The aqueous phase was separated and the pH adjusted to 2 by addition of HCI IN and extracted three times with AcOEt. The collected organic layers were washed with Brine, dried over anhydrous Na₂SO₄, filtered and the solvent evaporated *in vacuo.* The residue was purified by flash chromatography (Hex/AcOEt 6:4, Hex/AcOEt 3:7) to give compound **1z. Yield:** 94%. **¹H-NMR** (400 MHz, CDCl₃): δ 7.38-7.28 (m, 5H), 5.07 (s, 2H), 4.74 (bs, 1H), 3.17-3.15 (m, 2H), 2.31 (t, *J* = 7.2 Hz, 2H), 1.60-1.58 (m, 2H), 1.50-1.45 (m, 2H), 1.33-1.25 (m, 6H) ppm. **¹³C-NMR** (100 MHz, CDCl₃): δ 178.7, 156.4, 136.5, 128.4, 128.0, 128.0, 66.5, 41.4, 33.8, 29.7, 28.6, 26.4, 24.7 ppm. **MS (ES)** *m*/*z* = 609.0 [2M+Na]⁺, 316.0 [M+Na]⁺, 294.1 [M+H]⁺.

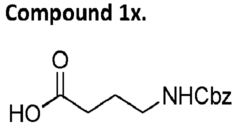

**Compound 1x** was prepared by the procedure described for **compound 1z** starting from 4-aminobutanoic acid (Sigma-Aldrich S.r.l., A2129). **Yield:** 84%. **¹H-NMR** (400 MHz, CDCl₃): δ 9.98 (bs, 1H), 7.34-7.14 (m, 5H), 5.06 (s, 2H), 3.20 (t, *J* = 4 Hz, 2H), 2.36-2.32 (m, 2H), 1.79 (t, *J* = 8 Hz, 2H) ppm. **¹³C-NMR** (100 MHz, CDCl₃): δ 178.2, 156.7, 136.4, 128.4, 128.0, 66.7, 40.2, 31.1, 24.8 ppm.

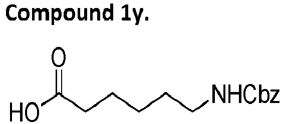

**Compound 1y** was prepared by the procedure described for **compound 1z** starting from 6-aminohexanoic acid (Sigma-Aldrich S.r.l., A2504). **Yield:** 89%. **¹H-NMR** (400 MHz, CDCl₃): δ 7.33-7.28 (m, 5H), 5.07 (s, 2H), 3.21-3.10 (m, 2H), 2.31 (t, *J* = 7.2 Hz, 2H), 1.68-1.56 (m, 2H), 1.55-1.42 (m, 2H), 1.39-1.26 (m, 2H) ppm. **¹³C-NMR** (100 MHz, CDCl₃): δ 178.9, 156.5, 128.4, 128.0, 67.0, 66.6, 60.4, 40.8, 33.4, 29.5, 26.0, 24.2, 20.9, 14.1 ppm.

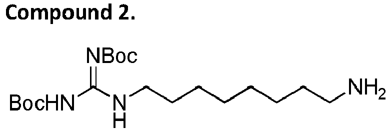

The 1,8-diaminooctane (Sigma-Aldrich S.r.l., D22401) (1490 mg, 10.34 mmol) was dissolved in CH₂Cl₂ with TEA (Sigma-Aldrich S.r.l., T0886) (528 µL, 3.80 mmol) and a solution of 1,3-di-boc-2-(trifluoromethylsulfonyl) guanidine (Sigma-Aldrich S.r.l., 15033) (1000 mg, 3.45 mmol) in CH₂Cl₂ was added dropwise. The mixture was stirred for 16 h at r.t. The solvent was evaporated *in vacuo,* and the residue was purified by flash chromatography (AcOEt/MeOH/TEA 8:2:2). **Yield** 82%. **¹H-NMR** (400 MHz, CDCl₃): δ 8.24 (bs, 1H), 3.34-3.27 (m, 2H), 2.70 (t, *J* = 7 Hz, 2H), 1.48 (bs, 2H), 1.42 (s, 9H), 1.41 (s, 9H), 1.26-1.20 (m, 12H) ppm. **¹³C-NMR** (100 MHz, CDCl₃): δ 163.7, 156.3, 153.4, 83.2, 79.4, 41.0, 40.8, 30.4, 30.1, 29.2, 28.6, 28.3, 28.0, 27.8, 27.5, 27.3, 26.9, 26.7, 26.6 ppm. **MS (ES)** *m*/*z* = 409.1 [M+Na]⁺, 387.1 [M+H]⁺, 287.1 [(M-1Boc)+H]⁺.

### General procedure for the coupling.

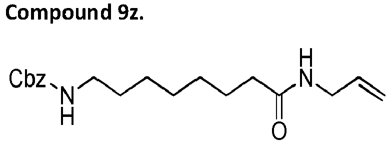

In a flask equipped with a magnetic stirrer, **1z** (3479 mg, 11.84 mmol) was dissolved in dry DMF (20 ml) at 0°C under N₂ atmosphere. Then HOBt (Sigma-Aldrich S.r.l., 54802) (1598 mg, 11.84 mmol), EDC (Sigma-Aldrich S.r.l., 39391) (2290 mg, 17.76 mmol), DIPEA (Sigma-Aldrich S.r.l., 387649) (2.47 mL, 14.21 mmol) and allylamine (Sigma-Aldrich S.r.l., 241075) (1 mL, 14.21 mmol) were subsequently added. The mixture was stirred for 15' at 0°C and then warmed at r. t. After 6 h, EDC (1527 mg, 11.84 mmol) was added and the mixture stirred at r. t. for 16 h. The reaction was quenched with NaHCO₃₍ₛₛ₎ and the aqueous phase was extracted with AcOEt. The organic layer was washed with LiCI 5% in H₂O and brine, dried over anhydrous Na₂SO₄, filtered and the solvent was evaporated *in vacuo* to afford compound **9z. Yield:** 74%. **¹H-NMR** (400 MHz, CDCl₃): δ 7.34-7.28 (m, 5H), 5.86-5.76 (m, 1H), 5.50 (bs, 1H), 5.17-5.09 (m, 2H), 5.07 (s, 2H), 4.72 (bs, 1H), 3.86 (t, *J* = 5.2 Hz, 2H), 3.16 (q, *J* = 6.4 Hz, 2H), 2.15 (t, *J* = 7.6 Hz, 2H), 1.66-1.57 (m, 2H), 1.50-1.42 (m, 2H), 1.33-1.25 (m, 6H) ppm. **¹³C-NMR** (100 MHz, CDCl₃): δ 172.8, 156.3, 136.5, 134.2, 128.4, 127.9, 127.6, 116.9, 66.4, 41.7, 40.8, 36.4, 29.7, 28.9, 28.7, 26.3, 25.4 ppm. **MS (ES)** *m*/*z* = 687.0 [2M+Na]⁺, 355.0 [M+Na]⁺, 333.1 [M+H]⁺.

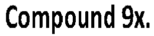

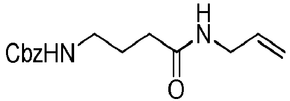

**Compound 9x** was prepared by the procedure described for **compound 9z** starting from compound **1x. Yield:** 95%. **¹H-NMR** (400 MHz, CDCl₃): δ 7.30-7.25 (m, 5H), 6.01 (bs, 1H), 5.82-5.73 (m, 1H), 5.15-5.06 (m, 2H), 5.04 (s, 2H), 3.81 (t, *J* = 5.6 Hz, 2H), 3.24-3.15 (m, 2H), 2.19 (t, *J* = 7.2 Hz, 2H), 1.83-1.76 (m, 2H) ppm. **¹³C-NMR** (100 MHz, CDCl₃): δ 172.6, 156.9, 136.5, 134.2, 128.4, 128.0, 127.9, 116.0, 66.5, 41.8, 40.3, 33.4, 26.0 ppm.

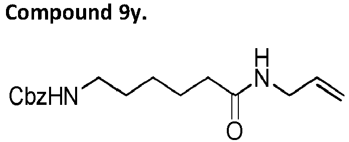

**Compound 9y** was prepared by the procedure described for **compound 9z** starting from starting from compound **1y.**

**Yield:** 75%. **¹H-NMR** (400 MHz, CDCl₃): δ 7.31-7.25 (m, 5), 5.84-5.74 (m, 1H), 5.11-5.05 (m, 4H), 4.96 (bs, 1H), 3.82 (t, *J* = 5.6 Hz, 2H), 3.14 (d, *J* = 6 Hz, 2H), 2.15 (t, *J* = 7.2 Hz, 2H), 1.65-1.58 (m, 2H), 1.50-1.44 (m, 2H), 1.31-1.27 (m, 2H) ppm. **¹³C-NMR** (100 MHz, CDCl₃): δ 172.7, 156.4, 136., 134.3, 128.4, 128.0, 66.5, 41.8, 40.7, 36.3, 29.6, 26.2, 25.1 ppm.

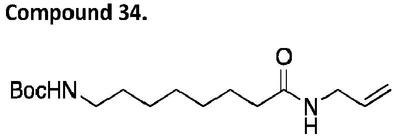

**Compound 34** was prepared by the procedure described for **compound 9z** starting from compound **33. Yield:** 89%. **¹H-NMR** (400 MHz, CDCl₃): δ 6.03 (bs, 1H), 5.80-5.71 (m, 1H), 5.12-5.03 (m, 2H), 4.60 (bs, 1H), 3.82-3.75 (m, 2H), 3.07-2.99 (m, 2H), 2.13 (t, *J* = 7.2 Hz, 2H), 1.61-1.50 (m, 4H), 1.37 (s, 9H), 1.30-1.19 (m, 6H) ppm. **¹³C-NMR** (100 MHz, CDCl₃): δ 173.0, 156.0, 134.3, 116.0, 78.9, 41.7, 40.4, 36.4, 29.9, 28.3, 26.4, 25.5 ppm.

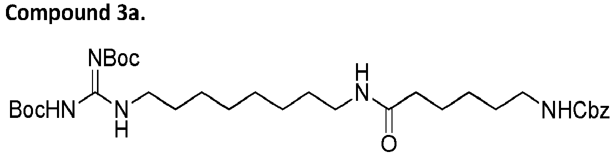

**Compound 3a** was prepared by the procedure described for **compound 9z** starting from compounds **2** and **1y.**

**Yield:** 71%. **¹H-NMR** (400 MHz, CDCl₃): δ 11.42 (bs, 1H), 8.20 (bs, 1H), 7.27-7.24 (m, 5H), 5.49 (bs, 1H), 5.00 (s, 2H), 4.83 (bs, 1H), 3.31-3.28 (m, 2H), 3.14-3.11 (m, 4H), 2.10-2.05 (m, 2H), 1.59-1.50 (m, 2H), 1.49-1.42 (m, 6H), 1.33 (s, 18H), 1.16-1.10 (m, 10H) ppm. **¹³C-NMR** (100 MHz, CDCl₃): 172.7, 163.52, 156.41, 156.0, 153.2, 136.5, 128.3, 127.9, 82.9, 79.1, 66.4, 40.8, 40.7, 39.3, 36.36, 29.5, 29.4, 28.2, 27.9, 26.7, 26.6, 26.2, 25.1 ppm. **MS (ES)** *m*/*z* = 656 [M+Na]⁺, 634 [M+H]⁺.

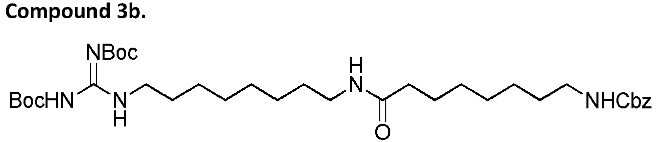

**Compound 3b** was prepared by the procedure described for **compound 9z** starting from compounds **2** and **1z.**

**Yield:** 71%. **¹H-NMR** (400 MHz, CDCl₃): δ 11.31 (bs, 1H), 8.09 (bs, 1H), 7.14-7.00 (m, 5H), 6.36 (bs, 1H), 5.37 (bs, 1H), 4.84 (s, 2H), 3.20-3.09 (m, 2H), 2.97-2.96 (m, 2H), 2.91-2.88 (m, 2H), 1.92-1.90 (m, 2H), 1.44-1.34 (m, 4H), 1.27 (s, 9H), 1.26 (s, 9H), 1.13-1.02 (m, 18H) ppm. **¹³C-NMR** (100 MHz, CDCl₃): δ 173.1, 163.3, 156.4, 155.9, 153.0, 136.6, 128.2, 128.0, 127.6, 82.8, 78.9, 66.0, 40.8, 40.7, 39.2, 36.2, 29.6, 29.3, 28.9, 28.7, 28.1, 28.0, 27.8, 27.5, 26.6, 26.5, 26.3, 25.4 ppm. **MS (ES)** *m*/*z* = 684.4 [M+Na]⁺, 662.4 [M+H]⁺.

### General procedure for the amide reduction.

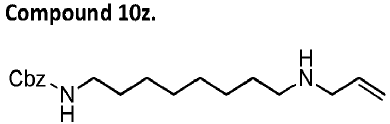

To a stirred solution of linear amide **9z** (1.03 g, 1.56 mmol) in dry DCM (29 mL) cooled at 0 °C a 1M solution of Diisobutylaluminium hydride (DIBAL-H) in DCM (3.12 mL, 3.12 mmol) (Sigma-Aldrich S.r.l., 214973) was added and the mixture was stirred at the same temperature for 1.5 h. Then it was allowed to warm to r.t. and stirred for 2 h. Additional DIBAL-H (4.68 ml, 4.68 mmol) was added and the mixture was stirred for further 1.5 h. The reaction was diluted with AcOEt (10 mL) and Rochelle salt saturated solution (20 mL) was added. Organic phase was separated and aqueous phase was extracted twice with AcOEt (10 mL). The combined organic phases were dried over Na₂SO₄ and evaporated under reduced pressure. The crude residue was purified with flash chromatography on silica gel, eluting with a 5/4/0.5/0.5 mixture of Hex/AcOEt/MeOH/TEA. **Yield:** 48%. **¹H-NMR** (400 MHz, CDCl₃): δ 7.33-7.28 (m, 5H), 5.92-5.83 (m, 1H), 5.16-5.11 (m, 2H), 5.07 (s, 2H), 3.22 (d, *J* = 4 Hz, 2H), 3.18-3.13 (m, 2H), 2.56 (t, *J* = 8 Hz, 2H), 1.48-1.41 (m, 4H), 1.27-1.23 (m, 8H) ppm. **¹³C-NMR** (100 MHz, CDCl₃): δ 156.3, 141.2, 134.2, 129.0, 128.1, 127.9, 116.0, 65.0, 52.1, 49.8, 41.9, 31.0, 29.9, 29.4, 27.0, 26.8 ppm. **MS (ES)** *m*/*z* = 659.0 [2M+Na]⁺, 341.0 [M+Na]⁺, 319.0 [M+H]⁺.

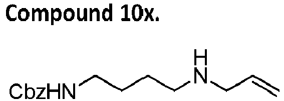

**Compound 10x** was prepared by the procedure described for **compound 10z** starting from compound **9x.**

**Yield:** 18%. **¹H-NMR** (400 MHz, CDCl₃): δ 7.27-7.22 (m, 5H), 5.86-5.76 (m, 1H), 5.40 (bs, 1H), 5.10-4.99 (m, 4H), 3.19-3.11 (m, 4H), 2.55-2.52 (m, 2H), 1.51-1.38 (m, 4H) ppm. **¹³C-NMR** (100 MHz, CDCl₃): δ 156.5, 136.5, 128.3, 127.9, 116.0, 66.3, 52.2, 48.6, 40.8, 27.7, 27.1 ppm.

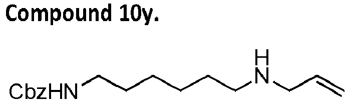

**Compound 10y** was prepared by the procedure described for **compound 10z** starting from compound **9y.**

**Yield:** 53%. **¹H-NMR** (400 MHz, CDCl₃): δ 7.31-7.25 (m, 5H), 5.91-5.81 (m, 1H), 5.15-4.98 (m, 4H), 4.85 (bs, 1H), 3.21 (d, *J* = 5.6 Hz, 2H), 3.13 (q, *J* = 6.4 Hz, 2H), 2.56 (t, *J* = 7.2 Hz, 2H), 2.14 (bs, 1H), 1.55-1.41 (m, 4H), 1.39-1.24 (m, 4H) ppm. **¹³C-NMR** (100 MHz, CDCl₃): δ 156.4, 136.6, 136.3, 128.4, 128.0, 116.2, 66.4, 60.3, 52.2, 49.0, 40.9, 29.8, 29.6, 26.8, 26.5, 20.9, 14.1 ppm.

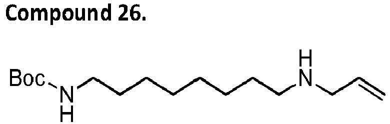

**Compound 26** was prepared by the procedure described for **compound 10z** starting from compound **34. Yield:** 35%. **¹H-NMR** (400 MHz, CDCl₃): δ 5.89-5.80 (m, 1H), 5.12-5.00 (m, 2H), 4.60 (bs, 1H), 3.18 (d, *J* = 5.6 Hz, 2H), 3.05-3.00 (m, 2H), 2.53 (t, *J* = 6.8 Hz, 2H), 1.52-1.47 (m, 4H), 1.37 (s, 9H), 1.30-1.19 (m, 8H) ppm. **¹³C-NMR** (100 MHz, CDCl₃): δ 155.9, 136.8, 115.6, 78.8, 52.4,49.3, 40.5, 29.9, 29.3, 29.1, 28.7, 28.3, 27.1, 26.6 ppm.

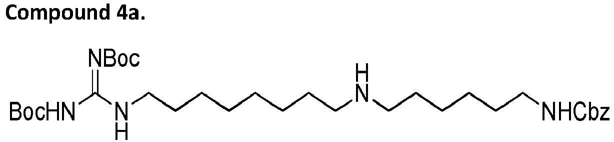

**Compound 4a** was prepared by the procedure described for compound **10z** starting from compound **3a. Yield:** 60%. **¹H-NMR** (400 MHz, CDCl₃): δ 11.46 (bs, 1H); 8.25 (s, 1H), 7.32-7.26 (m, 5H), 5.05 (s, 2H), 4.81 (bs, 1H), 3.36 (q, *J* = 6.8 Hz, 2H), 3.14 (q, *J* = 6.8 Hz, 2H), 2.55 (t, *J* = 6.8 Hz, 4H), 1.46 (s, 9H), 1.43 (s, 9H), 1.32-1.19 (m, 20H) ppm. **¹³C-NMR** (100 MHz, CDCl₃): δ 163.6, 156.0, 153.2, 136.6, 128.4, 128.0, 82.9, 79.1, 66.4, 49.9, 49.7, 40.9, 29.8, 29.3, 29.1, 28.9, 28.4, 28.2, 28.0, 27.2, 26.9, 26.7, 26.5 ppm. **MS (ES)** *m*/*z* = 620.0 [M+H]⁺.

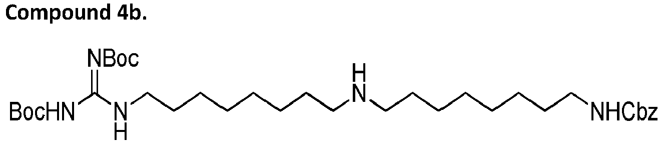

**Compound 4b** was prepared by the procedure described for **compound 10z** starting from compound **3b. Yield:** 65%. **¹H-NMR** (400 MHz, CDCl₃): δ 11.47 (bs, 1H), 8.26 (s, 1H), 7.33-7.29 (m, 5H), 5.07 (s, 2H), 3.37 (q, *J* = 6.4 Hz, 2H), 3.15 (q, *J* = 6.4 Hz, 2H), 2.59 (t, *J* = 7.2 Hz, 4H), 1.61-1.49 (m, 8H), 1.48 (s, 9H), 1.47 (s, 9H), 1.34-1.21 (m, 16H) ppm. **¹³C-NMR** (100 MHz, CDCl₃): δ 156.8, 154.5, 154.0, 153.5, 136.7, 128.5, 128.3, 128.1, 81.5, 80.3, 66.4, 47.8, 41.8, 40.4, 29.6, 28.9, 28.8, 28.6, 28.3, 28.2, 27.5, 27.5, 26.9, 26.8, 25.0, 24.9 ppm.

### General procedure for the macrocycle.

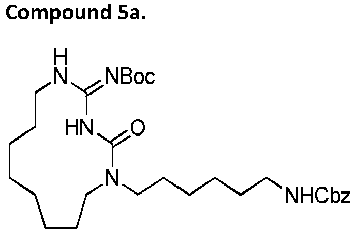

In a flask with magnetic stirrer, the linear amine **4a** (43 mg, 0.05 mmol) was dissolved in THF (26 mL) and TEA (7.4 µL, 0.05 mmol) was added. The mixture was stirred at reflux for 16 h. After cooling, the solvent was evaporated and the residue was solubilized with AcOEt and washed with H₂O and brine. Organic layer was dried over anhydrous Na₂SO₄, filtered and evaporated *in vacuo.* The residue was purified by flash chromatography (Hex/AcOEt 1:0, Hex/AcOEt 8:2). **Yield:** 24%. **¹H-NMR** (400 MHz, CDCl₃): δ 12.0 (s, 1H), 8.07 (s, 1H), 7.33-7.28 (m, 5H), 5.07 (s, 2H), 4.71(bs, 1H), 3.42 (t, *J* = 7.2 Hz, 2H), 3.34 (q, *J* = 6.8 Hz, 2H), 3.22 (t, *J* = 7.2 Hz, 2H), 3.16 (q, *J* = 6.8 Hz, 2H), 1.71-1.58 (m, 4H), 1.53-1.46 (m, 4H), 1.43 (s, 9H), 1.36-1.23 (m, 12H) ppm. **¹³C-NMR** (100 MHz, CDCl₃): δ 166.8, 163.9, 154.0, 153.5, 136.6, 128.4, 128.0, 82.0, 66.5, 47.0, 46.1, 40.9, 39.6, 29.8, 28.3, 28.0, 27.8, 26.7, 26.5, 26.1, 25.8, 25.2, 23.6; 23.5 ppm. **MS (ES)** *m*/*z* = 584.0 [M+K]⁺, 568.0 [M+Na]⁺, 546.0 [M+H]⁺.

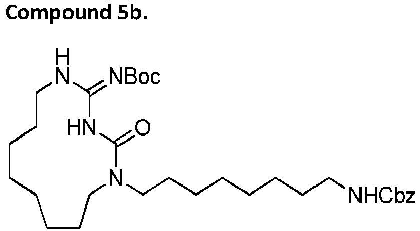

**Compound 5b** was prepared by the procedure described for **compound 5a** starting from compound **4b**. **Yield:** 35%. **¹H-NMR** (400 MHz, CDCl₃): δ 12.01 (bs, 1H), 8.02 (bs, 1H), 7.28-7.19 (m, 5H), 5.02 (s, 2H), 3.37 (t, *J* = 7.2 Hz, 2H), 3.31-3.26 (m, 2H), 3.16 (t, *J* = 7.6 Hz, 2H), 3.11-3.08 (m, 2H), 1.64-1.52 (m, 4H), 1.51-1.43 (m, 4H), 1.38 (s, 9H), 1.22-1.09 (m, 16H) ppm. **¹³C-NMR** (100 MHz, CDCl₃): δ 163.6, 156.0, 153.8, 153.2, 136.4, 127.7, 127.6, 81.6, 66.1, 46.7, 46.0, 40.7, 39.3, 29.5, 29.1, 28.9, 28.1, 27.5, 26.7, 26.3, 25.9, 25.5, 24.9, 23.4 ppm. **MS (ES)** *m*/*z* = 612.2 [M+K]⁺, 596.2 [M+Na]⁺, 574.3 [M+H]⁺.

### General procedure for the removal of the Cbz protecting group

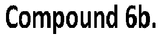

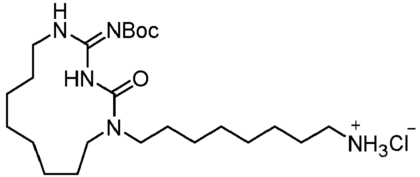

To a stirred solution of **5b** (0.06 g, 0.2 mmol) in isopropanol (15 mL), 10% Pd on carbon (Sigma-Aldrich S.r.l., 205699) (0.42 g, 0.04 mmol) and 2 drops of HCI 36% were added. The flask then was subjected to 3 cycles of vacuum followed by H₂ before being stirred under H₂ atmosphere for 4 h. The mixture was filtered through a plug of Celite (Sigma-Aldrich S.r.l., 22153), and then solvent was evaporated under reduced pressure. The crude product obtained was used directly in the next step without any further purification. **Yield** quantitative. **¹H NMR** (400 MHz CDCl₃): δ 12.01(bs, 1H), 8.08 (bs, 1H), 3.40-3.32 (m, 2H), 3.35-3.34 (m, 2H), 3.27-3.15 (m, 2H), 1.72-1.63 (m, 8H), 1.44 (s, 9H), 1.28 (s, 18H) ppm. **¹³C NMR** (100 MHz CDCl₃): δ 163.5, 153.4, 153.0, 81.2, 46.0, 45.2, 41.7, 34.3, 33.5, 29.8, 28.2, 27.6, 27.5, 26.8, 26.5, 25.8, 25.5, 24.9, 23.3 ppm. **MS (ES)** *m*/*z* = 440.4 [M + H]⁺, 462.4 [M + Na]⁺.

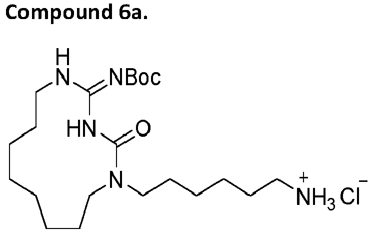

**Compound 6a** was prepared by the procedure described for **compound 6b** starting from compound 5a. **Yield** quantitative. **¹H NMR** (400 MHz CDCl₃): δ 12.09 (br s, NH), 8.07 (br s, NH), 3.44 (m, 2H), 3.35-3.34 (m, 2H), 3.23 (m, 4H), 1.63 (m, 8H), 1.44 (s, 9H), 1.28 (m, 12H) ppm. **¹³C NMR** (100 MHz CDCl₃): δ 163.9, 154.0, 153.5, 81.9, 46.9, 46.2, 41.9, 39.5, 30.22, 28.3, 28.0, 27.7, 27.3, 27.0, 26.1, 25.7, 25.2, 23.6, 23.5 ppm. **MS (ES)** *m*/*z* = 434.2 [M + Na]⁺, 412.1 [M + H]⁺.

### General procedure for the acid reduction.

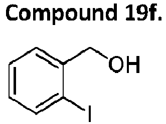

To a stirred solution of 2-iodobenzoic acid (Sigma-Aldrich S.r.l., 17675) (3 g, 12.09 mmol) in dry THF (40 mL) under N₂ atmosphere cooled to 0 °C, borane dimethyl sulfide complex (Sigma-Aldrich S.r.l., 199825) (3.44 mL, 36.29 mmol) was added dropwise. The mixture was allowed to gently warm at room temperature and stirred for 12h. Water and K₂CO₃ were carefully added, then the mixture was stirred for 30 min. The mixture was extracted with EtOAc (50 mL) and washed twice with NaOH IN (30 mL) and with Brine (30 mL). The organic phase was dried over Na₂SO₄, filtered and concentrated under reduced pressure, to give pure 2-iodobenzyl alcohol **19f** as a white solid. **Yield:** 98 %. **¹H-NMR** (400 MHz, CDCl₃): δ 7.80 (d, *J* = 8 Hz, 1H), 7.44 (d, *J* = 7.2 Hz, 1H), 7.35 (t, *J* = 7.6 Hz, 1H), 6.98 (t, *J* = 8 Hz, 1H), 4.66 (s, 2H) ppm. **¹³C-NMR** (100 MHz, CDCl₃): δ 139.0, 136.7, 128.9, 128.3, 126.5, 95.4, 64.5 ppm.

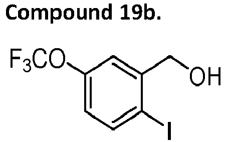

**Compound 19b** was prepared by the procedure described for **compound 19f** starting from 2-iodo-5-(trifluoromethoxy)benzoic acid (Chemspace Europe, Riga, Latvia, CSC010187405).

**Yield:** quantitative. **¹H NMR** (400 MHz, CDCl₃) δ 7.73 (d, J = 7.5 Hz, 1H), 6.91 (m, 1H), 6.79 (dd, J = 7.5, 2.0 Hz, 1H), 4.80 (s, 2H), 1.48 (bs, 1H). **¹³C NMR** (100 MHz, CDCl₃) δ 150.8, 140.9, 133.3, 120.6, 115.6, 113.7, 88.7, 60.4 ppm. **MS (ES)** *m*/*z* = 319.1 [M+H]⁺.

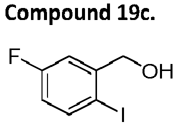

**Compound 19c** was prepared by the procedure described for **compound 19f** starting from 5-Fluoro-2-iodobenzoic acid (Sigma-Aldrich S.r.l., 678902). **Yield:** quantitative. **¹H NMR** (400 MHz, CDCl₃) δ 7.74 (dd, *J* = 8.6, 5.5 Hz, 1H), 7.25 (dd, *J* = 8.9, 3.7 Hz, 1H), 6.76 (td, *J* = 8.3, 3.0 Hz, 1H), 4.63 (s, 2H), 1.97 (s, 1H) ppm. **¹³C NMR** (100 MHz, CDCl₃) δ 140.1, 140.0, 116.4, 116.1, 115.5, 115.3, 68.7 ppm. **MS (ES)** *m*/*z* = 253.1 [M+H]⁺.

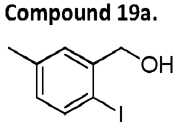

**Compound 19a** was prepared by the procedure described for **compound 19f** starting from 2-iodo-5-methylbenzoic acid (EnamineStore, Riga, Latvia, EN300-62484). **Yield:** quantitative. **¹H-NMR** (400 MHz, CDCl₃): δ 7.65 (d, *J* = 8 Hz, 1H), 7.24 (s, 1H), 6.80 (d, *J* = 8 Hz, 1H), 4.60 (s, 2H), 2.29 (s, 3H), 2.10 (bs, 1H) ppm. **¹³C-NMR** (100 MHz, CDCl₃): δ 145.9, 140.1, 134.6, 129.4, 126.3, 91.8, 59.8, 21.2 ppm.

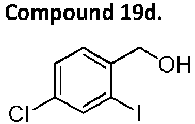

**Compound 19d** was prepared by the procedure described for **compound 19f** starting from 4-Chloro-2-iodobenzoic acid (Sigma-Aldrich S.r.l., 560146) **Yield:** 92 %. **¹H-NMR** (400 MHz, CDCl₃): δ 7.81 (d, *J* = 1.4 Hz, 1H), 7.39 (d, *J* = 8.2 Hz, 1H), 7.35 (dd, *J* = 8.3, 1.5 Hz, 1H), 4.64 (d, *J* = 6.1 Hz, 2H), 1.55 (s, 1H) ppm. **¹³C-NMR** (100 MHz, CDCl₃): δ 140.7, 139.4, 134.4, 130.1, 127.9, 98.3, 68.7 ppm. **MS (ES)** *m*/*z* = 269.4 [M+H]⁺.

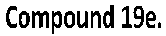

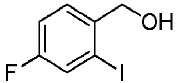

**Compound 19e** was prepared by the procedure described for **compound 19f** starting from 4-Fluoro-2-iodobenzoic acid (Sigma-Aldrich S.r.I., 678910) **Yield:** quantitative. **¹H NMR** (400 MHz, CDCl₃): δ 7.55 (d, *J* = 7.9 Hz, 1H), 7.45 - 7.38 (m, 1H), 7.08 (d, *J* = 8.3 Hz, 1H), 4.65 (s, 2H), 1.87 (s, 1H) ppm. **¹³C NMR** (100 MHz, CDCl₃): δ 129.18, 129.10, 126.14, 125.91, 115.41, 115.20, 68.38 ppm. **MS (ES)** *m*/*z* = 253.1 [M+H]⁺.

### General procedure for the synthesis of chloride.

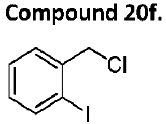

To a stirred solution of 2-iodobenzyl alcohol **19f** (2.78 g, 11.88 mmol) in DCM (80 mL) at 0°C, triethylamine (1.66 mL, 11.88 mmol) and 4-dimethylaminopyridine (Sigma-Aldrich S.r.I., 107700) (1.74 g, 14.26 mmol) were added. Then p-toluenesulfonyl chloride (Sigma-Aldrich S.r.I., 240877) (4,07 g, 21,38 mmol) was added portionwise, and the solution was stirred for 30 min at 0°C. Then it was left at room temperature and stirred overnight. The solvent was evaporated and the crude residue was purified with flash chromatography on silica gel, eluting with hexane to give compound **20f. Yield:** 56%. **¹H-NMR** (400 MHz, CDCl₃): δ 7.85 (d, *J* = 7.6 Hz, 1H), 7.47 (d, *J* = 8 Hz, 1H), 7.34 (t, *J* = 7.2 Hz, 1H), 6.99 (d, *J* = 8 Hz, 1H), 4.66 (s, 2H) ppm. **¹³C-NMR** (100 MHz, CDCl₃): δ 139.8, 135.6, 129.7, 129.3, 126.7, 96.8, 44.2 ppm.

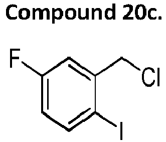

**Compound 20c** was prepared by the procedure described for **compound 20f** starting from compound **19c. Yield** 58%. **¹H NMR** (400 MHz, CDCl₃): δ 7.75 (dd, *J* = 8.8, 5.6 Hz, 1H), 7.20 (dd, *J* = 9.6, 3.2 Hz, 1H), 6.74 (td, *J* = 8.0, 2.8 Hz, 1H) 4.57 (s, 2H) ppm. **¹³C NMR** (100 MHz, CDCl₃): δ 164.2, 161.8, 141.8, 141.7, 140.9, 140.8, 117.5, 117.4, 117.3, 117.2, 91.9, 50.3 ppm. **MS (ES)** *m*/*z* = 271.1 [M+H]+.

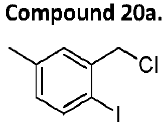

**Compound 20a** was prepared by the procedure described for **compound 20f** starting from compound **19a. Yield:** 59%. **¹H-NMR** (400 MHz, CDCl₃): δ 7.70 (d, *J* = 7.6 Hz, 1H), 7.28 (s, 1H), 6.81 (d, *J* = 8 Hz, 1H), 4.62 (s, 2H), 2.29 (s, 3H) ppm. **¹³C-NMR** (100 MHz, CDCl₃): δ 141.2, 140.3, 134.9, 130.8, 130.2, 106.5, 45.4, 21.2 ppm.

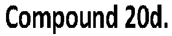

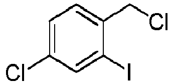

**Compound 20d** was prepared by the procedure described for **compound 20f** starting from compound **19d. Yield:** 55%. **¹H-NMR** (400 MHz, CDCl₃): δ 7.76 (s, 1H), 7.29 (d, *J* = 8 Hz, 1H), 7.02 (d, *J* = 4 Hz, 1H), 4.52 (s, 2H) ppm. **¹³C-NMR** (100 MHz, CDCl₃): δ 140.4, 138.6, 133.7, 131.4, 131.0, 115.9, 45.4 ppm.

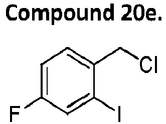

**Compound 20e** was prepared by the procedure described for **compound 20f** starting from compound **19e. Yield:** 51%. **¹H-NMR** (400 MHz, CDCl₃): δ 7.35 (s, 1H), 7.18 (d, *J* = 7.1 Hz, 1H), 7.10 (d, *J* = 7.7 Hz, 1H), 4.77 (s, 2H) ppm. **¹³C-NMR** (100 MHz, CDCl₃): δ 159.8, 134.7, 129.04, 128.98, 122.83, 115.35, 43.42 ppm.

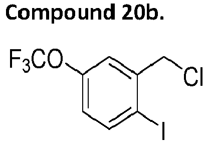

**Compound 20b** was prepared by the procedure described for **compound 20f** starting from compound **19b. Yield:** 45%. **¹H NMR** (400 MHz, CDCl₃) δ 7.88 (d, J = 7.5 Hz, 1H), 6.61 (m, 2H), 4.58 (s, 2H). ¹³C **NMR** (100 MHz, CDCl₃) δ 150.7, 140.9, 133.2, 120.5, 115.5, 113.7, 88.7, 39.3 ppm. **MS (ES)** *m*/*z =* 337.2 [M+H]+.

### General procedure for the chloride substitution.

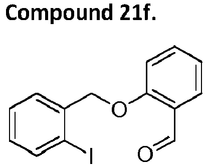

To a stirred solution of salicylaldehyde (Sigma-Aldrich S.r.I., S356) (0,5 mL, 4,69 mmol) in CH₃CN (10 mL), K₂CO₃ (0,71 g, 5,16 mmol), Nal (0,18 g, 1,17 mmol) and 1-(chloromethyl)-2-iodobenzene **20f** (1.30 g, 5,16 mmol) were added. The mixture was stirred at reflux for 12 h. Solvent was then evaporated under reduced pressure and the residue dissolved in AcOEt (5 mL). NaOH 1N (10 mL) was added and the mixture stirred for 10 min at room temperature. Organic phase was then separated and the aqueous layer was extracted with AcOEt (3x10 mL). The combined organic phases were washed with Brine, dried over Na₂SO₄, filtered and concentrated under reduce pressure to give pure compound. **Yield** 92%. **¹H NMR** (400 MHz, CDCl₃): δ 10.58 (s, 1H), 7.87 (t, 1H, *J* = 7.6 Hz), 7.56 (t, 1H, *J* = 7.6 Hz), 7.50 (m, 1H), 7.38 (t, 1H, *J* = 7.2), 7.08-7.02 (m, 2H), 5.16 (s, 2H) ppm. ¹³C **NMR** (100 MHz, CDCl₃): δ 189.40, 160.79, 142.16, 139.52, 135.90, 129.60, 129.20, 128.56, 128.17, 125.30, 121.09, 113.03, 92.91, 67.91 ppm.

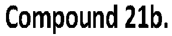

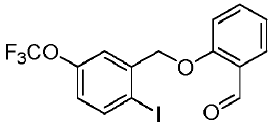

**Compound 21b** was prepared by the procedure described for **compound 21f** starting from salicylaldehyde (Sigma-Aldrich S.r.I., S356) and compound **20b. Yield:** 82%. ¹H **NMR** (400 MHz CDCl₃): δ 10.56 (s, 1H), 7.88 (d, 2H, *J* = 8.0 Hz), 7.56 (m, 1H), 7.41 (m, 1H), 7.10 (m, 1H), 7.02 (d, 1H, *J* = 11.6 Hz), 6.95 (d, 1H, *J* = 8.4 Hz), 5.12 (s, 2H) ppm. ¹³C **NMR** (100 MHz, CDCl₃): δ 190.7, 142.0, 137.4, 135.4, 130.6, 123.5, 123.3, 123.1, 122.6, 114.4, 75.2 ppm. **MS (ES)** *m*/*z* = 477.3 [M+MeOH+Na]+.

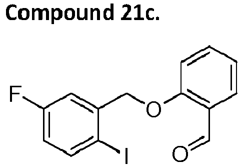

**Compound 21c** was prepared by the procedure described for **compound 21f** starting from salicylaldehyde (Sigma-Aldrich S.r.I., S356) and compound **20c. Yield** 86%. **¹H NMR** (400 MHz, CDCl₃): δ 10.53 (s, 1H), 7.82 (dd, *J* = 8.0, 1.6 Hz, 1H), 7.75 (dd, *J* = 8.4, 5.2 Hz, 1H), 7.51 (td, *J* = 8.8, 1.6 Hz, 1H), 7.23 (dd, *J* = 9.6, 2.8 Hz, 1H), 7.04 (t, *J* = 7.6 Hz, 1H), 6.95 (d, *J* = 8.0 Hz, 1H), 6.76 (td, *J* = 8.4, 3.2 Hz, 1H), 5.04 (s, 2H) ppm. ¹³C **NMR** (100 MHz, CDCl₃): δ 189.2, 164.4, 161.9, 160.1, 140.5, 140.4, 135.9, 129.0, 125.1, 121.5, 117.1, 117.0, 116.0, 116.8, 112.9, 89.0, 73.7 ppm. **MS (ES)** *m*/*z* = 411.1 [M+MeOH+Na]⁺.

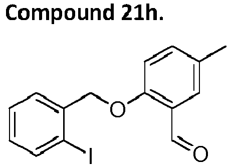

**Compound 21h** was prepared by the procedure described for **compound 21f** starting from 2-Hydroxy-5-methylbenzaldehyde (Sigma-Aldrich S.r.I., 454664) and compound **20f. Yield:** 91%. **¹H-NMR** (400 MHz, CDCl₃): δ 10.54 (s, 1H), 7.86 (d, *J* = 7.6 Hz, 1H), 7.65 (d, *J* = 1.2 Hz, 1H), 7.48 (d, *J* = 7.6 Hz, 1H), 7.38-7.33 (m, 2H), 7.04 (t, *J* = 8 Hz, 1H), 6.92 (d, *J* = 8 Hz, 1H), 5.12 (s, 2H), 2.31 (s, 3H) ppm. **¹³C-NMR** (100 MHz, CDCl₃): δ 189.8, 139.4, 138.4, 136.5, 130.7, 129.8, 128.4, 128.6, 113.1, 74.4, 20.9 ppm.

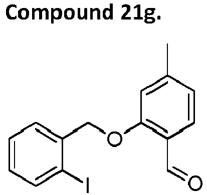

**Compound 21g** was prepared by the procedure described for **compound 21f** starting from 2-Hydroxy-4-methylbenzaldehyde (Sigma-Aldrich S.r.I., W369705) and compound **20f. Yield:** 88%. **¹H-NMR** (400 MHz, CDCl₃): δ 10.45 (s, 1H), 7.84 (d, *J* = 7.6 Hz, 1H), 7.72 (d, *J* = 8 Hz, 1H), 7.48 (d, *J* = 7.6 Hz, 1H), 7.35 (t, *J* = 7.2 Hz, 1H), 7.01 (t, *J* = 8 Hz, 1H), 6.85-6.80 (m, 2H), 5.09 (s, 2H), 2.39 (s, 3H) ppm. **¹³C-NMR** (100 MHz, CDCl₃): δ 189.2, 160.7, 147.4, 139.4, 138.3, 129.8, 128.7, 128.6, 128.5, 123.0, 122.3, 113.5, 97.2, 72.2, 22.3 ppm.

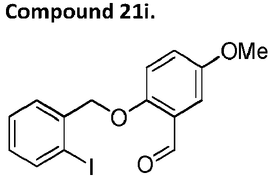

**Compound 21i** was prepared by the procedure described for **compound 21f** starting from 2-Hydroxy-5-methoxybenzaldehyde (Sigma-Aldrich S.r.I., 146862) and compound **20f. Yield** 93%. **¹H NMR** (400 MHz, CDCl₃): δ 10.53 (s, 1H), 7.85 (d, 1H), 7.46 (d, 1H, *J* = 7.2), 7.38-7.34 (m, 2H), 7.11 (dd, 1H, *J* = 8.8 Hz, *J* = 2.8 Hz), 7.03 (t, 1H, *J* = 6.4 Hz), 6.97 (d, 1H, *J* = 8.8 Hz), 5.09 (s, 2H), 3.79 (s, 3H) ppm. **¹³C NMR** (100 MHz, CDCl₃): δ 189.3, 155.3, 154.0, 139.4, 138.4, 129.8, 128.7, 128.4, 125.5, 123.3, 115.0, 110.5, 97.3, 75.0, 55.7 ppm. **MS (ES)** *m*/*z* = 759.2 [2M+Na]⁺, 737.2 [2M+H]⁺, 422.8 [M+MeOH+Na]⁺, 390.9 [M+Na]⁺.

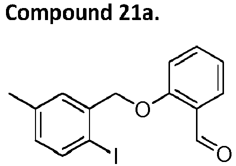

**Compound 21a** was prepared by the procedure described for **compound 21f** starting from salicylaldehyde (Sigma-Aldrich S.r.l., S356) and compound **20a. Yield:** 97%. **¹H-NMR** (400 MHz, CDCl₃): δ 10.57 (s, 1H), 7.86 (d, *J* = 8 Hz, 1H), 7.72 (d, *J* = 8 Hz, 1H), 7.54 (t, *J* = 8.4 Hz, 1H), 7.30 (s, 1H), 7.07-7.02 (m, 2H), 6.86 (d, *J* = 7.6 Hz, 1H), 5.11 (s, 2H), 2.30 (s, 3H) ppm. **¹³C-NMR** (100 MHz, CDCl₃): δ 187.5, 161.4, 141.4, 140.2, 135.2, 134.1, 131.6, 129.9, 128.1, 125.6, 121.1, 115.7, 85.8, 70.1, 21.2 ppm. **MS (ES)** *m*/*z* = 374.9 [M+Na]⁺.

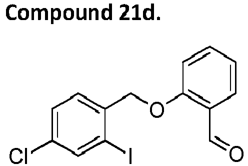

**Compound 21d** was prepared by the procedure described for **compound 21f** starting from salicylaldehyde (Sigma-Aldrich S.r.I., S356) and compound **20d. Yield:** 95%. **¹H-NMR** (400 MHz, CDCl₃): δ 7.81 (d, *J* = 7.4 Hz, 1H), 7.77 (d, *J* = 1.4 Hz, 1H), 7.52 (t, *J* = 7.5 Hz, 1H), 7.29 (s, 1H), 7.14-7.08 (m, 2H), 7.03 (d, *J* = 7.5 Hz, 1H), 5.22 (s, 2H) ppm. **¹³C-NMR** (100 MHz, CDCl₃): δ 187.5, 161.4, 140.7, 140.6, 135.2, 133.6, 131.6, 130.6, 127.6, 125.6, 121.1, 115.7, 91.6, 70.3 ppm.

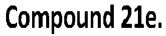

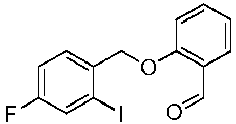

**Compound 21e** was prepared by the procedure described for **compound 21f** starting from salicylaldehyde (Sigma-Aldrich S.r.I., S356) and compound **20e. Yield:** 93%. **¹H-NMR** (400 MHz, CDCl₃): δ 7.82 (d, *J* = 7.5, 1H), 7.51 (d, *J* = 1.6 Hz, 1H), 7.40 (d, *J* = 8.1, 1H), 7.23 (s, 1H), 7.20-7.10 (m, 2H), 7.04 (d, *J* = 7.5, 1H), 5.22 (s, 2H) ppm. **¹³C-NMR** (100 MHz, CDCl₃): δ 190.4, 161.6, 159.5, 157.1, 133.3, 131.8, 130.6, 129.2, 128.4, 123.4, 114.3, 94.8, 94.7, 76.5 ppm.

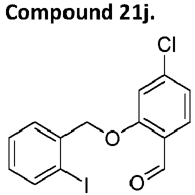

**Compound 21j** was prepared by the procedure described for **compound 21f** starting from 4-chloro-2-hydroxybenzaldehyde (EnamineStore, Riga, Latvia, EN300- 98543) and compound **20f. Yield** 84%. **¹H NMR** (400 MHz, CDCl₃): δ 10.46 (s, 1H), 7.88 (d, 1H, *J* = 7.6 Hz), 7.79 (d, 1H, *J* = 8.8 Hz), 7.47 (d, 1H, *J* = 6.4 Hz), 7.39 (t, 1H, *J* = 7.6 Hz), 7.08-7.03 (m, 3H), 5.12 (s, 2H) ppm. **¹³C NMR** (100 MHz, CDCl₃): δ 188.3, 160.9, 142.0, 141.8, 139.6, 139.5, 137.5, 130.1, 129.8, 129.6, 129.0, 123.7, 121.2, 74.6 ppm.

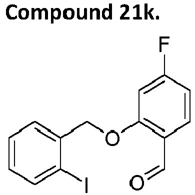

**Compound 21k** was prepared by the procedure described for **compound 21f** starting from 4-fluoro-2-hydroxybenzaldehyde (EnamineStore, Riga, Latvia, EN300- 97011) and compound **20f. Yield** 91%. **¹H NMR** (400 MHz, CDCl₃): *δ* 10.45 (s, 1H), 7.89 (d, *J* = 4 Hz, 1H), 7.87 (d, *J* = 8 Hz, 1H), 7.47 (d, *J* = 8 Hz, 1H), 7.38 (t, *J* = 7.8 Hz, 1H), 7.06 (t, *J* = 14 Hz, 1H), 6.77-6.72 (m, 2H), 5.12 (s, 2H), 1.23 (s, 1H) ppm. **¹³C NMR** (100 MHz, CDCl₃): *δ* 187.96, 168.77, 166.22, 162.27, 139.53, 137.48, 130.80, 128.62, 121.96, 108.71, 101.03, 97.31, 76.93, 74.62 ppm.

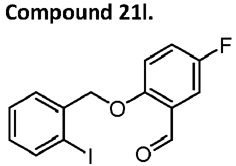

**Compound 21k** was prepared by the procedure described for **compound 21f** starting from 5-fluoro-2-hydroxybenzaldehyde (EnamineStore, Riga, Latvia, EN300- 50297) and compound **20f. Yield** 86%. **¹H NMR** (400 MHz, CDCl₃): *δ* 10.504 (d, *J* = 3.2 Hz, 1H), 7.88 (d, *J* = 8 Hz, 1H), 7.53 (dd, *J* = 4, 1.1 Hz, 1H), 7.46 (d, *J* = 8Hz, 1H), 7.375 (t, *J* = 7.4 Hz, 1H), 7.27-7.21 (m, 1H), 7.08-6.99 (m, 1H), 5.14 (s, 2H) ppm. **¹³C NMR** (100 MHz, CDCl₃): *δ* 188.6, 166.77, 166.20, 163.28, 140.63, 137.51, 131.86, 129.11, 120.96, 108.45, 103.03, 96.51, 78.43, 75.37 ppm.

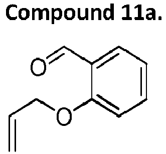

**Compound 11a** was prepared by the procedure described for **compound 21f** starting from salicylaldehyde (Sigma-Aldrich S.r.I., S356) and Allyl bromide (Sigma-Aldrich S.r.I., A29585). **Yield** 90%. **¹H NMR** (400 MHz, CDCl₃): δ 10.53 (s, 1H), 7.83 (dd, 1H, *J* = 7.7, 1.9 Hz), 7.51 (dd, 1H, *J* = 7.6, 1.9 Hz), 7.02 (t, 1H, *J* = 7.5 Hz), 6.96 (d, 1H, *J* = 8.4 Hz), 6.06 (m, 1H), 5.44 (d, 1H, *J* = 17.3), 5.32 (d, 1H, *J* = 10.5 Hz), 4.65 (d, 2H, *J* = 5.1Hz) ppm. ¹³C **NMR** (100 MHz, CDCl₃): δ ppm 188.9, 160.6, 135.6, 132.2, 127.8, 124.7, 120.5, 117.5, 112.7, 68.7 ppm.

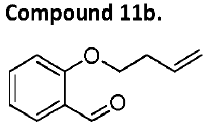

**Compound 11b** was prepared by the procedure described for **compound 21f** starting from salicylaldehyde (Sigma-Aldrich S.r.I., S356) and 4-Bromo-1-butene (Sigma-Aldrich S.r.I., 167851) **Yield:** 41%. **¹H-NMR** (400 MHz, CDCl₃): δ 10.45 (s, 1H), 7.78-7.76 (m, 1H), 7.49-7.45 (m, 1H), 6.98-6.91 (m, 2H), 5.90-5.80 (m, 1H), 5.16-5.07 (m, 2H), 4.08 (t, *J* = 6.8 Hz, 2H), 2.58-2.53 (m, 2H) ppm. **¹³C NMR** (100 MHz, CDCl₃): δ 189.7, 161.4, 135.8, 133.9, 133.6, 128.0, 124.8, 120.5, 117.4, 112.5, 67.5, 33.3 ppm. **MS (ES)** *m*/*z* = 375.0 [2M+Na]⁺, 199.0 [M+Na]⁺, 177.0 [M+H]⁺.

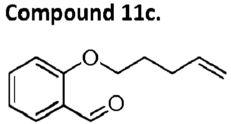

**Compound 11c** was prepared by the procedure described for **compound 21f** starting from salicylaldehyde (Sigma-Aldrich S.r.l., S356) and 5-Bromo-1-pentene (Sigma-Aldrich S.r.I., 253901) **Yield:** 99%. **¹H-NMR** (400 MHz, CDCl₃): δ 10.32 (s, 1H), 7.64-7.62 (m, 1H), 7.32 (t, *J* = 7.2 Hz, 1H), 6.81-6.76 (m, 2H), 5.70-5.60 (m, 1H), 4.89-4.81 (m, 2H), 3.85-3.82 (m, 2H), 2.11-2.05, (q, *J* = 6.8 Hz, 2H), 1.75-1.68 (m, 2H) ppm. **¹³C-NMR** (100 MHz, CDCl₃): δ 189.2, 161.2, 137.2, 135.7, 127.9, 124.7, 120.2, 115.2, 112.5, 67.5, 29.9, 28.0 ppm. **MS (ES)** *m*/*z* = 403.0 [2M + Na]⁺, 213.0 [M + Na]⁺, 190.9 [M+H]⁺.

### General procedure for the synthesis of vinyl derivatives.

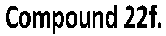

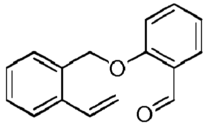

In a flask with magnetic stirrer, the iodide **21f** (1.11g, 3.28 mmol) was dissolved in dryTHF (46 mL) under N₂ atmosphere. Palladium acetate (Sigma-Aldrich S.r.I., 205869) (73 mg, 0.33 mmol) and triphenylphosphine (Sigma-Aldrich S.r.l., T84603) (172 mg, 0.66 mmol) were added. Then tributyl(vinyl)tin (Sigma-Aldrich S.r.I., 271438) (1134 µL, 3.93 mmol) was added dropwise and the mixture was stirred at reflux for 16 h. After cooling, the mixture was filtered through a plug of Celite and the solvent was evaporated *in vacuo.* The residue was diluted with AcOEt and washed with NaHCO₃₍ₛₛ₎ and brine. The organic layers were dried over anhydrous Na₂SO₄, filtered and the solvent was evaporated in *vacuo.* The residue was purified by flash chromatography (Hex/AcOEt 1:0; Hex/AcOEt 9:1). **Yield:** 90%. **¹H-NMR** (400 MHz, CDCl₃): δ 10.47 (s, 1H), 7.85 (d, 1H, *J* = 7.7), 7.57-7.52 (m, 2H), 7.42 (d, 1H, *J* = 6.8 Hz), 7.36 (t, 1H, *J* = 7.2 Hz), 7.30 (d, 1H, *J* = 8.0 Hz), 7.07-7.01 (m, 1H), 6.99 (dd, 1H, *J* = 17.2, 11.2 Hz), 5.70 (d, 1H, *J* = 18.0 Hz), 5.34 (d, 1H, *J* = 10.8 Hz), 5.22 (s, 2H) ppm. **¹³C-NMR** (100 MHz, CDCl₃): δ 189.6, 160.9, 137.2, 135.8, 133.6, 128.8, 128.3, 127.9, 126.2, 125.3, 121.0, 117.0, 113.0, 77.1, 76.8, 68.8 ppm.

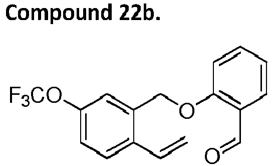

**Compound 22b** was prepared by the procedure described for **compound 22f** starting from compound **21b. Yield:** 60%. **¹H NMR** (400 MHz CDCl₃): δ 10.47 (s, 1H), 7.85 (d, 1H, *J* = 7.6 Hz), 7.55 (m, 2H), 7.32 (s, 1H), 7.19 (d, 1H, *J* = 8.4 Hz), 7.08-7.03 (m, 2H), 6.88 (dd, 1H, *J* = 17.2 and 10.8 Hz), 5.68 (d, 1H, *J* = 17.2 Hz), 5.39 (d, 1H, *J* = 10.8 Hz), 5.19 (s, 2H) ppm. **¹³C NMR** (100 MHz, CDCl₃): δ 189.3, 160.4, 148.6, 135.8, 135.5, 134.7, 132.2, 128.7, 127.8, 125.3, 121.7, 121.4, 120.9, 120.8, 119.1, 118.0, 112.8, 68.0 ppm. **MS (ES)** *m*/*z* = 477.3 [M+MeOH+Na]+.

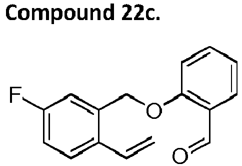

**Compound 22c** was prepared by the procedure described for **compound 22f** starting from compound **21c. Yield:** 75%. **¹H NMR** (400 MHz, CDCl₃): δ 10.50 (s, 1H), 7.87 (dd, *J* = 7.7, 1.7 Hz, 1H), 7.59 - 7.49 (m, 2H), 7.18 (dd, *J* = 9.3, 2.6 Hz, 1H), 7.10-7.01 (m, 3H), 6.88 (dd, *J* = 17.3, 11.0 Hz, 1H), 5.64 (d, *J* = 17.3 Hz, 1H), 5.35 (d, *J* = 11.0 Hz, 1H), 5.20 (s, 2H) ppm. **¹³C NMR** (100 MHz, CDCl₃): δ 189.39, 135.78, 132.42, 128.60, 128.11, 121.29, 116.98, 115.58, 112.82, 67.99 ppm. **MS (ES)** *m*/*z* = 311.1 [M+MeOH+Na]+. 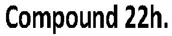 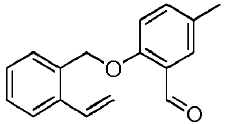 **Compound 22h** was prepared by the procedure described for **compound 22f** starting from compound **21h. Yield** 90%. **¹H-NMR** (400 MHz, CDCl₃): δ 10.44 (s, 1H), 7.64 (s, 1H), 7.55 (d, *J* = 7.6 Hz, 1H), 7.41 (d, *J* = 7.6 Hz, 1H), 7.38-7.26 (m, 3H), 6.99-6.92 (m, 2H), 5.68 (d, *J* = 17.2 Hz, 1H), 5.34 (d, *J* = 10.8 Hz, 1H), 5.18 (s, 2H), 2.30 (s, 3H) ppm. **¹³C-NMR** (100 MHz, CDCl₃): δ 189.8, 156.6, 137.6, 135.1, 134.1, 133.8, 132.5, 129.2, 128.9, 128.2, 127.8, 126.7, 125.7, 114.3, 113.1, 69.8, 20.7 ppm.

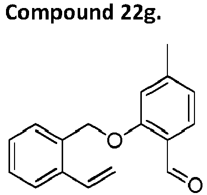

**Compound 22g** was prepared by the procedure described for **compound 22f** starting from compound **21g. Yield** 85%. **¹H-NMR** (400 MHz, CDCl₃): δ 10.37 (s, 1H), 7.72 (d, *J* = 8 Hz, 1H), 7.53 (d, *J* = 7.6 Hz, 1H), 7.40 (d, *J* = 7.6 Hz, 1H), 7.32 (t, *J* = 7.6 Hz, 1H), 7.27 (t, *J* = 6.8 Hz, 1H), 6.98-6.91 (m, 1H), 6.86 (s, 1H), 6.82 (d, *J* = 8 Hz, 1H), 5.67 (d, *J* = 17.6 Hz, 1H), 5.32 (d, *J* = 11.2 Hz, 1H), 5.16 (s, 2H), 2.37 (s, 3H) ppm. **¹³C-NMR** (100 MHz, CDCl₃): δ 189.3, 161.0, 147.3, 137.2, 133.6, 132.8, 128.9, 128.8, 128.3, 127.8, 126.2, 123.0, 122.0, 117.0, 113.5, 68.7, 22.3 ppm.

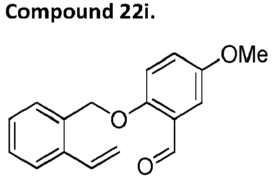

**Compound 22i** was prepared by the procedure described for **compound 22f** starting from compound **21i. Yield** 93%. **¹H NMR** (400 MHz, CDCl₃): δ 10.41 (s, 1H), 7.54 (d, 1H, *J* = 7.6), 7.39-7.27 (m, 3H), 7.12-7.10 (m, 1H), 7.03-6.92 (m, 2H), 5.69 (d, 1H, *J* = 17.2), 5.34 (d, 1H, *J* = 10.8), 5.16 (s, 2H), 3.77 (s, 3H) ppm. ¹³C **NMR** (100 MHz, CDCl₃): δ 189.4, 155.7, 153.9, 137.1, 133.5, 132.9, 129.0, 128.8, 127.8, 126.2, 125.6, 123.3, 116.9, 115.1, 110.2, 69.6, 55.7 ppm. **MS (ES)** *m*/*z* = 323.1 [M+MeOH+Na]⁺, 291.0 [M+Na]⁺.

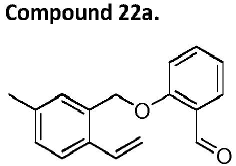

**Compound 22a** was prepared by the procedure described for **compound 22f** starting from compound **21a. Yield:** 90%. **¹H-NMR** (400 MHz, CDCl₃): δ 10.47 (s, 1H), 7.84 (d, *J* = 7.6 Hz, 1H), 7.56 (t, *J* = 1.6 Hz, 1H), 7.52 (d, *J* = 1.6 Hz, 1H), 7.22 (s, 1H), 7.16 (d, *J* =7.6 Hz, 1H), 7.09-7.02 (m, 2H), 6.96-6.89 (m, 1H), 5.64 (d, *J* = 17.2 Hz, 1H), 5.28 (d, *J* = 10.8 Hz, 1H), 5.17 (s, 2H), 2.35 (s, 3H) ppm. **¹³C NMR** (100 MHz, CDCl₃): δ 187.5, 161.4, 138.3, 137.4, 137.3, 136.2, 135.2, 131.6, 128.2, 127.5, 127.4, 125.6, 121.1, 115.7, 113.0, 70.1, 21.2 ppm.

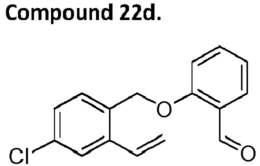

**Compound 22d** was prepared by the procedure described for **compound 22f** starting from compound **21d. Yield** 76%. **¹H-NMR** (400 MHz, CDCl₃): δ 10.44 (s, 1H), 7.84 (d, *J* = 7.6 Hz, 1H), 7.56-7.52 (m, 2H), 7.37-7.34 (m, 1H), 7.27-7.24 (m, 1H), 7.10-7.04 (m, 2H), 6.93-6.86 (m, 1H), 5.71 (d, *J* = 17.6 Hz, 1H), 5.40 (d, *J* = 10.8 Hz, 1H), 5.16 (s, 2H) ppm. **¹³C-NMR** (100 MHz, CDCl₃): δ 189.4, 160.6, 138.8, 135.8, 134.7, 132.4, 131.2, 130.2, 128.5, 127.8, 126.2, 125.2, 121.2, 118.3, 112.8, 68.0 ppm. **MS (ES)** m/z = 273.1 [M+H]⁺.

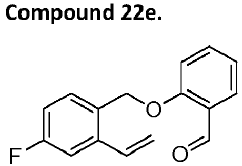

**Compound 22e** was prepared by the procedure described for **compound 22f** starting from compound **21e. Yield** 90%. **¹H NMR** (400 MHz, CDCl₃): δ 10.44 (s, 1H), 7.85 (dd, *J* = 7.6, 1.4 Hz, 1H), 7.58 - 7.53 (m, 1H), 7.39 (dd, *J* = 8.4, 5.8 Hz, 1H), 7.26 (dd, *J* = 9.6, 2.9 Hz, 1H), 7.09 (s, 2H), 7.06 (s, 2H), 7.04 (s, 1H), 7.02 - 6.89 (m, 2H), 5.71 (d, *J* = 17.3 Hz, 1H), 5.41 (d, *J* = 11.0 Hz, 1H), 5.17 (s, 2H) ppm; **¹³C NMR** (100 MHz, CDCl₃): δ 189.5, 135.8, 132.6, 131.0, 130.9, 128.5, 121.2, 118.1, 114.7, 114.5, 113.0, 112.9, 112.8, 68.2 ppm; **MS (ES)** *m*/*z* = 257.1 [M+H]+.

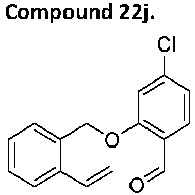

**Compound 22j** was prepared by the procedure described for **compound 22f** starting from compound **21j. Yield** 92%. **¹H NMR** (400 MHz, CDCl₃): δ 10.36 (s, 1H), 7.78 (d, *J* = 8.3 Hz, 1H), 7.56 (d, *J* = 7.6 Hz, 1H), 7.39 (m, 2H), 7.32 (d, *J* = 7.4 Hz, 1H), 7.09 (s, 1H), 7.03 (d, *J* = 8.3 Hz, 1H), 6.94 (dd, *J* = 17.3, 11.0 Hz, 1H), 5.70 (d, *J* = 17.3 Hz, 1H), 5.36 (d, *J* = 11.0 Hz, 1H), 5.19 (s, 2H) ppm. **¹³C NMR** (100 MHz, CDCl₃): δ 188.4, 161.2, 141.7, 137.3, 133.4, 132.0, 129.5, 129.1, 129.0, 127.9, 126.4, 123.7, 121.6, 117.3, 113.6, 69.2, ppm. **MS (ES)** *m*/*z* = 327.1 [M+MeOH+Na]⁺, 295.0 [M+Na]⁺.

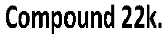

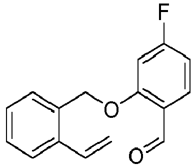

**Compound 22k** was prepared by the procedure described for **compound 22f** starting from compound **21k. Yield** 98%. **¹H NMR** (400 MHz, CDCl₃): δ 10.35 (s, 1H), 7.86 (t, *J* = 4 Hz, 1H), 7.56 (d, *J* = 7.6, 1H), 7.40 (d, *J* = 7.2 Hz, 1H), 7.36 (d, *J* = 7.6 Hz, 1H), 7.31 (d, *J* = 7.2 Hz, 1H), 7.935 (dd, *J* = 17.2 Hz, 1.7 Hz, 1H) 6.79-6.71 (m, 2H), 5.69 (d, *J* = 17.6 Hz, 1H), 5.36 (d, *J* = 10.8 Hz, 1H), 5.19 (s, 2H) ppm. **¹³C NMR** (100 MHz, CDCl₃): *δ* 189.42, 157.31, 156.58, 154.23, 134.55, 128.12, 127.33, 125.89, 122.37, 122.16, 118.71, 115.64, 114.23, 113.90, 77.88, 70.47 ppm.

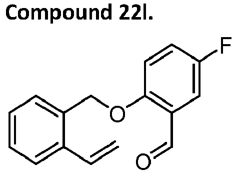

**Compound 22l** was prepared by the procedure described for **compound 22f** starting from compound **21l. Yield** 91%. **¹H NMR** (400 MHz, CDCl₃): *δ* 10.39 (s, 1H), 7.55 (d, *J* = 8 Hz, 1H), 7.50 (dd, *J* = 8.4, 1.3 Hz, 1H), 7.38 (d, *J* = 7.6 Hz, 1H), 7.35 (d, *J* = 7.2 Hz, 1H), 7.30-7.20 (m, 1H), 7.04 (dd, *J* = 8.8, 1.4 Hz, 1H), 7.95 (dd, *J* = 17.2, 1.9 Hz, 1H), 5.69 (d, *J* = 17.2 Hz, 1H), 5.35 (d, *J* = 11.2 Hz, 1H), 5.19 (s, 1H) ppm. **¹³C NMR** (100 MHz, CDCl₃): *δ* 188.62, 158.28, 157.15, 155.89, 133.44, 128.96, 127.87, 126.30, 122.46, 122.21, 117.13, 114.83, 114.75, 114.13, 113.90, 69.57 ppm.

### General procedure for the synthesis of the oxime.

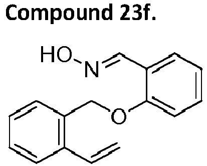

To a stirred solution of aldehyde **22f** (334 mg, 1,84 mmol) in EtOH (25mL), pyridine (Sigma-Aldrich S.r.l., 270970) (180 µL, 2,21 mmol) and hydroxylamine hydrochloride (Sigma-Aldrich S.r.I., 255580) (0,32 g, 4,6 mmol) were added. The mixture was heated at reflux for 2,5 h. After cooling solvent was evaporated. The residue was dissolved in AcOEt (20mL), washed with Brine (2x15 mL), dried over Na₂SO₄, filtered and concentrated under reduce pressure. **Yield:** 96%. **¹H-NMR** (400MHz, CDCl₃): δ 8.52 (s, 1H), 8.31 (bs, 1H), 7.75 (d, 1H, *J* = 8.0 Hz), 7.55 (d, 1H, *J* = 7.6 Hz), 7.40 (m, 1H), 7.41-7.26 (m, 3H), 6.95 (m, 3H), 5.69 (d, 1H, *J* = 17.6 Hz), 5.34 (d, 1H, *J* = 10.8 Hz), 5.14 (s, 2H) ppm. **¹³C-NMR** (100 MHz, CDCl₃): δ 156.6, 146.3, 137.1, 133.6, 133.1, 131.2, 128.9, 128.6, 127.8, 126.6, 126.1, 121.2, 116.9, 112.5, 68.6 ppm. **MS (ES)** *m*/*z* = 276.0 [M+Na]⁺, 254.0 [M+H]⁺.

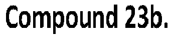

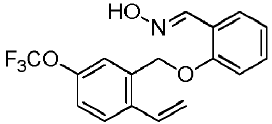

**Compound 23b** was prepared by the procedure described for **compound 23f** starting from compound **22b. Yield:** quantitative. **¹H NMR** (400 MHz CDCl₃): δ 8.72 (bs, 1H), 8.49 (s, 1H), 7.74 (d, 1H, *J* = 7.6 Hz), 7.54 (d, 1H, *J* = 8.4 Hz), 7.34 (t, 1H, *J* = 7.2 Hz), 7.29 (s, 1H), 7.18 (d, 1H, *J* = 8.4 Hz), 6.99 (t, 1H, *J* = 7.6 Hz), 6.94 (d, 1H, *J* = 8.4 Hz), 6.88 (dd, 1H, *J* = 17.2 and 10.8 Hz), 5.68 (d, 1H, *J* = 17.2 Hz), 5.39 (d, 1H, *J* = 10.8 Hz), 5.11 (s, 2H) ppm. **¹³C NMR** (100 MHz, CDCl₃): δ 156.3, 148.6, 146.2, 135.6, 135.2, 132.4, 131.2, 127.7, 126.9, 121.7, 121.5, 121.1, 120.9, 120.7, 119.1, 117.8, 112.5, 68.0 ppm. **MS (ES)** *m*/*z* = 438.3 [M+H]+.

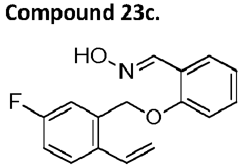

**Compound 23c** was prepared by the procedure described for **compound 23f** starting from compound **22c. Yield** 93%. **¹H NMR** (400 MHz, CDCl₃): δ 8.56 (s, 1H), 7.77 (d, *J* = 7.3 Hz, 1H), 7.51 (dd, *J* = 8.2, 5.7 Hz, 1H), 7.35 (t, *J* = 7.1 Hz, 1H), 7.18 - 7.12 (m, 1H), 7.07 - 6.83 (m, 4H), 5.64 (d, *J* = 17.3 Hz, 1H), 5.36 (d, *J* = 10.9 Hz, 1H), 5.12 (s, 2H) ppm. **¹³C NMR** (100 MHz, CDCl₃): δ 163.5, 161.1, 156.3, 146.2, 135.5, 135.4, 132.8, 131.2, 128.0, 126.9, 121.4, 116.8, 115.4, 115.1, 112.5, 68. 9 ppm. **MS (ES)** *m*/*z* = 272.1 [M+H]+.

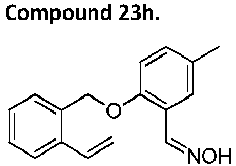

**Compound 23h** was prepared by the procedure described for **compound 23f** starting from compound **22h. Yield:** 92%. **¹H-NMR** (400 MHz, CDCl₃): δ 8.51 (s, 1H), 7.56-7.54 (m, 2H), 7.40 (d, *J* = 7.2 Hz, 1H), 7.35-7.26 (m, 2H), 7.14 (d, *J* = 8.4 Hz, 1H), 6.99-6.92 (m, 1H), 6.88 (d, *J* = 8.4 Hz, 1H), 5.69 (d, *J* = 17.6 Hz, 1H), 5.35 (d, *J* = 11.2 Hz, 1H), 5.11 (s, 2H), 2.29 (s, 3H) ppm. **¹³C-NMR** (100 MHz, CDCl₃): δ 154.7, 146.5, 137.1, 133.7, 131.8, 130.5, 128.8, 128.5, 127.8, 127.2, 126.9, 126.1, 120.6, 116.8, 112.6, 68.8, 20.4 ppm.

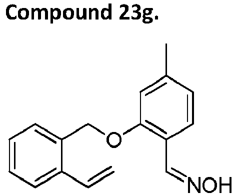

**Compound 23g** was prepared by the procedure described for **compound 23f** starting from compound **22g. Yield:** 97%. **¹H-NMR** (400 MHz, CDCl₃): δ 8.48 (s, 1H), 7.62 (d, *J* = 7.6 Hz, 1H), 7.54 (d, *J* = 7.2 Hz, 1H), 7.40 (d, *J* = 7.2 Hz, 1H), 7.34-7.26 (m, 2H), 6.83-6.77 (m, 2H), 5.68 (d, *J* = 17.2 Hz, 1H), 5.34 (d, *J* = 10.8 Hz, 1H), 5.09 (s, 2H), 2.34 (s, 3H) ppm. **¹³C-NMR** (100 MHz, CDCl₃): δ 156.6, 146.4, 141.8, 137.1, 133.8, 133.3, 129.0, 128.4, 127.3, 126.4, 126.1, 121.7, 118.2, 116.9, 113.2, 68.7, 21.9 ppm.

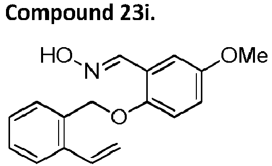

**Compound 23i** was prepared by the procedure described for **compound 23f** starting from compound **22i. Yield** 98%. **¹H NMR** (400 MHz, CDCl₃): δ 8.80 (br, 1H), 8.50 (s, 1H), 7.56 (d, 1H, *J* = 7.6 Hz,), 7.39-7.24 (m, 4H), 7.00-6.86 (m, 3H), 5.70 (d, *J* = 17.6 Hz, 1H), 5.35 (d, *J* = 10.8 Hz), 5.08 (s, 2H), 3.77 (s, 3H) ppm. **¹³C NMR** (100 MHz, CDCl₃): δ 153.9, 151.2, 146.3, 137.1, 133.7, 133.4, 128.9, 128.5, 127.8, 126.0, 121.8, 117.6, 116.8, 114.5, 110.2, 69.6, 55.7 ppm. **MS (ES)** *m*/*z* = 321.9 [M+K]⁺, 306.0 [M+Na]⁺, 284.0 [M+H]⁺.

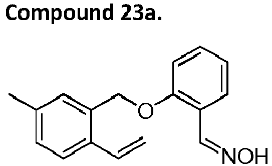

**Compound 23a** was prepared by the procedure described for **compound 23f** starting from compound **22a. Yield:** 86%. **¹H-NMR** (400 MHz, CDCl₃): δ 8.50 (s, 1H), 7.74 (d, *J* = 7.2 Hz, 1H), 7.45 (d, *J* = 8 Hz, 1H), 7.34 (t, *J* = 7.2 Hz, 1H), 7.19 (s, 1H), 7.15 (d, *J* = 8 Hz, 1H), 6.99-8.88 (m, 3H), 5.64 (d, *J* =17.2 Hz, 1H), 5.28 (d, *J* = 11.2 Hz, 1H). 5.09 (s, 2H), 2.34 (s, 3H) ppm. **¹³C-NMR** (100 MHz, CDCl₃): δ 163.9, 159.5, 138.3, 137.4,137.3, 136.2, 130.8, 129.8, 128.2, 127.5, 127.4, 122.4, 120.8, 115.4, 113.0, 70.1, 21.2 ppm.

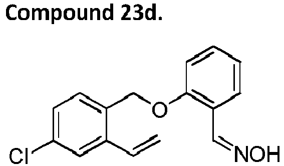

**Compound 23d** was prepared by the procedure described for **compound 23f** starting from compound **22d. Yield:** 92%. **¹H-NMR** (400 MHz, CDCl₃): δ 8.48 (s, 1H), 7.77 (d, *J* = 7.6 Hz, 1H), 7.53 (s, 1H), 7.38-7.33 (m, 2H), 7.29-7.25 (m, 1H), 7.04-6.95 (m, 2H), 6.92-6.85 (m, 1H), 5.71 (d, *J* = 17.2 Hz, 1H), 5.41 (d, *J* = 10.8 Hz, 1H), 5.10 (s, 2H) ppm. **¹³C-NMR** (100 MHz, CDCl₃): δ 172.4, 146.5, 131.4, 130.2, 127.1, 126.2, 121.4, 118.2, 112.4, 93.9, 68.0 ppm. **MS (ES)** m/z = 288.1 [M+H]⁺.

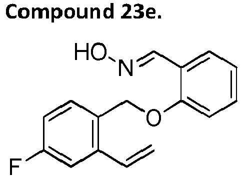

**Compound 23e** was prepared by the procedure described for **compound 23f** starting from compound **22e.Yield** 97%. **¹H NMR** (400 MHz, CDCl₃): δ 8.50 (s, 1H), 7.75 (d, *J* = 7.7 Hz, 1H), 7.39 - 7.32 (m, 2H), 7.25 (dd, *J* = 9.8, 2.2 Hz, 1H), 7.02 - 6.94 (m, 4H), 6.94 - 6.87 (m, 1H), 5.71 (d, *J* = 17.3 Hz, 1H), 5.41 (d, *J* = 10.9 Hz, 1H), 5.09 (s, 2H) ppm; **¹³C NMR** (100 MHz, CDCl₃): δ 164.1, 161.7, 156.5, 146.2, 139.4, 139.4, 132.7, 131.2, 131.0, 130.9, 129.0, 126.7, 121.3, 121.0, 117.9, 114.6, 114.4, 112.8, 112.6, 112.5, 68.0 ppm. **MS (ES)** *m*/*z* = 272.1 [M+H]⁺.

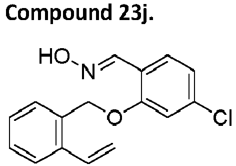

**Compound 23j** was prepared by the procedure described for **compound 23f** starting from compound **22j. Yield** 71%. **¹H NMR** (400 MHz, CDCl₃): δ 8.43 (s, 1H), 8.01 (br, 1H), 7.66 (d, 1H, *J* = 8.0 Hz), 7.55 (d, 1H, *J* = 7.6 Hz), 7.39-7.34 (m, 2H), 7.30 (d, 1H, *J* = 8.0 Hz), 6.99 (d, 1H, *J* = 1.6 Hz), 6.95-6.87 (m, 2H), 5.69 (d, *J* = 17.6 Hz, 1H), 5.36 (d, *J* = 10.8 Hz, 1H), 5.10 (s, 2H) ppm. **¹³C NMR** (100 MHz, CDCl₃): δ 157.0, 145.6, 137.3, 136.8, 133.5, 132.4, 129.0, 128.9, 127.9, 127.4, 126.3, 121.5, 119.5, 117.2, 113.1, 69.0 ppm. **MS (ES)** *m*/*z* = 310.0 [M+Na]⁺, 288.0 [M+H]⁺.

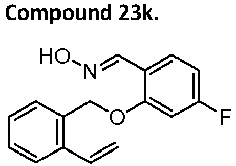

**Compound 23k** was prepared by the procedure described for **compound 23f** starting from compound **22k. Yield** 91%. **¹H NMR** (400 MHz, CDCl₃): *δ* 8.42 (s, 1H), 7.70 (t, *J* = 8 Hz, 1H), 7.55 (d, *J* = 7.6 Hz, 1H), 7.39-7.33 (m, 2H), 7.29 (d, *J* = 7.2 Hz, 1H), 6.92 (q, *J* = 10.8 Hz, 1H), 6.72-6.65 (m, 2H), 5.68 (d, *J* = 17.6 Hz, 1H), 5.36 (d, *J* = 10.8 Hz, 1H), 5.09 (s, 2H) ppm. **¹³C NMR** (100 MHz, CDCl₃): *δ* 203.06, 192.69, 165.88, 163.39, 157.86, 145.61, 137.24, 133.48, 132.39, 128.85, 127.86, 126.26, 117.15, 108.15, 100.50, 68.93 ppm.

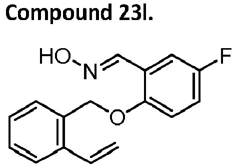

**Compound 23l** was prepared by the procedure described for **compound 23f** starting from compound **221. Yield** 98%. **¹H NMR** (400 MHz, CDCl₃): *δ* 8.22 (s, 1H), 7.61 (t, *J* = 8 Hz, 1H), 7.55 (d, *J* = 7.6 Hz, 1H), 7.39-7.63 (m, 2H), 7.33 (d, *J* = 7.2 Hz, 1H), 6.84 (q, *J* = 10.8 Hz, 1H), 6.72-6.65 (m, 2H), 5.51 (d, *J* = 17.6 Hz, 1H), 5.43 (d, *J* = 10.8 Hz, 1H), 5.23 (s, 2H) ppm. **¹³C NMR** (100 MHz, CDCl₃): *δ* 203.06, 192.69, 165.88, 163.45, 157.77, 145.61, 137.24, 133.54, 132.39, 128.97, 127.86, 126.26, 117.15, 108.15, 101.50, 68.46 ppm.

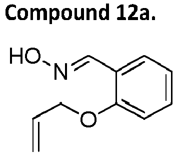

**Compound 12a** was prepared by the procedure described for **compound 23f** starting compound **11a. Yield** 99%. **¹H NMR** (400 MHz, CDCl₃): δ 9.01 (bs, 1H), 8.55 (s, 1H), 7.71 (dd, 1H, *J* = 7.7, 1.7 Hz), 7.31 (td, 1H, *J* = 7.4, 2.0 Hz), 6.95 (t, 1H, *J* = 7.6 Hz), 6.89 (d, 1H, *J* = 8.3 Hz), 6.04 (m, 1H), 5.40 (dd, 1H, *J* = 17.3, 1.6 Hz), 5.32 - 5.25 (m, 1H), 4.58 (dt, 2H, *J* = 5.2, 1.6 Hz) ppm. **¹³C NMR** (100 MHz, CDCl₃): δ 156.6, 146.6, 146.3, 132.8, 131.2, 130.9, 126.7, 121.3, 120.9, 120.6, 117.7, 117.7, 112.3, 112.4, 112.1, 69.2 ppm.

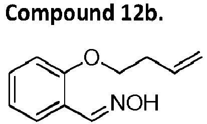

**Compound 12b** was prepared by the procedure described for **compound 23f** starting from compound **11b. Yield:** quantitative. **¹H-NMR** (400 MHz, CDCl₃): δ 8.50 (s, 1H), 7.69-7.66 (m, 1H), 7.29-7.25 (m, 1H), 6.92-6.83 (m, 2H), 5.91-5.80 (m, 1H), 5.15-5.06 (m, 2H), 4.00 (t, *J* = 6.8 Hz, 2H), 2.54-2.51 (m, 2H) ppm. **¹³C-NMR** (100 MHz, CDCl₃): δ 156.9, 146.4, 146.1, 134.2, 131.6, 131.2, 131.0, 126.5, 121.1, 120.8, 120.6, 120.4, 117.2, 112.5, 112.0, 111.8, 68.3, 68.0, 67.6, 67.3, 66.9, 66.6, 33.5 ppm.

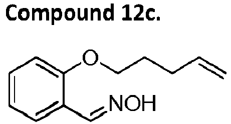

**Compound 12c** was prepared by the procedure described for **compound 23f** starting from compound **11c. Yield:** quantitative. **¹H NMR** (400 MHz, CDCl₃): δ 8.58 (s, 1H), 7.73 (d, *J* = 7.6 Hz, 1H), 7.33 (t, *J* = 8.8 Hz, 1H), 7.03-6.91 (m, 1H), 6.89 (d, *J* = 8.4 Hz, 1H), 5.91-5.81 (m, 1H), 5.15-4.98 (m, 2H), 4.02 (t, *J* = 6.4 Hz, 2H), 2.26 (q, *J* = 6.9 Hz, 2H), 1.92 (m, 2H) ppm. **¹³C NMR** (100 MHz, CDCl₃): δ 157.0, 146.5, 137.6, 131.2, 126.7, 120.8, 115.3, 112.1, 112.0, 67.6, 30.1, 28.2 ppm. **MS (ES)** *m*/*z* = 228.1 [M+Na]⁺, 206.1 [M+H]+.

### General procedure for the synthesis of the amine.

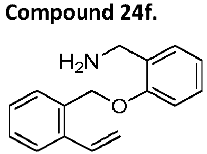

To a stirred solution of oxime **23f** (251 mg, 0,99 mmol) in THF (12 ml), Zn (Sigma-Aldrich S.r.I., 243469) (0,65 g, 9,89 mmol) was added. HCI 2N (5 ml) was added and the mixture was stirred at reflux for 2 h. The reaction mixture was cooled to room temperature, filtered on a Celite pad to remove the excess of metallic zinc and the filtrate was concentrate under reduced pressure. The residue was dissolved in AcOEt (20ml) and extracted with HCI 3N (3x10mL). The aqueous phase was basified with solid potassium carbonate and extracted with DCM (3x20mL). The combined organic phases were dried over Na₂SO₄ and filtered. Solvent was evaporated under reduce pressure to give the primary amine which was used in the next step without any further purification. **Yield:** 99%. **¹H-NMR** (400 MHz, CDCl₃): δ 7.56 (d, 1H, *J* = 7.7 Hz), 7.42 (d, 1H, *J* = 7.4 Hz), 7.34 (d, 1H, *J* = 7.5 Hz), 7.29 (d, 1H, *J* = 7.3 Hz), 7.23 (d, 2H, *J* = 7.1 Hz), 7.02 - 6.90 (m, 3H), 5.70 (d, 1H, *J* = 17.4 Hz), 5.33 (d, 1H, *J* = 11.0 Hz), 5.12 (s, 2H), 3.81 (s, 2H), 1.62 (bs, 2H) ppm. **¹³C-NMR** (100 MHz, CDCl₃): δ 156.4, 137.0, 133.8, 133.7, 131.8, 129.3, 129.2, 128.9, 128.8, 128.5, 128.3, 128.1, 127.9, 126.0, 120.9, 116.6, 111.4 ppm. **MS (ES)** *m*/*z* = 186.1 [M+Na]⁺, 164.1 [M+H]⁺.

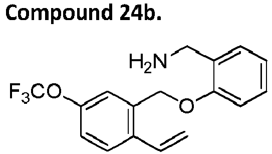

**Compound 24b** was prepared by the procedure described for **compound 24f** starting from compound **23b. Yield:** quantitative. **¹H NMR** (400 MHz CDCl₃): δ 7.53 (d, 1H, *J* = 8.4 Hz), 7.25 (m, 1H), 7.21 (d, 2H, *J* = 7.6 Hz), 7.14 (d, 1H, *J* = 8.8 Hz), 6.94-6.82 (m, 3H), 5.66 (d, 1H, *J* = 17.6 Hz), 5.36 (d, 1H, *J* = 11.2 Hz), 5.08 (s, 2H), 3.78 (s, 2H), 2.24 (bs, 2H) ppm. **¹³C NMR** (100 MHz, CDCl₃): δ 156.1, 148.6, 135.4, 132.3, 129.4, 128.5, 127.7, 127.2, 121.6, 121.0, 120.5, 117.7, 111.8, 111.3, 67.4, 42.0 ppm. **MS (ES)** *m*/*z* = 323.9 [M+H]+.

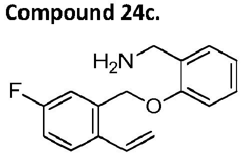

**Compound 24c** was prepared by the procedure described for **compound 24f** starting from compound **23c. Yield:** 95%. **¹H NMR** (400 MHz, CDCl₃): δ 7.51 (dd, *J* = 8.5, 5.8 Hz, 1H), 7.29 - 7.15 (m, 2H), 7.05 - 6.83 (m, 5H), 5.64 (d, *J* = 17.3 Hz, 1H), 5.33 (d, *J* = 11.0 Hz, 1H), 5.09 (s, 2H), 3.86 (s, 2H), 1.62 (s, 2H) ppm. **¹³C NMR** (100 MHz, CDCl₃): δ 163.5, 161.1, 156.1, 136.1, 132.6, 128.6, 128.0, 127.8, 121.2, 116.5, 115.2, 115.0, 114.9, 111.3, 67.3, 42.4 ppm. **MS (ES)** *m*/*z* = 258.1 [M+H]+.

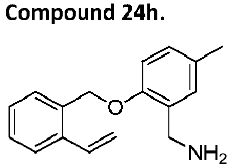

**Compound 24h** was prepared by the procedure described for **compound 24f** starting from compound **23h. Yield:** 47%. **¹H-NMR** (400 MHz, CDCl₃): δ 7.57 (d, *J* = 8 Hz, 1H), 7.41 (d, *J* = 4.4 Hz, 1H), 7.35-7.24 (m, 2H), 7.05-7.00 (m, 2H), 6.98-6.93 (m, 1H), 6.86 (d, *J* = 8 Hz, 1H), 5.70 (d, *J* = 17.2 Hz, 1H), 5.33 (d, *J* = 10.8 Hz, 1H), 5.08 (s, 2H), 3.78 (s, 2H), 2.29 (s, 3H) ppm. **¹³C-NMR** (100 MHz, CDCl₃): δ 137.0, 133.9, 133.7, 131.7, 130.1, 129.5, 128.8, 128.4, 128.2, 127.8, 126.0, 116.5, 111.4, 68.2, 42.5, 20.4 ppm.

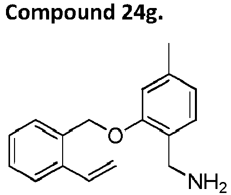

**Compound 24g** was prepared by the procedure described for **compound 24f** starting from compound **23g. Yield:** 97%. **¹H-NMR** (400 MHz, CDCl₃): δ 7.54 (d, *J* = 7.6 Hz, 1H), 7.39 (d, *J* = 7.2 Hz, 1H), 7.33-7.21 (m, 2H), 7.07 (d, *J* = 7.2 Hz, 1H), 6.97-6.93 (m, 1H), 6.77 (s, 1H), 6.72 (d, *J* = 7.6 Hz, 1H), 5.68 (d, *J* = 17.6 Hz, 1H), 5.30 (d, *J* = 11.2 Hz, 1H), 5.06 (s, 2H), 3.73 (s, 2H), 2.32 (s, 3H) ppm. **¹³C-NMR** (100 MHz, CDCl₃): δ 156.4, 138.2, 137.0, 133.7, 128.9, 128.7, 128.5, 127.8, 126.0, 121.4, 116.6, 112.3, 68.0, 42.2, 21.5 ppm.

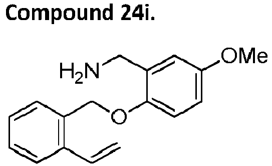

**Compound 24i** was prepared by the procedure described for **compound 24f** starting from compound **23i. Yield** 81%. **¹H NMR** (400 MHz, CDCl₃): δ 7.53 (d, 1H, *J* = 8.0 Hz), 7.37 (d, 1H, *J* = 8.0 Hz), 7.31-7.22 (m, 2H), 6.94 (dd, 1H, *J* = 17.2, 11.2 Hz), 6.85-6.83 (m, 2H), 6.72-6.70 (m, 1H), 5.68 (d, 1H, *J* = 17.2 Hz), 5.30 (d, 1H, *J* = 10.8 Hz), 5.02 (s, 2H), 3.76 (s, 2H), 3.72 (s, 3H), 2.91 (br, 2H) ppm. **¹³C NMR** (100 MHz, CDCl₃): δ 153.8, 150.6, 139.2, 136.9, 133.9, 133.7, 133.2, 129.5, 128.8, 128.4, 127.8, 127.1, 125.9, 116.5, 115.0, 112.8, 112.5, 111.9, 68.6, 55.6, 42.6 ppm. **MS (ES)** *m*/*z* = 270.1 [M+H]⁺.

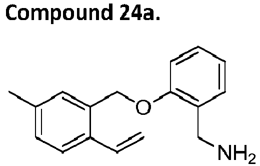

**Compound 24a** was prepared by the procedure described for **compound 24f** starting from compound **23a. Yield:** 93%. **¹H-NMR** (400 MHz, CDCl₃): δ 7.46 (d, *J* = 7.6 Hz, 1H), 7.21-7.13 (m, 4H), 6.98-6.89 (m, 3H), 5.65 (d, *J* = 17.6 Hz, 1H), 5.26 (d, *J* = 10.8 Hz, 1H), 5.07 (s, 2H), 3.80 (s, 2H), 2.35 (s, 3H), 1.61 (bs, 2H) ppm. **¹³C-NMR** (100 MHz, CDCl₃): δ 158.6, 138.3, 137.4, 137.3, 136.2, 128.3, 128.2, 127.9, 127.7, 127.6, 127.5, 122.1, 113.2, 113.0, 70.1, 42.8, 21.2 ppm.

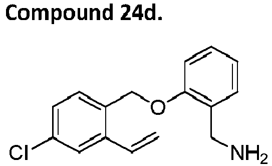

**Compound 24d** was prepared by the procedure described for **compound 24f** starting from compound **23d. Yield:** 91%. **¹H-NMR** (400 MHz, CDCl₃): δ 7.54 (d, *J* = 1.2 Hz, 1H), 7.36-7.34 (m, 1H), 7.26-7.22 (m, 3H), 6.98-6.86 (m, 3H), 5.72 (d, *J* = 17.2 Hz, 1H), 5.39 (d, *J* = 10.8 Hz, 1H), 5.06 (s, 2H), 3.82 (s, 2H), 1.64 (s, 2H) ppm. **¹³C-NMR** (100 MHz, CDCl₃): δ 156.2, 138.6, 134.4, 132.6, 132.2, 132.1, 130.2, 128.6, 128.0, 127.7, 126.0, 121.1, 117.8, 111.3, 67.4, 42.4 ppm. **MS (ES)** *m*/*z* = 274.1 [M+H]⁺.

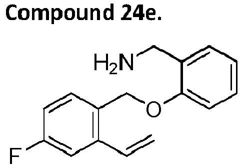

**Compound 24e** was prepared by the procedure described for **compound 24f** starting from compound **23e. Yield** 95%. **¹H-NMR** (400 MHz, CDCl₃): δ 7.38 (dd, *J* = 8.1 Hz, 1H), 7.30 - 7.20 (m, 3H), 7.03 - 6.88 (m, 4H), 5.72 (d, *J* = 17.3 Hz, 1H), 5.39 (d, *J* = 11.0 Hz, 1H), 5.06 (s, 2H), 3.81 (s, 2H), 1.64 (s, 2H) ppm. **¹³C-NMR** (100 MHz, CDCl₃): δ 164.1, 161.6, 156.3, 139.3, 139.2, 132.8, 132.1, 130.9, 130.8, 129.6, 128.6, 128.0, 121.0, 117.6, 114.6, 114.4, 112.7, 112.5, 111.3, 67.4, 42.5 ppm; **MS (ES)** *m*/*z* = 258.1 [M+H]⁺.

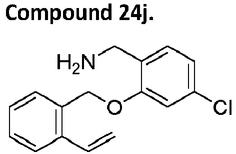

**Compound 24j** was prepared by the procedure described for **compound 24f** starting from compound **23j. Yield** 81%. **¹H NMR** (400 MHz, MeOD): δ 7.58 (d, *J* = 7.7 Hz, 1H), 7.40 (d, *J* = 7.4 Hz, 1H), 7.30 (t, *J* = 7.6 Hz, 1H), 7.27 (d, *J* = 7.6 Hz, 1H), 7.18 (d, *J* = 8.0 Hz, 1H), 7.09 (d, *J* = 1.7 Hz, 1H), 7.00 (dd, *J* = 17.4, 11.0 Hz, 1H), 6.91 (dd, *J* = 8.0, 1.8 Hz, 1H), 5.72 (d, *J* = 17.4 Hz, 1H), 5.31 (d, *J* = 12.0 Hz, 1H), 5.12 (s, 2H), 3.66 (s, 2H). **¹³C NMR** (100 MHz, CDCl₃): δ 158.8, 139.1, 136.6, 133.7, 129.4, 129.2, 128.5, 127.5, 125.6, 120.3, 115.4, 112.0, 68.3, 40.5 ppm. **MS (ES)** *m*/*z* = 296.0 [M+Na]⁺, 274.1 [M+H]⁺.

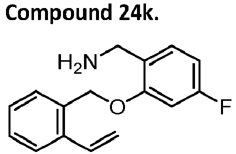

**Compound 24k** was prepared by the procedure described for **compound 24f** starting from compound **23k. Yield** 98%. **¹H NMR** (400 MHz, MeOD): *δ* 7.58 (dd, *J* = 7.7, 1.3 Hz, 1H), 7.40 (dd, *J* = 7.4, 1.5 Hz, 1H), 7.33 (d, *J* = 7.6 Hz, 1H), 7.29-7.23 (m, 1H), 7.18 (d, *J* = 8.0 Hz, 1H), 7.09 (d, *J* = 1.9 Hz, 1H), 7.00 (dd, *J* = 17.4, 11.0 Hz, 1H), 6.91 (dd, *J* = 8.0, 2.0 Hz, 1H), 5.71 (dd, *J* = 17.4, 1.3 Hz, 1H), 5.31 (dd, *J* = 11.0, 1.3 Hz, 1H), 5.12 (s, 2H), 4.80 (s, 1H), 3.67 (s, 2H) ppm. **¹³C NMR** (100 MHz, MeOD): d 157.4, 138.6, 137.2, 133.7, 129.4, 129.1, 128.5, 127.5, 125.5, 120.3, 115.3, 112.0, 68.2, 40.4 ppm.

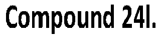

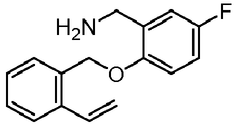

**Compound 24l** was prepared by the procedure described for **compound 24f** starting from compound **23l. Yield** 89%. **¹H NMR** (400 MHz, MeOD): *δ* 7.61 (d, *J* = 7.7 Hz, 1H), 7.41 (dd, *J* = 7.6, 1.5 Hz, 1H), 7.35 (d, *J* = 7.4 Hz, 1H), 7.28-7.22 (m, 1H), 7.15 (d, *J* = 8.2 Hz, 1H), 7.04 (d, *J* = 1.9 Hz, 1H), 7.01 (dd, *J* = 17.4, 11.5 Hz, 1H), 6.89 (dd, *J* = 8.5, 2.4 Hz, 1H), 5.71 (dd, *J* = 17.4, 11.3 Hz, 1H), 5.28 (dd, *J* = 10.8, 1.3 Hz, 1H), 5.12 (s, 2H), 4.84 (s, 1H), 3.67 (s, 2H) ppm. **¹³C NMR** (100 MHz, MeOD): δ 157.4, 138.6, 137.4, 133.9, 129.8, 129.3, 128.6, 127.9, 125.7, 120.6, 115.8, 112.3, 68.6, 40.7 ppm.

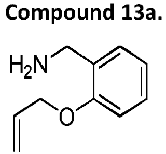

**Compound 13a** was prepared by the procedure described for **compound 24f** starting from compound **12a. Yield** 92%. **¹H-NMR** (400 MHz, CDCl₃): δ 7.25-7.16 (m, 2H), 6.91 (t, 1H, *J* = 7.6 Hz), 6.84 (d, 1H, *J* = 8.8 Hz), 6.06 (m, 1H), 5.41 (dd, 1H, *J* = 17.2, 1.6 Hz), 5.27 (dd, 1H, *J* = 10.4, 1.2 Hz), 4.56 (dt, 2H, *J* = 5.1, 1.6 Hz), 3.84 (bs, 2H) ppm. **¹³C-NMR** (100 MHz, CDCl₃): δ 156.7, 134.4, 128.6, 128.0, 127.9, 120.5, 116.7, 111.0, 67.0, 42.4, 34.5 ppm.

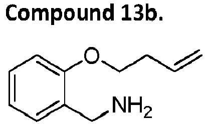

**Compound 13b** was prepared by the procedure described for **compound 24f** starting from **12b. Yield:** 98%. **¹H-NMR** (400 MHz, CDCl₃): δ 7.18-7.14 (m, 2H), 6.88-6.79 (m, 2H), 5.91-5.81 (m, 1H), 5.17-5.06 (m, 2H), 4.01 (t, *J* = 6 Hz, 2H), 3.76 (s, 2H), 2.55-2.50 (m, 2H) ppm. **¹³C NMR** (100 MHz, CDCl₃): δ 156.7, 134.4, 128.6, 128.0, 127.9, 120.5, 116.7, 111.0, 67.0, 42.4, 34.5 ppm. **MS (ES)** *m*/*z* = 377.0 [2M+Na]⁺, 200.0 [M+Na]⁺, 178.0 [M+H]⁺.

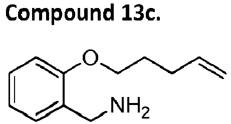

**Compound 13c** was prepared by the procedure described for **compound 24f** starting from compound **12c. Yield:** 99%. **¹H-NMR** (400 MHz, CDCl₃): δ 7.21-7.17 (m, 4H), 6.88 (t, *J* = 7.6 Hz, 1H), 6.83 (d, *J* = 8.8 Hz, 1H), 5.90-5.80 (m, 1H), 5.04-4.98 (m, 2H), 4.00 (t, *J* = 6.4 Hz, 2H), 3.82 (s, 2H), 2.24 (q, *J* = 6.8 Hz, 2H), 2.03 (bs, 2H), 1.94-1.87 (m, 2H) ppm. **¹³C-NMR** (100 MHz, CDCl₃): δ 156.8, 137.6, 131.2, 128.6, 128.1, 120.4, 115.2, 111.0, 67.0, 42.6, 30.2, 28.4 ppm. **MS (ES)** *m*/*z* = 405.0 [2M+Na]⁺, 214.0 [M+Na]⁺, 192.1 [M+H]⁺.

### General procedure for the synthesis of the guanidine derivatives.

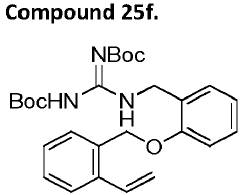

To a stirred solution of primary amine **24f** (251 mg, 1,05 mmol) in THF (10 mL), N,N'-Di-Boc-1H-pyrazole-1-carboxamidine (Sigma-Aldrich S.r.l., 434167) (0,42 g, 1,.36 mmol) and DIPEA (0,09 mL, 0.52 mmol) were added. The mixture was stirred at room temperature for 12 h. After cooling AcOEt (20mL) was added and the organic phase was washed with water and Brine. After drying over Na₂SO₄ the mixture was filtered and concentrated under reduce pressure. The crude residue was purified with flash chromatography on silica gel, eluting with 10% AcOEt/Hex to give pure compound. **Yield:** 78%. **¹H-NMR** (400 MHz, CDCl₃): δ 11.47 (s, 1H), 8.67 (s, 1H), 7.52 (d, 1H, *J* = 7.5 Hz), 7.43 (d, 1H, *J* = 7.3 Hz), 7.31 (t, 2H, *J* = 7.2 Hz), 7.25 (m, 2H), 7.00-6.91 (m, 3H), 5.66 (d, 1H, *J* = 17.4 Hz), 5.33 (d, 1H, *J* = 11.1 Hz), 5.15 (s, 2H), 4.63 (d, 2H, *J* = 5.6 Hz), 1.49 (s, 9H), 1.44 (s, 9H) ppm. **¹³C-NMR** (100 MHz, CDCl₃): δ 163.6, 156.7, 156.3, 156.0, 152.9, 136.8, 133.7, 129.8, 128.9, 128.7, 128.2, 127.7, 126.0, 120.9, 116.7, 111.6, 82.7, 79.0, 68.1, 40.6, 28.3, 28.0 ppm. **MS (ES)** *m*/*z* = 504.1 [M+Na]⁺, 482.1 [M+H]⁺.

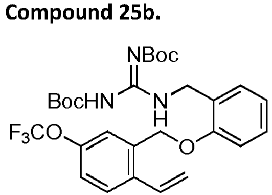

**Compound 25b** was prepared by the procedure described for **compound 25f** starting from compound **24b. Yield:** 75%. **¹H NMR** (400 MHz CDCl₃): δ 11.46 (s, 1H), 8.68 (s, 1H), 7.52 (d, 1H, *J* = 8.4 Hz), 7.33-7.23 (m, 3H), 7.15 (d, 1H, *J* = 8.4 Hz), 6.97-6.85 (m, 3H), 5.65 (d, 1H, *J* = 17.6 Hz), 5.37 (d, 1H, *J* = 11.2 Hz), 5.12 (s, 2H), 4.64 (d, 2H, *J* = 5.2 Hz), 1.48 (s, 9H), 1.41 (s, 9H) ppm. **¹³C NMR** (100 MHz, CDCl₃): δ 157.3, 154.1, 154.0, 144.0, 143.1, 136.4, 135.8, 129.1, 128.8, 127.2, 119.6, 117.9, 117.2, 114.1, 112.7, 80.3, 79.8, 67.9, 40.5, 28.2 ppm. **MS (ES)** *m*/*z* = 323.9 [M+H]⁺.

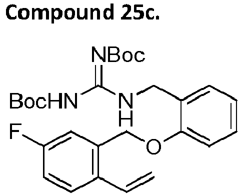

**Compound 25c** was prepared by the procedure described for **compound 25f** starting from compound **24c.** Yield: 87%. **¹H NMR** (400 MHz, CDCl₃): δ 11.50 (s, 1H), 8.76 - 8.71 (m, 1H), 7.47 (dd, *J* = 8.1, 6.0 Hz, 1H), 7.33 (d, *J* = 7.4 Hz, 1H), 7.28 - 7.17 (m, 2H), 7.02 - 6.83 (m, 4H), 5.60 (d, *J* = 17.3 Hz, 1H), 5.32 (d, *J* = 11.0 Hz, 1H), 5.12 (s, 2H), 4.66 (d, *J* = 5.2 Hz, 2H), 1.50 (s, 9H), 1.44 (s, 9H) ppm. **¹³C NMR** (100 MHz, CDCl₃): δ 163.6, 161.1, 156.5, 153.0, 135.9, 132.6, 130.0, 127.8, 125.9, 121.1, 116.6, 114.9, 111.6, 82.8, 79.0, 67.4, 40.7, 28.2, 28.9 ppm. **MS (ES)** *m*/*z* = = 500.2 [M+H]⁺.

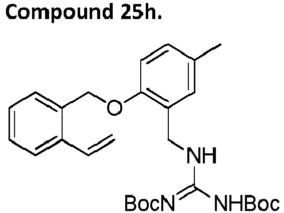

**Compound 25h** was prepared by the procedure described for **compound 25f** starting from compound **24h. Yield:** 71%. **¹H-NMR** (400 MHz, CDCl₃): δ 11.46 (bs, 1H), 8.67 (bs, 1H), 7.51 (d, *J* = 7.6 Hz, 1H), 7.44 (d, *J* = 7.2 Hz, 1H), 7.32-7.23 (m, 2H), 7.13 (s, 1H), 7.04-6.93 (m, 2H), 6.83 (d, *J* = 8 Hz, 1H), 5.66 (d, *J* = 17.6 Hz, 1H), 5.32 (d, *J* = 10.8 Hz, 1H), 5.12 (s, 2H), 4.61 (d, *J* = 5.2 Hz, 2H), 2.27 (s, 3H), 1.50 (s, 9H), 1.44 (s, 9H) ppm. **¹³C-NMR** (100 MHz, CDCl₃): δ 155.9, 154.6, 152.9, 136.8, 133.8, 130.7, 130.1, 129.2, 128.6, 128.2, 127.7, 125.9, 125.5, 116.6, 111.6, 82.7, 68.2, 40.7, 28.3, 28.0, 20.4 ppm.

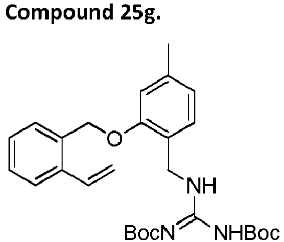

**Compound 25g** was prepared by the procedure described for **compound 25f** starting from compound **24g. Yield:** 46 %. **¹H-NMR** (400 MHz, CDCl₃): δ 11.44 (s, 1H), 8.61 (s, 1H), 7.50 (d, *J* = 8 Hz, 1H), 7.41 (d, *J* = 8 Hz, 1H), 7.30-7.21 (m, 2H), 7.17 (d, *J* = 7.2 Hz, 1H), 6.99-6.92 (m, 1H), 6.75 (s, 1H), 6.73 (d, *J* = 7.6 Hz, 1H), 5.64 (d, *J* = 17.6 Hz, 1H), 5.30 (d, *J* = 11.2 Hz, 1H), 5.10 (s, 2H), 4.57 (d, *J* = 4.8 Hz, 2H), 2.30 (s, 3H), 1.47 (s, 9H), 1.40 (s, 9H) ppm. **¹³C-NMR** (100 MHz, CDCl₃): δ 156.7, 155.8, 152.9, 136.9, 133.8, 133.6, 129.8, 128.7, 128.2, 127.7, 125.9, 122.8, 121.4, 116.6, 112.5, 82.6, 68.0, 40.5, 28.3, 28.0, 21.5 ppm.

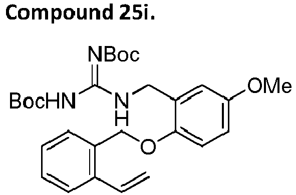

**Compound 25i** was prepared by the procedure described for **compound 25f** starting from compound **24i. Yield:** 65%. **¹H-NMR** (400 MHz, CDCl₃): δ 11.47 (s, 1H), 8.66 (br, 1H), 7.48 (d, 1H, *J* = 7.6 Hz), 7.40 (d, 1H, *J* = 7.6 Hz), 7.28-7.19 (m, 2H), 6.98-6.91 (m, 2H), 6.82 (d, 1H, *J* = 9.2 Hz), 6.72 (dd, 1H, *J* = 8.8, 2.8 Hz), 5.63 (d, 1H, *J* = 17.2 Hz), 5.29 (d, 1H, *J* = 10.8 Hz), 5.05 (s, 2H), 4.58 (d, 2H, *J* = 5.6 Hz), 3.71 (s, 3H), 1.46 (s, 9H), 1.41 (s, 9H) ppm. **¹³C-NMR** (100 MHz, CDCl₃): δ 163.6, 156.0, 153.7, 152.9, 150.8, 136.8, 133.8, 128.7, 128.2, 127.7, 127.1, 125.9, 116.6, 115.9, 113.3, 112.9, 82.7, 79.0, 68.8, 55.6, 40.5, 28.2, 28.0, 25.5 ppm. **MS (ES)** *m*/*z* = 534.2 [M + Na]⁺, 512.2 [M + H]⁺.

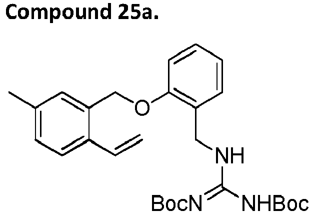

**Compound 25a** was prepared by the procedure described for **compound 25f** starting from compound **24a. Yield:** 67%. **¹H-NMR** (400 MHz, CDCl₃): δ 8.66 (bs, 1H), 7.41 (d, *J* = 8 Hz, 1H), 7.31 (d, *J* = 7.6 Hz, 1H), 7.26-7.23 (m, 2H), 7.11 (d, *J* = 8 Hz, 1H), 6.96-6.87 (m, 3H), 5.61 (d, *J* = 17.6 Hz, 1H), 5.26 (d, *J* = 11.6 Hz, 1H), 5.10 (s, 2H), 4.64 (d, *J* = 5.2 Hz, 2H), 2.32 (s, 3H), 1.48 (s, 9H), 1.42 (s, 9H) ppm. **¹³C-NMR** (100 MHz, CDCl₃): δ 163.6, 156.8, 156.0, 152.9, 138.0, 137.5, 134.0, 133.6, 133.3, 129.8, 129.3, 129.0, 128.9, 125.9, 120.8, 115.7, 111.6, 111.4, 82.6, 79.0, 68.2, 40.6, 28.9, 28.3, 28.0, 21.1 ppm.

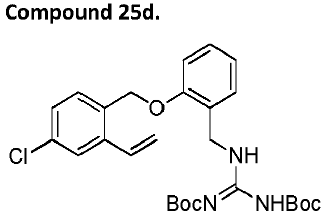

**Compound 25d** was prepared by the procedure described for **compound 25f** starting from compound **24d. Yield:** 70%. **¹H-NMR** (400 MHz, CDCl₃): δ 11.47 (bs, 1H), 8.67 (bs, 1H), 7.49 (s, 1H), 7.39 (d, *J* = 8.0 Hz, 1H), 7.33 (d, *J* = 7.6 Hz, 1H), 7.28-7.22 (m, 2H), 6.97-6.87 (m, 3H), 5.68 (d, *J* = 17.2 Hz, 1H), 5.39 (d, *J* = 10.8 Hz, 1H), 5.10 (s, 2H), 4.64 (d, *J* = 5.6 Hz, 2H), 1.50 (s, 9H), 1.44 (s, 9H) ppm. **¹³C-NMR** (100 MHz, CDCl₃): δ 156.4, 152.8, 138.2, 132.6, 130.1, 129.9, 128.9, 127.6, 126.0, 121.0, 118.0, 111.5, 82.7, 67.4, 40.6, 28.2, 27.9 ppm. **MS (ES)** *m*/*z* = 516.2 [M+H]⁺.

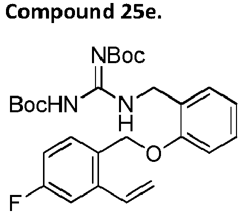

**Compound 25e** was prepared by the procedure described for **compound 25f** starting from compound **24e.** Y**ield** 90%. **¹H-NMR** (400 MHz, CDCl₃): δ 11.50 (s, 1H), 8.66 (s, 1H), 7.41 (d, *J* = 8.2 Hz, 1H), 7.33 (d, *J* = 7.3 Hz, 1H), 7.29 - 7.18 (m, 2H), 6.95 (t, *J* = 8.2 Hz, 4H), 5.68 (d, *J* = 17.3 Hz, 1H), 5.38 (d, *J* = 11.0 Hz, 1H), 5.09 (s, 2H), 4.63 (d, *J* = 5.3 Hz, 2H), 1.50 (s, 9H), 1.45 (s, 9H) ppm. **¹³C-NMR** (100 MHz, CDCl₃): δ 164.0, 163.6, 161.5, 156.6, 155.9, 152.9, 132.8, 130.9, 130.8, 129.8, 129.4, 128.9, 125.9, 121.0, 117.7, 114.6, 114.3, 112.6, 112.4, 111.5, 82.7, 79.0, 67.4, 40.6, 28.2, 27.9 ppm. **MS (ES)** *m*/*z* = 500.2 [M+H]⁺.

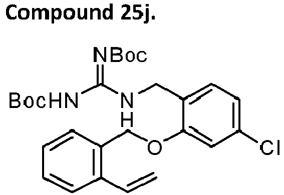

**Compound 25j** was prepared by the procedure described for **compound 25f** starting from compound **24j. Yield:** 75%. **¹H-NMR** (400 MHz, CDCl₃): δ 11.45 (s, 1H), 8.64 (s, 1H), 7.52 (d, *J* = 7.5 Hz, 1H), 7.41 (d, *J* = 7.2 Hz, 1H), 7.32 (t, *J* = 7.1 Hz, 1H), 7.26 (m, 2H), 6.96 (d, *J* = 11.1 Hz, 1H), 6.94 - 6.92 (m, 1H), 6.91 (d, *J* = 1.8 Hz, 1H), 5.66 (d, *J* = 17.3 Hz, 1H), 5.34 (d, *J* = 11.0 Hz, 1H), 5.10 (s, 2H), 4.59 (d, *J* = 5.4 Hz, 2H), 1.48 (s, 9H), 1.44 (s, 9H). **¹³C-NMR** (100 MHz, CDCl₃): δ 157.1, 155.9, 152.9, 137.0, 134.2, 133.6, 132.7, 130.5, 128.9, 128.6, 127.8, 126.1, 124.6, 120.8, 117.0, 112.3, 82.9, 79.2, 68.4, 40.0, 28.2, 28.0 ppm. **MS (ES)** *m*/*z* = 538.2 [M + Na]⁺, 516.2 [M + H]⁺.

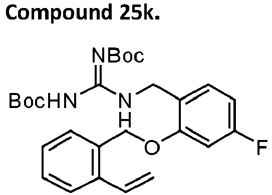

**Compound 25k** was prepared by the procedure described for **compound 25f** starting from compound **24k. Yield:** 70%. **¹H NMR** (400 MHz, CDCl₃): δ 11.45 (s, 1H), 8.64 (s, 1H), 7.41 (d,J = 7.7 Hz, 1H), 7.38 (d, J = 7.5 Hz, 1H), 7.32 (t, *J* = 8.0 Hz, 1H), 7.28-7.24 (m, *J* = 2H), 6.68-6.60 (m, 1H), 5.66 (dd, *J* = 17.4, 1.3 Hz, 1H), 5.34 (dd, *J* = 11.0, 1.3 Hz, 1H), 5.10 (s, 2H), 4.59 (d, *J* = 5.5 Hz, 2H), 1.48 (s, 9H) ppm. **¹³C NMR** (100 MHz, CDCl₃): δ 157.1, 155.9, 152.9, 137.0, 134.2, 133.6, 132.7, 130.5, 128.9, 128.5, 127.8, 126.1, 124.5, 120.8, 117.0, 112.3, 82.9, 68.4, 39.9, 28.2, 27.9 ppm.

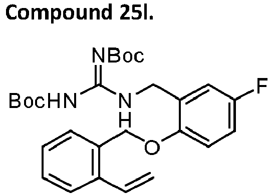

**Compound 25l** was prepared by the procedure described for **compound 25f** starting from compound **24l. Yield:** 62%. **¹H NMR** (400 MHz, CDCl₃): δ 11.45 (s, 1H), 8.60 (s, 1H), 7.41 (d, *J* = 7.5 Hz, 1H), 7.32 (d, *J* = 7.5 Hz, 1H), 7.29 (t, *J* = 8.0 Hz, 1H), 7.27-7.24 (m, *J* = 2H), 6.94 (dd, *J* = 7.4, 1.2 Hz 1H), 6.68-6.60 (m, 2H), 5.66 (d, *J* = 17.4 Hz, 1H), 5.34 (d, *J* = 11.0 Hz, 1H), 5.10 (s, 2H), 4.57 (d, *J* = 5.5 Hz, 2H), 1.44 (s, 9H) ppm. **¹³C NMR** (100 MHz, CDCl₃): δ 203.1, 165.9, 162.9, 147.0, 134.2, 133.6, 132.7, 130.5, 129.9, 128.5, 127.8, 126.1, 122.5, 121.8, 117.0, 113.3, 85.9, 69.4, 39.9, 30.2, 28.9 ppm.

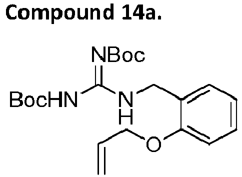

**Compound 14a** was prepared by the procedure described for **compound 25f** starting from compound **13a. Yield** 75%. **¹H-NMR** (400 MHz, CDCl₃): δ 7.28 (d, 1H, *J* = 7.5 Hz, 1H), 7.23 (t, 1H, *J* = 7.6 Hz), 6.90 (t, 1H, *J* = 7.5 Hz), 6.85 (d, 1H, *J* = 8.2 Hz), 6.12-6.022 (m, 1H), 5.38 (d, 1H, *J* = 16.8 Hz), 5.25 (d, 1H, *J* = 16.8 Hz), 4.63 (d, 2H, *J* = 5.2 Hz), 4.58 (d, 2H, *J* = 5.2 Hz), 1.50 (s, 9H), 1.45 (s, 9H) ppm. **¹³C-NMR** (100 MHz, CDCl₃): δ 163.4, 156.4, 155.8, 152.9, 132.9, 129.4, 128.7, 125.5, 120.4, 117.1, 111.3, 82.3, 78.5, 68.5, 40.6, 28.0, 27.7 ppm. **MS (ES)** *m*/*z* = 428.1 [M+Na]⁺, 406.2 [M+H]⁺.

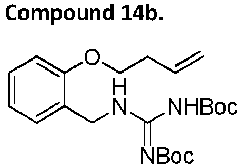

**Compound 14b** was prepared by the procedure described for **compound 25f** starting from compound **13b. Yield:** 78%. **¹H-NMR** (400 MHz, CDCl₃): δ 11.47 (bs, 1H), 8.71 (bs, 1H), 7.25-7.17 (m, 2H), 6.87-6.80 (m, 2H), 5.92-5.82 (m, 1H), 5.13-5.02 (m, 2H), 4.57 (d, *J* = 5.2 Hz, 2H), 4.00 (t, *J* = 6.8 Hz, 2H), 2.57-2.52 (m, 2H), 1.46 (s, 9H), 1.42 (s, 9H) ppm. **¹³C NMR** (100 MHz, CDCl₃): δ 163.6, 156.9, 155.8, 152.9, 134.4, 129.8, 128.8, 125.4, 120.3, 116.9, 111.0, 82.5, 78.9, 67.1, 40.9, 33.4, 28.3, 27.8 ppm. **MS (ES)** *m*/*z* = 861.0 [2M+Na]⁺, 441.9 [M+Na]⁺,419.9 [M+H]⁺.

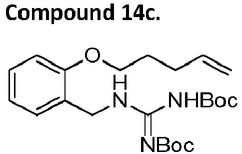

**Compound 14c** was prepared by the procedure described for **compound 25f** starting from compound **13c. Yield:** 85%. **¹H-NMR** (400 MHz, CDCl₃): δ 11.46 (bs, 1H), 8.71 (bs, 1H), 7.18-7.11 (m, 2H), 6.80-6.74 (m, 2H), 5.77-5.70 (m, 1H), 4.98-4.94 (d, *J* = 17.2 Hz, 1H), 4.90-4.87(d, *J* = 10.4 Hz, 1H), 3.94-3.90 (t, *J* = 6.4 Hz, 2H), 2.19-2.14 (q, *J* = 6.8 Hz, 2H), 1.85-1.81 (m, 2H), 1.40 (s, 9H), 1.36 (s, 9H) ppm. **¹³C-NMR** (100 MHz, CDCl₃): δ 163.4, 157.0, 155.9, 152.8, 137.6, 129.6, 128.9, 125.3, 120.3, 115.0, 111.0, 82.6, 78.9, 67.0, 40.9, 30.0, 28.1, 28.1, 27.8 ppm. **MS (ES)** *m*/*z* = 889.0 [2M+Na]⁺, 456.0 [M+Na]⁺, 434.0 [M+H]⁺.

### General procedure for the synthesis of amidinourea derivatives.

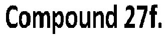

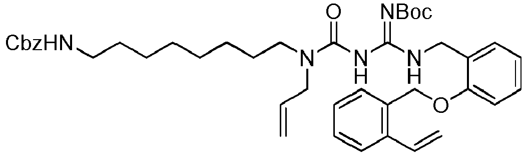

To a stirred solution of protected guanidine **25f** (246 mg, 0.51 mmol) in dry THF (10 mL), a solution of linker **10z** (0.26 g, 0.82 mmol) in dry THF (5mL) and triethylamine (0.07 mL, 0.51 mmol) were added dropwise under N₂ atmosphere. The mixture was heated at reflux and stirred for 12 h. After cooling AcOEt (25mL) was added and the organic phase was washed with water and Brine. After drying over Na₂SO₄ the mixture was filtered and concentrated under reduce pressure. The crude residue was purified with flash chromatography on silica gel, eluting with 20% AcOEt/Hex to give pure compound. **Yield** 73%. **¹H-NMR** (400 MHz CDCl₃): δ 12.28 (s, 1Hz), 8.41 (s, 1H), 7.52 (d, 1H, *J* = 7.5 Hz), 7.44 (d, 1H, *J* = 7.4 Hz), 7.35 (m, 5H), 7.32 (d, 1H, *J* = 6.9 Hz), 7.29 (d, 2H, *J* = 7.3 Hz), 7.27-7.21 (m, 1H), 7.04-6.98 (m, 1H), 6.94 (d, 2H, *J* = 9.7 Hz), 5.85-5.69 (m, 1H), 5.66 (d, 1H, *J* = 16.4 Hz), 5.32 (d, 1H, *J* = 10.8 Hz), 5.15 (s, 2H), 5.09 (m, 3H), 5.01 (d, 1H, *J* = 9.9 Hz), 4.55 (dd, 2H, *J* = 14.8, 6.0 Hz), 4.05 (d, 1H, *J* = 5.3 Hz), 3.89 (d, 1H, *J* = 5.3 Hz), 3.36 (t, 1H, *J* = 7.6 Hz), 3.21 (t, 1H, *J* = 8.0 Hz), 3.16-3.11 (m, 2H), 1.51 (m, 4H), 1.42 (s, 9H), 1.27-1.15 (m, 8H) ppm. **¹³C-NMR** (100 MHz, CDCl₃): δ 163.68, 156.46, 154.06, 153.88, 153.27, 135.33, 134.73, 133.74, 129.30, 128.97, 128.70, 128.64, 128.44, 128.28, 128.02, 127.77, 126.80, 125.95, 120.73, 116.65, 115.47, 111.51, 81.88, 68.07, 66.48, 50.46, 48.45, 47.53, 45.66, 41.04, 39.99, 29.88, 29.35, 29.21, 28.53, 28.04, 26.98, 26.91, 26.64 ppm. **MS (ES)** *m*/*z* = 747.9 [M+Na]⁺, 725.9 [M+H]⁺.

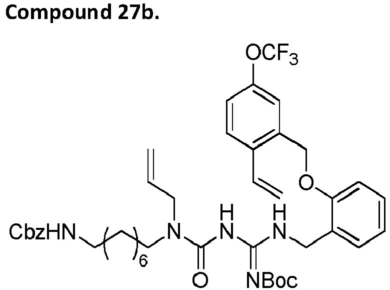

**Compound 27b** was prepared by the procedure described for **compound 27f** starting from compounds **25b** and **10z. Yield:** 77%. **¹H NMR** (400 MHz, CDCl₃): δ 12.27 (d, 1H, *J* = 10.0 Hz), 8.43 (s, 1H), 7.52 (d, 1H, *J* = 8.4 Hz), 7.32-7.14 (m, 9H), 6.95-6.85 (m, 3H), 5.83-5.68 (m, 1H), 5.65 (d, 1H, *J* = 16.0 Hz), 5.36 (d, 1H, *J* = 11.2 Hz), 5.10 (s, 2H), 5.06 (m, 3H), 5.01 (m, 1H), 4.57 (d, 1H, *J* = 5.8 Hz), 4.53 (d, 1H, *J* = 5.9 Hz), 4.04 (d, 1H, *J* = 5.8 Hz), 3.89 (d, 1H, *J* = 5.6 Hz), 3.35 (t, 1H, *J* = 7.5 Hz), 3.20 (t, 1H, *J* = 7.7 Hz), 3.13 (m, 2H), 1.40 (s, 9H), 1.25 (m, 12H) ppm. **¹³C NMR** (100 MHz, CDCl₃): δ 156.1, 154.0, 153.3, 135.7, 135.3, 134.6, 132.4, 129.4, 129.1, 128.4, 128.0, 127.5, 126.9, 121.1, 120.8, 120.4, 117.6, 115.4, 111.4, 81.9, 67.4, 66.4, 50.4, 48.4, 47.5, 45.6, 41.0, 39.9, 29.8, 29.3, 29.1, 28.5, 27.9, 26.9, 26.6, 14.1 ppm. **MS (ES)** *m*/*z* = 810.6 [M+H]⁺.

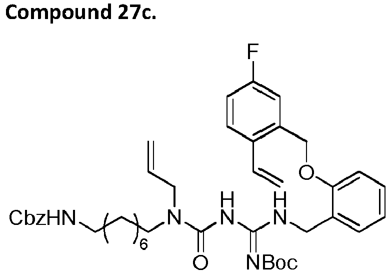

**Compound 27c** was prepared by the procedure described for **compound 27f** starting from compounds **25c** and **10z.** Y**ield:** 79%. **¹H NMR** (400 MHz, CDCl₃): δ 12.32 (d, *J* = 10.1 Hz, 1H), 8.49 (d, *J* = 4.4 Hz, 1H), 7.48 (dt, *J* = 12.9, 6.5 Hz, 1H), 7.38 - 7.18 (m, 6H), 7.02 - 6.82 (m, 5H), 5.87 - 5.68 (m, 1H), 5.61 (d, *J* = 17.3 Hz, 1H), 5.32 (d, *J* = 11.0 Hz, 1H), 5.19 - 4.98 (m, 6H), 4.59 (dd, *J* = 16.6, 5.6 Hz, 2H), 4.09 (d, *J* = 5.5 Hz, 1H), 3.92 (d, *J* = 5.1 Hz, 1H), 3.45 - 3.36 (m, 2H), 3.26 - 3.20 (m, 2H), 3.19 - 3.09 (m, 2H), 1.48 (d, *J* = 9.0 Hz, 4H), 1.43 (s, 13H), 1.28 (s, 6H) ppm. **¹³C NMR** (100 MHz, CDCl₃): δ 163.6, 161.1, 156.2, 154.0, 153.3, 136.0, 135.3, 134.7, 132.6, 129.4, 129.1, 128.5, 128.4, 128.0, 127.8, 127.7, 126.8, 121.0, 116.6, 115.5, 115.4, 115.1, 114.9, 111.5, 81.9, 67.4, 66.4, 50.4,48.4,47.5,45.6, 41.0, 40.1, 29.8, 29.1, 28.5, 28.0, 26.9, 26.6 ppm. **MS (ES)** *m*/*z* = 744.4 [M+H]⁺.

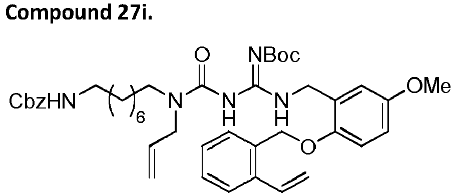

**Compound 27i** was prepared by the procedure described for **compound 27f** starting from compounds **25i** and **10z.Yield** 88%. **¹H-NMR** (400 MHz, CDCl₃): δ 12.33 (d, 1H, *J* = 10.4 Hz), 8.39 (d, 1H, *J* = 6.0 Hz), 7.48 (d, 1H, *J* = 7.6 Hz), 7.39 (d, 1H, *J* = 7.2 Hz), 7.27-7.20 (m, 5H), 6.94 (dd, 1H, *J* = 16.0, 8.0 Hz), 6.83-6.79 (m, 2H), 6.70-6.66 (m, 1H), 5.82-5.66 (m, 1H), 5.63 (d, 1H, *J* = 17.2 Hz), 5.29-5.26 (d, 1H, *J* = 10.8 Hz), 5.09-4.97 (m, 7H), 4.50 (dd, 2H, *J* = 17.6, 5.6 Hz), 4.03 (d, 1H, *J* = 5.2 Hz), 3.88 (d, 1H, *J* = 5.2 Hz), 3.67 (s, 3H), 3.35 (t, 1H, *J* = 7.2 Hz), 3.20 (t, 1H, *J* = 7.2 Hz), 3.35 (m, 2H), 1.40 (m, 15H), 1.18 (m, 7H) ppm. **¹³C-NMR** (100 MHz, CDCl₃): δ 163.7, 163.6, 156.4, 154.0, 153.9, 153.7, 153.2, 150.6, 150.5, 136.7, 135.3, 134.7, 133.9, 133.7, 128.6, 128.6, 128.3, 128.2, 128.0, 127.9, 127.7, 125.8, 116.4, 115.5, 115.4, 112.7, 112.4, 112.2, 81.9, 68.8, 66.3, 60.2, 55.5, 50.4, 48.4, 47.5, 45.6, 41.0, 30.0, 29.8, 29.3, 29.2, 28.5, 28.2, 28.1, 28.0, 26.9, 26.6, 20.8, 14.1 ppm. **MS (ES)** *m*/*z* = 778.3 [M + Na]⁺, 756.3 [M + H]⁺.

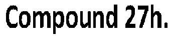

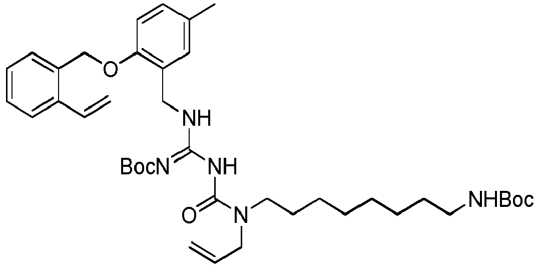

**Compound 27h** was prepared by the procedure described for **compound 27f** starting from compounds **25h** and **26.** Y**ield:** 76%. **¹H-NMR** (400 MHz, CDCl₃): δ 12.30 (s, 1H), 8.40 (bs, 1H), 7.51 (d, *J* = 7.2 Hz, 1H), 7.43 (d, *J* = 7.2 Hz, 1H), 7.31-7.22 (m, 2H), 7.06 (s, 1H), 7.01-6.93 (m, 2H), 6.81 (d, *J* = 7.6 Hz, 1H), 5.83-5.70 (m, 1H), 5.65 (d, *J* = 17.6 Hz, 1H), 5.31 (d, *J* = 10.8 Hz, 1H), 5.10-4.99 (m, 6H), 4.51 (dd, *J* = 5.2, 16.4 Hz, 2H), 4.08 (d, *J* = 4 Hz, 1H), 3.90 (d, *J* = 5.6 Hz, 1H), 3.37 (t, *J* = 3.2 Hz, 1H), 3.21 (t, *J* = 4 Hz, 1H), 3.10-2.99 (m, 2H), 2.25 (s, 3H), 1.53-1.48 (m, 4H), 1.42 (s, 9H), 1.41 (s, 9H), 1.27-1.21 (m, 8H) ppm. **¹³C-NMR** (100 MHz, CDCl₃): δ 203.1, 155.9, 154.4, 154.0, 153.2, 136.8, 135.3, 134.7, 133.7, 130.2, 130.0, 129.8, 128.7, 128.2, 127.7, 126.5, 125.9, 116.5, 115.4, 111.5, 81.8, 68.2, 50.4, 48.4, 47.5, 45.6, 40.0, 29.9, 29.3, 29.2, 28.5, 28.4, 28.0, 26.9, 26.7, 20.4 ppm.

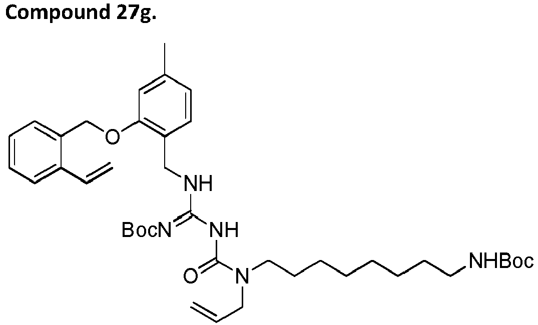

**Compound 27g** was prepared by the procedure described for **compound 27f** starting from compounds **25g** and **26. Yield:** 72%. **¹H-NMR** (400 MHz, CDCl₃): δ 12.28 (d, *J* = 8.8 Hz, 1H), 8.37 (s, 1H), 7.52 (d, *J* = 7.6 Hz, 1H), 7.45 (d, *J* = 7.2 Hz, 1H), 7.33-7.24 (m, 2H), 7.14 (d, *J* = 7.2 Hz, 1H), 7.01-6.94 (m, 1H), 6.77-6.72 (m, 2H), 5.83-5.69 (m, 1H), 5.67 (d, *J* = 17.6 Hz, 1H), 5.32 (d, *J* = 10.8 Hz, 1H), 5.28-5.09 (m, 6H), 4.50 (dd, *J* = 5.6, 14.4 Hz, 2H), 4.07(d, *J* = 5.2 Hz, 1H), 3.90 (d, *J* = 5.2 Hz, 1H), 3.37 (t, *J* = 7.2 Hz, 1H), 3.21 (d, *J* = 7.2 Hz, 1H), 3.10-3.03 (m, 2H), 2.32 (s, 3H), 1.61-1.57 (m, 4H), 1.42 (s, 9H), 1.41 (s, 9H), 1.27-1.22 (m, 8H) ppm. **¹³C-NMR** (100 MHz, CDCl₃): δ 161.0, 157.3, 157.0, 154.3, 148.4, 139.4, 138.0, 135.5, 134.8, 132.0, 129.5, 128.1, 128.0, 127.9, 126.6, 125.7, 124.9, 117.0, 116.6, 113.5, 82.5, 79.8, 68.7, 48.6, 45.3, 42.1, 40.4, 29.6, 28.8, 28.6, 28.5, 28.4, 28.0, 27.2, 27.0, 21.3 ppm.

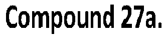

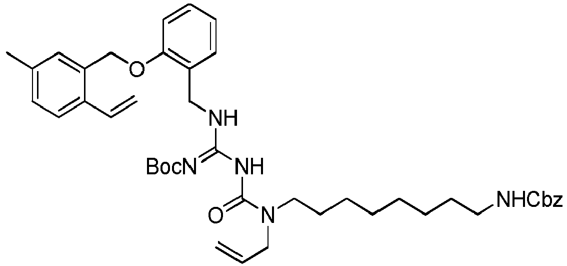

**Compound 27a** was prepared by the procedure described for **compound 27f** starting from compounds **25a** and **10z. Yield:** 78%. **¹H-NMR** (400 MHz, CDCl₃): δ 12.31 (d, *J* = 10.0 Hz, 1H), 8.40 (bs, 1H), 7.43 (d, *J* = 7.6 Hz, 1H), 7.34-7.32 (m, 5H), 7.29-7.20 (m, 3H), 7.11 (d, *J* = 7.6 Hz, 1H), 6.96-6.89 (m, 3H), 5.86-5.66 (m, 1H), 5.61 (d, *J* = 17.6 Hz, 1H), 5.25 (d, *J* = 10.8, 1H), 5.12-5.07 (m, 5H), 4.98 (d, *J* = 10.4 Hz, 1H), 4.55 (dd, *J* = 5.6, 15.2 Hz, 2H), 4.05 (d, *J* = 4.8 Hz, 1H), 3.90 (d, *J* = 4.8 Hz, 1H), 3.36 (t, *J* = 7.2 Hz, 1H),3.21 (t, *J* = 7.6 Hz, 1H), 3.14-3.10 (m, 2H), 2.32 (s, 3H), 1.41 (s, 9H), 1.26-1.18 (m, 12H) ppm. **¹³C-NMR** (100 MHz, CDCl₃): δ 163.8, 163.7, 156.5, 156.3, 154.0, 153.9, 153.2, 137.5, 136.7, 135.3, 134.7, 134.1, 133.6, 133.4, 129.4, 129.3, 129.0, 128.9, 128.4, 128, 126.8, 125.9, 120.7, 115.6, 115.4, 111.5, 81.8, 68.2, 66.4, 50.4, 48.4, 47.5, 45.6, 41.0, 40.0, 29.8, 29.3, 29.2, 28.5, 28.1, 28, 26.9, 26.8, 26.6, 20.9 ppm. **MS (ES)** *m*/*z* = 340.5 [M+2H]⁺.

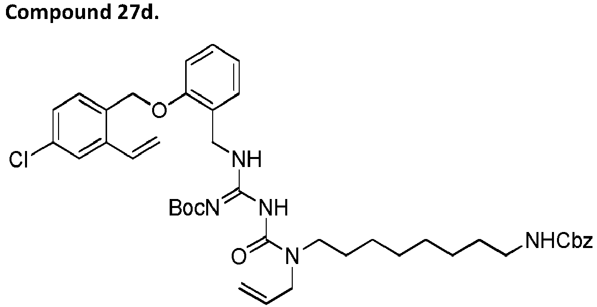

**Compound 27d** was prepared by the procedure described for **compound 27f** starting from compounds **25d** and **10z. Yield:** 66%. **¹H-NMR** (400 MHz, CDCl₃): δ 12.34 (d, *J* = 10 Hz, 1H), 8.43 (bs, 1H), 7.51 (d, *J* = 1.6 Hz, 1H), 7.41-7.34 (m, 5H), 7.34-7.25 (m, 4H), 6.95-6.88 (m, 3H), 5.84-5.74 (m, 1H), 5.69 (d, *J* = 17.2 Hz, 1H), 5.39 (d, *J* = 11.2 Hz, 1H), 5.14-5.01 (m, 6H), 4.56 (dd, *J* = 5.6, 15.6 Hz, 2H), 4.08 (d, *J* = 5.2 Hz, 1H), 3.92 (d, *J* = 5.2 Hz, 1H), 3.39 (t, *J* = 7.2 Hz, 1H), 3.24 (t, *J* = 7.6 Hz, 1H), 3.17-3.12 (m, 2H), 1.51-1.49 (m, 4H), 1.43 (s, 9H), 1.29-1.20 (m, 8H) ppm. **¹³C-NMR** (100 MHz, CDCl₃): δ 163.7, 156.3, 153.8, 153.3, 138.5, 135.3, 134.7, 134.2, 132.6, 132.1, 130.1, 129.4, 129.2, 128.6, 128.4, 128.0, 127.7, 126.7, 125.9, 120.9, 117.9, 115.5, 115.5, 111.4, 81.9, 67.4, 66.4, 50.4, 48.4, 47.5, 45.6, 41.0, 40.0, 29.8, 29.3, 29.2, 28.5, 28.1, 28.0, 27.0, 26.6 ppm. **MS (ES)** *m*/*z* = 760.4 [M+H]⁺.

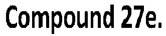

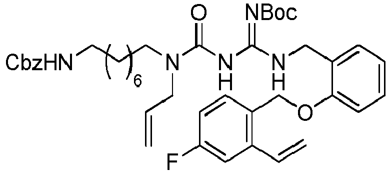

**Compound 27e** was prepared by the procedure described for **compound 27f** starting from compounds **25e** and **10z. Yield** 83%. **¹H-NMR** (400 MHz, CDCl₃): δ 12.31 (d, *J* = 9.6 Hz, 1H), 8.44 - 8.38 (m, 1H), 7.42 (dd, *J* = 8.1, 6.1 Hz, 1H), 7.34 (d, *J* = 4.2 Hz, 2H), 7.32 - 7.20 (m, 4H), 6.99 - 6.89 (m, 4H), 5.87 - 5.72 (m, 1H), 5.69 (d, *J* = 17.4 Hz, 1H), 5.38 (d, *J* = 11.0 Hz, 1H), 5.16 - 4.99 (m, 6H), 4.54 (dd, *J* = 14.6, 5.6 Hz, 2H), 4.07 (d, *J* = 5.2 Hz, 1H), 3.91 (d, *J* = 4.9 Hz, 1H), 3.38 (t, *J* = 7.4 Hz, 2H), 3.26 - 3.20 (m, 2H), 3.20 - 3.10 (m, 2H), 1.48 (d, *J* = 8.2 Hz, 4H), 1.43 (s, 13H), 1.28 (s, 6H) ppm. **¹³C-NMR** (100 MHz, CDCl₃): δ 163.98, 156.34, 153.99, 153.25, 139.15, 139.08, 138.03, 136.62, 135.30, 134.67, 132.83, 130.84, 129.45, 129.14, 128.51, 128.40, 127.97, 126.76, 120.84, 117.67, 115.48, 115.43, 114.57, 114.36, 112.64, 112.42, 111.42, 81.89, 67.43, 66.45, 50.41, 48.43, 47.48, 45.64, 41.00, 39.99, 29.83, 29.29, 29.12, 28.49, 28.07, 27.98, 26.93, 26.59 ppm. **MS (ES)** *m*/*z* = 744.4 [M+H]⁺.

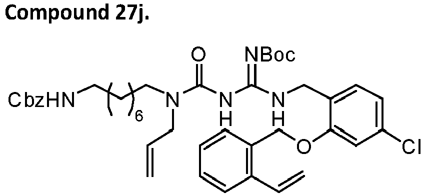

**Compound 27j** was prepared by the procedure described for **compound 27f** starting from compounds **25j** and **10z. Yield** 52%. **¹H-NMR** (400 MHz, CDCl₃): δ 12.29 (d, *J* = 13.0 Hz, 1H), 8.40 (s, 1H), 7.53 (d, *J* = 7.6 Hz, 1H), 7.42 (d, *J* = 7.4 Hz, 1H), 7.34-7.33 (m, 5H), 7.31 - 7.27 (m, 2H), 7.17 (dd, *J* = 7.5, 4.0 Hz, 1H), 6.96 (d, *J* = 11.0 Hz, 1H), 6.93 - 6.88 (m, 2H), 5.78 (m, 1H), 5.67 (d, *J* = 17.3 Hz, 1H), 5.34 (d, *J* = 11.0 Hz, 1H), 5.09 (s, 2H), 5.07 (s, 2H), 5.04 - 4.96 (m, 1H), 4.73 (br, 1H), 4.50 (d, *J* = 5.5 Hz, 1H), 4.47 (d, *J* = 5.5 Hz, 1H), 4.02 (d, *J* = 4.7 Hz, 1H), 3.89 (d, *J* = 4.3 Hz, 1H), 3.32 (t, *J* = 7.2 Hz, 1H), 3.20 (t, *J* = 7.2 Hz, 1H), 3.14 (m, 2H), 1.49 (m, 4H), 1.42 (s, 9H), 1.31 - 1.12 (m, 8H). **¹³C-NMR** (100 MHz, CDCl₃): δ 163.6, 156.8, 153.9, 153.3, 137.0, 134.6, 133.6, 132.9, 130.0, 129.4, 128.8, 128.6, 128.4, 128.1, 128.0, 127.8, 126.1, 125.5, 120.7, 116.9, 115.5, 115.4, 112.2, 82.1, 68.4, 66.5, 60.3,48.4,41.0, 39.4, 29.3, 29.2, 29.4, 28.0, 26.9, 26.6, 23.8. ppm. **MS (ES)** *m*/*z* = 781.3 [M + Na]⁺, 759.3 [M + H]⁺.

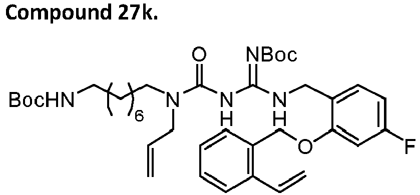

**Compound 27k** was prepared by the procedure described for **compound 27f** starting from compounds **25k** and **26. Yield** 59%. **¹H NMR** (400 MHz, CDCl₃): δ 12.30 (d, *J* = 7.4 Hz, 1H), 8.4 (s, 1H), 7.52 (d, *J* = 8 Hz, 1H), 7.41 (d, *J* = 5.4 Hz, 1H), 7.34 (d, *J* = 7.1 Hz, 1H), 7.27 (t, *J* = 10.5 Hz, 1H), 6.99-6.83 (m, 4H), 5.66 (d, *J* = 12,2 Hz, 1H), 5.33 (d, *J* = 8 Hz, 1H), 5.9 (s, 2H), 5.03-4.98 (m, 2H), 4.51 (dd, *J* = 17.4, 3.2 Hz, 2H), 4.00 (dd, *J* = 13.4, 1.8 Hz, 2H), 3.2 (dd, *J* = 17.4, 2.2 Hz, 2H), 3.06 (s, 2 H), 1.50 (s, 9H), 1.48 (s, 9H), 1.40-1.32 (m, 8H) ppm. **¹³C NMR** (100 MHz, CDCl₃): δ 164.04, 161.63, 153.83, 153.28, 136.95, 135.24, 134.63, 133.58, 132.88, 130.10, 128.68, 128.50, 127.79, 126.09, 122.54, 120.26, 116.92, 115.41, 106.8, 100.09, 99.83, 81.96, 68.38, 50.39, 48.43, 47.50, 45.66, 40.80, 40.54, 39.44, 29.95, 29.1, 28.35, 28.00, 26.94, 26.67, 23.76 ppm.

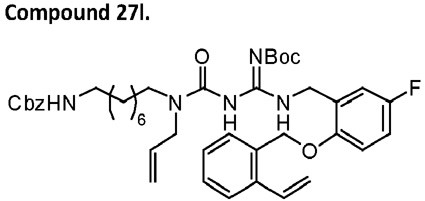

**Compound 27l** was prepared by the procedure described for **compound 27f** starting from compounds **25l** and **10z. Yield** 75%. **¹H NMR** (400 MHz, CDCl₃): δ 12.28 (s, 1Hz), 8.41 (s, 1H), 7.52 (d, *J* = 7.5 Hz, 1H), 7.44 (d, *J* = 7.4 Hz, 1H), 7.35 (m, 5H), 7.32 (d, *J* = 6.9 Hz, 1H), 7.29 (d, *J* = 7.3 Hz, 2H), 7.27-7.21 (m, 1H), 7.04-6.98 (m, 1H), 6.94 (d, *J* = 9.7 Hz, 2H), 5.85-5.69 (m, 1H), 5.66 (d, *J* = 16.4 Hz, 1H), 5.32 (d, *J* = 10.8 Hz, 2H), 5.15 (s, 2H), 5.09 (m, 3H), 5.01 (d, *J* = 9.9 Hz, 1H), 4.55 (dd, *J* = 14.8, 6.0 Hz, 2H), 4.05 (d, *J* = 5.3 Hz, 1H), 3.89 (d, *J* = 5.3 Hz, 1H), 3.36 (t, *J* = 7.6 Hz, 1H), 3.21 (t, *J* = 8.0 Hz, 1H), 3.16-3.11 (m, 2H), 1.51 (m, 4H), 1.42 (s, 9H), 1.27-1.15 (m, 8H) ppm. **¹³C NMR** (100 MHz, CDCl₃): δ 163.68, 156.46, 154.06, 153.88, 153.27, 135.33, 134.73, 133.74, 129.30, 128.97, 128.70, 128.64, 128.44, 128.28, 128.02, 127.77, 126.80, 125.95, 120.73, 116.65, 115.47, 111.51, 81.88, 68.07, 66.48, 50.46 48.45, 47.53, 45.66, 41.04, 39.99, 29.88, 29.35, 29.21, 28.53, 28.04, 26.98, 26.91, 26.64 ppm.

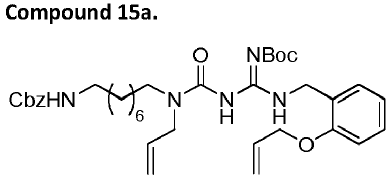

**Compound 15a** was prepared by the procedure described for **compound 27f** starting from compounds **14a** and **10z. Yield** 68%. **¹H-NMR** (400 MHz, CDCl₃): δ 12.29 (s, 1H), 8.50 (s, 1H), 7.26-7.21 (m, 5H), 7.18-7.12 (m, 2H), 6.88 (t, *J* = 7.6 Hz, 1H), 6.76 (d, 1H, *J* = 8.4 Hz), 6.07 (m, 1H), 5.77 (m, 1H), 5.39 (d, 1H, *J* = 17.6 Hz), 5.26 (d, 1H, *J* = 11.2 Hz), 5.13-5.02 (m, 4H), 4.57 (d, 2H, *J* = 5.6 Hz), 4.54 (d, 2H, *J* = 5.6 Hz), 4.09 (d, 1H, *J* = 5.6 Hz), 3.91 (d, 1H, *J* = 5.2 Hz), 3.41 (t, 1H, *J* = 7.2 Hz), 3.22 (t, 1H, *J* = 7.2 Hz), 3.15 (t, 2H, *J* = 7.2 Hz), 1.56 (m, 4H), 1.43 (s, 9H), 1.21-1.16 (m, 8H) ppm. **¹³C-NMR** (100 MHz, CDCl₃): δ 163.6, 156.3, 153.9, 153.1, 136.7, 135.3, 134.6, 129.2, 128.8, 128.2, 128.0, 127.8, 127.0, 120.4, 117.1, 115.4, 111.4, 81.7, 68.6, 66.1, 50.3, 48.3, 40.9, 40.2, 29.8, 29.2, 28.5, 27.9, 27.8, 26.8, 26.5 ppm. **MS (ES)** *m*/*z* = 672.3.0 [M + Na]⁺, 688.2 [M + K]⁺, 650.3 [M + H]⁺.

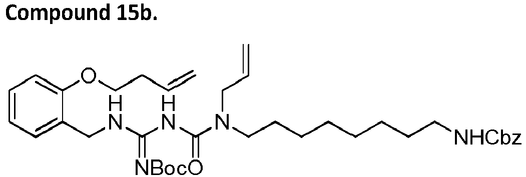

**Compound 15b** was prepared by the procedure described for **compound 27f** starting from compounds **14b** and **10z. Yield**: 83%. **¹H-NMR** (400 MHz, CDCl₃): δ 12.28 (d, *J* = 12 Hz, 1H), 8.48 (q, *J* = 8 Hz, 1H), 7.31-7.27 (m, 5H), 7.21-7.15 (m, 2H), 6.86-6.79 (m, 2H), 5.92-5.85 (m, 1H), 5.81-5.73 (m, 1H), 5.16-5.00 (m, 6H), 4.50 (dd, *J* = 8, 12 Hz, 2H), 4.08 (d, *J* = 4 Hz, 1H), 4.00 (t, *J* = 8 Hz, 2H), 3.88 (d, *J* = 4 Hz, 1H), 3.40 (t, *J* = 4 Hz, 1H), 3.20 (t, *J* = 4 Hz, 1H), 3.13-3.08 (m, 2H), 2.58-2.53 (m, 2H), 1.51-1.47 (m, 4H), 1.40 (s, 9H), 1.24-1.16 (m, 8H) ppm. **¹³C NMR** (100 MHz, CDCl₃): δ 163.7, 156.7, 156.3, 153.9, 153.2, 136.7, 135.3, 134.7, 129.3, 129.0, 128.6, 128.5, 128.3, 126.7, 120.2, 116.9, 115.4, 110.9, 81.7, 67.0, 66.3, 50.4, 48.4, 40.9, 40.3, 33.5, 29.8, 29.4, 29.3, 28.5, 27.9, 26.9, 26.6 ppm. **MS (ES)** *m*/*z* = 663.9 [M+H]⁺, 685.9 [M+Na]⁺.

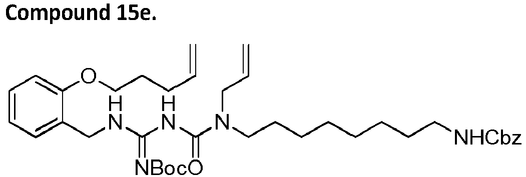

**Compound 15e** was prepared by the procedure described for **compound 27f** starting from compounds **14c** and **10z. Yield:** quantitative. **¹H-NMR** (400 MHz, CDCl₃): δ 12.30 (d, *J* = 10 Hz, 1H), 8.50 (bs, 1H), 7.30-7.23 (m, 5H), 7.19 (t, *J* = 6.4 Hz, 2H), 6.85-6.79 (m, 2H), 5.87-70 (m, 2H), 5.10-4.94 (m, 6H), 4.51 (dd, *J* = 5.2 and 14.4 Hz, 2H), 4.09 (d, *J* = 2.8 Hz, 1H), 3.96 (t, *J* = 6 Hz, 2H), 3.90 (d, *J* = 2 Hz, 1H), 3.41 (t, *J* = 2 Hz, 1H), 3.21 (t, *J* = 1.6 Hz, 1H), 3.15-3.06 (m, 2H), 2.23 (q, *J* = 2.4 Hz, 2H), 1.93-1.88 (m, 2H), 1.59-1.45 (m, 4H), 1.42 (s, 9H), 1.15-1.32 (m, 8H) ppm. **¹³C NMR** (100 MHz, CDCl₃): δ 163.8, 157.0, 156.3, 154.0, 153.8, 153.2, 137.7, 136.7, 135.3, 134.7, 129.4, 128.8, 128.5, 128.2, 127.9, 126.3, 120.0, 115.4, 110.9, 81.8, 67.0, 66.4, 50.4, 48.4, 47.5, 45.6, 41.0, 40.4, 30.1, 29.9, 28.1, 27.9, 27.7, 27.0, 26.6 ppm. **MS (ES)** *m*/*z* = 678.3 [M+H]⁺, 700.3 [M+Na]⁺, 716.2 [M+K]⁺.

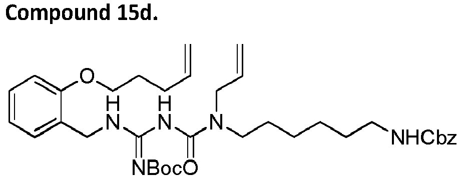

**Compound 15d** was prepared by the procedure described for **compound 27f** starting from compounds **14c** and **10y. Yield:** 98%. **¹H-NMR** (400 MHz, CDCl₃): δ 12.30 (d, *J* = 18.4 Hz, 1H), 8.52 (bs, 1H), 7.33-7.28 (m, 5H), 7.21-7.18 (m, 2H), 6.87-6.82 (m, 2H), 5.91-5.72 (m, 2H), 5.12-4.96 (m, 6H), 4.53 (dd, *J* = 5.6 and 12 Hz, 2H), 4.11 (d, *J* = 3.6 Hz, 1H), 3.99 (t, *J* = 6.4 Hz, 2H), 3.90 (d, *J* = 4.8 Hz, 1H), 3.42 (t, *J* = 6.8 Hz, 1H), 3.23 (t, *J* = 7.2 Hz, 1H), 3.18-3.08 (m, 2H), 2.25 (q, *J* = 6.8 Hz, 2H), 1.93 (q, *J* = 6.8 Hz, 2H), 1.55-1.45 (m, 4H), 1.42 (s, 9H), 1.36-1.25 (m, 4H) ppm. **¹³C NMR** (100 MHz, CDCl₃): δ 163.8, 157.0, 153.8, 153.2, 151.3, 137.8, 136.6, 135.3, 134.7, 129.4, 128.9, 128.4, 126.3, 120.1, 115.5, 115.1, 111.0, 81.9, 67.1, 66.4, 60.3, 50.4, 47.4, 45.5, 41.0, 40.3, 30.1, 28.5, 28.3, 28.0, 26.5, 20.9, 14.1 ppm.

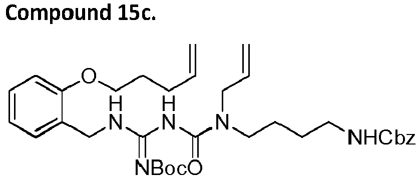

**Compound 15c** was prepared by the procedure described for **compound 27f** starting from compounds **14c** and **10x. Yield:** 77%. **¹H-NMR** (400 MHz, CDCl₃): δ 12.24 (d, *J* = 26.8 Hz, 1H), 8.51 (bs, 1H), 7.31-7.27 (m, 5H), 7.22-7.16 (m, 2H), 6.85-6.78 (m, 2H), 5.79-5.74 (m, 2H), 5.09-4.94 (m, 6H), 4.50 (t, *J* = 6 Hz, 2H), 4.08 (d, *J* = 5.2 Hz, 1H), 3.961 (961 (q, *J* = 6 Hz, 2H), 3.86 (d, *J* = 5.2 Hz, 1H), 3.41 (t, *J* = 6.8 Hz, 1H), 3.23 (t, *J* = 7.2 Hz, 1H), 3.17 (d, *J* = 6 Hz, 1H), 3.05 (d, *J* = 6 Hz, 1H), 2.29-2.15 (m, 2H), 1.44-1.40 (m, 4H), 1.39 (s, 9H) ppm. **¹³C-NMR** (100 MHz, CDCl₃): δ 163.8, 156.9, 154.1, 153.2, 137.8, 135.2, 134.5, 129.4, 128.4, 127.9, 126.2, 120.1, 115.6, 115.1, 110.9, 81.9, 67.0, 66.4, 60.3, 50.4, 48.4, 46.9, 45.0, 40.8, 40.4, 30.1, 28.3, 28.0, 27.3, 25.8, 25.4, 20.9, 14.1 ppm.

### General procedure for the synthesis of macrocycle.

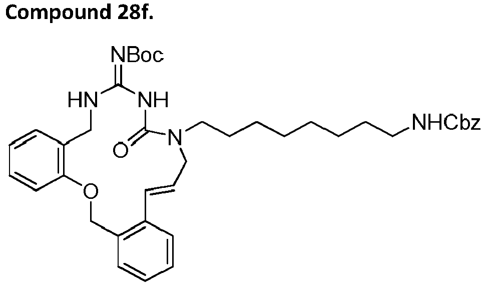

To a stirred solution of amidinourea derivative **27f** (247 mg, 0,34 mmol) in degassed DCM (170 mL, 2 mM solution), a solution of 2^{nd} generation Grubb's catalyst (Sigma-Aldrich S.r.l., 569747) (58 mg, 0,2 mmol) in degassed DCM (2 mL) was added dropwise under N₂ atmosphere. The mixture was heated at reflux and stirred overnight. After cooling solvent was evaporated under reduced pressure. The crude residue was purified with flash chromatography on silica gel, eluting with 15% AcOEt/Hex to give the macrocycle compound as mixture of E/Z isomers. **Yield** 75%. **¹H-NMR** (400 MHz, CDCl₃): δ 12.27 (s, 1H), 8.40 (s, 1H), 7.58 (d, 1H, *J* = 8.0 Hz), 7.34 (m, 5H), 7.30 (d, 2H, *J* = 7.1 Hz), 7.23 (m, 3H), 6.98 (d, 1H, *J* = 7.6 Hz), 6.93 (t, 1H, *J* = 7.6 Hz), 6.58 (d, 1H, *J* = 15.6 Hz), 6.16 (dt, 1H, *J* = 15.6, 5.2 Hz), 5.15 (s, 2H), 5.08 (s, 2H), 4.69 (bs, 1H), 4.61 (d, 2H, *J* = 6.4 Hz), 3.92 (d, 2H, *J* = 5.2 Hz), 3.35 (t, 2H, *J* = 7.6 Hz), 3.16 (q, 2H, *J* = 6.4 Hz), 1.57 (s, 4H), 1.46 (m, 9H), 1.30-1.15 (s, 6H) ppm. **¹³C-NMR** (100 MHz, CDCl₃): δ 163.7, 155.3, 154.2, 153.6, 138.1, 137.8, 136.9, 129.6, 129.4, 129.1, 128.8, 128.7, 128.3, 126.5, 122.9, 118.6, 99.9, 82.2, 72.2, 66.5, 45.1, 44.1, 43.4, 40.9, 31.1, 29.3, 29.2, 28.5, 27.3 ppm. **MS (ES)** *m*/*z* = 698.2 [M+H]⁺.

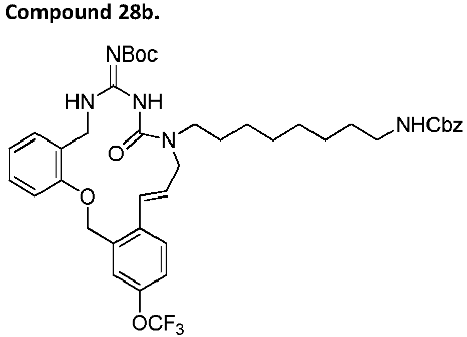

**Compound 28b** was prepared by the procedure described for **compound 28f** starting from compound **27b. Yield:** 36%. **¹H NMR** (400 MHz, CDCl₃): δ 12.25 (s, 1H), 8.40 (m, 1H), 7.56 (d, 1H, *J* = 8.4 Hz), 7.32 (m, 5H), 7.24 (m, 1H), 7.19 (m, 2H), 7.14 (m, 1H), 6.95 (m, 2H), 6.52 (d, 1H, *J* = 15.6 Hz), 6.13 (dt, 1H, *J* = 15.6 and 5.6 Hz), 5.12 (s, 2H), 5.07 (s, 2H), 4.70 (bs, 1H), 4.58 (d, 2H, *J* = 6.4 Hz), 3.90 (m, 2H), 3.33 (t, 2H, *J* = 7.6 Hz), 3.15 (m, 2H), 1.53 (m, 4H), 1.46 (s, 9H), 1.24 (m, 8H) ppm. **¹³C-NMR** (100 MHz, CDCl₃): δ 160.0, 157.0, 156.7, 154.3, 150.8, 150.7, 150.6, 150.5, 148.4, 137.0, 134.8, 134.1, 130.4, 130.0, 128.6, 128.4, 128.1, 128.1, 127.8, 126.8, 125.4, 123.9, 121.7, 121.3, 119.6, 117.6, 117.6, 117.4, 115.6, 115.6, 115.6, 113.3, 82.2, 68.7, 66.0, 46.9, 45.8, 40.7, 40.2, 29.8, 28.6, 28.5, 28.1, 28.0, 27.2, 26.9. **MS (ES)** *m*/*z* = 782.5 [M+H]⁺.

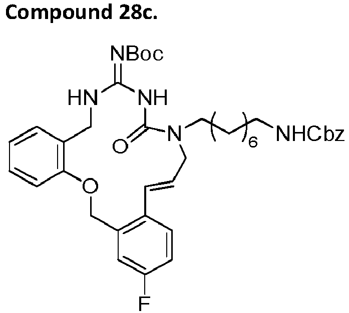

**Compound 28c** was prepared by the procedure described for **compound 28f** starting from compound 27c. Yield: 80%. **¹H NMR** (400 MHz, CDCl₃): δ 12.30 (s, 1H), 8.41 (t, *J* = 6.2 Hz, 1H), 7.53 (dd, *J* = 8.5, 5.6 Hz, 1H), 7.33 (d, *J* = 4.3 Hz, 3H), 7.31 - 7.18 (m, 3H), 7.08 - 6.91 (m, 5H), 6.52 (d, *J* = 15.6 Hz, 1H), 6.08 (dt, *J* = 15.6, 5.7 Hz, 1H), 5.09 (d, *J* = 5.2 Hz, 4H), 4.85 (s, 1H), 4.60 (d, *J* = 6.2 Hz, 2H), 3.91 (d, *J* = 5.0 Hz, 2H), 3.39 - 3.31 (m, 2H), 3.16 (d, *J* = 6.3 Hz, 2H), 1.56 (s, 3H), 1.49 (s, 2H), 1.47 (s, 12H), 1.31 (s, 10H) ppm. **¹³C NMR** (100 MHz, CDCl₃): δ 164.0, 162.6, 160.1, 156.3, 155.4, 154.1, 153.5, 136.7, 135.6, 135.5, 133.2, 133.1, 128.4, 128.0, 127.9, 127.7, 127.6, 127.2, 127.1, 126.3, 126.0, 121.1, 117.2, 117.0, 115.7, 115.5, 112.2, 82.2, 69.8, 66.4, 60.3, 51.8, 48.0, 41.0, 39.3, 29.8, 29.6, 29.3, 29.1, 28.7, 28.0, 26.9, 26.6 ppm. **MS (ES)** *m*/*z* = 716.4 [M+H]⁺.

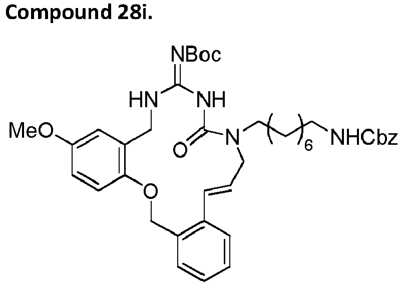

**Compound 28i** was prepared by the procedure described for **compound 28f** starting from compound **27i. Yield** 68%. **¹H-NMR** (400 MHz, CDCl₃): δ 12.24 (s, 1H), 8.35 (t, *J* = 6.0, 6.0 Hz, 1H), 7.56 (d, *J* = 8.0 Hz, 1H), 7.33-7.19 (m, 7H), 6.89 (d, *J* = 8.8 Hz, 1H), 6.79 (d, *J* = 2.8 Hz, 1H), 6.71 (dd, *J* = 8.4, 2.8 Hz, 1H), 6.58 (d, *J* = 15.6 Hz, 1H), 6.15 (dt, *J* = 15.6, 5.6 Hz, 1H), 5.07 (s, 3H), 4.78 (s, 1H), 4.57 (d, *J* = 6.4 Hz, 2H), 3.92 (d, *J* = 5.6 Hz, 2H), 3.74 (s, 4H), 3.34 (m, 2H), 3.15 (m, 3H), 1.55 (m, 2H), 1.45 (m, 8H), 1.30 (s, 9H) ppm. **¹³C-NMR** (100 MHz, CDCl₃): δ 164.0, 156.3, 154.0, 153.5, 149.9, 136.9, 136.6, 133.9, 130.5, 129.2, 128.8, 128.6, 128.4, 128.0,127.2, 126.9, 125.4,113.6, 113.0, 111.2, 82.2, 71.3, 66.4, 55.6, 52.0, 48.0,41.0, 39.4, 29.8, 29.3, 29.2, 28.7, 28.0, 26.9, 26.6 ppm. **MS (ES)** *m*/*z* = 750.0 [M + Na]+, 728.1 [M + H]⁺.

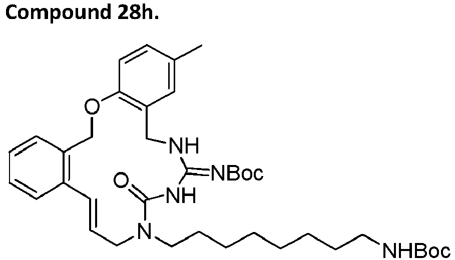

**Compound 28h** was prepared by the procedure described for **compound 28f** starting from compound **27h.**

**Yield:** 31%. **¹H-NMR** (400 MHz, CDCl₃): δ 12.26 (s, 1H), 8.35 (bs, 1H), 7.56 (d, *J* = 7.6 Hz, 1H), 7.33-7.27 (m, 2H), 7.19 (t, *J* = 7.6 Hz, 1H), 7.00-6.97 (m, 2H), 6.87 (d, *J* = 8 Hz, 1H), 6.61 (d, *J* = 16.5 Hz, 1H), 6.18-6.11 (m, 1H), 5.10 (s, 2H), 4.57 (d, *J* = 6.4 Hz, 2H), 4.00-3.92 (m, 2H), 3.35 (t, *J* = 7.2 Hz, 2H), 3.12-3.05 (m, 2H), 2.26 (s, 3H), 1.61-1.53 (m, 4H), 1.46 (s, 9H), 1.42 (s, 9H), 1.30-1.24 (m, 8H) ppm. **¹³C-NMR** (100 MHz, CDCl₃): δ 164.1, 154.0, 153.6, 137.0, 133.8, 130.4, 130.2, 128.8, 128.5, 127.8, 127.4, 127.2, 127.1, 126.8, 125.4, 112.5, 82.2, 78.9, 70.6, 68.5, 51.9, 48.0, 40.5, 39.4, 30.0, 29.6, 29.4, 29.2, 28.7, 28.4, 28.0, 27.0, 26.7, 20.9 ppm.

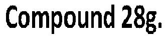

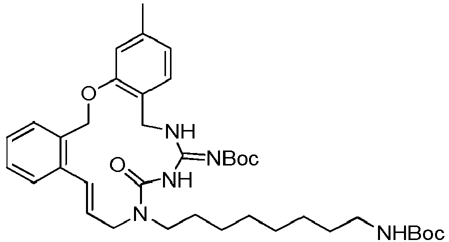

**Compound 28g** was prepared by the procedure described for **compound 28f** starting from compound **27g. Yield:** 41%. **¹H-NMR** (400 MHz, CDCl₃): δ 12.22 (s, 1H), 8.38 (bs, 1H), 7.65 (d, *J* = 6.4 Hz, 1H), 7.32-7.28 (m, 2H), 7.20 (t, *J* = 7.2 Hz, 1H), 7.06 (d, *J* = 7.6 Hz, 1H), 6.80 (s, 1H), 6.74 (d, *J* = 7.6 Hz, 1H), 6.61 (d, *J* = 15.2 Hz, 1H), 6.18-6.11 (m, 1H), 5.13 (s, 2H), 4.55 (d, *J* = 6.4 Hz, 1H), 4.01-3.92 (m, 2H), 3.35 (t, *J* = 7.2 Hz, 2H), 3.12-3.06 (m, 2H), 2.33 (s, 3H), 1.59-1.52 (m, 4H), 1.46 (s, 9H), 1.42 (s, 9H), 1.30-1.20 (m, 8H) ppm. **¹³C-NMR** (100 MHz, CDCl₃): δ 160.0, 157.6, 157.3, 154.3, 148.4, 139.1, 138.7, 136.5, 130.4, 130.3, 129.3, 128.2, 128.0, 127.5, 127.4, 125.4, 125.0, 113.5, 82.5, 79.8, 69.1, 46.9, 45.4, 40.7, 40.4, 29.7, 28.6, 28.5, 28.4, 28.1, 28.0, 27.2, 26.9, 21.4 ppm.

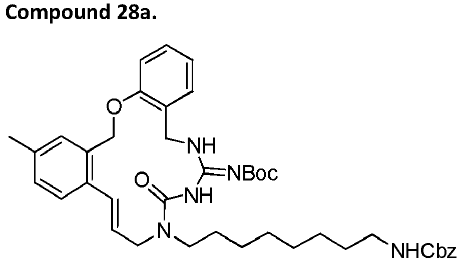

**Compound 28a** was prepared by the procedure described for **compound 28f** starting from compound **27a. Yield:** 69%. **¹H-NMR** (400 MHz, CDCl₃): δ 12.24 (s, 1H), 8.35 (t, *J* = 6 Hz, 1H), 7.44 (d, *J* = 8 Hz, 1H), 7.31-7.26 (m, 5H), 7.22-7.16 (m, 2H), 7.12-7.07 (m, 2H), 6.96-6.89 (m, 2H), 6.49 (d, *J* = 15.6 Hz, 1H), 6.09-6.05 (m, 1H), 5.08 (s, 2H), 5.05 (s, 2H), 4.70 (bs, 1H), 4.57 (d, *J* = 6.4 Hz, 2H), 3.86 (d, *J* = 5.6 Hz, 2H), 3.31 (t, *J* = 7.2 Hz, 2H), 3.13 (q, *J* = 6.4 Hz, 2H), 2.31 (s, 3H), 1.43 (s, 9H), 1.27-1.23 (m, 12H) ppm. **¹³C-NMR** (100 MHz, CDCl₃): δ 171.0, 164.1, 156.4, 155.7, 154.0, 153.5, 136.7, 136.6, 134.1, 133.6, 131.0, 130.6, 129.5, 128.9, 128.4, 127.9, 127.8, 127.5, 127.1, 126.5, 125.5, 125.2, 124.9, 121.2, 82.1, 70.6, 66.6, 66.4, 52.0, 49.5, 48.0, 41.0, 39.3, 29.8, 29.6, 29.3, 29.2, 28.8, 28.7, 28.2, 27.9, 27.7, 26.9, 26.6, 26.3, 25.5, 21.0, 20.9 ppm. **MS (ES)** *m*/*z* = 711.9 [M+H]⁺.

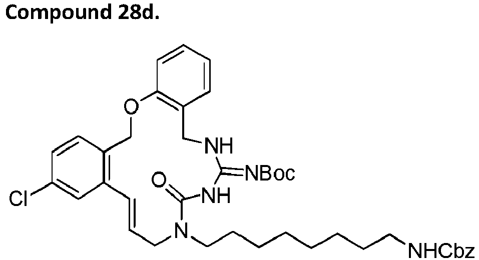

**Compound 28d** was prepared by the procedure described for **compound 28f** starting from compound **27d. Yield:** 57%. **¹H-NMR** (400 MHz, CDCl₃): δ 12.27 (s, 1H), 12.11 (s, 1H), 8.41 (d, *J* = 5.3 Hz, 2H), 7.53 (s, 1H), 7.34 (s, 4H), 7.32-7.13 (m, 9H), 6.96 (t, *J* = 8.2 Hz, 4H), 6.52 (d, *J* = 15.6 Hz, 1H), 6.25-6.10 (m, 2H), 5.11 (s, 2H), 5.08 (s, 4H), 4.75 (s, 1H), 4.64 (d, *J* = 5.6 Hz, 1H), 4.58 (d, *J* = 5.8 Hz, 3H), 3.93 (d, *J* = 3.8 Hz, 2H), 3.48-3.40 (m, 1H), 3.39-3.31 (m, 2H), 3.29-3.22 (m, 1H), 3.17 (d, *J* = 5.3 Hz, 4H), 1.56 (s, 2H), 1.49 (s, 6H), 1.47 (s, 15H), 1.35-1.20 (m, 21H) ppm. **¹³C-NMR** (100 MHz, CDCl₃): δ 155.4, 154.1, 153.5, 138.8, 134.8, 132.1, 131.7, 130.2, 128.4, 128.0, 127.6, 126.6, 126.4, 125.9, 125.6, 121.0, 112.2, 82.2, 69.5, 66.4, 51.7, 48.0, 41.0, 39.3, 29.8, 29.3, 29.2, 28.6, 28.0, 26.9, 26.6 ppm. **MS (ES)** *m*/*z* = 732.4 [M+H]⁺.

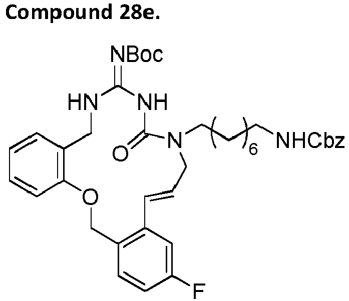

**Compound 28e** was prepared by the procedure described for **compound 28f** starting from compound **27e. Yield** 54%. **¹H-NMR** (400 MHz, CDCl₃): δ 12.29 (s, 1H), 12.13 (s, 1H), 8.41 (t, *J* = 5.9 Hz, 1H), 7.37 - 7.18 (m, 9H), 6.97 (t, *J* = 8.0 Hz, 3H), 6.88 (d, *J* = 8.4 Hz, 1H), 6.54 (d, *J* = 15.5 Hz, 1H), 6.15 (t, *J* = 5.7 Hz, 1H), 5.11 (s, 2H), 5.08 (s, 2H), 4.82 (s, 1H), 4.65 (d, *J* = 5.8 Hz, 1H), 4.59 (d, *J* = 6.1 Hz, 2H), 3.92 (d, *J* = 4.7 Hz, 2H), 3.49 - 3.43 (m, 1H), 3.40 - 3.32 (m, 2H), 3.29 - 3.23 (m, 1H), 3.17 (d, *J* = 5.9 Hz, 3H), 2.48 (d, *J* = 8.0 Hz, 1H), 1.56 (s, 2H), 1.50 (s, 4H), 1.47 (s, 11H), 1.35 - 1.23 (m, 13H) ppm. **¹³C-NMR** (100 MHz, CDCl₃): δ 164.3, 164.0, 163.4, 161.8, 156.3, 155.5, 155.3, 154.1, 153.5, 139.4, 139.3, 136.7, 132.1, 132.0, 130.3, 130.1, 129.7, 128.4, 128.0, 127.9, 127.7, 127.6, 127.3, 127.0, 126.3, 125.6, 121.2, 121.0, 113.5, 113.3, 112.4, 112.1, 82.2, 69.9, 69.6, 66.4, 60.3, 51.7, 48.0, 47.7, 46.6, 41.0, 39.3, 39.2, 32.6, 29.8, 29.6, 29.3, 29.2, 28.7, 28.0, 26.9, 26.6 ppm. **MS (ES)** *m*/*z* = 716.4 [M+H]⁺.

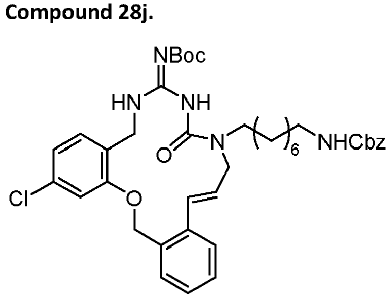

**Compound 28j** was prepared by the procedure described for **compound 28f** starting from compound **27j. Yield** 78%. **¹H-NMR** (400 MHz, CDCl₃): δ 12.27 (s, 1H), 8.39 (s, 1H), 7.56 (d, *J* = 7.7 Hz, 1H), 7.38 - 7.28 (m, 5H), 7.23 (d, *J* = 7.5 Hz, 2H), 7.15 (d, *J* = 7.4 Hz, 1H), 7.10 (d, *J* = 8.1 Hz, 1H), 6.96 (s, 1H), 6.91 (d, *J* = 8.1 Hz, 1H), 6.49 (d, *J* = 15.6 Hz, 1H), 6.15 (dt, *J* = 15.6, 5.6 Hz, 1H), 5.13 (s, 2H), 5.08 (s, 2H), 4.76 (br, 1H), 4.52 (d, *J* = 6.2 Hz, 2H), 3.92 (m, 2H), 3.35 (t, *J* = 8.0 Hz, 2H), 3.16 (q, *J* = 6.3 Hz, 2H), 1.54 (m, 2H), 1.46 (s, 9H), 1.30-1.20 (m, 10H). **¹³C-NMR** (100 MHz, CDCl₃): δ 156.1, 154.1, 137.0, 133.0, 130.4, 129.1, 129.0, 128.4, 128.2, 128.1, 128.0, 127.0, 126.7, 125.6, 125.2, 120.8, 113.0, 70.7, 66.5, 60.3, 51.9, 48.1, 41.0, 29.9, 29.6, 29.3, 29.2, 28.6, 28.0, 26.9, 26.6, 23.8 ppm.

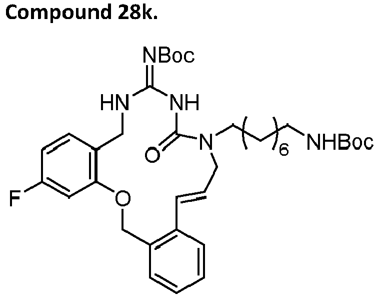

**Compound 28k** was prepared by the procedure described for **compound 28f** starting from compound **27k. Yield** 28%. **¹H NMR** (400 MHz, CDCl₃): δ 12.25 (s, 1H), 8.33 (s, 1H), 7.53 (d, *J* = 7.78 Hz, 1H), 7.35-29 (m, 2H), 7.28 (t, *J* = 8 Hz, 1H), 7.19 (t, *J* = 10.3 Hz, 1H), 6.69 (d, *J* = 8.3 Hz, 1H), 6.67-6.51 (m, 2H), 6.12 (dd, *J* = 17.2, 3.1 Hz, 1H), 5.10 (s, 2H), 4.51 (d, *J* = 10.4 Hz, 2H), 3.93 (d, *J* = 7.3 Hz, 2H), 3.33 (t, *J* = 7.6 Hz, 2H), 3.05 (s, 2H), 1.43 (s, 9H), 1.40 (s, 9H), 1.22 (s, 8H) ppm. **¹³C NMR** (100 MHz, CDCl₃): δ 225.29, 183.90, 171.17, 164.12, 163.18, 154.08, 153.62, 137.19, 130.47, 129.11, 128.90, 127.12, 126.97, 126,88, 126.75, 125.73, 123.42, 107.06, 101.07, 100.87, 82.37, 70.55, 60.39, 51.81, 48.12, 39.06, 30.01, 29.45, 29.22, 29.15, 28.74, 28.11, 27.34, 26.74, 23.86, 21.01, 20.81 ppm.

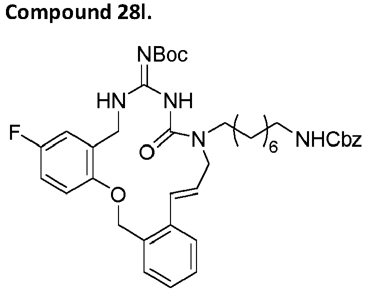

**Compound 28l** was prepared by the procedure described for **compound 28f** starting from compound **27l. Yield** 26%. **¹H NMR** (400 MHz, CDCl₃): δ 12.27 (s, 1H), 8.40 (s, 1H), 7.58 (d, *J* = 8.0 Hz, 1H), 7.34 (m, 5H), 7.30 (d, *J* = 7.1 Hz, 2H), 7.23 (m, 3H), 6.98 (d, *J* = 7.6 Hz, 1H), 6.93 (t, *J* = 7.6 Hz, 1H), 6.58 (d, *J* = 15.6 Hz, 1H), 6.16 (dt, *J* = 15.6, 5.2 Hz, 1H), 5.15 (s, 2H), 5.08 (s, 2H), 4.69 (s, 1H), 4.61 (d, *J* = 6.4 Hz, 1H), 3.92 (d, *J* = 5.2 Hz, 2H), 3.35 (t, *J* = 7.6 Hz, 2H), 3.16 (q, *J* = 6.4 Hz, 2H), 1.57 (s, 4H), 1.46 (m, 9H), 1.30-1.15 (s, 6H) ppm. **¹³C NMR** (100 MHz, CDCl₃): δ 163.68, 155.26, 154.22, 153.56, 138.10, 137.80, 136.89, 129.55, 129.41, 129.11, 128.82, 128.73, 128.32, 126.49, 122.91, 118.56, 99.92, 82.22, 72.21, 66.50, 45.10, 44.14, 43.35, 40.91, 31.11, 29.34, 29.22, 28.47, 27.32 ppm.

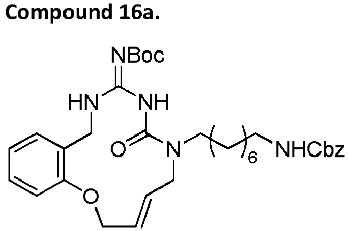

**Compound 16a** was prepared by the procedure described for **compound 28f** starting from compound **15a. Yield** 50%. **¹H-NMR** (400 MHz, CDCl₃): δ 12.15 (s, 1H), 8.64 (s, 1H), 7.33-7.16 (5H, m), 7.02-7.89 (4H, m), 5.38-5.32 (2H, m), 5.07-5.05 (2H, m), 4.65-4.45 (4H, m), 3.80-3.70 (2H, m), 3.17-3.14 (4H, m), 1.43 (9H, m), 1.26 (12H, m) ppm. **¹³C NMR** (100 MHz, CDCl₃): δ 163.7, 156.5, 154.1, 153.9, 153.3, 135.3, 134.7, 133.7, 129.3, 129.0, 128.7, 128.6, 128.4, 128.3, 128.0, 127.8, 126.8, 126.0, 120.7, 116.6, 115.5, 111.5, 81.9, 68.1, 66.5, 50.5, 48.4, 47.5, 45.7, 41.0, 40.0, 29.9, 29.4, 29.2, 28.5, 28.0, 27.0, 26.9, 26.6 ppm. **MS (ES)** *m*/*z* = 747.9 [M+Na]⁺, 725.9 [M+H]⁺.

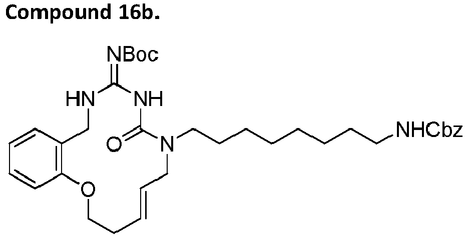

**Compound 16b** was prepared by the procedure described for **compound 28f** starting from compound **15b. Yield:** 26%. **¹H-NMR** (400 MHz, CDCl₃): (Mixture E/Z isomers) δ 12.07 (d, *J* = 10.4 Hz, 1H), 8.39 (s, 0.5H), 8.22 (s, 0.5H), 7.31-7.28 (m, 5H), 7.22-7.14 (m, 2H), 6.92-6.84 (m, 2H), 5.40-5.38 (m, 1H), 5.35-5.34 (m, 1H), 5.04 (s, 2H), 4.61 (d, *J* = 6 Hz, 1H), 4.52 (d, *J* = 6 Hz, 1H), 4.22 (t, *J* = 4.4 Hz, 1H), 4.08 (t, *J* = 5.2 Hz, 1H), 3.88 (d, *J* = 6 Hz, 1H), 3.77 (d, *J* = 6 Hz, 1H), 3.25 (t, *J* = 7.6 Hz, 1H), 3.20 (t, *J* = 7.6 Hz, 1H), 3.13-3.09 (m, 2H), 2.38-2.34 (m, 1H), 2.31-2.28 (m, 1H), 1.46-1.44 (m, 4H), 1.42 (s, 4.5H), 1.40 (s, 4.5H), 1.25-1.10 (m, 8H) ppm. **¹³C-NMR** (100 MHz, CDCl₃): δ 163.4, 156.3, 155.5, 154.5, 153.1, 136.6, 131.9, 128.7, 128.3, 128.1,127.9,127.4, 125.6,124.8,120.7, 114.3, 82.0, 67.6, 66.3, 50.4,48.2, 40.9, 31.7, 29.8, 29.2, 29.0, 28.6, 28.0, 27.3, 26.8, 26.5 ppm. **MS (ES)** *m*/*z* = 658.0 [M+Na]⁺, 636.0 [M+H]⁺.

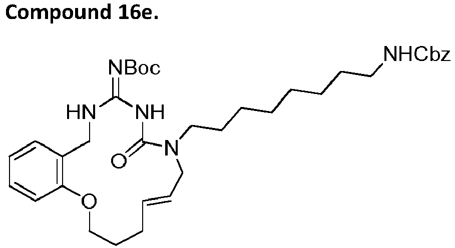

**Compound 16e** was prepared by the procedure described for **compound 28f** starting from compound **15e. Yield:** 57%. **¹H-NMR** (400 MHz, CDCl₃): (Mixture E/Z isomers) δ 12.26 (s, 0.5H), 12.09 (s, 0.5H), 8.31 (s, 0.5H), 7.83 (s, 0.5H), 7.33-7.27 (m, 5H), 7.22-7.16 (m, 2H), 6.89-6.82 (m, 1H), 6.77 (d, *J* = 8.4 Hz, 1H), 6.05-5.94 (m, 0.5H), 5.56-5.48 (m, 1H), 5.38-5.33 (m, 0.5H), 5.03 (s, 2H), 4.80 (bs, 1H), 4.64 (d, *J* = 4.8 Hz, 2H), 4.15 (d, *J* = 6.4 Hz, 1H), 3.99-3.92 (m, 3H), 3.29 (t, *J* = 7.2 Hz, 1H), 3.25 (t, *J* = 7.2 Hz, 1H), 3.14-3.06 (m, 2H), 2.43-2.32 (m, 1H), 2.20-2.11 (m, 1H), 1.88-1.79 (m, 1H), 1.75-1.69 (m, 1H), 1.59-1.42 (m, 4H), 1.39 (s, 4.5H), 1.36 (s, 4.5H), 1.26-1.15 (m, 8H) ppm. **¹³C-NMR** (100 MHz, CDCl₃): δ 164.1, 163.4, 157.3, 156.4, 153.5, 153.3, 152.6, 136.7, 134.1, 132.0, 130.6, 129.2, 128.7, 128.4, 127.9, 127.0, 126.2, 125.4, 125.2, 120.5, 120.2, 111.6, 110.9, 82.0, 81.8, 69.6, 66.7, 66.4, 50.4, 47.2, 44.7, 42.7, 41.0, 40.1, 38.6, 32.2, 29.8, 29.6, 29.3, 29.2, 28.7, 28.0, 27.8, 27.0, 26.9, 26.6, 24.3 ppm. **MS (ES)** *m*/*z* = 650.3 [M+H]⁺, 672.2 [M+H]⁺, 688.3 [M+K]⁺.

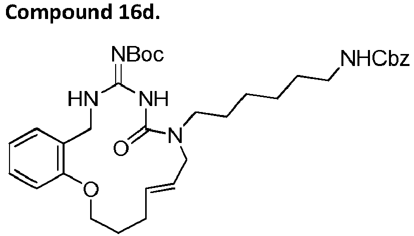

**Compound 16d** was prepared by the procedure described for **compound 28f** starting from compound **15d. Yield:** 79%. **¹H-NMR** (400 MHz, CDCl₃): (Mixture E/Z isomers) δ 12.27 (s, 0.5H), 12.09 (s, 0.5), 8.34 (s, 0.5), 7.87 (s, 0.5H), 7.36-7.29 (m, 5H), 7.24-7.20 (m, 2H), 6.95-6.87 (m, 1H), 6.81 (d, *J* = 8 Hz, 1H), 6.07-5.98 (m, 0.5H), 5.61-5.50 (m, 1H), 5.35-5.44 (m, 0.5H), 5.07 (s, 2H), 4.82 (bs, 1H), 4.67 (d, *J* = 4.8 Hz, 2H), 4.18 (d, *J* = 6 Hz, 1H), 4.02-3.96 (m, 3H), 3.33 (t, *J* = 6.8 Hz, 1H), 3.27 (t, *J* = 6.8 Hz, 1H), 3.10- 3.11 (m, 2H), 2.49-2.40 (m, 1H), 2.27-2.18 (m, 1H), 1.90-1.81 (m, 1H), 1.80-1.74 (m, 1H), 1.59-1.47 (m, 4H), 1.43 (s, 4.5H), 1.40 (s, 4.5H), 1.33-1.22 (m, 4H) ppm. **¹³C-NMR** (100 MHz, CDCl₃): δ 164.1, 157.3, 156.4, 153.5, 153.3, 152.6, 136.7, 134.2, 132.0, 130.6, 129.2, 128.7, 128.4, 128.0, 127.9, 127.0, 126.2, 125.4, 125.2, 120.5, 120.2, 111.6, 110.9, 82.0, 81.9, 69.6, 66.7, 66.4, 60.3, 50.4, 47.0, 44.6, 42.7, 40.9, 40.1, 38.6, 32.2, 29.8, 29.6, 29.1, 28.6, 28.0, 27.7, 27.1, 26.7, 26.6, 26.5, 24.3, 20.9, 14.1 ppm.

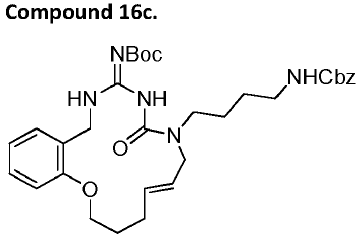

**Compound 16c** was prepared by the procedure described for **compound 28f** starting from compound **15c. Yield:** 70%. **¹H-NMR** (400 MHz, CDCl₃): (Mixture E/Z isomers) δ 12.19 (s, 0.5H), 12.02 (s, 0.5H), 8.33 (s, 0.5H), 7.85 (s, 0.5H), 7.33-7.24 (m, 5H), 7.22-7.16 (m, 2H), 6.88 (t, *J* = 7.2 Hz, 1H), 6.79 (d, *J* = 8 Hz, 1H), 6.04-5.96 (m, 0.5H), 5.59-4.45 (m, 1H), 5.37-5.32 (m, 0.5H), 5.04 (s, 2H), 4.97 (bs, 0.5H), 4.90 (bs, 0.5H), 4.64 (d, *J* = 5.2 Hz, 2H), 4.14 (d, *J* = 5.2 Hz, 1H), 3.99 (t, *J* = 4.8 Hz, 2H), 3.92 (d, *J* = 5.6 Hz, 1H), 3.30-3.20 (m, 2H), 3.19-3.08 (m, 2H), 2.43-2.37 (m, 1H), 2.19-2.11 (m, 1H), 1.77-1.69 (m, 2H), 1.53-1.45 (m, 4H), 1.40 (s, 4.5H), 1.37 (s, 4.5H) ppm. **¹³C-NMR** (100 MHz, CDCl₃): δ 164.2, 163.4, 157.3, 156.4, 153.6, 153.5, 153.2, 152.7, 136.6, 134.3, 132.2, 134.3, 130.6, 129.2, 128.7, 128.4, 127.9, 127.0, 126.1, 125.4, 125.0, 120.5, 111.6, 110.9, 82.1, 81.9, 69.6, 66.7, 66.4, 60.3, 56.0, 50.4, 46.6, 44.2, 42.7, 40.8, 40.1, 38.6, 32.2, 29.6, 29.1, 28.0, 27.3, 26.0, 25.0, 24.3, 14.1 ppm.

### General procedure for the double bond reduction.

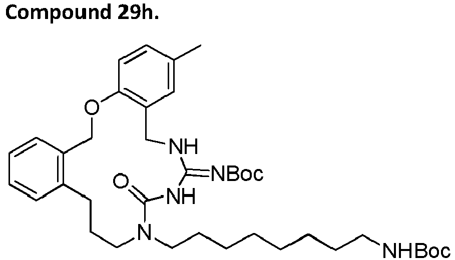

In a flask with magnetic stirrer, macrocycle compound **28h** (122 mg, 0.17 mmol) was dissolved in isopropanol (19 mL) and 10% palladium on carbon (36 mg, 0.03 mmol). The flask was subjected to three cycles of vacuum followed by flush of H₂ before being stirred under H₂ atmosphere for 3h. The mixture was filtered through a plug of Celite, and then solvent was evaporated *in vacuo.* The crude product obtained was used directly in the next step without any further purification. **Yield:** quantitative. **¹H-NMR** (400 MHz, CDCl₃): δ 12.11 (s, 1H), 8.48 (s, 1H), 7.33 (d, *J* = 5.6 Hz, 1H), 7.28 (d, *J* = 7.2 Hz, 1H), 7.27-7.18 (m, 2H), 7.02 (s, 1H), 6.95 (d, *J* = 8 Hz, 1H), 6.86 (d, *J* = 7.6 Hz, 1H), 5.00 (s, 2H), 4.52 (d, *J* = 5.2 Hz, 2H), 3.21 (t, *J* = 4 Hz, 2H), 3.15 (t, *J* = 5.6 Hz, 2H), 3.09-3.04 (m, 2H), 2.52 (t, *J* = 4.8 Hz, 2H), 2.26 (s, 3H), 1.99-1.93 (m, 2H), 1.64-1.52 (m, 4H), 1.46 (s, 9H), 1.42 (s, 9H), 1.27-1.22 (m, 8H) ppm. **¹³C-NMR** (100 MHz, CDCl₃): δ 160.7, 157.3, 156.5, 154.3, 148.4, 137.9, 135.0, 134.9, 129.9, 129.2, 128.3, 126.9, 126.6, 125.8, 114.2, 82.5, 79.8, 69.3, 45.5, 44.9, 40.7, 40.4, 31.5, 29.7, 28.6, 28.5, 28.4, 28.1, 28.0, 27.2, 26.9, 26.6, 20.9 ppm.

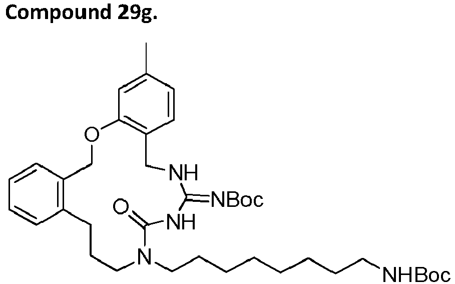

**Compound 29g** was prepared by the procedure described for **compound 29h** starting from compound **28g. Yield:** quantitative. **¹H-NMR** (400 MHz, CDCl₃): δ 12.20 (s, 1H), 8.49 (s, 1H), 7.30 (d, *J* = 7.2 Hz, 1H), 7.25 (d, *J* = 6.8 Hz, 1H), 7.20 (d, *J* = 6.8 Hz, 1H), 7.11 (d, *J* = 7.6 Hz, 1H), 6.80 (s, 1H), 6.74 (d, *J* = 7.6 Hz, 1H), 5.02 (s, 2H), 4.51 (d, *J* = 6 Hz, 2H), 3.21 (t, *J* = 6.8 Hz, 2H), 3.14 (t, *J* = 8 Hz, 2H), 3.10-3.03 (m, 2H), 2.53 (t, *J* = 7.6 Hz, 2H), 2.32 (s, 3H), 2.02-1.95 (m, 2H), 1.63-1.52 (m, 4H), 1.46 (s, 9H), 1.42 (s, 9H), 1.28-1.23 (m, 8H) ppm. **¹³C-NMR** (100 MHz, CDCl₃): δ 153.6, 134.1, 130.7, 129.9, 128.9, 126.4, 125.8, 121.2, 111.7, 82.1, 78.9, 69.4, 68.5, 53.3, 49.8, 47.3, 40.6, 37.9, 30.8, 30.3, 30.0, 29.6, 29.4, 29.2, 28.7, 28.4, 28.1, 27.0, 26.7, 25.3, 21.4 ppm.

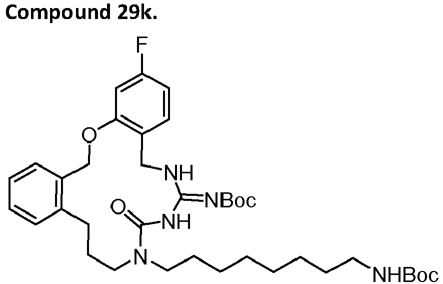

**Compound 29k** was prepared by the procedure described for **compound 29h** starting from compound **28k. Yield:** quantitative. **¹H NMR** (400 MHz, CDCl₃): δ 12.20 (s, 1H), 8.49 (s, 1H), 7.30 (d, *J* = 7.2 Hz, 1H), 7.25 (d, *J* = 6.8 Hz, 1H), 7.20 (d, *J* = 6.8 Hz, 1H), 7.11 (d, *J* = 7.6 Hz, 1H), 6.80 (s, 1H), 6.74 (d, *J* = 7.6 Hz, 1H), 5.02 (s, 2H), 4.51 (d, *J* = 6 Hz, 2H), 3.21 (t, *J* = 6.8 Hz, 2H), 3.14 (t, *J* = 8 Hz, 2H), 3.10-3.03 (m, 2H), 2.53 (t, *J* = 7.6 Hz, 2H), 2.02-1.95 (m, 2H), 1.63-1.52 (m, 4H), 1.46 (s, 9H), 1.42 (s, 9H), 1.28-1.23 (m, 8H) ppm. **¹³C NMR** (100 MHz, CDCl₃): δ 201.45, 167.34, 165,27, 160.98, 153.6, 134.1, 130.7, 129.9, 128.9, 126.4, 125.8, 121.2, 111.7, 82.1, 78.9, 69.4, 68.5, 53.3, 49.8, 47.3, 40.63, 39.87, 37.9, 30.8, 30.3, 30.0, 29.6, 29.4, 29.2, 28.7, 28.4, 28.1, 27.0, 26.7, 25.3, 21.4 ppm.

### General procedure for the deprotection of compounds 29g, 29h and 29j.

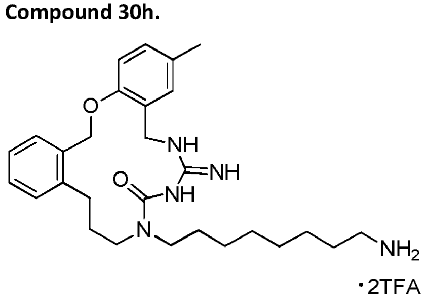

In a flask with magnetic stirrer, **29h** (6 mg, 0.01 mmol) was dissolved in dry CH₂Cl₂ (1.8 mL) under N₂ atmosphere. Then freshly distilled TFA (Sigma-Aldrich S.r.l., T6508) (10% v/v, 200 µL) was added dropwise and the mixture was stirred at room temperature for 7h. The reaction mixture was concentrated *in vacuo.* The residue was decanted several times with Et₂O and Hexane affording the desired compound as trifluoroacetate salt in quantitative yield. **Yield:** quantitative. **¹H-NMR** (400 MHz, MeOD): 7.24 (d, *J* = 7.3, 1H), 7.24-7.19 (m, 1H), 7.18 - 7.12 (m, 2H), 7.04 (d, *J* = 7.5 Hz, 1H), 6.91 (d, *J* = 7.4 Hz, 1H), 6.79 (d, *J* = 7.5 Hz, 1H), 5.11 (s, 2H), 4.42 (s, 2H), 3.53-3.44 (m, 2H), 3.27 (t, *J* = 7.1 Hz, 2H), 2.76 (t, *J* = 7.1 Hz, 2H), 2.68-2.62 (m, 2H), 2.35 (s, 3H), 1.92-1.85 (m, 2H), 1.77-1.67 (m, 4H), 1.37 - 1.24 (m, 8H) ppm. **¹³C-NMR** (100 MHz, MeOD): δ 160.1, 156.5, 155.7, 139.3, 135.2, 134.2, 131.6, 128.7, 128.2, 127.1, 127.1, 126.9, 126.6, 113.4, 70.1,46.0,45.5,41.8,40.2, 32.9, 30.6, 28.8, 28.7, 27.4, 26.9, 26.8, 26.7, 20.8 ppm.

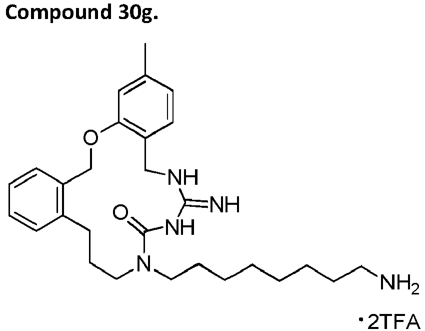

**Compound 30g** was prepared by the procedure described for **compound 30h** starting from compound **29g. Yield:** quantitative. **¹H-NMR** (400 MHz, MeOD): δ 7.43 (d, *J* = 6.8 Hz, 1H), 7.35-7.30 (m, 2H), 7.25 (t, *J* = 7.2 Hz, 1H), 7.14 (d, *J* = 7.6 Hz, 1H), 7.03 (s, 1H), 6.79 (d, *J* = 7.2 Hz, 1H), 5.04 (s, 2H), 4.34 (s, 2H), 3.70 (t, *J* = 6 Hz, 2H), 2.88 (t, *J* = 7.2 Hz, 2H), 2.82-2.77 (m, 2H), 2.34 (s, 3H), 2.11-2.06 (m, 2H), 1.53-1.49 (m, 4H), 1.38-1.27 (m, 8H) ppm. **¹³C-NMR** (100 MHz, MeOD): δ 160.1, 156.7, 156.5, 139.3, 138.7, 135.2, 131.6, 128.2, 127.4, 127.1, 126.6, 124.8, 123.2, 112.8, 70.0, 46.0, 45.5, 41.8, 39.7, 32.9, 30.6, 28.8, 28.8, 27.4, 27.0, 26.8, 26.7, 21.5 ppm.

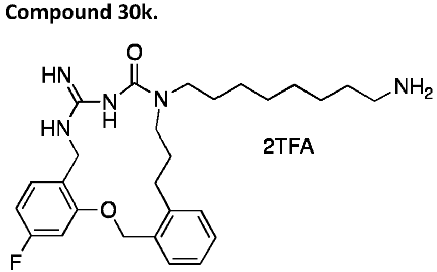

**Compound 30k** was prepared by the procedure described for **compound 30h** starting from compound **29k** and was used in the next step without isolation.

### General procedure for the synthesis of guanylated compounds.

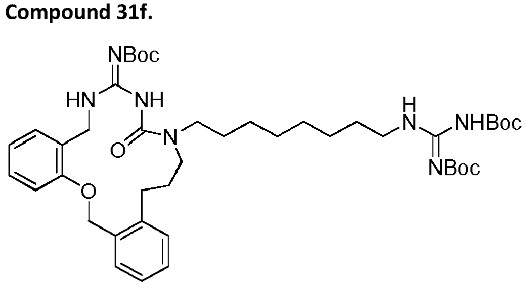

In a flask with magnetic stirrer, protected macrocycle **28f** (118 mg, 0.17 mmol) was dissolved in isopropanol (19 mL). Then 10% palladium on carbon (36 mg, 0.03 mmol) and two drops of HCl 36% were added. The flask was subjected to three cycles of vacuum followed by flush of H₂ before being stirred under H₂ atmosphere for 3h. The mixture was filtered through a plug of Celite, and then solvent was evaporated *in vacuo.* The crude product **30f** obtained was used directly in the next step without any further purification.

In a flask with magnetic stirrer, the amine **30f** (40 mg, 0.07 mmol) was dissolved in THF (5 mL). Then, *N,N'*-di-boc-pyrazolo-guanidine (33 mg, 0.09 mmol) and DIPEA (12 µL, 0.07 mmol) were added and the mixture was stirred at room temperature for 16 h. The reaction was diluted with AcOEt and washed with H₂O. The organic phase was dried over anhydrous Na₂SO₄, filtered and the solvent was evaporated in *vacuo.* The residue was purified by flash chromatography (Hex/AcOEt 1:0; Hex/AcOEt 8:2). **Yield (calculated on two steps):** 62%. **¹H-NMR** (400 MHz, CDCl₃): δ 12.20 (s, 1H), 11.49 (s, 1H), 8.52 (t, 1H, *J* = 6.4 Hz), 8.27 (s, 1H), 7.27 (d, 1H, *J* = 12.8 Hz), 7.24 (d, 1H, *J* = 6.0 Hz), 7.21 (m, 2H), 7.17 (m, 2H), 6.98 (d, 1H, *J* = 8.4 Hz), 6.93 (t, 1H, *J* = 7.6 Hz), 5.12 (s, 2H), 4.56 (d, 2H, *J* = 6.4 Hz), 3.37 (q, 2H, *J* = 6.8 Hz), 3.21 (t, 2H, *J* = 7.6 Hz), 3.13 (t, 2H, *J* = 8.0 Hz), 2.52 (t, 2H, *J* = 8.0 Hz), 1.97 (m, 2H), 1.52 (m, 4H), 1.48 (s, 9H), 1.47 (s, 9H), 1.46 (s, 9H), 1.29-1.25 (m, 8H) ppm. **¹³C-NMR** (100 MHz, CDCl₃): δ 203.1, 163.6, 163.5, 156.0, 155.5, 153.6, 153.3, 142.7, 134.0, 130.7, 129.9, 128.9, 127.7, 127.4, 126.5, 125.8, 120.6, 110.6, 82.9, 82.1, 79.1, 69.4, 60.3, 49.9, 47.3, 40.9, 38.1, 30.8, 30.2, 29.6, 29.3, 29.2, 28.9, 28.7, 28.2, 28.1, 28.0, 27.0, 26.8, 20.9, 14.1 ppm. **MS (ES)** *m*/*z* = 831.3 [M+Na]⁺.

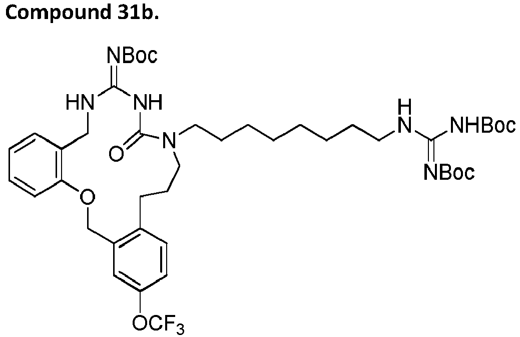

**Compound 31b** was prepared by the procedure described for **compound 31f** starting from compound **28b. Yield:** 78%. **¹H NMR** (400 MHz, CDCl₃): δ 12.22 (s, 1H), 11.51 (s, 1H), 8.56 (t, 1H, *J* = 6.4 Hz), 8.33 (bs, 1H), 7.32 - 7.26 (m, 2H), 7.26 - 7.21 (m, 2H), 7.21 - 7.16 (m, 1H), 6.97 (t, 2H, *J* = 7.3 Hz), 5.05 (s, 2H), 4.58 (d, 2H, *J* = 6.4 Hz), 3.42 (d, 2H, *J* = 6.4 Hz), 3.23 (t, 2H, *J* = 7.6 Hz), 3.19 - 3.11 (m, 2H), 2.61 - 2.49 (m, 2H), 2.04 - 1.91 (m, 2H), 1.53 - 1.48 (m, 12H), 1.27 (m, 9H) ppm. **¹³C-NMR** (100 MHz, CDCl₃): δ 160.8, 156.7, 155.6, 154.4, 154.3, 151.5, 151.3, 151.2, 151.1, 151.0, 147.9, 137.0, 135.7, 128.4, 127.8, 127.7, 126.3, 123.9, 121.7, 121.4, 119.6, 117.4, 116.2, 116.2, 115.6, 115.6, 115.6, 114.0, 81.9, 81.8, 80.2, 68.9, 45.5, 44.6,41.6,40.7, 31.4, 28.8, 28.6, 28.5, 28.2, 28.2, 28.1, 27.3, 26.7, 26.6 ppm. **MS (ES)** *m*/*z* = 892.4 [M+H]⁺, 914.3 [M+Na]⁺.

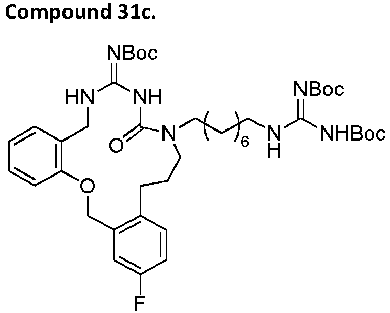

**Compound 31c** was prepared by the procedure described for **compound 31f** starting from compound **28c. Yield** 36%. **¹H NMR** (400 MHz, CDCl₃): δ 12.20 (s, 1H), 11.49 (s, 1H), 8.54 (t, *J* = 6.0 Hz, 1H), 8.34 (s, 1H), 5.00 (s, 2H), 4.55 (d, *J* = 6.4 Hz, 2H), 3.4 (m, 2H), 3.20 (t, *J* = 7.6 Hz, 2H), 3.12 (t, *J* = 8.0 Hz, 2H), 2.49 (t, *J* = 8.0 Hz, 2H), 1.93 (m, 2H), 1.53 (m, 4H), 1.49 (s, 9H), 1.48 (s, 9H), 1.47 (s, 9H), 1.29-1.24 (s, 8H) ppm. **¹³C NMR** (100 MHz, CDCl₃) δ 163.4, 161.8, 155.8, 155.3, 153.6, 138.2, 135.8, 131.3, 127.7, 127.5, 126.6, 120.8, 117.3, 117.1, 115.7, 110.6, 82.2, 68.9,49.7,47.3, 38.1, 30.8, 29.6, 29.3, 29.2, 28.9, 28.6, 28.2, 28.1, 28.0, 27.0, 26.7 ppm. **MS (ES)** *m*/*z* = 826.6 [M+H]⁺.

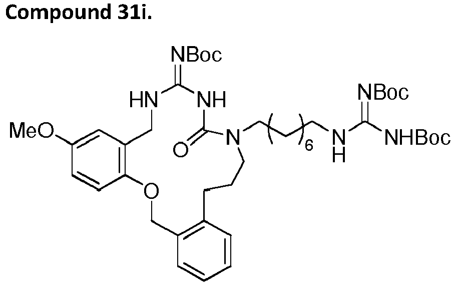

**Compound 31i** was prepared by the procedure described for **compound 31f** starting from compound **28c. Yield** 50% (over two steps). **¹H-NMR** (400 MHz, CDCl₃): δ 12.19 (s, 1H), 11.48 (s, 1H), 8.49 (t, 1H, *J* = 6.4 Hz), 8.27 (s, 1H), 7.34 (d, 1H, *J* = 7.6 Hz), 7.24 (d, 1H, *J* = 6.0 Hz), 7.21-7.17 (m, 2H), 6.89 (d, 1H, *J* = 8.8 Hz), 6.83 (m, 1H), 6.68 (dd, 1H, *J* = 8.8, 2.8 Hz), 5.00 (s, 2H), 4.51 (d, 2H, *J* = 6.4 Hz), 3.74 (s, 3H), 3.37 (q, 2H, *J* = 6.8 Hz), 3.21 (t, 2H, *J* = 7.6 Hz), 3.14 (m, 2H), 2.52 (t, 2H, *J* = 8.0 Hz), 1.96 (m, 2H), 1.50 (m, 4H), 1.48 (s, 9H), 1.47 (s, 9H), 1.46 (s, 9H), 1.29-1.25 (m, 8H) ppm. **¹³C-NMR** (100 MHz, CDCl₃): δ 203.1, 163.6, 163.4, 156.0, 153.6, 153.5, 153.3, 149.8, 142.7, 134.0, 130.6, 129.9, 129.1, 128.9, 125.8, 113.4, 111.4, 111.1, 82.9, 82.1, 79.1, 69.9, 55.5, 49.8, 47.3, 40.9, 38.1, 30.8, 30.2, 29.6, 29.3, 29.2, 28.9, 28.7, 28.2, 28.1, 28.0, 27.0, 26.8 ppm. **MS (ES)** *m*/*z* = 861.2 [M+Na]⁺.

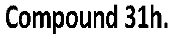

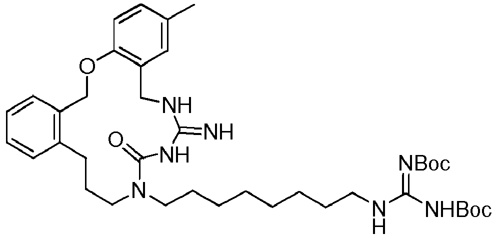

**Compound 31h** was prepared by the procedure described for **compound 31f** starting from compound **30h. Yield:** 45%. **¹H-NMR** (400 MHz, CDCl₃): δ 11.51 (s, 1H), 8.69 (s, 1H), 7.25 - 7.13 (m, 4H), 7.04 (d, *J* = 7.9, 1H), 6.96 - 6.91 (m, 1H), 6.78 (d, *J* = 7.5 Hz, 1H), 5.09 (s, 2H), 4.42 (d, *J* = 6.6 Hz, 2H), 3.47 - 3.38 (m, 4H), 3.26 (t, *J* = 7.1 Hz, 2H), 2.77 (t, *J* = 7.1 Hz, 2H), 2.32 (s, 3H), 1.92-1.85 (m, 2H), 1.58-1.51 (m, 4H), 1.47 (s, 9H), 1.46 (s, 9H), 1.38 - 1.23 (m, 8H) ppm. **¹³C-NMR** (100 MHz, CDCl₃): δ 161.5, 159.6, 156.5, 155.4, 154.3, 151.5, 137.9, 135.0, 134.8, 129.9, 129.2, 128.3, 127.8, 126.5, 126.4, 125.7, 114.2, 82.5, 80.2, 69.2, 45.5, 44.9, 42.0, 41.8, 31.6, 28.8, 28.6, 28.5, 28.2, 28.1, 28.0, 27.1, 26.9, 26.6, 20.9 ppm.

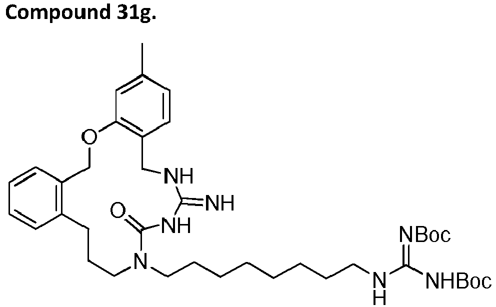

**Compound 31g** was prepared by the procedure described for **compound 31f** starting from compound **30h. Yield:** 35%. **¹H-NMR** (400 MHz, CDCl₃): δ 11.46 (s, 1H), 8.26 (s, 1H), 7.33 (d, *J* = 7.6 Hz, 1H), 7.28 (d, *J* = 7.2 Hz, 1H), 7.22-7.25 (m, 1H), 7.18 (t, *J* = 7.2 Hz, 1H), 7.09-7.02 (m, 1H), 6.78 (s, 1H), 6.68 (d, *J* = 7.2 Hz, 1H), 5.00 (s, 2H), 4.39 (s, 2H), 3.36-3.32 (m, 2H), 3.25-3.11 (m, 4H), 2.61-2.52 (m, 2H), 2.29 (s, 3H), 2.01-1.92 (m, 2H), 1.59-1.50 (m, 4H), 1.47 (s, 9H), 1.46 (s, 9H), 1.25-1.18 (m, 8H) ppm. **¹³C-NMR** (100 MHz, CDCl₃): δ 206.9, 163.6, 156.0, 153.2, 134.1, 130.6, 128.8, 125.7, 121.2, 112.0, 82.9, 79.1, 69.4, 47.0, 40.9, 30.8, 29.6, 29.2, 28.9, 28.6, 28.2, 28.0, 26.9, 26.7, 23.8, 21.4 ppm.

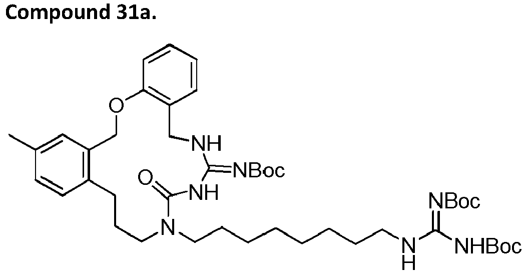

**Compound 31a** was prepared by the procedure described for **compound 31f** starting from compound **28a. Yield (calculated on two steps):** 59%. **¹H-NMR** (400 MHz, CDCl₃): δ 12.19 (s, 1H), 11.48 (s, 1H), 8.49 (t, *J* = 6.4 Hz, 1H), 8.25 (s, 1H), 7.19 (d, *J* = 7.6 Hz, 1H), 7.15-7.06 (m, 4H), 6.93 (d, *J* = 8.4 Hz, 1H), 6.88 (t, *J* = 7.2 Hz, 1H), 4.95 (s, 2H), 4.51 (d, *J* = 6.4 Hz, 2H), 3.34 (q, *J* = 6.8 Hz, 2H), 3.17 (t, *J* = 7.6 Hz, 2H), 3.07 (t, *J* = 8 Hz, 2H), 2.43 (t, *J* = 8 Hz, 2H), 2.28 (s, 3H), 1.92-1.89 (m, 2H), 1.51-1.48 (m, 4H), 1.45 (s, 9H), 1.43 (s, 9H), 1.42 (s, 9H), 1.25-1.17 (m, 8H) ppm. **¹³C-NMR** (100 MHz, CDCl₃): δ 163.6, 163.4, 156.0, 155.6, 153.6, 153.2, 139.6, 135.2, 133.8, 131.4, 129.8, 129.6, 127.7, 127.4, 126.4, 120.5, 110.6, 82.9, 82.1, 79.1, 69.5, 64.6, 49.9, 47.3, 40.9, 38.1, 30.9, 29.9, 29.6, 29.3, 29.2, 28.9, 28.6, 28.2, 28.1, 28.0, 26.0, 26.7, 20.8, 14.1 ppm. **MS (ES)** *m*/*z* = 822.3 [M+H]⁺, 411.6 [M+2H]²⁺.

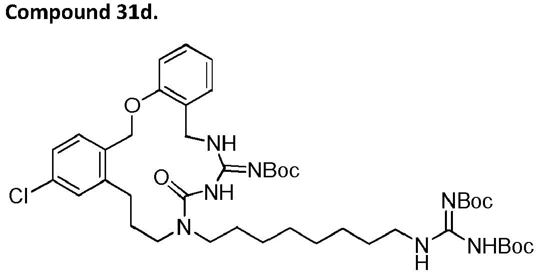

**Compound 31d** was prepared by the procedure described for **compound 31f** starting from compound **28d. Yield (calculated on two steps):** 41%. **¹H-NMR** (400 MHz, CDCl₃): δ 12.20 (s, 1H), 11.48 (s, 1H), 8.53 (t, *J* = 6 Hz, 1H), 8.30 (s, 1H), 7.29-7.23 (m, 3H), 7.19-7.15 (m, 2H), 6.94-6.92 (m, 2H), 5.00 (s, 2H), 4.54 (d, *J* = 6.4 Hz, 2H), 3.38 (q, *J* = 6.8 Hz, 2H), 3.20 (t, *J* = 7.6 Hz, 2H), 3.12 (t, *J* = 8 Hz, 2H), 2.49 (t, *J* =8 Hz, 2H), 1.99-1.91 (m, 4H), 1.48 (s, 9H), 1.47 (s, 9H), 1.46 (s, 9H), 1.37-1.19 (m, 10H) ppm. **¹³C-NMR** (100 MHz, CDCl₃): δ 163.4, 156.0, 155.3, 153.6, 153.2, 144.6, 134.6, 132.5, 131.9, 129.9, 128.4, 127.7, 127.4, 126.6, 125.9, 120.8, 110.5, 83.0, 82.2, 68.6, 49.7, 47.3, 41.0, 38.1, 30.6, 30.1, 29.3, 29.2, 28.9, 28.6, 28.2, 28.1, 28.0, 27.0, 26.7, 25.2, 23.9 ppm.

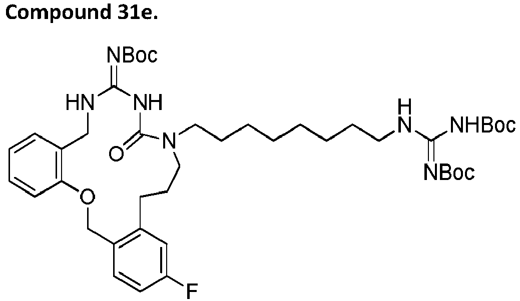

**Compound 31e** was prepared by the procedure described for **compound 31f** starting from compound **28e. Yield (calculated on two steps):** 41%. **¹H-NMR** (400 MHz, CDCl₃): δ 8.86 - 8.79 (m, 1H), 7.28 - 7.19 (m, 2H), 7.01 (d, *J* = 7.7 Hz, 1H), 6.93 (d, *J* = 7.5 Hz, 1H), 6.84 (d, *J* = 7.8 Hz, 1H), 5.10 (d, *J* = 0.9 Hz, 1H), 4.63 (d, *J* = 6.7 Hz, 1H), 3.48 - 3.38 (m, 2H), 3.28 (t, *J* = 7.1 Hz, 1H), 2.79 (d, *J* = 7.1 Hz, 1H), 1.89 (p, *J* = 7.1 Hz, 1H), 1.56 (p, *J* = 7.1 Hz, 2H), 1.46 (d, *J* = 5.2 Hz, 13H), 1.38 - 1.23 (m, 4H) ppm. **¹³C-NMR** (100 MHz, CDCl₃): δ 160.8, 156.7, 155.4, 154.4, 154.3, 151.5, 148.4, 128.4, 127.9, 127.9, 127.7, 127.7, 121.4, 115.7, 115.6, 114.4, 114.3, 113.8, 82.5, 82.5, 80.6, 69.2, 45.5, 44.8, 41.6, 40.7, 31.4, 31.4, 28.7, 28.6, 28.5, 28.4, 28.3, 28.2, 28.20, 28.1, 28.0, 27.2, 26.9, 26.6 ppm.

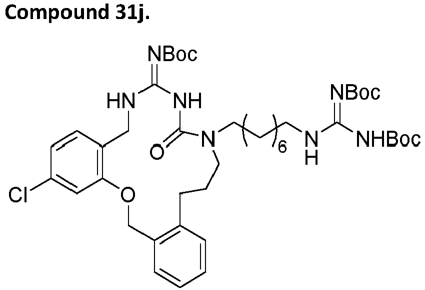

**Compound 31j** was prepared by the procedure described for **compound 31f** starting from compound **28j. Yield (calculated on two steps):** 31%. **¹H NMR** (400 MHz, CDCl₃): *δ* 12.13 (s, 1H), 11.45 (s, 1H), 8.50 (s, 1H), 8.39 (s, 1H), 7.33 (m, 2H), 7.25 (m, 1H), 7.21 (d, *J* = 7.1 Hz, 1H), 7.13 (d, J = 8.0 Hz, 1H), 6.96 (s, 1H), 6.89 (d, J = 7.6 Hz, 1H), 5.01 (s, 2H), 4.46 (d, *J* = 6.0 Hz, 2H), 3.40 (m, 2H), 3.19 (t, *J* = 7.6 Hz, 2H), 3.10 (t, *J* = 8.0 Hz, 2H), 2.46 (m, 2H), 1.94 (m, 2H), 1.51 (m, 4H), 1.48-1.43 (m, 27h), 1.17 (s, 8H) ppm. **¹³C-NMR** (100 MHz, CDCl₃): δ 163.3, 153.6, 142.7, 133.2, 132.6, 130.7, 130.0, 129.2, 125.9, 120.5, 111.3, 69.7, 49.8, 47.4, 40.8, 37.9, 33.8, 30.8, 30.2, 29.3, 29.2, 28.9, 28.4, 28.2, 28.0, 27.9, 27.0, 26.7.

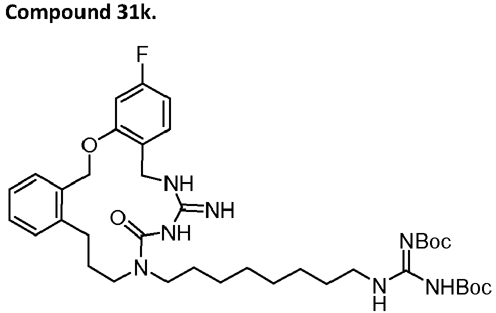

**Compound 31k** was prepared by the procedure described for **compound 31f** starting from compound **30k. Yield (calculated on two steps):** 47%. **¹H NMR** (400 MHz, CDCl₃): *δ* 11.46 (s, 1H), 8.27 (s, 1H), 7.34-7.29 (m, 2H), 7.25 (d, *J* = 7.5 Hz, 1H), 7.18 (t, *J* = 10.6 Hz, 1H), 7.10 (t, *J* = 13.4 Hz, 1H), 6.68 (d, *J* = 7.8 Hz, 1H), 6.53 (t, *J* = 10.4 Hz, 1H), 5.03 (s, 2H), 4.37 (s, 2H), 3.37-3.32 (m, 2H), 3.21 (t, *J* = 14.5 Hz, 2H), 3.15 (s, 2H), 2.51 (t, *J* = 17.6 Hz, 2H), 1.86 (s, 2H), 1.62-1.47 (m, 4H), 1.48 (s, 9H), 1.47 (s, 9H), 1.25-1.21 (m, 8H) ppm. **¹³C-NMR** (100 MHz, CDCl₃): δ 164.2, 163.1, 162.2, 158.8, 154.0, 147.5, 141.2, 136.8, 130.9, 130.8, 129.3, 128.4, 127.1, 126.2, 124.9, 108.1, 102.2, 79.8, 68.7, 44.9, 44.4, 41.5, 41.1, 31.9, 29.0, 29.0, 28.2, 28.1, 27.5, 27.4, 26.6 ppm.

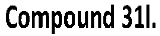

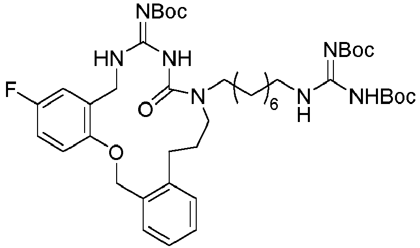

**Compound 31l** was prepared by the procedure described for **compound 31f** starting from compound **28l. Yield (calculated on two steps):** 21%. **¹H NMR** (400 MHz, CDCl₃): *δ* 12.18 (s, 1H), 8.50 (s, 1H), 8.27 (s, 1H), 7.30 (d, *J* = 7.4 Hz, 1H), 7.25 (d, *J* = 7.5 Hz, 1H), 7.21 (d, *J* = 7.1 Hz, 1H), 7.11 (m, 1H), 6.96 (d, *J* = 7.05 Hz, 1H), 6.88-6.84 (m, 2H), 5.02 (s, 2H), 4.50 (d, *J* = 8.2 Hz, 2H), 3.37 (d, *J* = 8.6 Hz, 2H), 3.21 (t, *J* = 10.4 Hz, 2H), 3.11 (t, *J* = 10.9 Hz, 2H), 2.49 (t, *J* = 9.8 Hz, 2H), 1.97-1.93 (m, 2H), 1.53-1.44 (m, 4H), 1.29 (s, 9H), 1.23 (s, 9H), 1.21 (s, 9H), 1.19 (s, 8H) ppm. **¹³C-NMR** (100 MHz, CDCl₃): δ 167.1, 161.3, 158.8, 154.1, 154.0, 147.5, 144.4, 141.2, 136.8, 129.3, 128.4, 128.3, 128.2, 127.1, 126.2, 116.6, 116.4, 115.5, 114.9, 114.8, 80.4, 68.7, 44.9, 44.4, 41.1, 40.4, 31.7, 29.0, 28.9, 28.4, 28.3, 28.2, 27.5, 27.4, 26.8, 26.6.

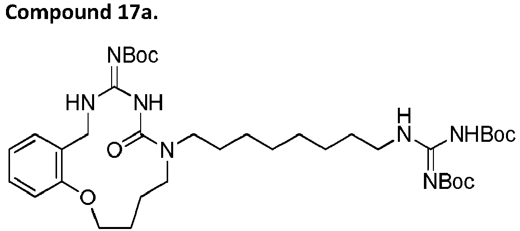

**Compound 17a** was prepared by the procedure described for **compound 31f** starting from compound **16a. Yield** 15%. **¹H-NMR** (400 MHz, CDCl₃): δ 12.04 (s, 1H), 11.48 (s, 1H), 8.49 (s, 1H), 8.26 (s, 1H), 7.25 (m, 1H), 7.19 (t, 1H, *J* = 7.6 Hz), 6.91 (t, 1H, *J* = 7.6 Hz), 6.81 (d, 1H, *J* = 8.4 Hz), 4.66 (m, 2H), 4.06 (m, 2H), 3.73 (m, 1H), 3.38-3.35 (m, 4H), 3.21 (m, 3H), 1.81 (m, 4H) 1.49 (s, 9H), 1.48 (s, 9H), 1.44 (s, 9H), 1.26 (m, 12H) ppm. **¹³C-NMR** (100 MHz, CDCl₃): δ 157.6, 156.6, 154.5, 154.4, 154.3, 153.9, 153.2, 128.8, 127.9, 126.0, 121.4, 112.7, 81.5, 80.3, 69.3, 46.0, 45.9, 41.5, 40.3, 28.9, 28.8, 28.7, 28.3, 28.2, 28.1, 27.5, 27.4, 26.9, 24.9, 24.6 ppm. **MS (ES)** *m*/*z* = 754.3 [M+Na]⁺.

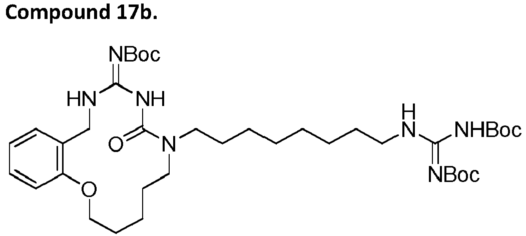

**Compound 17b** was prepared by the procedure described for **compound 31f** starting from compound **16b. Yield (calculated on two steps):** 46%. **¹H-NMR** (400 MHz, CDCl₃): δ 12.19 (s, 1H), 11.45 (s, 1H), 8.51 (t, *J* = 6 Hz, 1H), 8.23 (s, 1H), 7.22 (d, *J* = 6.4 Hz, 1H), 7.14 (t, *J* = 8 Hz, 1H), 6.89 (t, *J* = 7.6 Hz, 1H), 6.81 (d, *J* = 8.4 Hz, 1H), 4.64 (d, *J* = 6.4 Hz, 2H), 4.07-4.02 (m, 2H), 3.36-3.29 (m, 4H), 3.16 (t, *J* = 7.2 Hz, 2H), 1.88-1.76 (m, 2H), 1.66-1.54 (m, 6H), 1.45 (s, 9H), 1.44 (s, 9H), 1.41 (s, 9H), 1.29-1.13 (m, 10H) ppm. **¹³C-NMR** (100 MHz, CDCl₃): δ 163.6, 156.0, 155.8, 153.5, 153.3, 128.8, 128.0, 120.7, 112.2, 82.9, 82.0, 79.1, 68.5, 49.0, 46.7, 41.0, 36.8, 31.1, 29.6, 29.3, 29.2, 28.9, 28.5, 28.2, 28.0, 27.0, 26.7, 26.6, 25.4 ppm.

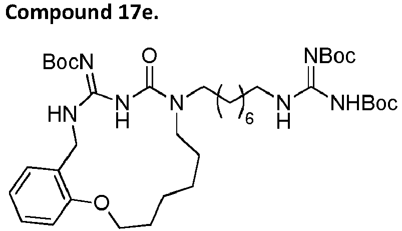

**Compound 17e** was prepared by the procedure described for **compound 31f** starting from compound **16e. Yield** 60%. **¹H-NMR** (400 MHz, CDCl₃): δ 12.37 (s, 1H), 11.47 (s, 1H), 8.26 (m, 1H), 8.08 (m, 1H), 7.21 (m, 2H), 6.88 (t, 1H, *J* = 7.2 Hz), 6.81 (d, 1H, *J* = 8.4 Hz), 4.66 (d, 2H, *J* = 5.2 Hz), 4.01 (m, 2H), 3.62 (t, 2H, *J* = 6.4 Hz), 3.36 (m, 2H), 3.23 (t, 2H, *J* = 7.6 Hz), 1.66 (m, 4H), 1.52 (m, 4H), 1.47 (s, 9H), 1.46 (s, 9H), 1.40 (s, 9H), 1.28 (m, 12H)ppm. **¹³C-NMR** (100 MHz, CDCl₃): δ 164.12, 163.55, 156.95, 155.99, 153.39, 153.21, 129.63, 128.84, 126.24, 120.25, 111.63, 82.84, 81.84, 79.04, 67.92, 44.70, 44.61, 40.88, 39.46, 29.31, 29.19, 28.87, 28.39, 28.22, 28.02, 27.07, 26.73, 26.33, 24.57, 24.30 ppm. **MS (ES)** *m*/*z* = 782.6 [M+Na]⁺, 760.6 [M+H]⁺.

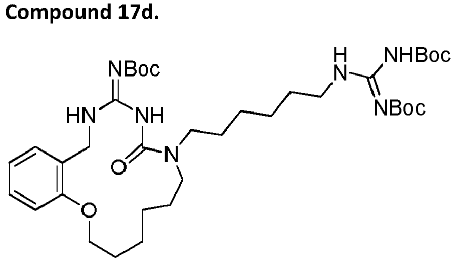

**Compound 17d** was prepared by the procedure described for **compound 31f** starting from compound **16d. Yield (calculated on two steps):** 77%. **¹H-NMR** (400 MHz, CDCl₃): δ 12.36 (s, 1H), 11.49 (s, 1H), 8.34 (s, 1H), 8.11 (s, 1H), 7.24-7.18 (m, 2H), 6.89 (t, *J* = 7.2 Hz, 1H), 6.83 (d, *J* = 8.8 Hz, 1H), 4.67 (d, *J* = 5.2 Hz, 2H), 4.02-3.95 (m, 2H), 3.63 (t, *J* = 6.4 Hz, 2H), 3.47-3.36 (m, 2H), 3.24 (t, *J* = 7.2 Hz, 2H), 1.70-1.66 (m, 4H), 1.55-1.52 (m, 4H), 1.49 (s, 9H), 1.48 (s, 9H), 1.42 (s, 9H), 1.36-1.32 (m, 8H) ppm. **¹³C-NMR** (100 MHz, CDCl₃): δ 157.6, 156.6, 154.5, 154.5, 154.4, 153.9, 153.2, 128.8, 127.9, 126.0, 121.4, 112.7, 81.5, 80.3, 69.4, 46.0, 45.9, 41.5, 40.3, 29.3, 28.6, 28.3, 28.2, 28.1, 27.5, 27.2, 27.0, 26.9, 26.8, 25.9 ppm.

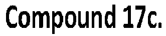

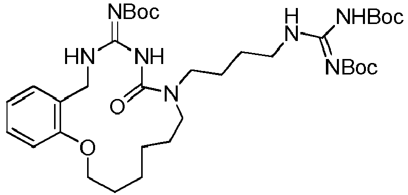

**Compound 17c** was prepared by the procedure described for **compound 31f** starting from compound **16c. Yield (calculated on two steps):** 49%. **¹H-NMR** (400 MHz, CDCl₃): δ 12.30 (s, 1H), 11.44 (s, 1H), 8.28 (s, 1H), 8.08 (s, 1H), 7.21-7.18 (m, 2H), 6.86 (t, *J* = 7.6 Hz, 1H), 6.79 (d, *J* = 8.4 Hz, 1H), 4.64 (d, *J* = 5.6 Hz, 2H), 3.99-3.91 (m, 2H), 3.60 (t, *J* = 6.8 Hz, 2H), 3.41-3.35 (m, 2H), 3.26 (t, *J* = 6.8 Hz, 2H), 1.69-1.57 (m, 4H), 1.55-1.50 (m, 4H), 1.45 (s, 9H), 1.44 (s, 9H), 1.39 (s, 9H), 1.33-1.25 (m, 4H) ppm. **¹³C-NMR** (100 MHz, CDCl₃): δ 203.1, 192.7, 157.0, 156.0, 153.5, 129.7, 128.9, 126.2, 120.3, 111.6, 82.0, 78.8, 67.9, 44.7, 44.1, 40.8, 39.5, 28.4, 28.2, 28.0, 26.6, 26.3, 25.3, 24.6, 24.3 ppm.

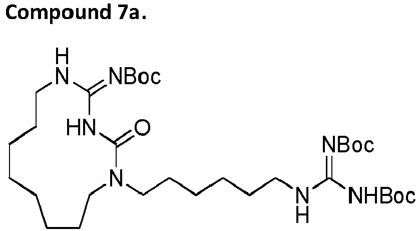

**Compound 7a** was prepared by the procedure described for **compound 31f** starting from compound **5a. Yield (calculated on two steps):** 42%. **¹H-NMR** (400 MHz, CDCl₃): δ 12.04 (s, 1H), 11.47 (bs, 1H), 8.34 (bs, 1H), 8.06 (s, 1H), 3.46-3.40 (m, 4H), 3.34 (q, *J* = 6.8 Hz, 2H), 3.22 (t, *J* = 7.2 Hz, 2H), 1.69-1.59 (m, 4H), 1.56-1.51 (m, 4H), 1.48 (s, 9H), 1.47 (s, 9H), 1.44 (s, 9H), 1.35-1.26 (m, 12H) ppm. **¹³C-NMR** (100 MHz, CDCl₃): δ 163.9, 154.1, 153.5, 82.0, 47.0, 46.2, 40.8, 39.6, 29.6, 28.9, 28.3, 28.2, 28.0, 26.8, 26.1, 25.8, 25.2, 23.7, 20.7, 14.5 ppm.

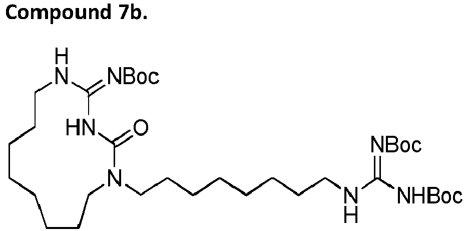

**Compound 7b** was prepared by the procedure described for **compound 31f** starting from compound 5b. **Yield (calculated on two steps):** 54%. **¹H-NMR** (400 MHz, CDCl₃): δ 12.06(s, 1H), 11.49 (s, 1H), 8.32 (s, 1H), 8.04 (s, 1H), 3.45-3.37 (m, 4H), 3.32 (q, *J* = 7.2 Hz, 2H), 3.20 (t, *J* = 7.2 Hz, 2H), 1.72-1.55 (m, 8H), 1.46 (s, 9H), 1.45 (s, 9H), 1.41 (s, 9H), 1.32-1.21 (m, 16H) ppm. **¹³C-NMR** (100 MHz, CDCl₃): δ 163.9, 154.0, 153.5, 81.9, 47.0, 46.3, 39.6, 37.4, 31.8, 29.6, 29.3, 28.2, 28.0, 27.3, 27.0, 26.7, 25.8, 25.2, 24.4, 23.5, 22.2, 14.2 ppm.

### General procedure for the synthesis of amidinourea terminal.

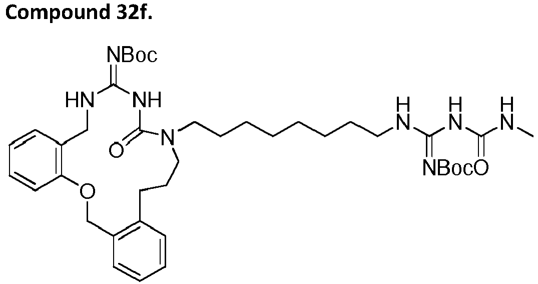

To a solution of di-Boc-protected guanidine **31f** (57 mg, 0,07 mmol) in THF (1 mL) in a glass tube, DIPEA (0,013 mL, 0,07 mmol) and a 40% solution of methylamine in H₂O (Sigma-Aldrich S.r.l., 426466) (0,06 mL, 0,724 mmol) were added. The tube was sealed and warmed to 80 °C overnight. Then the solvent was evaporated, and the crude was purified with flash chromatography on silica gel, eluting with 20% AcOEt in Hexane to give pure compound. **Yield** 35%. **¹H-NMR** (400 MHz, CDCl₃): δ 12.20 (s, 1H), 12.11 (s, 1H), 8.53 (t, 1H, *J* = 6.4 Hz), 7.95 (s, 1H), 7.36 (d, 1H, *J* = 7.6 Hz), 7.30 (d, 1H, *J* = 7.2 Hz), 7.25 (m, 2H), 7.18 (m, 2H), 6.98 (d, 1H, *J* = 8.0 Hz), 6.93 (t, 1H, *J* = 7.2 Hz), 5.11 (bs, 1H), 5.06 (s, 2H), 4.56 (d, 2H, *J* = 6.4 Hz), 3.28-3.18 (m, 4H), 3.13 (t, 2H, *J* = 8.4 Hz), 2.76 (d, 3H, *J* = 5.0 Hz), 2.52 (t, 2H, *J* = 8.4 Hz), 1.96 (m, 2H), 1.52 (m, 4H), 1.47 (s, 9H), 1.45 (s, 9H), 1.29-1.25 (m, 8H) ppm. **¹³C-NMR** (100 MHz, CDCl₃): δ 165.4, 163.4, 155.5, 154.0, 153.6, 153.4, 142.7, 134.0, 130.7, 129.8, 128.9, 127.7, 127.4, 126.5, 125.8, 120.5, 110.6, 82.1, 69.4, 53.3, 49.8, 47.3, 40.5, 38.1, 30.8, 30.3, 29.6, 29.4, 29.2, 29.0, 28.7, 28.1, 28.0, 27.0, 26.8, 26.7 ppm. **MS (ES)** *m*/*z* = 787.5 [M+Na]⁺.

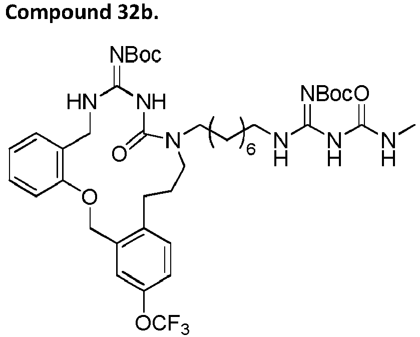

**Compound 32b** was prepared by the procedure described for **compound 32f** starting from compound **31b. Yield** 67%. **¹H NMR** (400 MHz, CDCl₃): δ 12.18 (s, 1H), 12.13 (s, 1H), 8.53 (s, 1H), 7.95 (bs, 1H), 7.21 (m, 2H), 7.18 (m, 1H), 7.16 (m, 2H), 6.95 (m, 2H), 5.02 (s, 2H), 4.54 (d, 2H, *J* = 6.4 Hz), 3. 62 (m, 2H), 3.21 (t, 2H, *J* = 8.0 Hz), 3.12 (t, 2H, *J* = 10.0 Hz), 2.75 (d, 3H, *J* = 5.6 Hz), 2.51 (t, 2H, *J* = 8.8 Hz), 1.95 (m, 2H), 1.52 (m, 4H), 1.46 (s, 9H), 1.45 (s, 9H), 1.28 (m, 8H) ppm. **¹³C NMR** (100 MHz, CDCl₃): δ 183.4, 163.4, 155.2, 154.0, 153.6, 153.5, 146.8, 141.4, 135.7, 131.1, 127.7, 127.5, 126.6, 123.0, 121.2, 120.9, 110.5, 82.2, 68.8, 49.7, 47.3, 38.1, 30.7, 29.7, 29.3, 29.1, 29.0, 28.6, 28.1, 28.0, 27.0, 26.7, 26.5 ppm. **MS (ES)** *m*/*z* = 849.3 [M+H]⁺, 871.4 [M+Na]⁺.

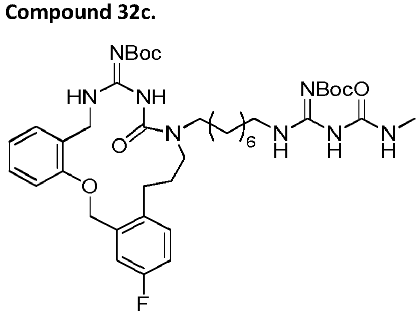

**Compound 32c** was prepared by the procedure described for **compound 32f** starting from compound **31c. Yield** 36%. **¹H NMR (400** MHz, CDCl₃): δ 12.19 (s, 1H), 12.13 (s, 1H), 8.54 (t, *J* = 6.4 Hz, 1H), 7.95 (bs, 1H), 7.25 (m, 1H), 7.19 (m, 2H), 7.07 (d, *J* = 9.2 Hz, 1H), 7.00 (t, *J* = 8.8 Hz, 1H), 6.96 (s, 1H), 6.94 (m, 2H), 5.00 (s, 2H), 4.55 (d, *J* = 6.0 Hz, 2H), 3.20 (t, *J* = 8.0 Hz, 4H), 3.12 (t, *J* = 8.0 Hz, 2H), 2.77 (s, 3H), 2.49 (t, *J* = 8.4 Hz, 2H), 1.92 (m, 2H), 1.59 (m, 4H), 1.46 (m, 18H), 1.28 (m, 8H) ppm. **¹³C NMR** (100 MHz, CDCl₃): δ 161.7, 161.5, 157.3, 153.3, 135.4, 135.4, 134.9, 129.6, 129.5, 129.4, 129.2, 127.9, 127.8, 115.7, 115.6, 115.4, 114.1, 113.8, 68.6, 68.5, 46.6, 45.0, 39.8, 28.4, 28.1, 28.0, 27.5, 27.4, 27.2, 26.4, 26.3, 26.0. ppm. **MS (ES)** *m*/*z* = 783.4 [M+H]⁺.

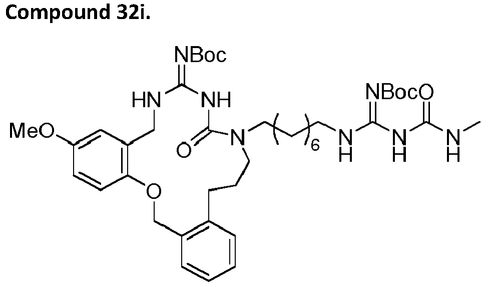

**Compound 32i** was prepared by the procedure described for **compound 32f** starting from compound **31i. Yield** 37%. **¹H-NMR** (400 MHz, CDCl₃): δ 12.19 (s, 1H), 12.12 (s, 1H), 8.49 (t, 1H, *J* = 6.4 Hz), 7.96 (s, 1H), 7.34 (d, 1H, *J* = 7.6 Hz), 7.29 (d, 1H, *J* = 7.2 Hz), 7.24 (m, 1H), 7.18 (m, 1H), 6.89 (d, 1H, *J* = 8.0 Hz), 6.83 (m, 1H), 6.68 (dd, 1H, *J* = 8.8, 2.8 Hz), 5.13 (bs, 1H), 5.00 (s, 2H), 4.52 (d, 2H, *J* = 6.4 Hz), 3.75 (s, 3H), 3.28-3.18 (m, 4H), 3.15 (t, 2H, *J* = 8.4 Hz), 2.76 (d, 3H, *J* = 5.0 Hz), 2.52 (t, 2H, *J* = 8.4 Hz), 1.95 (m, 2H), 1.51 (m, 4H), 1.46 (s, 9H), 1.45 (s, 9H), 1.29-1.25 (m, 8H) ppm. **¹³C-NMR** (100 MHz, CDCl₃): δ 163.4, 153.6, 149.8, 142.6, 134.2, 130.6, 129.8, 129.1, 128.8, 125.8, 113.4, 111.4, 111.1, 82.1, 69.8, 55.5, 49.8, 47.3, 40.5, 38.2, 30.8, 30.3, 29.4, 29.2, 29.0, 28.7, 28.1, 28.0, 27.0, 26.8, 26.6 23.8 ppm. **MS (ES)** *m*/*z* = 817.4 [M+Na]⁺.

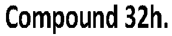

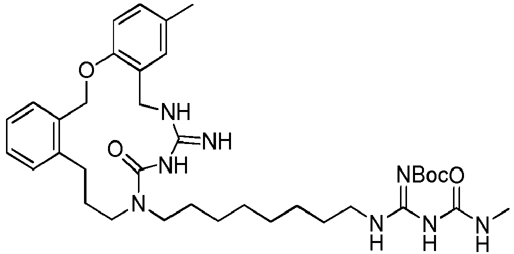

**Compound 32h** was prepared by the procedure described for **compound 32f** starting from compound **31h. Yield:** 47%. **¹H-NMR** (400 MHz, MeOD): δ 7.55-7.50 (m, 1H), 7.38 (d, *J* = 9.6 Hz, 1H), 7.29-7.25 (m, 2H), 7.20-7.17 (m, 1H), 7.04 (s, 1H), 6.98-6.92 (m, 2H), 5.06 (s, 2H), 4.42 (s, 2H), 3.46-3.41 (m, 4H), 3.25-3.16 (m, 2H), 2.66 (s, 3H), 2.58-2.52 (m, 2H), 2.25 (s, 3H), 1.97-1.92 (m, 2H), 1.65-1.53 (m, 4H), 1.49 (s, 9H), 1.34-1.28 (m, 8H) ppm. **¹³C-NMR** (100 MHz, MeOD): δ 161.8, 159.4, 157.5, 156.5, 154.3, 146.3, 137.9, 135.0, 134.9, 129.9, 129.2, 128.3, 126.9, 126.6, 125.7, 114.2, 82.5, 69.3, 45.5, 44.9, 42.1, 41.8, 31.5, 28.8, 28.6, 28.5, 28.1, 28.0, 27.2, 26.9, 26.8, 26.6, 20.9 ppm.

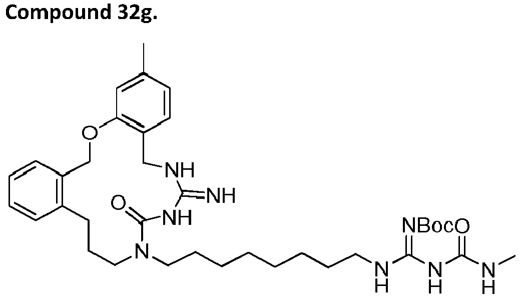

**Compound 32g** was prepared by the procedure described for **compound 32f** starting from compound **31g. Yield:** 35%. **¹H-NMR** (400 MHz, MeOD): δ 7.42-7.38 (m, 1H), 7.35-7.28 (m, 2H), 6.26-7.19 (m, 1H), 7.13 (d, *J* = 7.2 Hz, 1H), 7.01-6.97 (m, 1H), 6.76 (d, *J* = 7.6 Hz, 1H), 5.06 (s, 2H), 4.38 (s, 2H), 3.32-3.23 (m, 4H), 2.77-2.71 (m, 2H), 2.66 (s, 3H), 2.33 (s, 3H), 2.09-1.99 (m, 2H), 1.56-1.51 (m, 4H), 1.48 (s, 9H), 1.34-1.28 (m, 10H) ppm. **¹³C-NMR** (100 MHz, MeOD): δ 161.8, 159.4, 157.6, 157.5, 154.3, 146.3, 139.1, 137.9, 134.9, 129.9, 128.3, 127.4, 126.9, 125.4, 125.0, 113.5, 82.5, 69.3, 45.5, 44.9, 42.1, 41.7, 31.5, 28.8, 28.6, 28.5, 28.1, 28.0, 27.2, 26.9, 26.8, 26.6, 21.4 ppm.

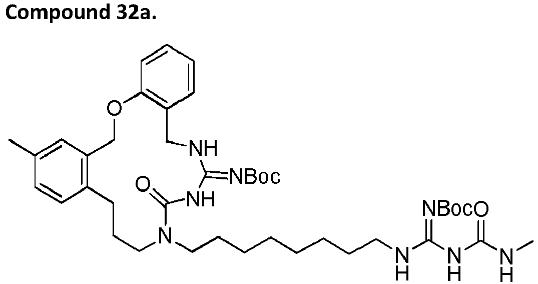

**Compound 32a** was prepared by the procedure described for **compound 32f** starting from compound **31a. Yield:** 88%. **¹H-NMR** (400 MHz, CDCl₃): δ 12.26 (s, 1H), 12.19 (s, 1H), 8.57 (t, *J* = 4 Hz, 1H), 8.00 (s, 1H), 7.27 (d, *J* = 8 Hz, 1H), 7.23-7.14 (m, 4H), 7.01 (d, *J* = 8 Hz, 1H), 6.96 (t, *J* = 8 Hz, 1H), 5.29 (s, 1H), 5.04 (s, 2H), 4.59 (d, *J* = 8 Hz, 2H), 3.32-3.23 (m, 4H), 3.19-3.16 (m, 2H), 2.78 (d, *J* = 4 Hz, 3H), 2.54-2.51 (m, 2H), 2.37 (s, 3H), 2.01-1.96 (m, 2H), 1.75-1.65 (m, 4H), 1.50 (s, 9H), 1.45 (s, 9H), 1.33-1.25 (m, 8H) ppm. **¹³C-NMR** (100 MHz, CDCl₃): δ 163.4, 155.6, 154.0, 153.6, 153.3, 139.5, 135.2, 133.8, 131.4, 129.8, 129.6, 127.7, 127.4, 126.4, 120.5, 110.5, 82.1, 69.4, 60.3, 49.8, 47.3, 40.5, 38.1, 30.9, 29.8, 29.6, 29.3, 29.2, 29.0, 28.6, 28.1, 28.0, 27.0, 26.8, 26.5, 20.9, 20.8, 14.1 ppm. **MS (ES)** *m*/*z* = 779.4 [M+H]⁺.

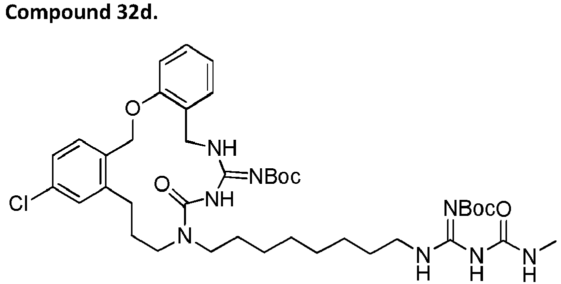

**Compound 32d** was prepared by the procedure described for **compound 32f** starting from compound **31d. Yield:** 68%. **¹H-NMR** (400 MHz, CDCl₃): δ 12.17 (s, 1H), 12.10 (s, 1H), 8.51 (t, *J* = 6 Hz, 1H), 7.27-7.20 (m, 3H), 7.15 (t, *J* = 7.6 Hz, 2H), 6.91 (t, *J* = 4 Hz, 2H), 4.98 (s, 2H), 4.51 (d, *J* = 6.4 Hz, 2H), 3.18 (t, *J* = 7.6 Hz, 4H), 3.09 (t, *J* = 8 Hz, 2H), 2.73 (d, *J* = 4.8 Hz, 3H), 2.46 (t, *J* = 8.4 Hz, 2H), 1.98-1.87 (m, 4H), 1.44 (s, 9H), 1.42 (s, 9H), 1.29-1.15 (m, 10H) ppm. **¹³C-NMR** (100 MHz, CDCl₃): δ 153.6, 134.6, 132.5, 131.9, 129.9, 127.7, 127.5, 126.6, 125.9, 120.8, 110.5, 82.2, 68.6, 49.7, 47.3, 38.1, 30.6, 30.2, 29.6, 29.3, 29.2, 29.0, 28.7, 28.1, 28.0, 27.0, 26.7, 26.6 ppm.

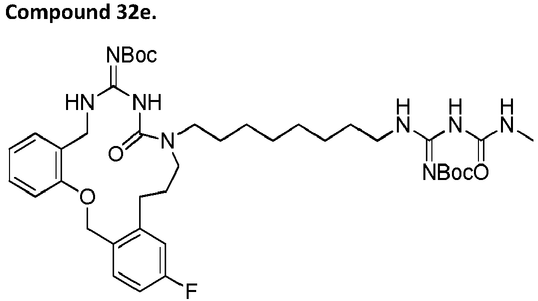

**Compound 32e** was prepared by the procedure described for **compound 32f** starting from compound **31e. Yield:** 68%. **¹H-NMR** (400 MHz, CDCl₃): δ 8.98 (t, *J* = 4.6 Hz, 1H), 8.83 (t, *J* = 6.7 Hz, 1H), 8.67 (s, 1H), 7.28 - 7.19 (m, 3H), 7.01 (d, *J* = 7.7 Hz, 1H), 6.96 - 6.89 (m, 2H), 6.88 - 6.81 (m, 2H), 6.68 (q, *J* = 3.1 Hz, 1H), 5.10 (d, *J* = 0.9 Hz, 2H), 4.63 (d, *J* = 6.7 Hz, 2H), 3.48 - 3.37 (m, 4H), 3.28 (t, *J* = 7.1 Hz, 2H), 2.79 (t, *J* = 7.1 Hz, 2H), 2.72 (d, *J* = 3.1 Hz, 3H), 1.89 (p, *J* = 7.1 Hz, 2H), 1.60 - 1.45 (m, 4H), 1.47 (s, 18H), 1.38 - 1.23 (m, 8H) ppm. **¹³C-NMR** (100 MHz, CDCl₃): δ 160.8, 157.5, 156.7, 154.4, 154.3, 148.4, 147.5, 128.6, 127.8, 127.7, 127.7, 125.8, 121.3, 115.7, 115.6, 114.4, 114.3, 113.8, 82.2, 82.2, 69.2, 45.5, 44.9, 41.7, 40.7, 31.3, 31.3, 28.8, 28.6, 28.5, 28.1, 28.1, 28.1, 28.0, 27.2, 26.9, 26.8, 26.6 ppm.

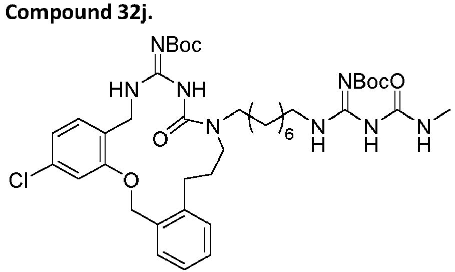

**Compound 32j** was prepared by the procedure described for **compound 32f** starting from compound **31j. Yield** 68%. **¹H-NMR** (400 MHz, CDCl₃): δ 12.18 (s, 1H), 8.50 (t, 1H, *J* = 6.4 Hz), 7.35 (d, *J* = 7.4 Hz, 1H), 7.33 - 7.30 (m, 1H), 7.25 (d, *J* = 9.2 Hz, 1H), 7.23 - 7.18 (m, 1H), 7.15 (d, *J* = 8.1 Hz, 1H), 6.97 (d, *J* = 1.9 Hz, 1H), 6.90 (d, *J* = 8.2 Hz, 1H), 5.02 (s, 2H), 4.47 (d, 2H, *J* = 6.0 Hz), 3.24-3.18 (m, 4H), 3.11 (t, 2H, *J* = 8.4 Hz), 2.78 (m, 3H), 2.49 (t, 2H, *J* = 8.4 Hz), 1.95 (m, 2H), 1.53 (m, 4H), 1.48-1.45 (m, 18H), 1.31-1.21 (m, 8H) ppm. **¹³C-NMR** (100 MHz, CDCl₃): δ 163.3, 152.8, 149.6, 143.0, 133.8, 130.8, 129.9, 129.2, 127.4, 125.9, 120.5, 111.3, 111.1, 82.0, 69.7, 49.9, 47.4, 40.9, 37.9, 30.9, 30.2, 28.7, 28.1, 28.0, 26.9, 26.5, 23.8, 20.7, 17.2 ppm.

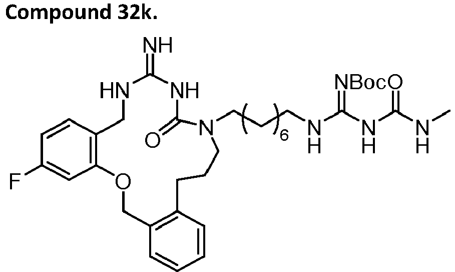

**Compound 32k** was prepared by the procedure described for **compound 32f** starting from compound **31k.** Yield 36%. **¹H NMR** (400 MHz, CDCl₃): *δ* 12.09 (s, 1H), 7.95 (s, 1H), 7.25 (q, *J* = 17.5 Hz, 1H), 7.20 (d, *J* = 7.3 Hz, 1H), 7.18 (t, *J* = 10.3 Hz, 1H), 7.11 (t, *J* = 17.8 Hz, 1H), 6.69 (d, *J* = 7.5 Hz, 1H), 6.54 (t, *J* = 10.7 Hz, 1H), 5.18 (s, 1H), 5.06 (s, 2H), 4.38 (s, 2H), 3.22 (s, 4H), 3.15 (s, 2H), 2.75 (d, *J* = 7.1 Hz, 3H), 2.51 (t, *J* = 15.6 Hz, 2H), 1.94, (s, 2H), 1.48 (s, 4H), 1.44 (s, 9H), 1.26-1.19 (m, 8H) ppm. **¹³C-NMR** (100 MHz, CDCl₃): δ 164.2, 163.1, 162.2, 158.7, 155.9, 154.0, 141.2, 140.6, 136.8, 130.8, 129.3, 128.4, 127.1, 126.2, 124.9, 124.9, 124.9, 107.9, 102.2, 80.3, 68.7, 44.9, 44.4, 41.5, 41.1, 31.9, 29.0, 29.0, 28.2, 27.5, 27.4, 26.8, 26.6, 26.2.

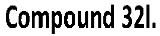

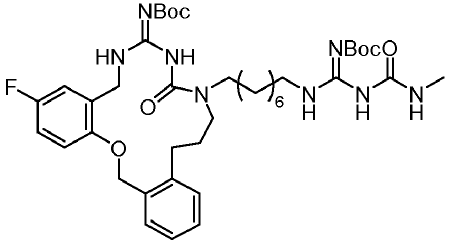

**Compound 32l** was prepared by the procedure described for **compound 32f** starting from compound **31l. Yield** 35%. **¹H NMR** (400 MHz, CDCl₃): *δ* 12.15 (s, 1H), 8.50 (s, 1H), 7.95 (s, 1H), 7.31 (d, *J* = 7.6 Hz, 1H), 7.25 (d, *J* = 7.8 Hz, 1H), 7.21 (d, *J* = 7.2 Hz, 1H), 7.18 (m, 1H), 6.96 (dd, *J* = 14.1, 2.05 Hz, 1H), 6.89-6.84 (m, 2H), 5.11 (s, 1H), 5.02 (s, 2H), 4.50 (d, *J* = 8.1 Hz, 2H), 3.27-3.19 (m, 4H), 3.17 (t, *J* = 15.4 Hz, 2H), 2.75 (t, *J* = 7.9 Hz, 3H), 2.49 (t, *J* = 10.8 Hz, 2H), 1.98-1.93 (m, 2H), 1.55-1.52 (m, 4H), 1.49 (s, 9H), 1.47 (s, 9H), 1.28 (s, 8H) ppm. **¹³C-NMR** (100 MHz, CDCl₃): δ 167.1, 160.3, 155.9, 154.8, 154.1, 154.0, 144.4, 141.2, 140.6, 136.8, 129.3, 128.4, 128.2, 127.1, 126.2, 116.6, 115.5, 114.8, 80.3, 68.7, 44.9, 44.4, 41.1, 40.5, 40.4, 31.9, 29.0, 28.4, 28.2, 27.3, 27.1, 26.8, 26.7, 26.4 ppm.

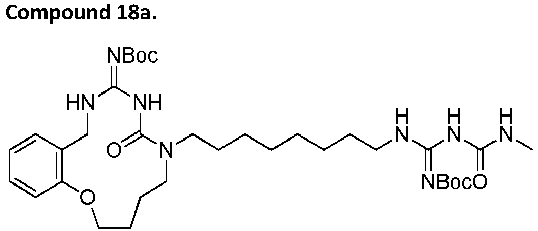

**Compound 18a** was prepared by the procedure described for **compound 32f** starting from compound **17a. Yield** 33%. **¹H-NMR** (400 MHz, CDCl₃): δ 12.03 (bs, 1H), 8.49 (s, 1H), 7.21 (m, 1H), 7.19 - 7.17 (m, 1H), 6.92 (t, 1H, *J* = 7.2 Hz), 6.81 (d, 1H, *J* = 8.4 Hz), 4.62 (m, 2H), 4.06 (m, 2H), 3.37 (m, 2H), 3.21 (m, 4H), 2.78 (s, 3H), 1.87 (bs, 2H), 1.80 (m, 2H), 1.57 (m, 4H), 1.47 (s, 9H), 1.44 (s, 9H), 1.28-1.15 (m, 8H) ppm. **¹³C-NMR** (100 MHz, CDCl₃): δ 158.7, 157.6, 156.6, 155.7, 154.5, 154.5, 153.2, 128.7, 127.9, 126.0, 121.2, 112.7, 81.5, 81.5, 69.3, 46.0, 46.0, 41.9, 40.3, 28.9, 28.8, 28.6, 28.2, 28.1, 27.5, 27.3, 26.9, 26.1, 24.9, 24.6 ppm. **MS (ES)** *m*/*z* = 711.5 [M+Na]⁺.

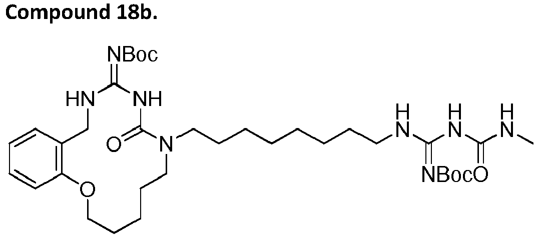

**Compound 18b** was prepared by the procedure described for **compound 32f** starting from compound **17b. Yield:** 56%. **¹H-NMR** (400 MHz, CDCl₃): δ 12.18 (s, 1H), 12.08 (s, 1H), 8.51 (s, 1H), 7.91 (s, 1H), 7.22 (d, *J* = 7.2 Hz, 1H), 7.15 (t, *J* = 8 Hz, 1H), 6.89 (t, *J* = 7.6 Hz, 1H), 6.82 (d, *J* = 8 Hz, 1H, 4.64 (d, *J* = 6.4 Hz, 2H), 4.06-4.02 (m, 2H), 3.35-3.28 (m, 2H), 3.25-3.20 (m, 2H), 3.18-3.14 (m, 2H), 2.72 (d, *J* = 4.8 Hz, 3H), 1.64-1.57 (m, 8H)1.42 (s, 9H), 1.41 (s, 9H), 1.29-1.18 (m, 10H) ppm. **¹³C-NMR** (100 MHz, CDCl₃): δ 165.4, 163.6, 155.8, 154.0, 153.5, 153.3, 128.8, 128.0, 121.0, 120.8, 112.2, 82.1, 82.0, 68.5, 60.3, 49.0, 46.7, 40.5, 36.8, 29.3, 29.1, 29.0, 28.5, 28.0, 27.0, 26.8, 26.6, 25.4, 24.6, 20.9, 14.1 ppm.

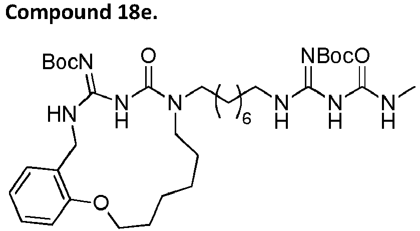

**Compound 18e** was prepared by the procedure described for **compound 32f** starting from compound **17e. Yield** 70%. **¹H-NMR** (400 MHz, CDCl₃): δ 12.37 (s, 1H), 12.11 (s, 1H), 8.09 (s, 1H), 7.94 (s, 1H), 7.22 (m, 1H), 6.89 (t, 1H, *J* = 7.2 Hz), 6.82 (d, 1H, *J* = 8.4 Hz), 5.09 (bs, 1H), 4.66 (d, 2H, *J* = 5.6 Hz), 3.99 (s, 2H), 3.63 (t, 2H, *J* = 6.4 Hz), 3.24 (t, 3H, *J* = 7.2) 2.76 (d, 2H, *J* = 4.4), 1.54 (m, 4H), 1.45 (s, 9H), 1.41 (s, 9H), 1.29-1.24 (m, 16H) ppm. **¹³C-NMR** (100 MHz, CDCl₃): δ 192.3, 171.7, 164.2, 157.0, 154.0, 153.4, 137.5, 129.7, 129.2, 128.9, 126.2, 120.3, 111.6, 81.9, 67.9, 51.0, 44.7, 44.6, 40.5, 39.5, 29.3, 29.2, 29.0, 28.4, 28.0, 27.1, 26.8, 26.5, 26.3, 24.6, 24.3 ppm. **MS (ES)** *m*/*z* = 739.5 [M+Na]⁺.

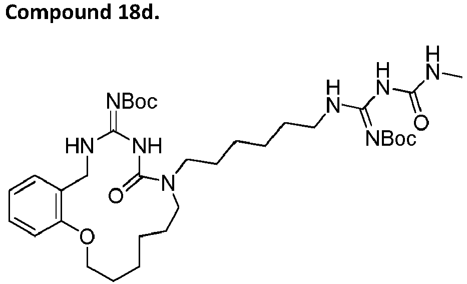

**Compound 18d** was prepared by the procedure described for **compound 32f** starting from compound **17d. Yield:** 50%. **¹H-NMR** (400 MHz, CDCl₃): δ 12.09 (s, 1H), 7.93 (bs, 1H), 7.24-7.21 (m, 1H), 7.16 (d, *J* = 7.2 Hz, 1H), 6.87-6.81 (m, 2H), 5.21 (bs, 1H), 4.44 (s, 2H), 4.01-3.95 (m, 2H), 3.65-3.59 (m, 2H), 3.25-3.18 (m, 4H), 2.74 (s, 3H), 1.55-1.48 (m, 4H), 1.44 (s, 9H), 1.35-1.24 (m, 4H) ppm. **¹³C-NMR** (100 MHz, CDCl₃): δ 158.6, 157.6,156.6,155.7,154.5,154.5, 153.2, 128.7, 127.9, 126.0, 121.2, 112.7, 81.5, 81.4, 69.5, 46.0, 45.9, 41.9, 40.3, 29.3, 28.6, 28.2, 28.1, 27.5, 27.2, 27.0, 26.9, 26.1, 25.9 ppm.

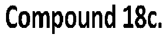

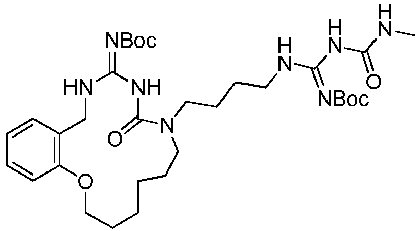

**Compound 18c** was prepared by the procedure described for **compound 32f** starting from compound **17c. Yield:** quantitative. **¹H-NMR** (400 MHz, CDCl₃): δ 12.29 (s, 1H), 12.08 (s, 1H), 8.08 (s, 1H), 7.25-7.17 (m, 2H), 6.86 (t, *J* = 7.2 Hz, 1H), 6.79 (d, *J* = 8.4 Hz, 1H), 4.64 (d, *J* = 5.2 Hz, 2H), 4.01-3.94 (m, 2H), 3.60 (t, *J* = 6.4 Hz, 2H), 3.34-3.21 (m, 4H), 2.73 (d, *J* = 4.4 Hz, 3H), 1.70-1.62 (m, 4H), 1.59-1.50 (m, 4H), 1.42 (s, 9H), 1.39 (s, 9H), 1.28-1.15 (m, 4H) ppm. **¹³C-NMR** (100 MHz, CDCl₃): δ 164.2, 157.0, 153.5, 129.7, 128.9, 126.2, 124.9, 120.3, 111.6, 82.0, 67.9, 44.6, 44.0, 40.6, 39.5, 30.1, 29.4, 29.0, 28.0, 27.3, 27.1, 26.5, 26.3, 25.8, 25.5, 25.2, 22.2, 14.3 ppm.

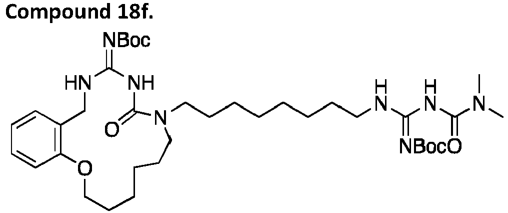

**Compound 18f** was prepared by the procedure described for **compound 32f** starting from compound **17e** and dimethylamine 40% solution in water (Sigma-Aldrich S.r.l., 426458). **Yield** 51%. **¹HNMR** (400MHz CDCl₃) δ ppm 12.37 (s, 1H), 12.27 (s, 1H), 8.09 (s, 1H), 7.95 (s, 1H), 7.22 (m, 2H), 6.88 (t, 1H, *J* = 7.2 Hz), 6.81 (d, 1H, *J* = 8.4 Hz), 4.66 (d, 2H, *J* = 5.2 Hz), 3.97 (s, 2H), 3.62 (m, 2H), 3.29 (m, 2H), 3.28 (m, 2H), 3.06 (s, 3H), 2.90 (s, 3H), 1.67 (m, 4H), 1.54 (m, 4H), 1.43 (s, 9H), 1.41 (s, 9H), 1.29 (m, 12H). **¹³C-NMR** (100 MHz, CDCl₃): δ 167.1, 160.4, 157.1, 154.1, 154.0, 144.4, 141.6, 129.3, 128.9, 127.2, 119.5, 112.4, 80.4, 80.3, 69.7, 45.0, 44.9, 41.1, 40.5, 38.1, 29.2, 29.1, 29.0, 28.4, 28.3, 28.2, 27.5, 27.4, 26.9, 26.8, 26.8, 26.4**LRMS** (ESI) m/z = 753.4 [M+Na]⁺.

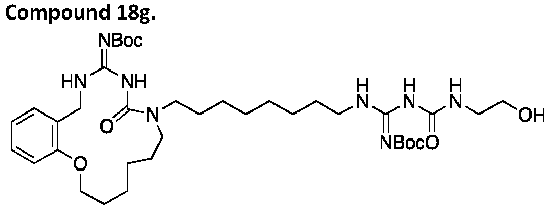

**Compound 18g** was prepared by the procedure described for **compound 32f** starting from compound **17e** and ethanolamine (Sigma-Aldrich S.r.l., 411000).**Yield** 54%. **¹H-NMR** (400MHz CDCl₃) δ ppm 12.34 (s, 1H), 11.92 (s, 1H), 8.09 (m, 2H), 7.23 (m, 2H), 6.89 (t, 1H, *J* = 7.2 Hz), 6.82 (d, 1H, *J* = 8.4 Hz), 5.57 (bs, 1H), 4.66 (d, 2H, *J* = 5.6 Hz), 3.98 (s, 2H), 3.70 (m, 2H), 3.63 (t, 2H, *J* = 6.8 Hz), 3.34 (m, 2H), 3.29 (m, 2H), 3.23 (m, 2H), 1.67 (m, 4H), 1.53 (m, 4H), 1.45 (s, 9H), 1.41 (s, 9H), 1.30 (m, 12H). **¹³C-NMR** (100 MHz CDCl₃) δ ppm 193.0, 172.4, 164.2, 157.0, 154.2, 153.4, 129.7, 129.9, 126.2, 120.3, 111.7, 81.9, 67.9, 63.17, 44.7, 43.0, 39.5, 29.3, 29.1, 28.9, 28.4, 28.0, 27.1, 26.6, 26.3, 24.6, 24.3. **LRMS** (ESI) m/z = 769.5 [M+Na]⁺, 746.5 [M+H]⁺.

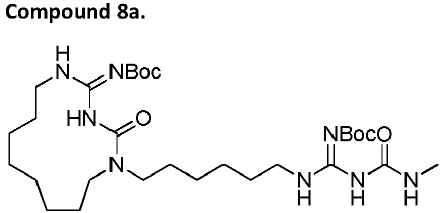

**Compound 8a** was prepared by the procedure described for **compound 32f** starting from compound 7a. **Yield:** 56%. **¹H-NMR** (400 MHz, CDCl₃): δ 12.04 (s, 1H), 8.07 (s, 1H), 3.43 (t, *J* = 5.6 Hz, 2H), 3.37-3.30 (m, 4H), 3.23 (t, *J* = 7.6 Hz, 2H), 2.78 (s, 3H), 1.62-1.53 (m, 8H), 1.44 (s, 9H), 1.42 (s, 9H), 1.39-1.25 (m, 12H) ppm. **¹³C-NMR** (100 MHz, CDCl₃): δ 158.5, 157.6, 155.7, 154.5, 154.4, 152.6, 81.5, 46.3, 46.0, 41.9, 41.7, 28.7, 28.7, 28.2, 28.1, 28.0, 27.5, 27.1, 27.0, 26.9, 26.8, 26.1 ppm.

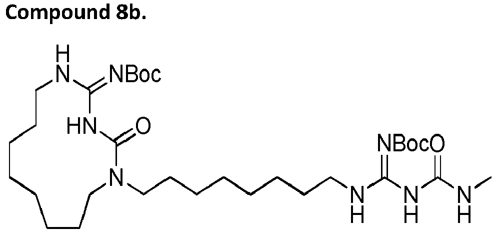

**Compound 8b** was prepared by the procedure described for **compound 32f** starting from compound **7b. Yield:** 54%. **¹H-NMR** (400 MHz, CDCl₃): δ 12.04 (s, 1H), 8.04 (s, 1H), 3.45-3-36 (m, 4H), 3.32 (q, *J* = 6.8 Hz, 2H), 3.20 (t, *J* = 8 Hz, 2H), 2.76 (s, 3H), 1.67-1.55 (m, 8H), 1.44 (s, 9H), 1.41 (s, 9H), 1.30-1.21 (m, 16) ppm. **¹³C-NMR** (100 MHz, CDCl₃): δ 164.0, 154.1, 153.6, 82.0, 53.3, 47.0, 46.3, 39.6, 31.8, 29.6, 29.3, 28.4, 28.0, 27.0, 26.5, 26.2, 25.8, 25.2, 23.5, 22.2, 14.1 ppm.

### General procedure for the final deprotection of compounds.

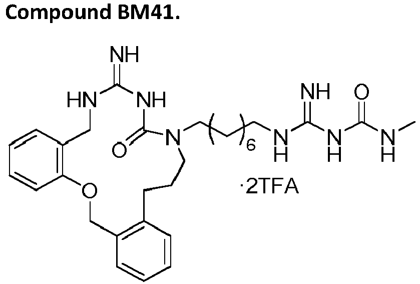

In a flask with magnetic stirrer, **32f** (6 mg, 0.01 mmol) was dissolved in dry CH₂Cl₂ (1.8 mL) under N₂ atmosphere. Then freshly distilled TFA (10% v/v, 200 µL) was added dropwise and the mixture was stirred at room temperature for 7h. The reaction mixture was concentrated *in vacuo.* The residue was decanted several times with Et₂O and Hexane affording the desired compound as trifluoroacetate salt in quantitative yield. **Yield:** quantitative. **¹H-NMR** (400 MHz, MeOD): δ 7.49 (d, 1H, *J* = 7.2 Hz), 7.40 (m, 3H), 7.33 (t, 2H, *J* = 7.6 Hz), 7.25 (d, 1H, *J* = 8.4 Hz), 7.02 (t, 1H, *J* = 7.2 Hz), 5.12 (bs, 2H), 4.45 (s, 2H), 3.34 (m, 4H), 3.29 (t, 2H, *J* = 6.8 Hz), 2.18 (t, 2H, *J* = 6.4 Hz), 2.80 (s, 3H), 1.66 (bs, 2H), 1.64 (m, 2H), 1.58 (m, 2H), 1.43-1.32 (m, 8H) ppm. **¹³C-NMR** (100 MHz, MeOD): δ 162.0, 154.7, 151.5, 131.3, 130.8, 130.1, 129.8, 129.4, 128.8, 126.0, 120.6, 119.5, 112.0, 40.9, 40.5, 29.2, 28.7, 28.6, 27.8, 27.3, 26.1, 26.0, 25.1, 24.5, 22.2 ppm. **MS (ES)** *m*/*z* = 587.4 [M+Na]⁺, 565.4 [M+H]⁺.

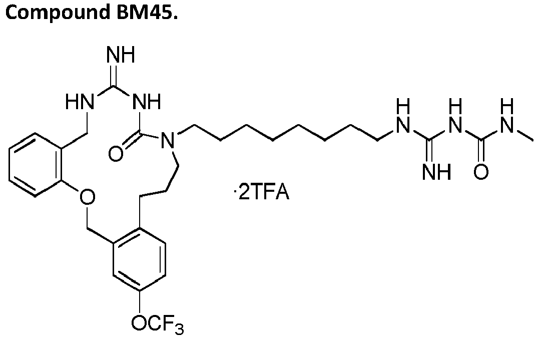

**Compound BM45** was prepared by the procedure described for **compound BM41** starting from compound **32b. Yield** quantitative. **¹H NMR** (400 MHz, MeOD): δ 7.33 (d, 1H, *J* = 17.6 Hz), 7.29 (m, 2H), 7.37 (m, 2H), 7.29 (m, 2H), 7.20 (d, 1H, *J* = 8.4 Hz), 6.99 (t, 1H, *J* = 7.2 Hz), 5.07 (s, 2H), 4.41 (s, 2H), 3.52 (m, 2H), 3.29 (m, 2H), 3.23 (t, 2H, *J* = 6.8 Hz), 2.81 (m, 2H), 2.74 (s, 3H), 2.11 (m, 2H), 1.61 (m, 4H), 1.51 (m, 4H), 1.33 (m, 4H) ppm. **¹³C NMR** (100 MHz, MeOD): δ 161.0, 160.8, 156.5, 156.4, 154.3, 149.1, 149.0, 148.9, 148.8, 136.8, 134.8, 128.7, 127.9, 127.5, 127.1, 123.8, 121.7, 121.3, 119.5, 119.2, 119.2, 118.9, 118.8, 117.4, 112.8, 70.0, 46.0, 45.5, 41.9, 40.1, 30.9, 28.9, 28.8, 28. 7, 27.5, 26.9, 26.6, 26.1, 24.93ppm. **MS (ES)** *m*/*z* = 649.3 [M+H]⁺.

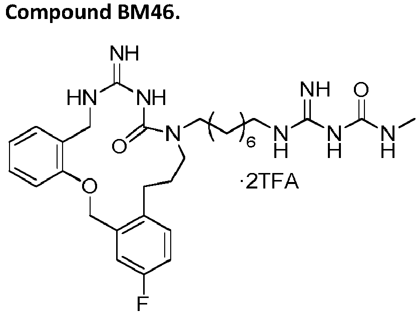

**Compound BM46** was prepared by the procedure described for **compound BM41** starting from compound **32c. Yield** 36%. **¹H NMR** (400 MHz, MeOD): δ 7.32 (m, 3H), 7.21 (m, 1H), 7.14 (m, 1H), 7.06 (m, 1H), 6.95 (m, 1H), 5.05 (s, 2H), 4.42 (s, 2H), 3.29 (m, 4H), 3.24 (t, *J* = 6.8 Hz, 2H), 2.74 (s, 3H), 2.01 (m, 2H), 1.60 (m, 2H), 1.51 (m, 2H), 1.32 (m, 10H) ppm. **¹³C NMR** (100 MHz, MeOD): δ 161.0, 160.00, 156.5, 156.4, 153.9, 129.8, 129.7, 128.7, 127.9, 126.2, 121.5, 116.3, 116.1, 113.9, 113.7, 112.8, 69.8, 69.7, 46.0, 45.5, 41.3, 40.1, 30.4, 29.0, 28.9, 28.8, 27.3, 27.0, 26.6, 26.1, 25.0 ppm. **MS (ES)** *m*/*z* = 583.5 [M+H]⁺.

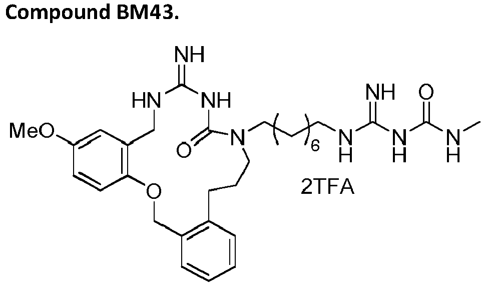

**Compound BM43** was prepared by the procedure described for **compound BM41** starting from compound **32i. Yield:** quantitative. **¹H-NMR** (400 MHz, MeOD): δ 7.49 (m, 1H), 7.40 (m, 2H), 7.31 (m, 1H), 7.17 (m, 1H), 6.97 (m, 2H), 5.08 (m, 2H), 4.43 (m, 2H), 3.82 (m, 3H), 3.36 (m, 4H), 3.30 (m, 4H), 2.87 (bs, 2H), 2.74 (m, 3H), 2.14 (m, 2H), 1.68 (m, 2H), 1.59 (m, 2H), 1.40-1.34 (m, 10H) ppm. **¹³C-NMR** (100 MHz, MeOD): δ ppm 162.0, 161.6, 154.6, 154.3, 153.9, 134.7, 131.0, 129.8, 128.7, 126.0, 124.8, 113.9, 54.8, 48.3, 48.1, 47.8, 47.6, 47.4, 47.2, 47.0, 40.9, 31.6, 29.3, 29.0, 28.7, 28.6, 27.8, 27.3, 26.1, 25.2, 22.3 ppm. **MS (ES)** *m*/*z* = 617.4 [M+Na]⁺, 595.5 [M+H]⁺.

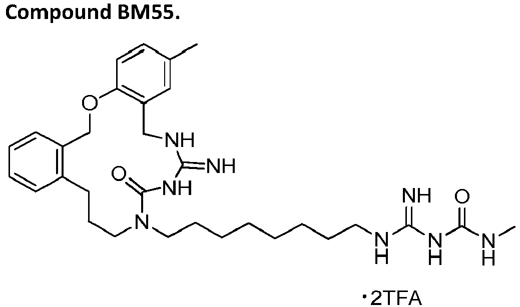

**Compound BM55** was prepared by the procedure described for **compound BM41** starting from compound **32h. Yield:** quantitative. **¹H-NMR** (400 MHz, MeOD): δ 7.63-7.55 (m, 1H), 7.45-7.40 (m, 1H), 7.37-7.30 (m, 2H), 7.27-7.21 (m, 1H), 7.13-7.08 (m, 2H), 5.09 (s, 2H), 4.36 (s, 2H), 3.59-3.55 (m, 2H), 3.43-3.38 (m, 2H), 3.26-3.22 (m, 2H), 2.82-2.76 (m, 2H), 2.74 (s, 3H), 2.27 (s, 3H), 2.02-1.96 (m, 2H), 1.61-1.53 (m, 4H), 1.40-1.34 (m, 10H) ppm. **¹³C-NMR** (100 MHz, MeOD): δ 160.8, 159.7, 159.4, 156.6, 155.2, 137.9, 135.0, 134.8, 129.3, 129.2, 128.2, 127.6, 126.7, 126.6, 125.7, 114.3, 69.3, 45.5, 45.0, 42.5, 41.8, 31.5, 28.8, 28.6, 28.6, 28.2, 27.2, 26.9, 26.8, 26.6, 20.9 ppm.

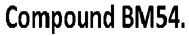

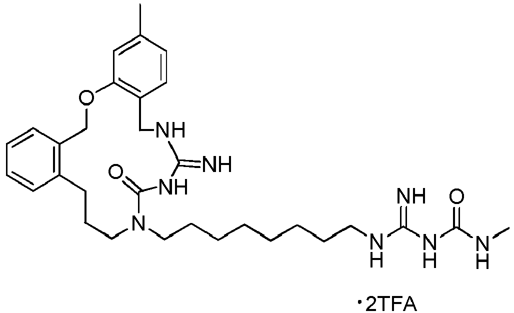

**Compound BM54** was prepared by the procedure described for **compound BM41** starting from compound **32g. Yield:** quantitative. **¹H-NMR** (400 MHz, MeOD): δ 7.42-7.33 (m, 3H), 7.26-7.19 (m, 1H), 7.13 (d, *J* = 7.2 Hz, 1H), 7.01 (s, 1H), 6.77 (d, *J* = 7.2 Hz, 1H), 5.05 (s, 2H), 4.35 (s, 2H), 3.32-3.26(m, 4H), 2.83-2.77 (m, 2H), 2.74 (s, 3H), 2.33 (s, 3H), 2.05-1.97 (m, 2H), 1.52-1.47 (m, 4H), 1.38-1.26 (m, 10H) ppm. **¹³C-NMR** (100 MHz, MeOD): δ 160.8, 159.7, 159.4, 157.2, 155.2, 139.1, 137.9, 134.8, 129.2, 128.2, 127.6, 126.7, 125.9, 125.0, 113.2, 69.3, 45.5, 45.0, 42.5, 41.7, 31.5, 28.8, 28.6, 28.6, 28.2, 27.2, 26.9, 26.8, 26.6, 21.3 ppm.

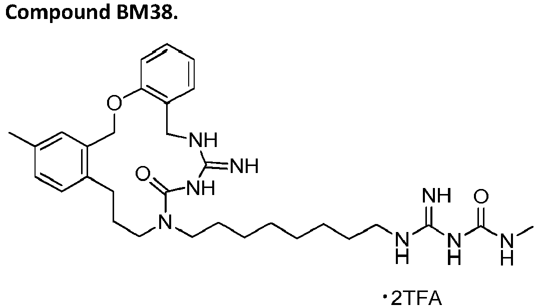

**Compound BM38** was prepared by the procedure described for **compound BM41** starting from compound **32a. Yield:** quantitative. **¹H-NMR** (400 MHz, MeOD): δ 7.34 (t, *J* = 7.6 Hz, 1H), 7.29-7.25 (m, 2H), 7.20-7.12 (m, 3H), 6.95 (t, *J* = 7.2 Hz, 1H), 5.01 (s, 2H), 4.39 (s, 2H), 3.49-3.43 (m, 2H), 3.22 (t, *J* = 6.8 Hz, 2H), 2.88-2.65 (m, 5H), 2.32 (s, 3H), 2.11-1.98 (m, 2H), 1.60-1.51 (m, 4H), 1.37-1.26 (m, 10H) ppm. **¹³C-NMR** (100 MHz, MeOD): δ 160.8, 159.7, 159.4, 156.73, 155.2, 137.6, 137.0, 134.4, 128.9, 128.8, 128.0, 127.1, 126.8, 125.3, 121.6, 114.1, 69.3, 45.5, 45.0, 42.5, 41.7, 31.5, 28.8, 28.6, 28.6, 28.2, 27.2, 26.9, 26.8, 26.6, 21.0 ppm. **MS (ES)** *m*/*z* = 579.3 [M+H]⁺, 290.1 [M+2H]⁺.

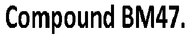

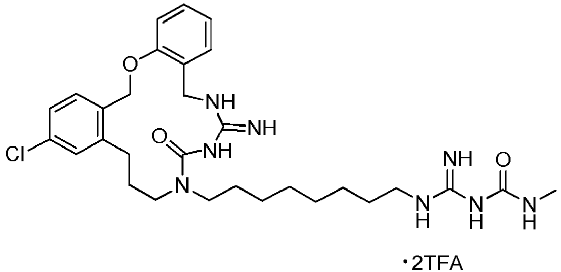

**Compound BM47** was prepared by the procedure described for **compound BM41** starting from compound **32d. Yield:** quantitative. **¹H-NMR** (400 MHz, MeOD): δ 7.47-7.42 (m, 1H), 7.41-7.37 (m, 2H), 7.35-7.29 (m, 2H), 7.23 (d, *J* = 8.4 Hz, 1H), 7.02 (t, *J* = 7.2 Hz, 1H), 5.08 (s, 2H), 4.45 (s, 2H), 3.59-3.52 (m, 2H), 3.28 (t, *J* = 6.8 Hz, 2H), 2.87-2.81 (m, 2H), 2.79 (s, 3H), 2.19-2.07 (m, 2H), 1.66-1.57 (m, 4H), 1.38-1.31 (m, 10H) ppm. **¹³C-NMR** (100 MHz, MeOD): δ 160.3, 160.1, 156.5, 156.5, 154.3, 141.0, 134.6, 132.8, 128.8, 128.1, 128.0, 127.9, 126.4, 126.2, 121.3, 112.8, 70.0, 46.0, 45.5, 41.9, 40.1, 30.4, 29.0, 28.9, 28.8, 27.5, 26.9, 26.6, 26.1, 24.9 ppm.

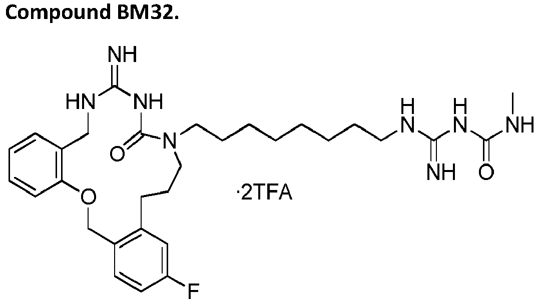

**Compound BM32** was prepared by the procedure described for **compound BM41** starting from compound **32e. Yield:** quantitative. **¹H-NMR** (400 MHz, MeOD): δ 7.35 - 7.29 (m, 1H), 7.20 - 7.13 (m, 1H), 6.99 - 6.86 (m, 5H), 5.29 (s, 2H), 5.00 (s, 2H), 4.46 (s, 2H), 3.48 (s, 3H), 3.25 - 3.13 (m, 4H), 2.79 - 2.73 (m, 2H), 2.56 - 2.49 (m, 2H), 2.00 - 1.93 (m, 2H), 1.63 - 1.53 (m, 2H), 1.51 - 1.41 (m, 2H), 1.34 - 1.20 (m, 10H) ppm. **¹³C-NMR** (100 MHz, MeOD): δ 161.0, 160.3, 156.5, 156.5, 154.3, 128.8, 128.3, 128.2, 127.9, 126.2, 121.6, 115.6, 115.4, 113.7, 113.6, 112.8, 69.2, 46.0, 45.5, 41.9, 40.1, 30.3, 30.3, 29.0, 28.9, 28.8, 27.5, 26.9, 26.6, 26.1, 24.9 ppm. **MS (ES)** *m*/*z* = 583.4 [M+H]⁺.

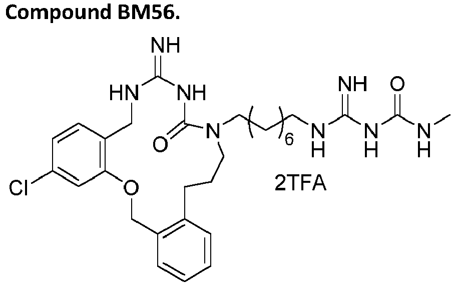

**Compound BM56** was prepared by the procedure described for **compound BM41** starting from compound **32j Yield:** quantitative. **¹H-NMR** (400 MHz, MeOD): δ 7.43-7.38 (m, 1H), 7.27 (m, 2H), 7.23-7.20 (m, 3H), 6.98 (d, *J* = 8.0 Hz, 1H), 5.07 (s, 2H), 4.37 (m, 2H), 3.52 (m, 2H), 3.29 (m, 4H), 2.78 (m, 2H), 2.74 (s, 3H), 2.07 (m, 2H), 1.59 (m, 2H), 1.53 (m, 2H), 1.37-1.26 (m, 8H) ppm. **¹³C-NMR** (100 MHz, MeOD): δ ppm 163.1, 162.2, 162.1, 158.5, 152.8, 141.2, 136.8, 132.6, 130.1, 129.3, 128.4, 128.1, 127.1, 126.2, 124.7, 115.1, 65.3, 44.8, 42.3, 41.4, 41.1, 32.8, 29.0, 28.9, 27.4, 26.5, 25.6, 25.2, 24.4, 22.2 ppm. **MS (ES)** *m*/*z* = 621.4 [M+Na]⁺, 599.4 [M+H]⁺.

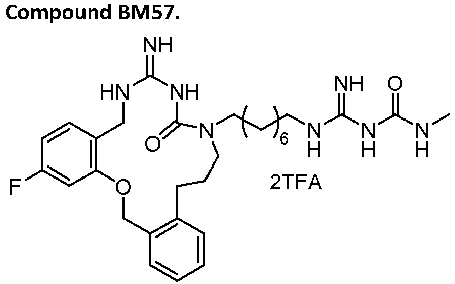

**Compound BM57** was prepared by the procedure described for **compound BM41** starting from compound **32k. Yield:** quantitative. **¹H NMR** (400 MHz, CDCl₃): *δ* 7.44 (d, *J* = 7.2 Hz, 1H), 7.35 (m, 2H), 7.29 (t, *J* = 10.4 Hz, 1H), 7.01 (d, *J* = 8.4 Hz, 1H), 6.69 (t, *J* = 15.4 Hz, 1H), 5.06 (s, 2H), 4.37 (s, 2H), 3.49 (s, 2H), 3.29 (s, 2H), 3.25-3.21 (t, *J* = 17.4 Hz, 2H), 2.78 (s, 2H), 2.74 (s, 3H), 2.13-2.10 (m, 2H), 1.60-1.57 (m, 2H), 1.54 (t, *J* = 10.5 Hz, 2H), 1.34-1.21 (m, 8H) ppm. **¹³C-NMR** (100 MHz, MeOD): δ ppm 164.2, 163.1, 162.2, 162.1, 158.7, 152.8, 141.2, 136.8, 130.8, 129.3, 128.4, 127.1, 126.2, 124.9, 107.9, 102.1, 68.8, 45.8,42.6,41.5,41.0, 31.9, 29.1, 29.0, 27.5, 27.4, 26.8, 25.5, 24.2, 23.2 ppm. **MS (ES)** *m*/*z* = 583.4 [M+H]⁺.

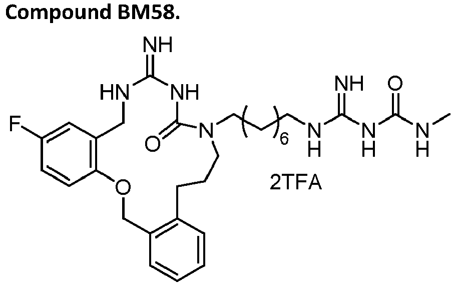

**Compound BM58** was prepared by the procedure described for **compound BM41** starting from compound **32I. Yield:** quantitative. **¹H-NMR** (400 MHz, MeOD): *δ* 7.41 (m, 1H), 7.32 (m, 2H), 7.23 (s, 1H), 7.09-7.04 (m, 3H), 5.06 (s, 2H), 4.50 (s, 2H), 3.29-3.24 (m, 6H), 2.74 (s, 3H), 2.70 (m, 2H), 2.03-1.94 (m, 2H), 1.59 (t, *J* = 10.6 Hz, 2H), 1.53 (t, *J* = 10.3 Hz, 2H), 1.52-1.34 (m, 8H) ppm. **¹³C-NMR** (100 MHz, MeOD): δ 163.1, 162.2, 162.1, 159.8, 154.8, 152.8, 141.2, 136.8, 129.3, 128.4, 128.2, 127.1, 126.2, 116.6, 115.3, 114.8, 65.6, 45.1, 43.4, 41.6, 39.9, 32.9, 29.2, 29.1, 27.5, 27.4, 26.8, 26.2, 25.8, 23.9 ppm. **MS (ES)** *m*/*z* = 583.4 [M+H]⁺.

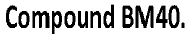

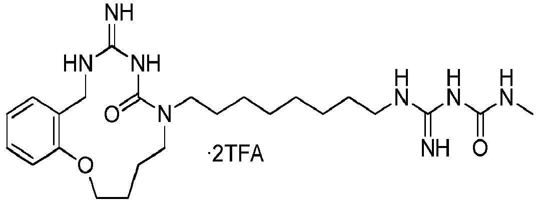

**Compound BM40** was prepared by the procedure described for **compound BM41** starting from compound **18a. Yield:** quantitative. **¹H-NMR** (400 MHz, MeOD): δ 7.28 - 7.17 (m, 2H), 6.94 - 6.82 (m, 2H), 4.62 (s,2H), 3.92 (m, 2H), 3.28 (m, 2H), 3.18 (m, 2H), 2.91 (m, 2H), 2.79 (s, 3H), 1.77 - 1.47 (m, 8H), 1.36 - 1.21 (m, 7H) ppm. **¹³C-NMR** (100 MHz, MeOD): δ 160.1, 160.0, 156.6, 156.5, 154.3, 129.3, 127.9, 126.7, 121.7, 112.7, 69.2, 46.3, 46.0, 41.9, 40.1, 28.9, 28.8, 28.7, 27.4, 27.4, 27.0, 26.1, 24.9, 24.6 ppm. **MS (ES)** *m*/*z* = 511.4 [M+Na]⁺, 489.3 [M+H]⁺.

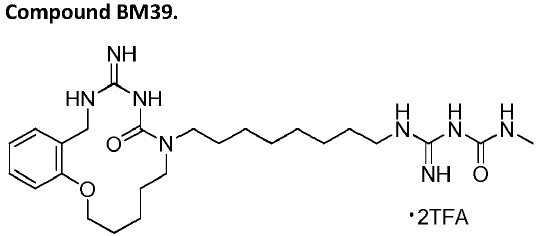

**Compound BM39** was prepared by the procedure described for **compound BM41** starting from compound **18b. Yield:** quantitative. **¹H-NMR** (400 MHz, MeOD): δ 7.26 (t, *J* = 7.6 Hz, 2H), 7.00 (d, *J* = 8.8 Hz, 1H), 6.98-6.89 (m, 1H), 4.48 (s, 2H), 4.11 (t, *J* = 4.4 Hz, 2H), 3.49-3.40 (m, 2H), 3.26-3.24 (m, 2H), 3.21 (t, *J* = 7.2 Hz, 2H), 2.71 (s, 3H), 1.61-1.48 (m, 8H), 1.35-1.24 (m, 10H) ppm. **¹³C-NMR** (100 MHz, MeOD): δ 160.1, 160.0, 156.6, 156.5, 154.3, 129.4, 127.9, 126.7, 121.7, 112.7, 68.9, 46.1, 46.0, 41.9, 40.1, 29.0, 28.9, 28.8, 28.7, 27.5, 27.0, 26.9, 26.1, 24.9, 24.7 ppm.

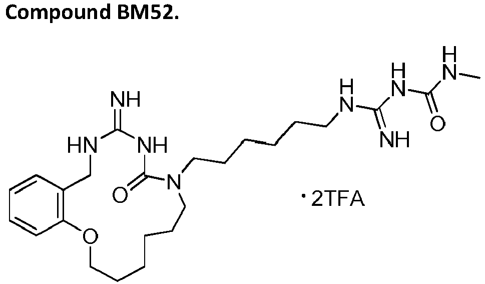

**Compound BM52** was prepared by the procedure described for **compound BM41** starting from compound **18d. Yield:** quantitative. **¹H-NMR** (400 MHz, MeOD): δ 7.34 (t, *J* = 7.6 Hz, 1H), 7.29 (d, *J* = 7.2 Hz, 1H), 6.97 (d, *J* = 8.4 Hz, 1H), 6.92 (t, *J* = 7.2 Hz, 1H), 4.40 (s, 2H), 4.02 (t, *J* = 4Hz, 2H), 3.55-3.46 (m, 2H), 3.35-3.28 (m, 2H), 3.24 (t, *J* = 7.2Hz, 2H), 2.74 (s, 3H), 1.82-1.74 (m, 2H), 1.70-1.65 (m, 2H), 1.63-1.56 (m, 4H), 1.43-1.33 (m, 4H) ppm. **¹³C-NMR** (100 MHz, MeOD): δ 160.1, 160.0, 156.6, 156.5, 154.3, 129.3, 127.9, 126.7, 121.7, 112.7, 69.4, 46.0, 45.9, 41.9, 40.1, 29.2, 28.9, 27.5, 27.2, 26.9, 26.8, 26.8, 26.1, 26.0 ppm.

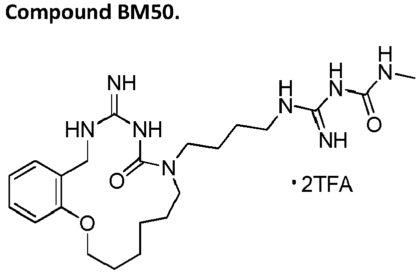

**Compound BM50** was prepared by the procedure described for **compound BM41** starting from compound **18c. Yield:** quantitative. **¹H-NMR** (400 MHz, MeOD): δ 7.31 (t, *J* = 7.6 Hz, 1H), 7.26 (d, *J* = 6.8 Hz, 1H), 6.94 (d, *J* = 8 Hz, 1H), 6.89 (t, *J* = 7.6 Hz, 1H), 4.38 (s, 2H), 3.99 (t, *J* = 4.4 Hz, 2H), 3.51-3.43 (m, 2H), 3.35-3.29 (m, 2H), 3.26 (t, *J* = 2 Hz, 2H), 2.71 (s, 3H), 1.77-1.69 (m, 2H), 1.68-1.60 (m, 2H), 1.50-1.39 (m, 4H), 1.35-1.24 (2H) ppm. **¹³C-NMR** (100 MHz, MeOD): δ 160.1, 160.0, 156.6, 156.5, 154.2, 129.2, 127.9, 126.7, 121.7, 112.7, 69.4, 45.8, 45.8, 40.9, 40.1, 29.2, 27.3, 27.2, 26.7, 26.1, 26.0, 25.0 ppm.

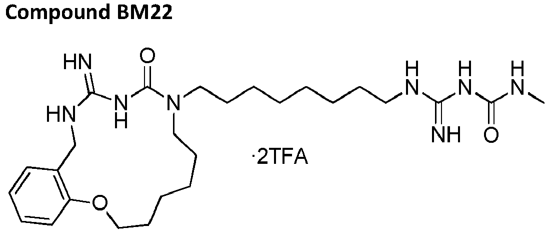

**Compound BM22** was prepared by the procedure described for **compound BM41** starting from compound **18e. Yield:** quantitative. **¹HNMR** (400 MHz, MeOD): δ 7.32 (t, 1H, *J* = 8.0 Hz), 7.27 (d, 1H, *J* = 7.2 Hz), 6.96 (d, 1H, *J* = 8.4 Hz), 6.921 (t, 1H, *J* = 7.6 Hz), 4.40 (s, 2H), 4.01 (m, 2H), 3.48 (m, 2H), 3.28 (m, 2H), 3.23 (t, 2H, *J* = 7.2 Hz) 2.73 (s, 3H), 1.75 (m, 2H), 1.60 (m, 2H), 1.55 (m, 2H), 1.48 (m, 2H), 1.44-1.37 (m, 2H), 1.33-1.19 (m, 10H) ppm. **¹³C-NMR** (100 MHz, MeOD): δ 160.1, 160.0, 156.6, 156.5, 154.3, 129.1, 127.9, 126.7, 122.8, 112.7, 69.4, 46.0, 45.9, 41.9, 40.1, 29.3, 28.9, 28.8, 28.7, 27.4, 27.2, 26.9, 26.9, 26.1, 25.9, 24.9. **MS (ES)** *m*/*z* = 539.2 [M+Na]⁺, 517.3 [M+H]⁺.

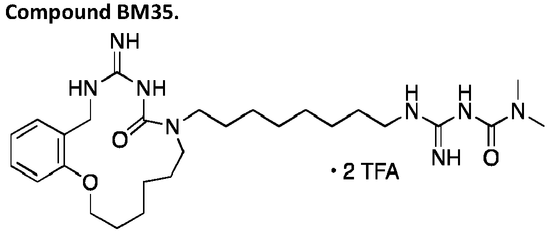

**Compound BM35** was prepared by the procedure described for **compound BM41** starting from compound **18f. Yield:** quantitative. **¹H-NMR** (400MHz MeOD) δ ppm 7.33 (t, 1H, *J* = 7.33 Hz), 7.28 (d, 1H, *J* = 7.2 Hz), 7.96 (d, 1H, *J* = 8.4 Hz), 6.91 (t, 1H, *J* = 7.6 Hz), 4.40 (s, 2H), 4.01 (t, 2H, *J* = 4.4 Hz), 3.48 (m, 2H), 3.27 (m, 2H), 2.98 (s, 6H), 1.76 (m, 2H), 1.65-1.58 (m, 4H), 1.55 (m, 4H), 1.45 (m, 2H), 1.37-1.26 (m, 10H). **¹³C-NMR** (100 MHz MeOD) δ ppm 163.7, 163.5, 159.1, 156.2, 155.8 ,155.5, 131.9, 131.8, 126.1, 123.5, 121.4, 112.4, 68.7, 47.1, 46.6, 44.7, 42.5, 36.6, 30.9, 30.7, 30.4, 30.2, 30.1, 29.2, 28.4, 28.0, 27.7, 27.6, 26.4. **LRMS** (ESI) m/z = 553.4 [M+Na]⁺, 530.4 [M+H]⁺.

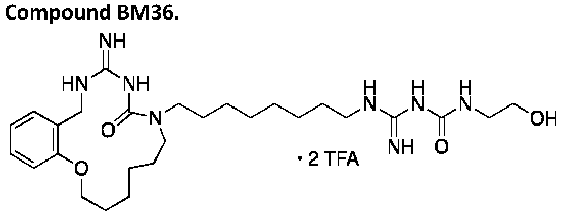

**Compound BM36** was prepared by the procedure described for **compound BM41** starting from compound **18g. Yield:** quantitative. **¹HNMR** (400MHz MeOD) δ ppm 7.33 (t, 1H, *J* = 8.0 Hz), 7.27 (d, 1H, *J* = 7.6 Hz), 6.96 (d, 1H, *J* = 8.4 Hz), 6.91 (t, 1H, *J* = 7.6 Hz), 4.40 (s, 2H), 4.01 (m, 2H), 3.59 (t, 2H, *J* = 5.2 Hz), 3.48 (bs, 1H), 3.32-3.29 (m, 6H), 3.18 (m, 2H), 1.76 (m, 2H), 1.65-1.47 (m, 10H), 1.38-1.33 (m, 6H). **¹³C-NMR** (100 MHz MeOD) δ ppm 163.1, 162.2, 162.1, 157.1, 153.6, 129.3, 128.9, 127.2, 119.5, 112.4, 69.7, 62.5, 45.0, 44.9, 42.4, 41.5, 41.1, 29.2, 29.1, 29.0, 27.5, 27.4, 26.9, 26.8, 26.8, 26.4. **LRMS** (ESI) m/z = 569.5 [M+Na]⁺, 546.5 [M+H]⁺.

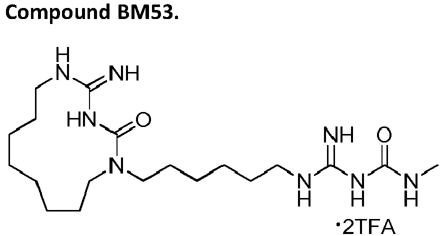

**Compound BM53** was prepared by the procedure described for **compound BM41** starting from compound **8a. Yield:** quantitative. **¹H-NMR** (400 MHz, MeOD): δ 3.50-3.42 (m, 2H), 3.39-3.29 (m, 4H), 3.28-3.22 (m, 2H), 2.74 (s, 3H), 1.65-1.59 (m, 8H), 1.45-1.29 (m, 12H) ppm. **¹³C-NMR** (100 MHz, MeOD): δ 161.0, 160.3, 156.5, 154.1, 46.1, 46.0, 41.9, 41.3, 29.1, 28.7, 27.6, 27.5, 27.4, 27.0, 26.9, 26.8, 26.4, 26.0, 24.4 ppm.

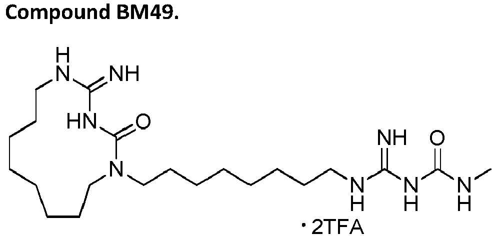

**Compound BM49** was prepared by the procedure described for **compound BM41** starting from compound **8b. Yield:** quantitative. **¹H-NMR** (400 MHz, MeOD): δ 3.49-3.38 (m, 2H), 3.35-3.29 (m, 4H), 3.22 (t, *J* = 7.2 Hz, 2H), 2.72 (s, 3H), 1.70-1.54 (m, 8H), 1.40-1.26 (m, 16H) ppm. **¹³C-NMR** (100 MHz, MeOD): δ 160.3, 160.0, 156.5, 154.2, 46.0, 45.9, 41.9, 41.1, 29.1, 29.0, 28.9, 28.7, 27.6, 27.5, 27.4, 26.9, 26.8, 26.4, 26.1, 24.8, 24.3 ppm.

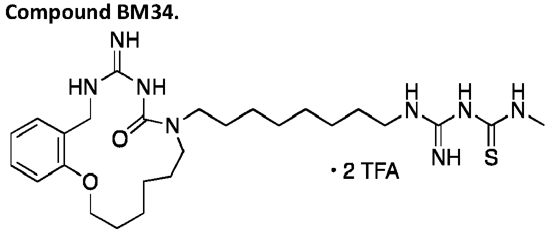

In a flask with magnetic stirrer, **17e** (30 mg, 0.04 mmol) was dissolved in dry CH₂Cl₂ (1.8 mL) under N₂ atmosphere. Then freshly distilled TFA (10% v/v, 200 µL) was added dropwise and the mixture was stirred at room temperature for 7h. The reaction mixture was concentrated *in vacuo.* The residue was decanted several times with Et₂O and Hexane. To this residue, solubilized in in dry DCM (2 mL) under N₂ atmosphere, DBU (Sigma-Aldrich S.r.l., 33482) (0,03 mL, 0,17 mmol) was added and, then, methyl isothiocyanate (Sigma-Aldrich S.r.l., 112771) (0,08 g, 0,11 mmol), and the mixture war stirred for 30 h. The mixture was then washed with H₂O, Brine, dried over Na₂SO₄, filtered and evaporated under reduced pressure. The crude residue was purified with flash chromatography on silica gel, eluting with 10% MeOH in DCM. The obtained compound was directly solubilized in a 10% solution of TFA in dry DCM (2 mL) under N₂ atmosphere and stirred for 8 h. The reaction mixture was then concentrated under reduced pressure. The residue was decanted several times with Et₂O and Hexane affording the desired compound **BM34** as trifluoroacetate salt. **Yield** 52% (overall yield). **¹HNMR** (400MHz MeOD) δ ppm 7.22 (m, 2H), 6.86 (m, 2H), 4.44 (s, 2H), 3.98 (m, 2H), 3.59 (m, 2H), 3.25 (m, 1H), 3.23-3.18 (m, 3H), 2.95 (s, 1.5H, rotamer), 2.82 (s, 1.5H, rotamer), 1.68 (m, 4H), 1.55 (m, 4H), 1.32 (m, 12H). **¹³CNMR** (100 MHz MeOD) δ ppm 158.3, 157.1, 129.5, 128.9, 119.8, 111.1, 67.4, 44.7, 44,3, 40.6, 29.6, 29.3, 29.0, 28.8, 28.6, 27.6, 26.6, 26.4, 24.9, 24.3. **LRMS** (ESI) m/z = 533.4 [M+H]⁺.

### BIOLOGICAL EVALUATION

### In vitro enzymatic assay

Inhibition constants (*Kᵢ* values) against human Acidic Mammalian chitinase (CycLex Co., Ltd., Nagano, Japan, CY-E1248) were determined using a fluorometric enzyme assay performed at 25 °C in assay buffer (40 mM Na₃PO₄, 20 mM Citric acid, BSA 1 mg/mL, pH 4.2). The enzyme activity was measured as the rate of hydrolysis of the fluorescent substrate 4-methylumbelliferyl-β-D-*N,N',N"*-triacetylchitotrioside (Sigma-Aldrich, M5639), followed using an excitation wavelength of 330 nm and an emission wavelength of 442 nm. Enzyme assays were carried out by preincubating the enzyme in the presence of variable concentrations of the tested inhibitor (10 to 100 µM) or in assay buffer (control) for 20 minutes. Enzyme activity was then determined following the variation of fluorescence after the addition of 20 µM substrate. The time-dependence of fluorescence variation determined at various inhibitor concentrations allowed to compute the *Kᵢ* values using a competitive model of inhibition as previously described (such as the Dixon graphical method, using substrate concentrations varying between 5 and 50 µM and inhibitor concentrations ranging 10 to 200 µM). Alternatively, the ratio *v*ₒ/*v*ᵢ (where *v*ₒ is the hydrolysis rate in the absence of inhibitor and *v*ᵢ is the hydrolysis rate in the presence of inhibitor) was plotted as a function of the inhibitor concentration, yielding linear data whose slope equals to *Kₘ*/(*Kₘ* + [S])*Kᵢ* (Docquier et al. J. Antimicrob. Chemother (2003) 51:257; Chou et al. Biochemistry (2006) 45:4444). Bisdionin F (Merck S.p.a., 112252), a known inhibitor of both AMCase and chitotriosidase (CHIT1) enzymes, was used as a reference compound in all assays.

Preferably, an active compound exhibits a *Kᵢ* lower than 50 µM and/or a % of inhibition at a fixed concentration (20, 50 or 100 µM) equal to or greater than 70 %. The % of inhibition can be defined as the fraction of inhibited enzyme expressed in percentage, and is computed as (1 - (*v*ᵢ/*v*₀)) × 100.

### Results

Compounds of the invention were tested against human AMCase. As shown in Table 1, they showed a good activity, especially those with two aromatic rings. Further, it is clear that the introduction of the second phenyl ring (substituted or not) is an added value, because its presence is probably linked to the possibility for the compounds to do other interactions, thus improving the binding with the enzyme.

**Table 1: Enzymatic assay against human AMCase**

| **Inhibitor** | **Structure** | **Ki (µM)** |
|---|---|---|
| **Bisdionin F** | 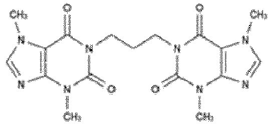 | 13.1 ± 0.6 |
| **BM22** | 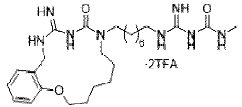 | 13.2 ± 0.1 |
| **BM32** | 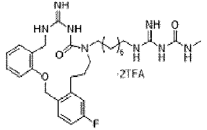 | 3.8 ± 0.6 |
| **BM38** | 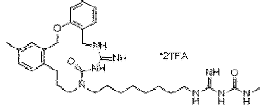 | 5.1 ± 0.3 |
| **BM39** | 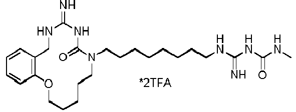 | 4.5 ± 0.5 |
| **BM40** | 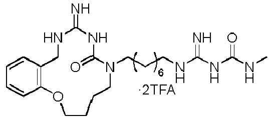 | 9.7 ± 0.3 |
| **BM41** | 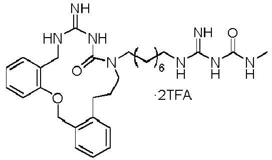 | 18.4 ± 0.7 |
| **BM43** | 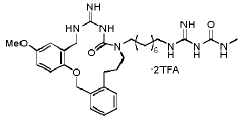 | 21.0 ± 0.1 |
| **BM46** | 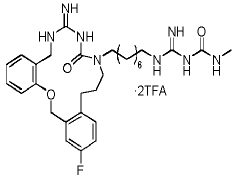 | 9.0 ± 0.4 |
| **BM47** | 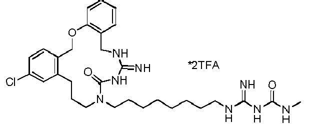 | 1.9 ± 0.2 |
| **BM49** | 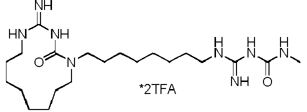 | 18.4 ± 0.7 |
| **BM50** | 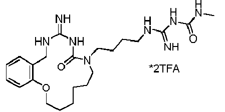 | 27.7 ± 0.3 |

### REFERENCES

(1) http://www.who.int/respiratory/asthma/en/.
(2) Locksley, R. M. Asthma and Allergic Inflammation. Cell 2010, 140 (6), 777-783.
(3) https://www.nhlbi.nih.gov/health-topics/asthma.
(4) Katzung, B. G.; Masters, S. B.; Trevor, A. J. Basic and Clinical Pharmacology, 12th editi.; 2012.
(5) Zhu, Z.; Zheng, T.; Homer, R. J.; Kim, Y. K.; Chen, N. Y.; Cohn, L.; Hamid, Q.; Elias, J. A. Acidic Mammalian Chitinase in Asthmatic Th2 Inflammation and IL-13 Pathway Activation. Science (80-.). 2004, 304 (5677), 1678-1682.
(6) Boot, R. G.; Blommaart, E. F.; Swart, E.; Ghauharali-van der Vlugt, K.; Bijl, N.; Moe, C.; Place, A.; Aerts, J. M. Identification of a Novel Acidic Mammalian Chitinase Distinct from Chitotriosidase. J. Biol. Chem. 2001, 276 (9), 6770-6778.
(7) Kawada, M.; Hachiya, Y.; Arihiro, A.; Mizoguchi, E. Role of Mammalian Chitinases in Inflammatory Conditions. Keio J. Med. 2007, 56 (1), 21-27.
(8) Elias, J. A.; Lee, C. G.; Zheng, T.; Ma, B.; Homer, R. J.; Zhu, Z. New Insights into the Pathogenesis of Asthma. J. Clin. Invest. 2003, 111 (3), 291-297.
(9) Miel, H.; Rault, S. Conversion of N,N'-Bis(Tert-Butoxycarbonyl)Guanidines to N-(N'-Tert-Butoxycarbonylamidino)Ureas. Tetrahedron Lett. 1998, 39 (12), 1565-1568.
(10) Prabhakaran, P.; Sanjayan, G. J. N,N',N"-Tri-Boc-Guanidine (TBG): A Common Starting Material for Both N-Alkyl Guanidines and Amidinoureas. Tetrahedron Lett. 2007, 48 (10), 1725-1727.
(11) Gradillas, A.; Pérez-Castells, J. Macrocyclization by Ring-Closing Metathesis in the Total Synthesis of Natural Products: Reaction Conditions and Limitations. Angew. Chemie Int. Ed. 2006, 45 (37), 6086-6101.
(12) Edlin, C. D.; Faulkner, J.; Quayle, P. Catalyst Economy. Part 2: Sequential Metathesis-Kharasch Sequences Using the Grubbs Metathesis Catalysts. Tetrahedron Lett. 2006, 47 (7), 1145-1151.
(13) Docquier, J.D.; Lamotte-Brasseur, J.; Galleni, M.; Amicosante, G.; Frère, J.M.; Rossolini, G.M. On functional and structural heterogeneity of VIM-type metallo-beta-lactamases. J Antimicrob Chemother 2003, 51 (2), 257-66.
(14) Chou, Y.T.; Yao, S.; Czerwinski, R.; Fleming, M.; Krykbaev, R.; Xuan, D.; Zhou, H.; Brooks, J.; Fitz, L.; Strand, J.; Presman, E.; Lin, L.; Aulabaugh, A.; Huang, X. Kinetic characterization of recombinant human acidic mammalian chitinase. Biochemistry 2006, 45 (14), 4444-54.

## Claims

1. A compound of formula (I): 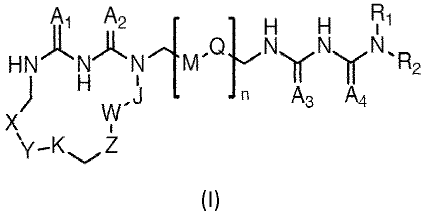 or a salt, a solvate, a tautomer or a stereoisomer thereof,
wherein:
n is a number from 0 to 3;
J is a linear saturated or unsaturated C₁-C₄ alkyl chain;
K is -CH₂-, O or S;
A₁, A₂, A₃, A₄ are the same or different and are each independently selected from the group consisting of: NH, O and S;
R₁ and R₂ are the same or different and are each independently selected from the group consisting of: H, substituted or unsubstituted C₁-C₆ alkyl, substituted or unsubstituted C₂-C₆ alkenyl, substituted or unsubstituted C₂-C₆ alkynyl, substituted or unsubstituted aryl and substituted or unsubstituted heteroaryl, wherein the substituted C₁-C₆ alkyl, the substituted C₂-C₆ alkenyl, the substituted C₂-C₆ alkynyl, the substituted aryl and the substituted heteroaryl are each independently substituted by one or more substituents each independently selected from the group consisting of: halogen, NO₂, substituted or unsubstituted phenyl, OR_{A}, C(O)OR_{B}, OC(O)R_{B}, SR_{A}, SO₃H, SOR_{A}, SO₂R_{A}, NR_{A}R_{B}, OP(O)(OR_{A}), OP(O)(OR_{A})(OR_{B}), OC(O)NR_{A}R_{B}, NR_{A}C(O)OR_{B}, C(O)NR_{A}R_{B}, NR_{A}C(O)R_{B}, N(COR_{A})(COR_{B}), C(O)NR_{A}C(O)R_{B}, C(O)ONR_{A}R_{B}, C(NOR_{A})R_{B}, C(O)R_{A}, CN, haloalkyl, CF₃, OCF₃, substituted or unsubstituted cycloalkyl, substituted or unsubstituted C₁-C₆ alkyl, substituted or unsubstituted C₂-C₆ alkenyl and substituted or unsubstituted C₂-C₆ alkynyl;
or R₁ and R₂ together with the nitrogen to which they are attached form a 4-7 membered saturated, partially unsaturated or aromatic ring optionally containing one or more additional heteroatoms independently selected from N, S and O, the ring being optionally substituted by one, two or more groups independently selected from: halogen, NO₂, substituted or unsubstituted phenyl, OR_{A}, C(O)OR_{B}, OC(O)R_{B}, SR_{A}, SO₃H, SOR_{A}, SO₂R_{A}, NR_{A}R_{B}, OP(O)(OR_{A}), OP(O)(OR_{A})(OR_{B}), OC(O)NR_{A}R_{B}, NR_{A}C(O)OR_{B}, C(O)NR_{A}R_{B}, NR_{A}C(O)R_{B}, N(COR_{A})(COR_{B}), C(O)NR_{A}C(O)R_{B}, C(O)ONR_{A}R_{B}, C(NOR_{A})R_{B}, C(O)R_{A}, CN, haloalkyl, CF₃, OCF₃, substituted or unsubstituted cycloalkyl, substituted or unsubstituted C₁-C₆ alkyl, substituted or unsubstituted C₂-C₆ alkenyl and substituted or unsubstituted C₂-C₆ alkynyl;
R_{A} and R_{B} are the same or different and are each independently selected from: H, unsubstituted or substituted C₁-C₆ alkyl, unsubstituted or substituted aralkyl, unsubstituted or substituted aryl, haloalkyl, or R_{A} and R_{B} together with the nitrogen, sulfur, or oxygen to which they are attached, form a 4-7 membered saturated, partially unsaturated or aromatic ring optionally containing one or more additional heteroatoms independently selected from N, S and O the ring being optionally substituted by one, two or more groups independently selected from halogen, substituted or unsubstituted C₁-C₆ alkyl, haloalkyl, OH and alkoxy;
X-Y, Z-W and M-Q are the same or different and are each independently selected from the group consisting of: -CH₂CH₂-, substituted or unsubstituted aryl and substituted or unsubstituted heteroaryl, wherein the substituted aryl and the substituted heteroaryl are each independently substituted by one or more substituents each independently selected from the group consisting of: halogen, NO₂, substituted or unsubstituted phenyl, OR_{A}, C(O)OR_{B}, OC(O)R_{B}, SR_{A}, SO₃H, SOR_{A}, SO₂R_{A}, NR_{A}R_{B}, OP(O)(OR_{A}), OP(O)(OR_{A})(OR_{B}), OC(O)NR_{A}R_{B}, NR_{A}C(O)OR_{B}, C(O)NR_{A}R_{B}, NR_{A}C(O)R_{B}, N(COR_{A})(COR_{B}), C(O)NR_{A}C(O)R_{B}, C(O)ONR_{A}R_{B}, C(NOR_{A})R_{B}, C(O)R_{A}, CN, haloalkyl, CF₃, OCF₃, substituted or unsubstituted cycloalkyl, substituted or unsubstituted C₁-C₆ alkyl, substituted or unsubstituted C₂-C₆ alkenyl and substituted or unsubstituted C₂-C₆ alkynyl,
wherein said compound is an Acidic Mammalian Chitinase (AMCase) inhibitor and/or a chitotriosidase (CHIT-1) inhibitor.

2. The compound according to claim 1, wherein X-Y and Z-W are the same or different and are each independently selected from the group consisting of: substituted or unsubstituted aryl and substituted or unsubstituted heteroaryl.

3. A compound of formula (I): 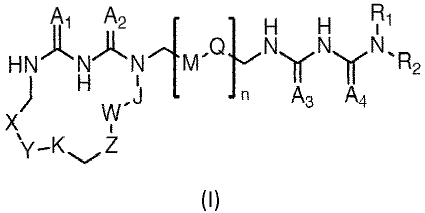 or a salt, a solvate, a tautomer or a stereoisomer thereof,
wherein:
n is a number from 0 to 3;
J is a linear saturated or unsaturated C₁-C₄ alkyl chain;
K is -CH₂-, O or S;
A₁, A₂, A₃, A₄ are the same or different and are each independently selected from the group consisting of: NH, O and S;
R₁ and R₂ are the same or different and are each independently selected from the group consisting of: H, substituted or unsubstituted C₁-C₆ alkyl, substituted or unsubstituted C₂-C₆ alkenyl, substituted or unsubstituted C₂-C₆ alkynyl, substituted or unsubstituted aryl and substituted or unsubstituted heteroaryl, wherein the substituted C₁-C₆ alkyl, the substituted C₂-C₆ alkenyl, the substituted C₂-C₆ alkynyl, the substituted aryl and the substituted heteroaryl are each independently substituted by one or more substituents each independently selected from the group consisting of: halogen, NO₂, substituted or unsubstituted phenyl, OR_{A}, C(O)OR_{B}, OC(O)R_{B}, SR_{A}, SO₃H, SOR_{A}, SO₂R_{A}, NR_{A}R_{B}, OP(O)(OR_{A}), OP(O)(OR_{A})(OR_{B}), OC(O)NR_{A}R_{B}, NR_{A}C(O)OR_{B}, C(O)NR_{A}R_{B}, NR_{A}C(O)R_{B}, N(COR_{A})(COR_{B}), C(O)NR_{A}C(O)R_{B}, C(O)ONR_{A}R_{B}, C(NOR_{A})R_{B}, C(O)R_{A}, CN, haloalkyl, CF₃, OCF₃, substituted or unsubstituted cycloalkyl, substituted or unsubstituted C₁-C₆ alkyl, substituted or unsubstituted C₂-C₆ alkenyl and substituted or unsubstituted C₂-C₆ alkynyl;
or R₁ and R₂ together with the nitrogen to which they are attached form a 4-7 membered saturated, partially unsaturated or aromatic ring optionally containing one or more additional heteroatoms independently selected from N, S and O, the ring being optionally substituted by one, two or more groups independently selected from: halogen, NO₂, substituted or unsubstituted phenyl, OR_{A}, C(O)OR_{B}, OC(O)R_{B}, SR_{A}, SO₃H, SOR_{A}, SO₂R_{A}, NR_{A}R_{B}, OP(O)(OR_{A}), OP(O)(OR_{A})(OR_{B}), OC(O)NR_{A}R_{B}, NR_{A}C(O)OR_{B}, C(O)NR_{A}R_{B}, NR_{A}C(O)R_{B}, N(COR_{A})(COR_{B}), C(O)NR_{A}C(O)R_{B}, C(O)ONR_{A}R_{B}, C(NOR_{A})R_{B}, C(O)R_{A}, CN, haloalkyl, CF₃, OCF₃, substituted or unsubstituted cycloalkyl, substituted or unsubstituted C₁-C₆ alkyl, substituted or unsubstituted C₂-C₆ alkenyl and substituted or unsubstituted C₂-C₆ alkynyl;
R_{A} and R_{B} are the same or different and are each independently selected from: H, unsubstituted or substituted C₁-C₆ alkyl, unsubstituted or substituted aralkyl, unsubstituted or substituted aryl, haloalkyl, or R_{A} and R_{B} together with the nitrogen, sulfur, or oxygen to which they are attached, form a 4-7 membered saturated, partially unsaturated or aromatic ring optionally containing one or more additional heteroatoms independently selected from N, S and O the ring being optionally substituted by one, two or more groups independently selected from halogen, substituted or unsubstituted C₁-C₆ alkyl, haloalkyl, OH and alkoxy;
M-Q is selected from the group consisting of: -CH₂CH₂-, substituted or unsubstituted aryl and substituted or unsubstituted heteroaryl, wherein the substituted aryl and the substituted heteroaryl are each independently substituted by one or more substituents each independently selected from the group consisting of: halogen, NO₂, substituted or unsubstituted phenyl, OR_{A}, C(O)OR_{B}, OC(O)R_{B}, SR_{A}, SO₃H, SOR_{A}, SO₂R_{A}, NR_{A}R_{B}, OP(O)(OR_{A}), OP(O)(OR_{A})(OR_{B}), OC(O)NR_{A}R_{B}, NR_{A}C(O)OR_{B}, C(O)NR_{A}R_{B}, NR_{A}C(O)R_{B}, N(COR_{A})(COR_{B}), C(O)NR_{A}C(O)R_{B}, C(O)ONR_{A}R_{B}, C(NOR_{A})R_{B}, C(O)R_{A}, CN, haloalkyl, CF₃, OCF₃, substituted or unsubstituted cycloalkyl, substituted or unsubstituted C₁-C₆ alkyl, substituted or unsubstituted C₂-C₆ alkenyl and substituted or unsubstituted C₂-C₆ alkynyl;
X-Y and Z-W are the same or different and are each independently selected from the group consisting of: substituted or unsubstituted aryl and substituted or unsubstituted heteroaryl, wherein the substituted aryl and the substituted heteroaryl are each independently substituted by one or more substituents each independently selected from the group consisting of: halogen, NO₂, substituted or unsubstituted phenyl, OR_{A}, C(O)OR_{B}, OC(O)R_{B}, SR_{A}, SO₃H, SOR_{A}, SO₂R_{A}, NR_{A}R_{B}, OP(O)(OR_{A}), OP(O)(OR_{A})(OR_{B}), OC(O)NR_{A}R_{B}, NR_{A}C(O)OR_{B}, C(O)NR_{A}R_{B}, NR_{A}C(O)R_{B}, N(COR_{A})(COR_{B}), C(O)NR_{A}C(O)R_{B}, C(O)ONR_{A}R_{B}, C(NOR_{A})R_{B}, C(O)R_{A}, CN, haloalkyl, CF₃, OCF₃, substituted or unsubstituted cycloalkyl, substituted or unsubstituted C₁-C₆ alkyl, substituted or unsubstituted C₂-C₆ alkenyl and substituted or unsubstituted C₂-C₆ alkynyl.

4. The compound according to claim 3, wherein said compound is a chitinase inhibitor, preferably said compound is an Acidic Mammalian Chitinase (AMCase) inhibitor and/or a chitotriosidase (CHIT-1) inhibitor.

5. The compound according to any one of previous claims, having general formula (II): 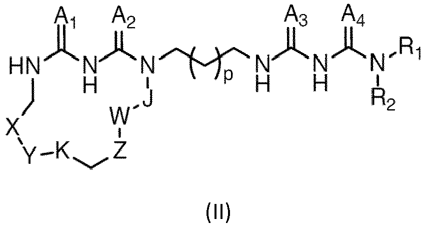 wherein p is a number from 0 to 6.

6. The compound according to any one of the preceding claims, **characterized by** at least one of the following:
- A₁ is NH,
- A₂ is O,
- A₃ is NH,
- A₄ is O or S.

7. The compound according to any one of the preceding claims, **characterized by** at least one of the following:
- R₁ and R₂ are independently H, methyl or CH₂CH₂OH,
- n is 1, 2 or 3,
- J is a C₁ alkyl chain, a C₂ alkyl chain or a C₃ alkyl chain,
- K is -CH₂- or O.

8. The compound according to any one of the previous claims being selected from:
| | | |
|---|---|---|
| **BM41** | 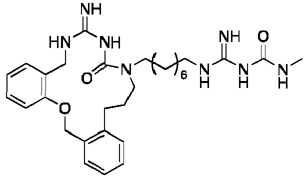 | 1-[N-(8-{16-imino-14-oxo-2-oxa-13,15,17-triazatricyclo[17.4.0.0^{4,9}]tricosa-1(19),4(9),5,7,20,22-hexaen-13-yl}octyl)carbamimidoyl]-3-methylurea |
| **BM46** | 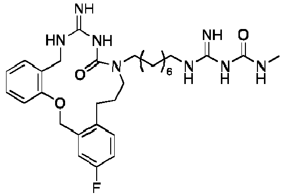 | 1-[N-(8-{6-fluoro-16-imino-14-oxo-2-oxa-13,15,17-triazatricyclo[17.4.0.0^{4,9}]tricosa-1(19),4(9),5,7,20,22-hexaen-13-yl}octyl)carbamimidoyl]-3-methylurea |
| **BM43** | 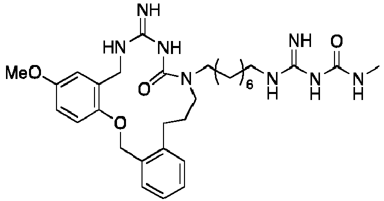 | 1-[N-(8-{16-imino-21-methoxy-14-oxo-2-oxa-13,15,17-triazatricyclo[17.4.0.0^{4,9}]tricosa-1(19),4(9),5,7,20,22-hexaen-13-yl}octyl)carbamimidoyl]-3-methylurea |
| **BM55** | 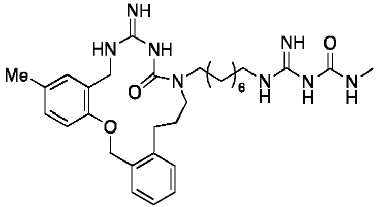 | 1-[N-(8-{16-imino-21-methyl-14-oxo-2-oxa-13,15,17-triazatricyclo[17.4.0.0^{4,9}]tricosa-1(19),4(9),5,7,20,22-hexaen-13-yl}octyl)carbamimidoyl]-3-methylurea |
| **BM54** | 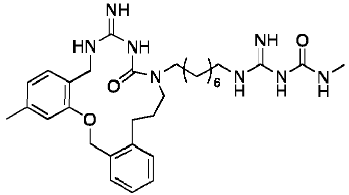 | 1-[N-(8-{16-imino-22-methyl-14-oxo-2-oxa-13,15,17-triazatricyclo[17.4.0.0^{4,9}]tricosa-1(19),4(9),5,7,20,22-hexaen-13-yl}octyl)carbamimidoyl]-3-methylurea |
| **BM38** | 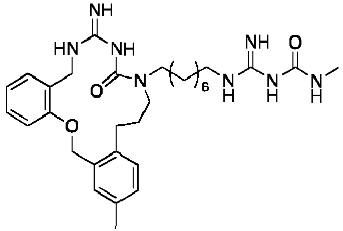 | 1-[N-(8-{16-imino-6-methyl-14-oxo-2-oxa-13,15,17-triazatricyclo[17.4.0.0^{4,9}]tricosa-1(19),4(9),5,7,20,22-hexaen-13-yl}octyl)carbamimidoyl]-3-methylurea |
| | | |
|---|---|---|
| **BM47** | 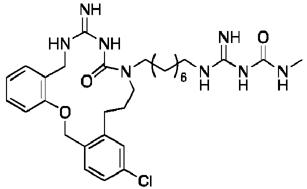 | 1-[N-(8-{7-chloro-16-imino-14-oxo-2-oxa-13,15,17-triazatricyclo[17.4.0.0^{4,9}]tricosa-1(19),4(9),5,7,20,22-hexaen-13-yl}octyl)carbamimidoyl]-3-methylurea |
| **BM32** | 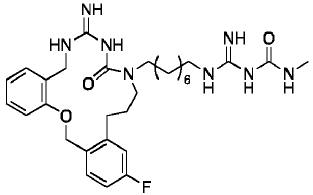 | 1-[N-(8-{7-fluoro-16-imino-14-oxo-2-oxa-13,15,17-triazatricyclo[17.4.0.0^{4,9}]tricosa-1(19),4(9),5,7,20,22-hexaen-13-yl}octyl)carbamimidoyl]-3-methylurea |
| **BM40** | 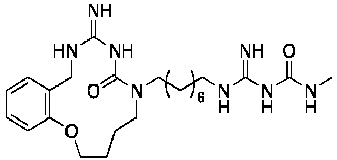 | 1-{N-[8-(9-imino-7-oxo-2,3,4,5,6,7,8,9,10,11-decahydro-1,6,8,10-benzoxatriazacyclotridecin-6-yl)octyl]carbamimidoyl}-3-methylurea |
| **BM39** | 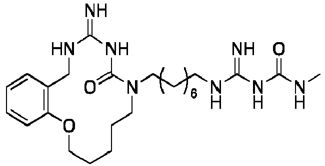 | 1-{N-[8-(10-imino-8-oxo-3,4,5,6,7,8,9,10,11,12-decahydro-2H-1,7,9,11-benzoxatriazacyclotetradecin-7-yl)octyl]carbamimidoyl}-3-methylurea |
| **BM52** | 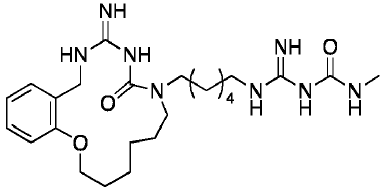 | 1-{N-[6-(11-imino-9-oxo-2,3,4,5,6,7,8,9,10,11,12,13-dodecahydro-1,8,10,12-benzoxatriazacyclopentadecin-8-yl)hexyl]carbamimidoyl}-3-methylurea |
| **BM50** | 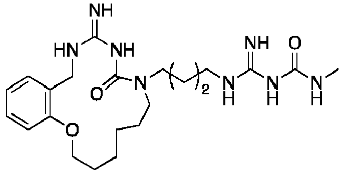 | 1-{N-[4-(11-imino-9-oxo-2,3,4,5,6,7,8,9,10,11,12,13-dodecahydro-1,8,10,12-benzoxatriazacyclopentadecin-8-yl)butyl]carbamimidoyl}-3-methylurea |
| **BM22** | 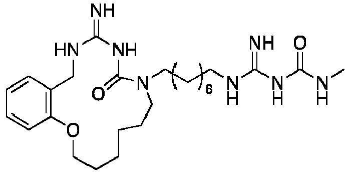 | 1-{N-[8-(11-imino-9-oxo-2,3,4,5,6,7,8,9,10,11,12,13-dodecahydro-1,8,10,12-benzoxatriazacyclopentadecin-8-yl)octyl]carbamimidoyl}-3-methylurea |
| **BM34** | 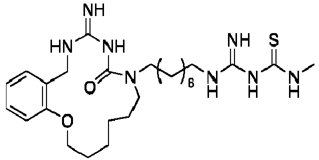 | 1-{N-[8-(11-imino-9-oxo-2,3,4,5,6,7,8,9,10,11,12,13-dodecahydro-1,8,10,12-benzoxatriazacyclopentadecin-8-yl)octyl]carbamimidoyl}-3-methylthiourea |
| | | |
|---|---|---|
| **BM35** | 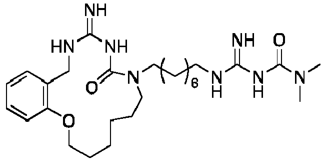 | 1-{N-[8-(11-imino-9-oxo-2,3,4,5,6,7,8,9,10,11,12,13-dodecahydro-1,8,10,12-benzoxatriazacyclopentadecin-8-yl)octyl]carbamimidoyl}-3,3-dimethylurea |
| **BM36** | 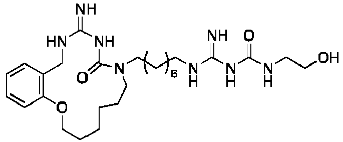 | 3-(2-hydroxyethyl)-1-{N-[8-(11-imino-9-oxo-2,3,4,5,6,7,8,9,10,11,12,13-dodecahydro-1,8,10,12-benzoxatriazacyclopentadecin-8-yl)octyl]carbamimidoyl}urea |
| **BM53** | 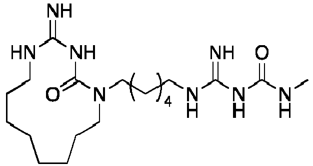 | 1-{N-[6-(4-imino-2-oxo-1,3,5-triazacyclotridecan-1-yl)hexyl]carbamimidoyl}-3-methylurea |
| **BM49** | 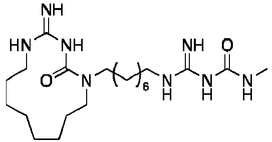 | 1-{N-[8-(4-imino-2-oxo-1,3,5-triazacyclotridecan-1-yl)octyl]carbamimidoyl}-3-methylurea |
| **BM45** | 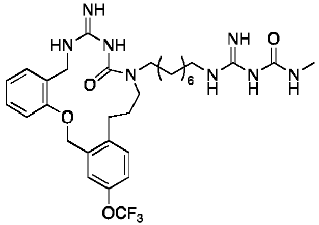 | 1-(N-{8-[16-imino-14-oxo-6-(trifluoromethoxy)-2-oxa-13,15,17-triazatricyclo[17.4.0.0^{4,9}]tricosa-1(19),4(9),5,7,20,22-hexaen-13-yl]octyl}carbamimidoyl)-3-methylurea |
| **BM56** | 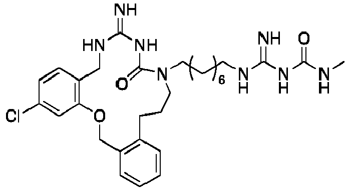 | 1-[N-(8-{22-chloro-16-imino-14-oxo-2-oxa-13,15,17-triazatricyclo[17.4.0.0^{4,9}]tricosa-1(19),4(9),5,7,20,22-hexaen-13-yl}octyl)carbamimidoyl]-3-methylurea |
| **BM57** | 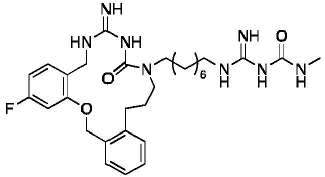 | 1-[N-(8-{22-fluoro-16-imino-14-oxo-2-oxa-13,15,17-triazatricyclo[17.4.0.0^{4,9}]tricosa-1(19),4(9),5,7,20,22-hexaen-13-yl}octyl)carbamimidoyl]-3-methylurea |
| | | |
|---|---|---|
| **BM58** | 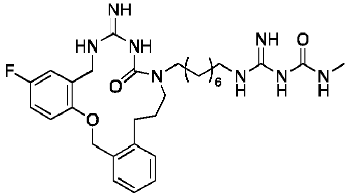 | 1-[N-(8-{21-fluoro-16-imino-14-oxo-2-oxa-13,15,17-triazatricyclo[17.4.0.0^{4,9}]tricosa-1(19),4(9),5,7,20,22-hexaen-13-yl}octyl)carbamimidoyl]-3-methylurea |
| **BM42** | 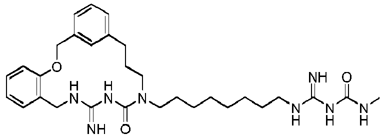 | 1-[N-(8-{12-imino-14-oxo-3-oxa-11,13,15-triazatricyclo[17.3.1.0^{4,9}]tricosa-1(23),4(9),5,7,19,21-hexaen-15-yl}octyl)carbamimidoyl]-3-methylurea |
| **BM48** | 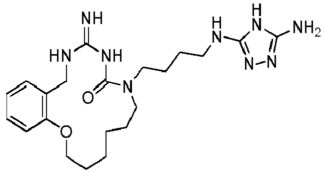 | 8-(4-((5-amino-4*H*-1,2,4-triazol-3-yl)amino)butyl)-11-imino-3,4,5,6,7,8,10,11,12,13-decahydrobenzo[*b*][1]oxa[5,7,9]triazacyclopentad ecin-9(2*H*)-one |

9. The compound as defined in any one of previous claims for use as a medicament.

10. The compound as defined in any one of claims 1, 2 or 5-8 for use as an Acidic Mammalian Chitinase (AMCase) inhibitor and/or as a chitotriosidase (CHIT-1) inhibitor or the compound as defined in any one of claims 3-8 for use as a chitinase inhibitor, preferably said chitinase is Acidic Mammalian Chitinase (AMCase) and/or chitotriosidase (CHIT-1).

11. The compound for use according to claim 9 or 10, for use in a method of treatment, prevention and/or amelioration of a Th-2-mediated disease, an IL-13-mediated disease, an IL-4-mediated disease, an IL-5-mediated disease or an IL-9-mediated disease.

12. The compound for use according to any one of claims 9 to 11, for use in a method of treatment, prevention and/or amelioration of any one or more of: asthma, bronchoconstriction, cough, shortness of breath, wheezing, chest tightness, wheezing dyspnea, airway narrowing, chronic airway narrowing, respiratory impairment, chronic respiratory impairment, bronchospasm, lung inflammation, idiopathic pulmonary fibrosis, allergic rhinitis, allergic lung disease, interstitial lung disease, chronic obstructive pulmonary disease (COPD), airway hyper-responsiveness, bronchial hyper-reactivity, Th2 immune response, conjunctivitis, sarcoidosis, vernal keratoconjunctivitis, seasonal allergic conjunctivitis, dry eye syndrome, chronic rhinosinusitis, chronic lung disease, adenoid hypertrophy, a nasal polyp, eosinophilic esophagitis, cancer, parasitosis.

13. A pharmaceutical composition comprising:
- the compound as defined in any one of claims 1 to 8,
- a vehicle, and
- optionally a further therapeutic agent,
preferably said further therapeutic agent is selected from the group consisting of: a chitinase inhibitor, an anti-chitinase antibody, a steroid, a membrane stabilizer, a 5LOX inhibitor, a leukotriene synthesis and receptor inhibitor, an inhibitor of IgE isotype switching or IgE synthesis, an IgG isotype switching or IgG synthesis, a b-agonist, an anti-Th2 cytokine, a tryptase inhibitor, aspirin, a COX inhibitor, methotrexate, an anti-TNF drug, retuxin, a PD4 inhibitor, a p38 inhibitor, a PDE4 inhibitor, a xanthine and an antihistamine.

14. A process for the preparation of the compound as defined in any one of claims 1 to 8 according to one of the following synthetic schemes:
- Scheme A, wherein X-Y, Z-W and M-Q are -CH₂CH₂-, K is -CH₂- and PG is a protecting group, comprising the following steps: 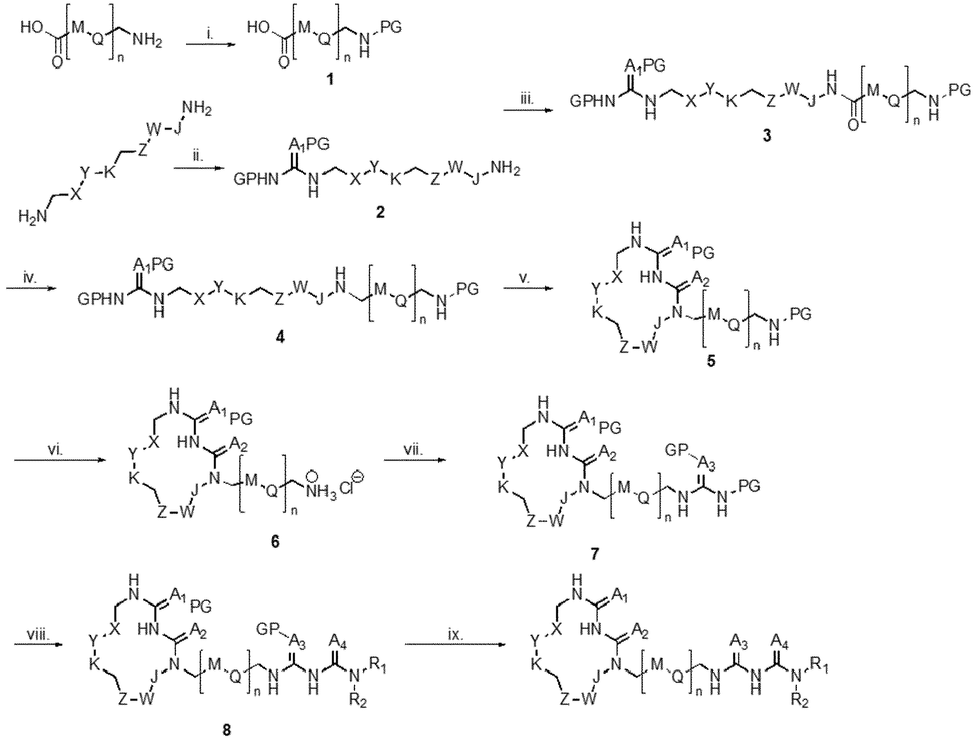
(i) Protection of the amino acid of formula H₂NCH₂[M-Q]ₙCOOH to obtain a protected compound of formula 1;
(ii) Addition-elimination reaction of the diamine of formula H₂NCH₂XYKCH₂ZWJNH₂ with a compound of formula 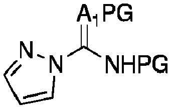 to obtain a compound of formula 2;
(iii) Coupling of the protected compound of formula 1 with the compound of formula 2 to obtain an amide compound formula 3;
(iv) Reduction of the amide compound of formula 3 to an amine compound of formula 4;
(v) Intramolecular reaction of the compound of formula 4 to obtain a macrocyclic compound of formula 5;
(vi) Deprotection of the macrocyclic compound of formula 5 to obtain a deprotected macrocyclic compound of formula 6;
(vii) Addition-elimination reaction of the deprotected macrocyclic compound of formula 6 with a compound of formula 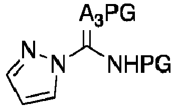 to obtain a compound of formula 7;
(viii) Nucleophilic substitution of the compound of formula 7 with a compound of formula NR₁R₂ to obtain a compound of formula 8;
(ix) Deprotection of the compound of formula 8 to obtain a compound as defined in any one of claims 1-8;
or Scheme B, wherein X-Y is a substituted or unsubstituted aryl or a substituted or unsubstituted heteroaryl, M-Q is -CH₂CH₂-, K is O or S and PG is a protecting group, comprising the following steps: 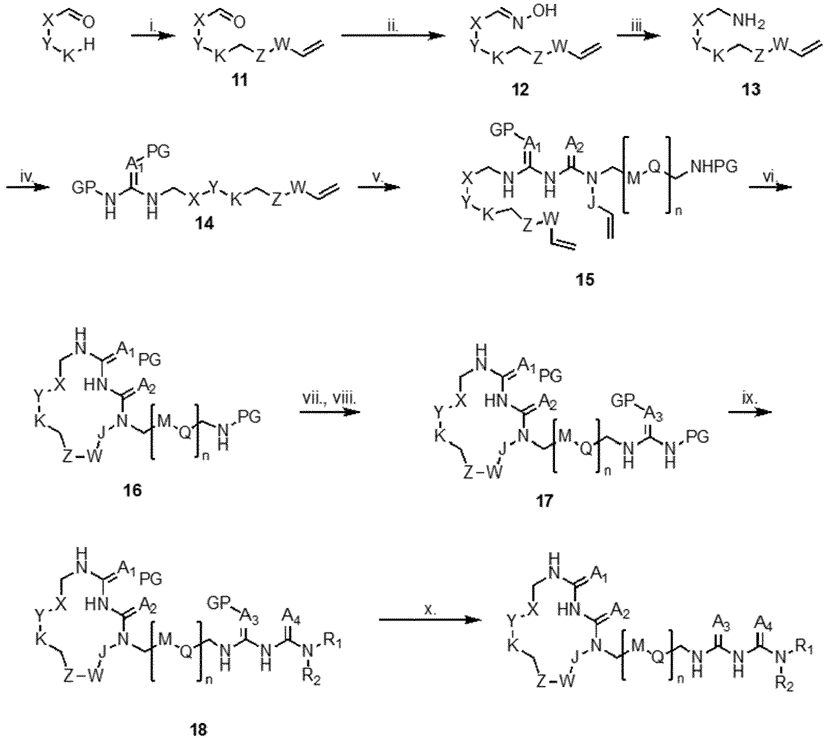
(i) Reaction of the aldehyde of formula KH-Y-X-CHO with a bromoalkene of formula H₂CCHWZCH₂Br to obtain a compound of formula 11;
(ii) Reaction of a compound of formula 11 with hydroxylamine or a salt thereof to obtain an oxime compound of formula 12;
(iii) Reduction of the oxime compound of formula 12 to obtain an amine compound of formula 13;
(iv) Addition-elimination reaction of the amine compound of formula 13 with a compound of formula 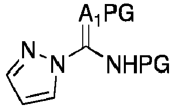 to obtain a compound of formula 14;
(v) Reaction of a compound of formula 14 with a linker compound of formula H₂CCHJNHCH₂[M-Q]ₙCH₂NHPG to obtain a compound of formula 15;
(vi) Intramolecular reaction of the compound of formula 15 to obtain a macrocyclic compound of formula 16;
(vii)-(viii) Reduction of the macrocyclic compound of formula 16 and subsequent addition-elimination reaction with a compound of formula 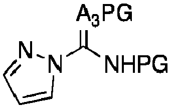 to obtain a macrocyclic compound of formula 17;
(ix) Nucleophilic substitution of the compound of formula 17 with a compound of formula NR₁R₂ to obtain a compound of formula 18;
(xi) Deprotection of the compound of formula 18 to obtain a compound as defined in any one of claims 1-8;
or Scheme C, wherein X-Y and W-Z are a substituted or unsubstituted aryl or a substituted or unsubstituted heteroaryl, M-Q is -CH₂CH₂- and K is O or S, comprising the following steps: 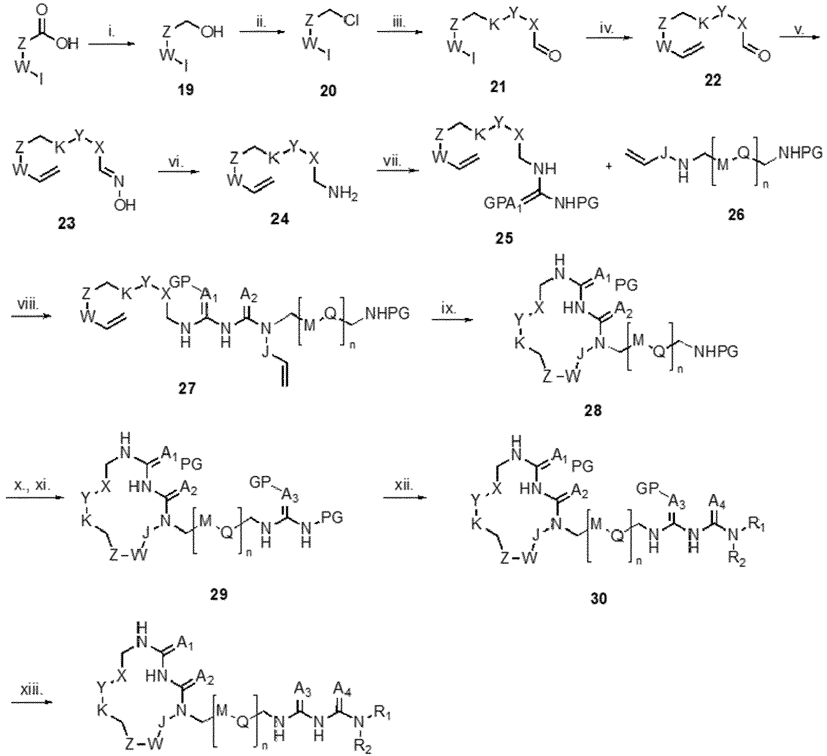
(i) Reduction of the carboxylic acid of formula I-W-Z-COOH to obtain a primary alcohol of formula 19;
(ii) Conversion of the primary alcohol of formula 19 to a chloride of formula 20;
(iii) Nucleophilic substitution of the chloride of formula 20 with a compound of formula KH-Y-X-CHO to obtain a compound of formula 21;
(iv) Conversion of the iodide of formula 21 to a vinyl compound of formula 22;
(v) Reaction of a compound of formula 22 with hydroxylamine or a salt thereof to obtain an oxime compound of formula 23;
(vi) Reduction of the oxime compound of formula 23 to obtain an amine compound of formula 24;
(vii) Addition-elimination reaction of the amine compound of formula 24 with a compound of formula 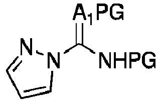 to obtain a compound of formula 25;
(viii) Reaction of the compound of formula 25 with a linker compound of formula 26 to obtain a compound of formula 27;
(ix) Intramolecular reaction of the compound of formula 27 to obtain a macrocyclic compound of formula 28;
(x) and (xi) Reduction of the macrocyclic compound of formula 28 and subsequent addition-elimination reaction with a compound of formula 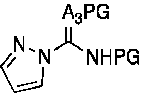 to obtain a macrocyclic compound of formula 29;
(xii) Nucleophilic substitution of the compound of formula 29 with a compound of formula NR₁R₂ to obtain a compound of formula 30;
(xiii) Deprotection of the compound of formula 30 to obtain a compound as defined in any one of claims 1-8.

15. A compound having one of the following formulas: 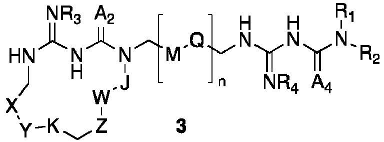 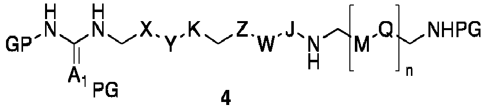 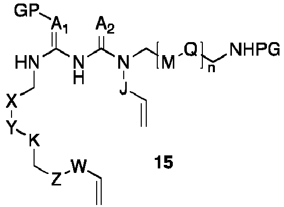 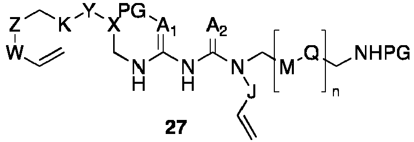 wherein n, J, K, A₁, A₂, A₃, A₄, R₁, R₂, X-Y, Z-W, M-Q are as defined above, R₃ is H or a protecting group, PG and R₄ are a protecting group, preferably said protecting group is Boc or Cbz.
